# EUROPEAN PATENT APPLICATION

(11) **EP 4 080 217 A2**
(43) Date of publication of application: **26.10.2022**
(21) Application number: 22165219.1
(22) Date of filing: 29.03.2022
(51) Int. Cl.: G01N 33/68, A61P 37/08, C07K 14/47, C07K 16/00

(54) **MILK ALLERGY ANTIGEN**

(30) Priority: 31.03.2021 JP 2021061611
(71) Applicant: Fujita Academy, Toyoake-shi, Aichi 470-1192 (JP); Hoyu Co., Ltd., Nagoya-shi, Aichi 461-8650 (JP)
(72) Inventor: YAGAMI, Akiko, Toyoake-shi, 470-1192 (JP); KONDO, Yasuto, Toyoake-shi, 470-1192 (JP); MATSUNAGA, Kayoko, Toyoake-shi, 470-1192 (JP); AOKI, Yuji, Nagakute-shi, 480-1136 (JP); HASEGAWA, Erika, Nagakute-shi, 480-1136 (JP); SAKAI, Tomomi, Nagakute-shi, 480-1136 (JP)
(74) Representative: Mewburn Ellis LLP

(57) **Abstract**

The present invention provides a novel milk allergy antigen, a milk allergy diagnosis method or diagnosis kit, a composition comprising the antigen, a milk or processed milk product with the antigen removed, and a tester composition for determining the presence or absence of the milk antigen in an object of interest. The present invention also pertains to an antigen epitope-comprising polypeptide, a kit for diagnosing an allergy comprising the polypeptide, diagnostic composition, a diagnosis method, a composition comprising the polypeptide, and a raw material or processed product in which the polypeptide-comprising antigen is eliminated or reduced. The present invention further pertains to a tester composition for determining the presence or absence of an antigen in an object of interest.

## Description

The present invention relates to a novel milk allergy antigen. The present invention also relates to a kit, a composition and a method for diagnosing milk allergy. The present invention further relates to a composition comprising the antigen and a raw material or processed product in which the antigen is eliminated or reduced. The present invention furthermore relates to a tester composition for determining the presence or absence of a milk antigen in an object of interest.

In addition, the present invention relates to an epitope-comprising polypeptide antigen. In addition, the present invention relates to a kit for diagnosing an allergy, a diagnostic composition, and a diagnosis method comprising the polypeptide. In addition, the present invention relates to a method for providing an indicator for diagnosing an allergy in a subject. In addition, the present invention relates to a composition comprising the polypeptide and a raw material or processed product in which the polypeptide is eliminated or reduced. Further, the present invention relates to a method for producing a raw material or processed product in which the polypeptide is eliminated or reduced. Furthermore, the present invention relates to a tester composition for determining the presence or absence of the polypeptide-comprising antigen in an object of interest.

An IgE antibody specific to a particular antigen (hereinafter, also referred to as an allergen) is produced in serum or tissues of a patient with allergy. This Ig E antibody interacts with the particular antigen and causes physiological consequences, which trigger an allergic reaction. Antigens refer to a food/foodstuff, etc., which causes an allergy symptom in a broad sense, and refer to proteins (hereinafter, also referred to as an allergen component) included in foods/foodstuffs, etc., to which specific IgE antibodies bind in a narrow sense.

In conventional allergy test agents, antigen reagents have often been prepared by simply grinding candidate food/foodstuff, etc. (PTL 1). Consequently, it is possible to detect the positive reaction in the allergy test only when, in a conventional antigen reagent comprising many types of allergen components, the content of an allergen component exceeds a threshold that allows determination of a positive reaction for binding to an IgE antibody. As a result, the diagnosis efficiency cannot be said to be sufficiently high.

In the allergen candidate foods/foodstuffs, some allergen components are pointed out and have been commercialized as test kits. To enhance the reliability of allergy test, the allergen components have to be identified exhaustively: however, the rate of detecting a patient by measuring the above allergen components is still insufficient. It is critical to identify a novel milk allergen(s) not only increasing the accuracy of a diagnostic agent but also providing a target for a low-allergen food, a low-allergen foodstuff, or a therapeutic agent.

Meanwhile, in the field of protein separation and purification, a method for separating and purifying various proteins from a small amount of a sample has recently been used, wherein a 2D electrophoresis consisting of isoelectric focusing as a first dimension, and SDS-PAGE (sodium dodecyl sulfate-polyacrylamide gel electrophoresis) as a second dimension is performed. The present applicants have hitherto developed a 2D electrophoresis with increased separation capacity (PTLs 2 to 5).

Allergen-specific IgE antibodies recognize and bind to epitopes which are particular amino acid sequences in allergen components. Although the allergen components have been analyzed at an epitope level in several cases (NPL 1), the number is so far very low. In addition, no allergy diagnosis kits using an epitope-comprising polypeptide are currently available in the market.
PTL1: Japanese Patent Laid-Open No. 2002-286716
PTL2: Japanese Patent Laid-Open No. 2011-33544
PTL3: Japanese Patent Laid-Open No. 2011-33546
PTL4: Japanese Patent Laid-Open No. 2011-33547
PTL5: Japanese Patent Laid-Open No. 2011-33548
NPL1: Matsuo, H., et al., J. Biol. Chem., (2004), Vol.279, No.13, pp.12135-12140

The invention provides a novel allergy protein antigen. In addition, the invention provides an allergy diagnosis method and diagnosis kit comprising the antigen. In addition, the invention provides a composition comprising the antigen and a raw material or processed product in which the antigen is eliminated or reduced. Further, the invention provides a tester composition for determining the presence or absence of the antigen in an object of interest.

In addition, the present invention provides an epitope-comprising polypeptide antigen. In addition, the present invention provides a kit, a composition and a method for diagnosing allergy comprising the polypeptide. In addition, the invention provides a method for providing an indicator for diagnosing an allergy in a subject. In addition, the invention provides a composition comprising the polypeptide and a raw material or processed product in which the polypeptide-comprising antigen is eliminated or reduced. Further, the invention relates to a method for producing a raw material or processed product in which the antigen is eliminated or reduced. Furthermore, the invention provides a tester composition for determining the presence or absence of the polypeptide-comprising antigen in an object of interest.

The present inventors have conducted intensive studies to solve the above problems and have identified an antigen(s) that causes a milk allergy. As a result, the inventors succeeded in identification of a novel protein antigen(s) to which an IgE antibody in the serum of a patient with milk allergy specifically binds.

Meanwhile, epitopes have relatively short amino acid sequences. Thus, if the same amino acid sequence is present in different allergen components, the IgE antibody can bind to a plurality of allergen components. The different allergen components share an epitope, so that the IgE antibody from the allergic patient can bind to the epitope in such components. Thus, the antigen has cross-reactivity. Accordingly, an epitope identified herein enables, for example, diagnosis and treatment of an allergy with cross-reactivity and detection of a plurality of allergen components comprising the epitope.

The term "antigen" used herein means and encompasses both a narrowly defined protein antigen and a protein-derived "epitope" unless otherwise indicated. When explicitly indicated, the "antigen" is used to mean either a protein or a protein-derived epitope.

Based on the above findings, the invention has been completed. Although not limited, the invention includes the following items.
[Item 1] A kit for diagnosing an allergy comprising at least one of the following polypeptides (E1) to (E25):
   (E1) a polypeptide comprising at least one amino acid sequence selected from amino acid sequences set forth in SEQ ID NOs: 27 to 58 and 1115;
   (E2) a polypeptide comprising at least one amino acid sequence selected from amino acid sequences set forth in SEQ ID NOs: 59 to 97;
   (E3) a polypeptide comprising at least one amino acid sequence selected from amino acid sequences set forth in SEQ ID NOs: 98 to 144;
   (E4) a polypeptide comprising at least one amino acid sequence selected from amino acid sequences set forth in SEQ ID NOs: 145 to 195;
   (E5) a polypeptide comprising at least one amino acid sequence selected from amino acid sequences set forth in SEQ ID NOs: 196 to 254 and 1116 to 1118;
   (E6) a polypeptide comprising at least one amino acid sequence selected from amino acid sequences set forth in SEQ ID NOs: 255 to 288;
   (E7) a polypeptide comprising at least one amino acid sequence selected from amino acid sequences set forth in SEQ ID NOs: 289 to 333 and 1119 to 1120;
   (E8) a polypeptide comprising at least one amino acid sequence selected from amino acid sequences set forth in SEQ ID NOs: 334 to 352;
   (E9) a polypeptide comprising at least one amino acid sequence selected from amino acid sequences set forth in SEQ ID NOs: 353 to 376 and 1121 to 1122;
   (E10) a polypeptide comprising at least one amino acid sequence selected from amino acid sequences set forth in SEQ ID NOs: 377 to 384;
   (E11) a polypeptide comprising at least one amino acid sequence selected from amino acid sequences set forth in SEQ ID NOs:385 to 411;
   (E12) a polypeptide comprising at least one amino acid sequence selected from amino acid sequences set forth in SEQ ID NOs: 412 to 450;
   (E13) a polypeptide comprising at least one amino acid sequence selected from amino acid sequences set forth in SEQ ID NOs: 451 to 500 and 1123 to 1124;
   (E14) a polypeptide comprising at least one amino acid sequence selected from amino acid sequences set forth in SEQ ID NOs: 501 to 604 and 1125 to 1127;
   (E15) a polypeptide comprising at least one amino acid sequence selected from amino acid sequences set forth in SEQ ID NOs: 605 to 632 and 1128;
   (E16) a polypeptide comprising at least one amino acid sequence selected from amino acid sequences set forth in SEQ ID NOs: 633 to 673;
   (E17) a polypeptide comprising at least one amino acid sequence selected from amino acid sequences set forth in SEQ ID NOs: 674 to 693;
   (E18) a polypeptide comprising at least one amino acid sequence selected from amino acid sequences set forth in SEQ ID NOs: 694 to 757 and 1129;
   (E19) a polypeptide comprising at least one amino acid sequence selected from amino acid sequences set forth in SEQ ID NOs: 758 to 791;
   (E20) a polypeptide comprising at least one amino acid sequence selected from amino acid sequences set forth in SEQ ID NOs: 792 to 868 and 1130;
   (E21) a polypeptide comprising at least one amino acid sequence selected from amino acid sequences set forth in SEQ ID NOs: 869 to 927;
   (E22) a polypeptide comprising at least one amino acid sequence selected from amino acid sequences set forth in SEQ ID NOs: 928 to 996;
   (E23) a polypeptide comprising at least one amino acid sequence selected from amino acid sequences set forth in SEQ ID NOs: 997 to 1037;
   (E24) a polypeptide comprising at least one amino acid sequence selected from amino acid sequences set forth in SEQ ID NOs: 1038 to 1075; or
   (E25) a polypeptide comprising at least one amino acid sequence selected from amino acid sequences set forth in SEQ ID NOs: 1076 to 1114 and 1131.
[Item 2] A composition for diagnosing an allergy comprising at least one polypeptide as defined in any of (E1) to (E25) in item 1.
[Item 3] A method for providing an indicator for diagnosing an allergy in a subject, comprising the steps of:
   (i) contacting a sample obtained from the subject with an antigen, wherein the sample is a solution comprising an IgE antibody;
   (ii) detecting binding between the IgE antibody present in the sample obtained from the subject and the antigen; and
   (iii) when the binding between the IgE antibody in the subject and the antigen is detected, an indicator of the fact that the subject is allergic is provided,
      wherein the antigen is at least one polypeptide as defined in any of (E1) to (E25) in item 1.
[Item 4] An antigen, which is at least one polypeptide as defined in any of (E1) to (E25) in item 1 and is causative of an allergy.
[Item 5] A composition comprising at least one antigen according to item 4.
[Item 6] The composition according to item 5, wherein the composition is intended for the treatment of an allergy.
[Item 7] A tester composition for determining the presence or absence of an antigen in an object of interest, comprising an antibody that binds to at least one polypeptide as defined in any of (E1) to (E25) in item 1.
[Item 8] A tester composition for determining the presence or absence of an antigen in an object of interest, comprising at least one primer comprising a portion of a nucleotide sequence, and/or a portion of a complementary strand thereof, of a nucleic acid encoding a polypeptide as defined in any of(E1) to (E25) in item 1.
[Item 9] A tester composition for determining the presence or absence of an IgE antibody in a subject, comprising a polypeptide as defined in any of (E1) to (E25) in item 1.
[Item 10] A method for determining the presence or absence of a polypeptide as defined in any of (E1) to (E25) in item 1 in a raw material or processed product, comprising detecting the polypeptide as defined in any of (E1) to (E25) in item 1 in the raw material or processed product.
[Item 11] A raw material or processed product in which an antigen is eliminated or reduced, wherein the antigen is at least one polypeptide as defined in any of (E1) to (E25) in item 1.
[Item 12] A method for producing a raw material or processed product in which an antigen is eliminated or reduced, the method comprising the step of confirming that the antigen has been eliminated or reduced in a production process of the raw material or the processed product, wherein the antigen is at least one polypeptide as defined in any of (E1) to (E25) in item 1.

The invention can provide a novel allergy (e.g., milk allergy) antigen. Since a novel allergen component which is causative of an allergy has been identified in the present invention, it has become possible to provide a highly sensitive allergy diagnosis method and diagnosis kit, a composition comprising the antigen, a raw material or processed product in which the antigen is eliminated or reduced, and a tester composition for determining the presence or absence of the antigen in an object of interest.

In addition, the invention can provide a novel polypeptide antigen comprising an epitope of a protein antigen. Use of a polypeptide in the invention makes it possible to provide a highly sensitive allergy (e.g., milk allergy) diagnosis kit, diagnostic composition, and diagnosis method, a composition comprising the polypeptide, a tester composition for determining the presence or absence of the polypeptide-comprising antigen in an object of interest, and a raw material or processed product in which the polypeptide is eliminated or reduced and a method for producing the raw material or processed product.

Fig. 1 is a photograph of a gel showing a protein electrophoresis pattern obtained by 2D-electrophoresis of proteins contained in milk. The left-side bands in the photograph are bands of molecular weight markers, and the numerical values on the left side in the photograph indicate respective molecular weights (KDa) of molecular weight markers. The numerals at the top of the photograph represent isoelectric points.

Fig. 2 is a photograph of an immunoblot of a 2D electrophoresis of proteins contained in milk, obtained with serum from a patient with milk allergy. Spots 1 to 14 where IgE antibodies in serum of the patient with milk allergy specifically reacted in comparison with a healthy individual are each enclosed with a white line.

Fig. 3 shows the results of examining cross-reactivity to peptides having the amino acid sequence of each epitope by ELISA using serum from a patient (P1) with milk allergy to.

Fig. 4 shows the results of checking cross-reactivity to peptides having the amino acid sequence of each epitope by ELISA using serum from a patient (P5) with milk allergy.

Fig. 5 shows the results of checking cross-reactivity to peptides having the amino acid sequence of each epitope by ELISA using serum from a patient (P8) with milk allergy.

Fig. 6 shows the results of examining cross-reactivity to peptides having the amino acid sequence of each epitope by ELISA using serum from a patient (P20) with milk allergy.

Hereinbelow, the invention will be described specifically. However, the invention is not limited to them.

Unless otherwise defined herein, scientific terms and technical terms used with respect to the invention have meanings commonly understood by those skilled in the art.

As used herein, the allergy refers to a state in which an *in vivo* unwanted hypersensitivity reaction occurs when a certain antigen re-enters into the body of an individual which has been sensitized with the certain antigen. Upon contact with an antigen or intake of the antigen, an allergic reaction may occur. Here, the contact refers to touch to an object, and refers to, in the case of a human body, in particular, attachment to the skin, mucosa (e.g., an eye, a lip), etc. In addition, the intake refers to incorporation into the body, and refers to incorporation by inhalation or through an oral route, etc. Generally, the allergic reaction caused by food ingestion refers to, in particular, a food allergy. In a preferred embodiment, the allergy may be food allergy. In many food allergy diseases, an IgE antibody specific to an antigen is produced in blood and tissues. The IgE antibody binds to a mast cell or basophil. When an antigen specific to such an IgE antibody re-enters into the body of a patient with allergic disease, the antigen binds to the IgE antibody bound to a mast cell or basophil to exert a physiological effect of the IgE antibody-antigen interaction. Examples of such a physiological effect include release of histamine, serotonin, heparin, eosinophil chemotactic factors, various leukotrienes or the like. These released substances can trigger an allergic reaction caused by the combination of an IgE antibody with a specific antigen. Specifically, the IgE antibody recognizes and binds to an epitope, which is a specific amino acid sequence in the specific antigen. As a result, the antigen-mediated allergic reaction appears via the above pathway.

The allergy of interest in the invention is not particularly limited as long as the allergy is against an allergen (antigen) comprising an epitope to be used. In an embodiment, examples of the allergen include milk, a dairy product, a processed milk product, meat, a processed meat product, grains, seafood, a fruit, a vegetable, nuts (seeds), edible grass, an egg, a processed egg, etc., that are ingested by living organisms or parasites that live in living organisms (especially humans).

The origin of the milk is not particularly limited. In an embodiment, milk derived from a cow, goat, sheep, or the like may be included. In an embodiment, the milk is cow's milk. Examples of the milk include, but are not limited to, raw milk, pasteurized milk, ingredient-adjusted milk, low-fat milk, or fat-free milk. The dairy product is a product produced by processing milk as a main raw material. Examples of the dairy product include cream, butter, butter oil, cheese, whey, whey concentrate, ice cream, milk concentrate, skimmed milk concentrate, unsweetened condensed milk, unsweetened skimmed condensed milk, sweetened condensed milk, sweetened skimmed condensed milk, whole milk powder, skimmed milk powder, cream powder, whey powder, protein concentrate whey powder, buttermilk powder, sweetened milk powder, prepared milk powder, prepared liquid milk, fermented milk such as yogurt, a lactobacillus beverage, or a milk drink. The processed milk product refers to a milk product produced by processing cow's milk as a main raw material or each food comprising the processed milk product as a raw material. Examples of the processed milk product include, but are not limited to, cream, butter, butter oil, cheese, whey, whey concentrate, ice cream, milk concentrate, skimmed milk concentrate, unsweetened condensed milk, unsweetened skimmed condensed milk, sweetened condensed milk, sweetened skimmed condensed milk, whole milk powder, skimmed milk powder, cream powder, whey powder, protein-enriched whey powder, buttermilk powder, sweetened milk powder, prepared milk powder, prepared liquid milk, fermented milk such as yogurt, a lactobacillus beverage, a milk drink, which are produced by processing milk, or a cake, pastry, custard pudding, milk agar, biscuit, cookie, snack, chocolate, bread, white sauce, potage, cream stew, gratin, curry roux, or stew roux, which contain the above material(s) as a raw material.

The type of meat is not particularly limited. In an embodiment, examples include bird meat (e.g., chicken, duck) or mammalian meat (e.g., pork, beef, ram). In an embodiment, the meat is derived from a mammal. In an embodiment, the meat is derived from a cow (*Bos taurus*)*.* The processed meat product refers to a product produced by processing meat as a main raw material or each food comprising the processed meat product as a raw material. Examples of the processed meat product include, but are not limited to, ham, sausage, bacon, bouillon, consomme, steak, grilled meat, roast beef, tartar steak, hamburg steak, hamburger, meatballs, or nuggets.

The grains generally refer to a foodstuff obtained from a plant and are edible seeds primarily comprising starch. The term "grains", in a narrow sense, refers only to the seeds of gramineous crops (cereals), but in a broader sense the term also includes the seeds of legumes (beans) and seeds of other crops. Among the grains in a broad sense, the seeds of dicotyledonous plants that are used as grains because they resemble the seeds of grass crops (the cereals of monocotyledons) are collectively called pseudocereals. Examples of the pseudocereals include those from buckwheat (*Polygonaceae*)*,* amaranth (*Amaranthaceae*)*,* or quinoa (*Chenopodium quinoa, Chenopodiaceae*).

In the invention, examples of the gramineous grains include, but are not limited to, bread wheat (*Triticum aestivum*)*,* barley, rye, oat, timothy, orchardgrass, *Anthoxanthum odoratum,* millet, foxtail millet, Japanese millet, corn, or adlay. Examples of the grains from the family *Polygonaceae* include those from *Fagopyrum esculentum.* In an embodiment, the antigen (allergen) is derived from grains. In an embodiment, the allergen is derived from gramineous grains. In an embodiment, the allergen is derived from wheat.

Examples of the seafood include, but are not limited to, shrimps or crabs belonging to the order *Decapoda.* Most of the organisms generally recognized as "crustaceans" are included in the order *Decapoda.* Other examples of the seafood include, but are not limited to, squids or octopuses belonging to the order *Teuthoidea* and the order *Octopoda.* Examples further include fish belonging to the family *Scombridae* or *Gadidae.* Examples furthermore include shellfish from the family *Venus.*

Examples of the fruit include, but are not limited to, a fruit belonging to the family *Actinidiaceae, Bromeliaceae, Anacardiaceae, Cucurbitaceae, Musaceae, Rutaceae,* or *Rosaceae.* Examples of the vegetable include a fruit belonging to the family *Solanaceae, Cucurbitaceae,* or *Lauraceae.* Examples of the nuts (seeds) include nuts (seeds) belonging to the family *Anacardiaceae, Rosaceae,* or *Juglandaceae* or nuts (e.g., peanuts) belonging to the family *Leguminosae.* Examples of the edible grass include edible grass belonging to the family *Asteraceae.* Examples of the grains include grains belonging to the family *Poaceae* or *Polygonaceae.*

In the invention, examples of the egg include, in addition to the above seafood egg, a bird egg. Examples of the bird egg include, but are not limited to, a quail egg or chicken egg. The processed egg product refers to a product produced by processing an egg as a main raw material or each food comprising the processed egg product as a raw material. Examples of the processed egg product include, but are not limited to, a processed fish roe product (e.g., salted cod roe, spicy cod roe, salted salmon roe, salmon roe pickled in soy sauce, a baby sardine, dried mullet roe) or a processed chicken egg product (e.g., mayonnaise, hollandaise sauce, quail egg boiled in water, boiled egg, boiled egg seasoned in soy sauce, omelet, thick omelet, rolled egg, steamed egg, omelet, omelet rice, baked egg, poached egg, quiche, scotch egg, French toast, cake, pastry, custard pudding, biscuit, cookie, snack, bread).

The parasites are not limited, and are, for example, parasites of the family *Anisakidae.* Examples of the parasites of the family *Anisakidae* include *Anisakis simplex.*

As used herein, the allergy refers to a state with an allergic reaction caused by, as an antigen, a protein, etc., contained in a raw material or processed product (e.g., milk or a processed milk product). In the allergy, an allergic reaction can occur in the case of contact with an antigen contained in a raw material or processed product (e.g., milk or a processed milk product) or in the case of ingestion of the antigen. In general, the allergic reaction occurring when a food is ingested refers to, in particular, a food allergy. Milk allergy may be a food allergy.

As used herein, the antigen is a substance that can trigger an allergic reaction. In the case of a protein contained in a raw material for a foodstuff, etc., the antigen is also referred to as an allergen component. The antigen is preferably a protein.

As used herein, the protein is a molecule with a structure in which naturally occurring amino acids are linked via a peptide bond. The number of amino acids contained in the protein is not particularly limited. As used herein, the term "polypeptide" also means a molecule with a structure in which naturally occurring amino acids are linked via a peptide bond. The number of amino acids contained in the polypeptide is not particularly limited. The "polypeptide" is a concept including a "protein". The polypeptide in which about 2 to 50 amino acids are linked via peptide bonds, in particular, is sometimes called a peptide.

In the case where an amino acid may have an enantiomer, the amino acid is in an L-form unless otherwise indicated. The nomenclature of amino acid sequences of proteins, polypeptides, or peptides used herein is based on standard usage or the nomenclature conventionally used in the art. The amino acid is represented by one-letter code; the leftward direction represents the amino terminal direction, and the rightward direction represents the carboxy terminal direction. Note that in the one-letter amino acid code, X may be any of substances each having an amino group and a carboxyl group and capable of bonding to amino acids at both ends, and may represent any of, in particular, naturally occurring 20 amino acids.

Alanine scanning (or "alanine/glycine scanning") is a method including: creating variants in which residues in a protein is mutated to alanine (glycine if the original amino acid is alanine) one by one; and site-specifically identifying which residue is important for the structure and the function of the protein. If the binding ability to IgE antibodies from patients remains even after replacement with alanine (glycine if the original amino acid is alanine), the residues are not important for the binding ability to IgE antibodies, and, in this case, the binding ability remains even after replacement of these residues to other amino acids. The binding ability to the IgE antibodies means the detection of the binding and reaction between an IgE antibody and an epitope of interest. In the sequence listing of the present application, a residue denoted by X is an amino acid residue at a site where, even after replacement of the residue with alanine (glycine if the original amino acid is alanine) by the alanine/glycine scanning shown in Example 4, the binding ability to IgE antibodies from patients with allergy remains. It is well-known to those skilled in the art that even when such a site is replaced with any other amino acids, the probability that the binding ability to the IgE antibodies remains is high. Specifically, the residue can be replaced with not only alanine or glycine but also any amino acid residues other than alanine.

The binding of the IgE to an antigen (epitope) and its maintenance are important for a subsequent allergic reaction, and electric charge, hydrophobic bonds, hydrogen bonds, and aromatic interaction of the epitope are responsible for the above binding and maintenance. Even after the loss of them due to alanine/glycine replacement, the binding and its maintenance may remain, which means that the amino acid is dispensable.

### Identification of Antigen

Proteins contained in milk were subjected to 2D electrophoresis under the conditions below to identify milk allergy antigens.

For the first dimension, electrophoresis was isoelectric focusing. The gel length was in the range from 5 to 10 cm. The pH range of the gel was from 3 to 10. The pH gradient of the gel in the electrophoresis direction was set while the entire gel length was set to 1, the gel length up to pH 5 was "a", the gel length between pH 5 and pH 7 was "b", and the gel length of pH 7 or higher was "c". In this case, used was a gel with "a" ranging from 0.15 to 0.3, "b" ranging from 0.4 to 0.7, and "c" ranging from 0.15 to 0.3. Specifically, an IPG gel Immobiline Drystrip (pH 3-10 NL), manufactured by GE Healthcare Bioscience, Inc. (hereinafter, abbreviated as GE Company), was used to conduct isoelectric focusing. The electrophoresis apparatus used was an IPGphor, manufactured by GE Company. The upper limit of a current value in the electrophoresis apparatus was set to 75 µA per gel. The voltage program included (1) a constant voltage step of providing a constant voltage of 300 V until 750 Vhr was reached (a change in current for 30 min of electrophoresis before end of the step was 5 µA); (2) gradually increasing the voltage up to 1000 V over 300 Vhr; (3) further gradually increasing the voltage up to 5000 V over 4500 Vhr; and (4) subsequently providing a constant voltage of 5000 V until the total Vhr reached 12000. In this way, 1D isoelectric focusing was performed.

In the second dimension, electrophoresis was a SDS-PAGE, in which the gel concentration at a base end in the electrophoresis direction was set to 3 to 6%, and the gel concentration at a portion on the distal end side in the electrophoresis direction was set to be higher than the gel concentration at a portion on the base end side in the electrophoresis direction. Such a polyacrylamide gel was used. Specifically, a NuPAGE 4-12% Bis-Tris Gels IPG well mini 1mm, manufactured by Life Technologies, Inc., was used to conduct SDS-PAGE. The electrophoresis apparatus used was a XCell SureLock Mini-Cell, manufactured by Life Technologies, Inc. The running buffer used was 50mM MOPS, 50mM Tris Base, 0.1% (w/v) SDS, and 1mM EDTA. The electrophoresis was performed at a constant voltage of 200 V for about 45 min.

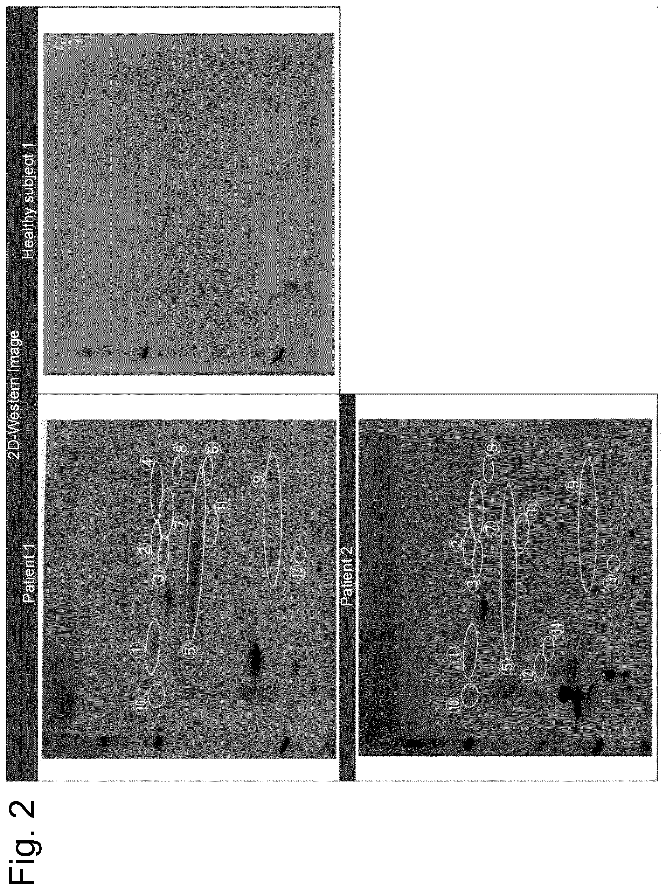

Milk proteins were subjected to 2D electrophoresis under the above conditions. The results have revealed that in the gel, the antigens of the following spots 1 to 14 specifically bind to an IgE antibodies of patients with milk allergy (Fig. 2).

### Antigens (Proteins)

The sequences contained in each spot were identified by mass spectrometry. The mass data obtained using a mass spectrometer was analyzed by comparing between the mass data and protein data of Uniprot, NCBI. The results have revealed that each of spots 1 to 14 corresponds to known sequences.

Information about each spot is summarized in Table 1 below.

**[Table 1-1]**

| | | | MW:66kDa |
|---|---|---|---|
| SPOT No. | | | ① |
| protein | Isoelectric point | Range | 2-8 |
| | | Preferably | 2.5-7 |
| | | More preferably | 3-6 |
| | Molecular weight | Range | 40-160 |
| | | Preferably | 50-110 |
| | | More preferably | 60-100 |
| | Searched database | | NCBI |
| | Biological species | | Cow (*Bos taurus*) |
| | accession No. | | AAN60017.1 |
| | Protein name | | immunoglobulin M heavy chain secretory form |
| | Full-length sequence (SEQ ID NO: 2) | | SQVQLRESGPSLVKPSQTLSLTCTVSGFSLSDYRVGWVRQAPGKALEWLGVMYNDGTTAYRPALLSRLRITRDNSKNQVALS LSSVTTEDTATYYCTRIIPVCTWPYGDYGCYDILGQGLLVTVSSEGESHPRVFPLVSCVSSPSDESTVALGCLARDFVPNSV SFSWKFNNSTVSSERFWTFPEVLRDGLWSASSQVVLPSSSAFQGPDDYLVCEVQHPKGGKTVGTVRVVATKAEVLSPVVSVF VPPRNSLSGDGNSKSSLICQATDFSPKQISLSWFRDGKRIVSGISEGQVETVQSSPVTFRAYSMLTITERDWLSQNVYTCQV EHNKETFQKNVSSSCDVAPPSPIGVFTIPPSFADIFLTKSAKLSCLVTNLASYDGLNISWSRQNGKALETHTYFERHLNDTF SARGEASVCSEDWESGEEFTCTVAHSDLPFPEKNTVSKPKDVAMKPPSVYLLPPTREQLSLRESASVTCLVKGFAPADVFVQ WLQRGEPVTKSKYVTSSPAPEPQDPSVYFVHSILTVAEEDWSKGETYTCVVGHEALPHMVTERTVDKSTGKPTLYNVSLVLS DTASTCY |
| DNA | Searched database | | GenBank |
| | accession No. | | AY145128.1 |
| | Full-length sequence (SEQ ID NO: 1) | | TCCCAGGTGCAGCTGC GGGAGTCGGGCCCAGCCTGGTGAAGCCCTCACAGACCCTCTCCCTCACCTGCACGGTCTCTGGATTCTC ATTAAGCGATTATCGTGTAGGCTGGGTCCGCCAGGCTCCAGGAAAGGCGCTGGAGTGGCTCGGTGTAATG TATAATGACGGAACCACTGCGTATAGGCCAGCCCTGCTATCCCGGCTCCGAATCACCAGGGACAACTCCA AGAACCAAGTCGCCCTGTCACTAAGCAGCGTAACAACTGAGGACACGGCCACATACTACTGTACAAGAAT AATTCCTGTGTGTACTTGGCCTTATGGTGACTATGGCTGTTATGACATCTTGGGCCAAGGACTCCTGGTC ACCGTCTCCTCAGAAGGTGAATCACACCCGAGAGTCTTCCCCCTGGTGTCCTGCGTGAGCTCCCCATCCG ATGAGAGCACGGTGGCCCTGGGCTGCCTGGCCCGGGACTTCGTGCCCAATTCAGTCAGCTTCTCCTGGAA GTTCAACAACAGCACAGTCAGCAGCGAGAGATTCTGGACCTTCCCCGAAGTCCTGAGGGACGGCTTGTGG TCGGCCTCCTCTCAGGTGGTCCTGCCCTCCTCAAGCGCCTTTCAAGGGCCGGATGACTACCTGGTGTGCG AAGTCCAGCACCCCAAGGGAGGAAAGACCGTCGGCACCGTGAGGGTGGTCGCTACAAAGGCGGAAGTGCT GTCCCCAGTCGTGAGTGTCTTTGTCCCGCCTCGCAACAGCCTCTCTGGTGACGGCAATAGCAAGTCCAGC CTCATCTGCCAGGCCACGGACTTCAGCCCCAAACAGATCTCCTTGTCCTGGTTTCGTGATGGAAAGCGGA TAGTGTCTGGCATTTCTGAAGGCCAGGTGGAGACTGTGCAGTCCTCACCCGTAACTTTCAGGGCCTACAG CATGCTGACCATCACGGAGAGAGACTGGCTCAGCCAGAACGTGTACACCTGCCAGGTAGAACACAACAAG GAAACCTTCCAGAAGAACGTGTCCTCCTCATGTGATGTTGCACCACCATCTCCCATCGGGGTCTTCACCA TCCCCCCATCCTTCGCCGACATCTTCCTCACGAAGTCAGCCAAGCTGTCCTGTCTGGTCACAAACCTGGC CTCCTATGATGGCCTGAACATCAGCTGGTCCCGTCAGAACGGCAAGGCCCTGGAGACCCACACGTATTTT GAGCGACACCTCAACGACACCTTCAGCGCCCGGGGTGAGGCCTCGGTCTGCTCGGAGGACTGGGAGTCCG GAGAGGAGTTCACGTGCACAGTGGCCCACTCGGACCTGCCCTTCCCAGAAAAGAACACCGTCTCCAAGCC CAAAGATGTCGCCATGAAACCGCCGTCCGTGTACCTGCTGCCTCCAACGCGGGAACAGCTGAGCCTGCGG GAGTCGGCCTCCGTCACCTGCCTGGTGAAGGGCTTCGCGCCCGCGGACGTGTTCGTGCAGTGGCTGCAGA GGGGGGAGCCCGTGACCAAGAGCAAGTACGTGACCAGCAGCCCGGCGCCCGAGCCCCAGGACCCCAGCGT GTACTTTGTGCACAGCATCCTGACGGTGGCCGAGGAGGACTGGAGCAAAGGGGAGACCTACACCTGCGTC GTGGGCCACGAGGCCCTGCCCCACATGGTCACCGAGCGGACCGTGGACAAGTCCACCGGTAAACCCACCC TGTACAACGTGTCCCTGGTCCTGTCTGACACAGCCAGCACCTGCTAC |

**[Table 1-2]**

| | | | MW:78kDa |
|---|---|---|---|
| SPOT No. | | | ②③ |
| protein | Isoelectric point | Range | 4-11 |
| | | Preferably | 4.5-10 |
| | | More preferably | 5-9 |
| | Molecular weight | Range | 40-160 |
| | | Preferably | 50-110 |
| | | More preferably | 60-100 |
| | Searched database | | Uniprot |
| | Biological species | | Cow (*Bos taurus*) |
| | accession No. | | G3X6N3 |
| | Protein name | | Beta-1 metal-binding globulin |
| | Full-length sequence (SEQ ID NO: 4) | | DPERTVRWCTISTHEANKCASFRENVLRILESGPFVSCVKK TSHMDCIKAISNNEADAVTLDGGLVYEAGLKPNNLKPVVAEFHGTKDNPQTHYYAVAVVK KDTDFKLNELRGKKSCHTGLGRSAGWNIPMGKLYKELPDPQESIQRAAANFFSASCVPCA DQSSFPKLCQLCAGKGTDKCACSNHEPYFGYSGAFKCLMEGAGDVAFVKHSTVFDNLPNP EDRKNYELLCGDNTRKSVDNYQECYLAMVPSHAVVARTVGGKEDVIWELLNHAQEHFGKD KPDNFQLFQSPHGKDLLFKDSADGFLKIPSKMDFELYLGYEYVTALQNLRESKPPDSSKD ECMVKWCAIGHQERTKCDRWSGFSGGAIECETAENTEECIAKIMKGEADAMSLDGGYLYI AGKCGLVPVLAENYKTEGESCKNTPEKGYLAVAVVKTSDANINWNNLKDKKSCHTAVDRT AGWNIPMGLLYSKINNCKFDEFFSAGCAPGSPRNSSLCALCIGSEKGTGKECVPNSNERY YGYTGAFRCLVEKGDVAFVKDQTVIQNTDGNNNEAWAKNLKKENFEVLCKDGTRKPVTDA ENCHLARGPNHAVVSRKDKATCVEKILNKQQDDFGKSVTDCTSNFCLFQSNSKDLLFRDD TKCLASIAKKTYDSYLGDDYVRAMTNLRQCSTSKLLEACTFHKP |
| DNA | Searched database | | GenBank |
| | accession No. | | NM_177484.3 mutant |
| | Full-length sequence (SEQ ID NO: 3) | | GACCCTGAGAGAA CTGTGAGATGGTGCACCATTTCAACTCATGAGGCCAATAAGTGTGCCAGTTTTCGTGAAAATGTGCTTCG TATCTTGGAGAGTGGTCCTTTTGTCTCCTGTGTGAAGAAAACCTCACACATGGATTGCATCAAGGCTATC TCGAATAACGAAGCAGATGCTGTGACGTTGGATGGAGGTTTGGTGTATGAGGCAGGCCTGAAGCCCAACA ACCTGAAGCCTGTGGTGGCAGAGTTCCATGGGACAAAAGACAACCCACAAACTCACTATTATGCTGTGGC GGTGGTGAAGAAGGACACTGACTTCAAGCTGAATGAGCTCAGAGGCAAGAAGTCCTGCCACACAGGCCTC GGCAGGTCCGCTGGGTGGAACATCCCCATGGGCAAACTTTATAAGGAATTGCCTGATCCACAGGAATCAA TTCAGAGAGCTGCGGCCAACTTCTTCTCTGCCAGCTGTGTCCCCTGTGCGGATCAATCATCATTTCCCAA ACTCTGTCAACTGTGTGCGGGGAAAGGGACAGACAAGTGTGCCTGCTCCAACCACGAACCATACTTCGGC TACTCAGGGGCCTTTAAATGTCTGATGGAGGGCGCTGGGGATGTGGCCTTTGTCAAGCACTCAACGGTAT TCGACAACCTGCCTAACCCAGAAGACAGGAAAAACTATGAGCTGCTCTGCGGGGACAACACCCGGAAGTC TGTAGATAATTACCAAGAATGCTACTTGGCAATGGTTCCTTCCCATGCGGTTGTGGCTCGAACTGTGGGC GGCAAGGAGGATGTGATCTGGGAACTTCTCAATCACGCCCAGGAACATTTTGGCAAAGATAAACCAGACA ATTTCCAGCTTTTCCAATCCCCTCATGGGAAGGACCTGCTGTTTAAGGACTCTGCTGATGGGTTTTTAAA GATTCCTTCTAAGATGGATTTTGAGCTGTACTTGGGATATGAATATGTCACTGCTCTTCAGAATCTAAGA GAAAGTAAACCCCCGGACTCCTCAAAGGATGAGTGCATGGTGAAGTGGTGTGCAATTGGTCACCAGGAGA GGACAAAGTGTGATCGGTGGAGTGGGTTCAGCGGCGGGGCAATAGAGTGTGAAACAGCAGAGAACACTGA AGAGTGCATCGCCAAGATCATGAAAGGAGAAGCTGATGCCATGAGCTTGGACGGAGGCTACCTCTACATA GCAGGCAAATGTGGCCTGGTGCCTGTCCTGGCAGAGAACTACAAAACTGAGGGTGAAAGTTGCAAGAACA CACCAGAAAAAGGGTATCTTGCTGTAGCTGTGGTTAAAACATCAGATGCTAATATCAACTGGAACAATCT GAAAGACAAGAAGTCCTGCCACACTGCAGTAGACAGAACCGCTGGCTGGAACATCCCCATGGGTTTGCTC TACAGCAAGATTAATAACTGTAAATTTGATGAATTTTTCAGCGCAGGTTGTGCACCTGGATCTCCGCGAA ATTCCAGTCTCTGTGCTCTGTGCATTGGCTCAGAGAAGGGTACAGGAAAGGAGTGTGTTCCCAACAGCAA TGAAAGATACTATGGCTATACAGGGGCTTTCAGGTGTCTGGTGGAGAAGGGAGACGTGGCCTTTGTGAAG GACCAGACTGTCATACAGAACACTGACGGAAATAATAATGAAGCATGGGCAAAAAATCTGAAGAAGGAAA ATTTTGAAGTACTATGCAAAGATGGCACCAGGAAACCTGTGACAGATGCTGAGAACTGCCACCTGGCCCG AGGCCCGAATCATGCTGTGGTCTCACGGAAAGATAAGGCAACTTGTGTGGAGAAAATATTAAACAAACAG CAGGATGATTTTGGAAAATCTGTAACCGACTGCACGAGCAATTTTTGTTTATTCCAATCAAATTCCAAGG ACCTTCTGTTCAGGGATGACACTAAATGTTTGGCTTCAATTGCGAAAAAAACATATGACTCCTACTTAGG GGATGACTACGTCAGAGCTATGACCAACCTGAGACAATGCTCAACCTCAAAACTCCTGGAAGCATGCACT TTCCACAAACCT |

**[Table 1-3]**

| | | | MW:83kDa |
|---|---|---|---|
| SPOT No. | | | ④ |
| protein | Isoelectric point | Range | 6-12 |
| | | Preferably | 6.5-11 |
| | | More preferably | 7-10 |
| | Molecular weight | Range | 40-160 |
| | | Preferably | 50-110 |
| | | More preferably | 60-100 |
| | Searched database | | Uniprot |
| | biological species | | Cow (*Bos taurus*) |
| | accession No. | | G3MXZ0 |
| | Protein name | | Lactoperoxidase |
| | Full-length sequence (SEQ ID NO: 6) | | DTIAQAASTTTISDAVSKV KIQVNKAFLDSRTRLKTTLSSEAPTTQQLSEYFKHAKGRTRTAIRNGQVWEESLKRLRRD TTLTNVTDPSLDLTALSWEVGCGAPVPLVKCDENSPYRTITGDCNNRRSPALGAANRALA RWLPAEYEDGLALPFGWTQRKTRNGFRVPLAREVSNKIVGYLDEEGVLDQNRSLLFMQWG QIVDHDLDFAPETELGSNEHSKTQCEEYCIQGDNCFPIMFPKNDPKLKTQGKCMPFFRAG FVCPTPPYQSLAREQINAVTSFLDASLVYGSEPSLASRLRNLSSPLGLMAVNQEAWDHGL AYLPFNNKKPSPCEFINTTARVPCFLAGDFRASEQILLATAHTLLLREHNRLARELKKLN PHWNGEKLYQEARKILGAFIQIITFRDYLPIVLGSEMQKWIPPYQGYNNSVDPRISNVFT FAFRFGHMEVPSTVSRLDENYQPWGPEAELPLHTLFFNTWRIIKDGGIDPLVRGLLAKKS KLMNQDKMVTSELRNKLFQPTHKIHGFDLAAINLQRCRDHGMPGYNSWRGFCGLSQPKTL KGLQTVLKNKILAKKLMDLYKTPDNIDIWIGGNAEPMVERGRVGPLLACLLGRQFQQIRD GDRFWWENPGVFTEKQRDSLQKVSFSRLICDNTHITKVPLHAFQANNYPHDFVDCSTVDK LDLSPWASREN |
| DNA | Searched database | | GenBank |
| | accession No. | | XM_024979549.1 mutant |
| | Full-length sequence (SEQ ID NO: 5) | | gacacaattgcacaggccgccagcaccaccaccatctctgatgctgtgagtaaggtcaagatccaggtcaacaaggc cttcctggattcccggaccaggctgaagacgaccttgagctctgaggcacccaccacccaacagctctcagagtact tcaagcacgcaaagggccggacccgcacggccattcgcaacgggcaggtgtgggaggagtccttaaagaggctgagg cgggacacaaccctgaccaacgtcacagaccctagcctggacttgactgcactctcctgggaggtgggctgcggtgc cccggttcctctggtgaaatgtgatgaaaacagcccttaccgcaccatcacgggagactgtaataacaggaggagcc ccgcactgggcgccgccaacagggcgctggcgcgctggctgccggcggagtacgaggacgggctcgccctgcccttc ggctggacgcagaggaagacgcgcaacggcttccgcgtcccgctggcccgggaggtatccaacaaaattgtaggcta cctggacgaagagggtgttctggaccaaaacaggtccctgctcttcatgcagtggggtcagattgtggaccacgacc tggacttcgccccggaaacggaactggggagcaacgagcactctaaaacccagtgtgaggagtactgtatccaggga gacaactgcttccccatcatgttcccgaaaaatgatcccaagttgaagactcaagggaaatgcatgcctttcttccg agccgggtttgtctgccccactccaccttaccagtcgttggcccgagaacagatcaatgctgtgacctccttcctgg acgccagcttagtgtacggctctgagcccagtctggccagccgtctccggaacctcagcagcccgctgggcctcatg gctgtcaaccaagaagcctgggaccacgggctggcctacctgccctttaacaacaagaagccgagcccctgtgagtt catcaacaccaccgcccgcgtgccctgtttcctggcgggagattttcgagcctcagagcagattctgctggccactg cccacaccctccttctccgggagcacaaccggctggccagagaactaaagaaactcaaccctcactggaatggagag aagctctaccaggaagcccggaaaatcctgggagccttcatacagatcatcacctttagggactacctacccattgt gctaggtagtgagatgcagaagtggatcccgccctaccaaggctataataactctgtggatccccgaatttccaatg tcttcacctttgccttccgctttggccacatggaggttccctccactgtgtcccgcctggatgagaattaccagcca tggggtccggaagcagagctccccctacacaccctcttcttcaacacctggaggataatcaaagacggtggaattga ccctctggtgcggggtctgctggccaagaagtccaaactgatgaatcaggataaaatggtgacgagtgagctgcgca acaagcttttccagcccactcacaagatccacggctttgacctggctgctatcaacttacagcgttgccgagaccat gggatgcctgggtacaactcctggaggggcttctgtggcctctcacagcccaagacactgaaggggctgcagactgt gctgaagaacaagatactggctaagaagttaatggatctctataagacccccgacaacattgacatctggattggag gcaacgctgagcccatggtagaaaggggccgggtggggccactcctggcctgcctcctagggaggcaattccagcag atacgtgatggggacaggttctggtgggagaaccctggggtcttcactgagaagcagcgggactctctacagaaagt gtccttctcacgcctcatctgtgacaacacccacatcacgaaggtcccgctgcatgccttccaggccaacaactacc cacatgactttgtggattgctcaaccgttgataagctggatctctcaccctggcctccaggagaat |

**[Table 1-4]**

| | | | MW:51kDa |
|---|---|---|---|
| SPOT No. | | | ⑤ |
| protein | Isoelectric point | Range | 4-12 |
| | | Preferably | 4.5-11 |
| | | More preferably | 5-10 |
| | Molecular weight | Range | 40-110 |
| | | Preferably | 45-100 |
| | | More preferably | 50-80 |
| | Searched database | | NCBI |
| | Biological species | | Cow (*Bos taurus*) |
| | accession No. | | S22080 |
| | Protein name | | Ig heavy chain precursor (B/MT.4A.17.H5.A5) |
| | Full-length sequence (SEQ ID NO: 8) | | SQVQLRESGPSLVKPSQTLSLTCTVSGFSLSSYALTWVRQAPGKALEWVGGI TSGGTTYYNPALKSRLSITKENSKSQVSLSVSSVTPEDTATYYCARSTYGEVGDGAIADAWGQGLLVTVS SASTTAPKVYPLSSCCGDKSSSTVTLGCLVSSYMPEPVTVTWNSGALKSGVHTFPAVLQSSGLYSLSSMV TVPGSTSGQTFTCNVAHPASSTKVDKAVDPTCKPSPCDCCPPPELPGGPSVFIFPPKPKDTLTISGTPEV TCVVVDVGHDDPEVKFSWFVDDVEVNTATTKPREEQFNSTYRVVSALRIQHQDWTGGKEFKCKVHNEGLP APIVRTISRTKGPAREPQVYVLAPPQEELSKSTVSLTCMVTSFYPDYIAVEWQRNGQPESEDKYGTTPPQ LDADSSYFLYSKLRVDRNSWQEGDTYTCVVMHEALHNHYTQKSTSKSAGK |
| DNA | Searched database | | GenBank |
| | accession No. | | KT761812.1 mutant |
| | Full-length sequence (SEQ ID NO: 7) | | TCCCAGGTGCAGCTGC GGGAGTCGGGCCCAGCCTGGTGAAGCCCTCACAGACCCTCTCCCTCACGTGCACGGTGTCTGGATTCTC ATTGAGCAGCTATGCTCTAACCTGGGTCCGCCAGGCTCCAGGGAAGGCGCTGGAGTGGGTTGGTGGTATA ACCAGTGGTGGAACCACATACTATAATCCAGCCCTGAAATCCCGGCTCAGCATCACCAAGGAGAACTCCA AGAGCCAAGTCTCTCTGTCAGTGAGCAGCGTGACACCTGAGGACACAGCCACATACTACTGTGCAAGAAG TACTTATGGTGAGGTTGGTGATGGTGCCATCGCCGATGCCTGGGGCCAAGGACTCCTGGTCACCGTCTCC TCAGCCTCCACCACAGCCCCGAAAGTCTACCCTCTGAGTTCTTGCTGCGGGGACAAGTCCAGCTCCACCG TGACCCTGGGCTGCCTGGTCTCCAGCTACATGCCCGAGCCGGTGACCGTGACCTGGAACTCGGGTGCCCT GAAGAGCGGCGTGCACACCTTCCCGGCTGTCCTTCAGTCCTCCGGGCTGTACTCTCTCAGCAGCATGGTG ACCGTGCCCGGCAGCACCTCAGGACAGACCTTCACCTGCAACGTAGCCCACCCGGCCAGCAGCACCAAGG TGGACAAGGCTGTTGATCCCACATGCAAACCATCACCCTGTGACTGTTGCCCACCCCCTGAGCTCCCCGG AGGACCCTCTGTCTTCATCTTCCCACCGAAACCCAAGGACACCCTCACAATCTCGGGAACGCCCGAGGTC ACGTGTGTGGTGGTGGACGTGGGCCACGATGACCCCGAGGTGAAGTTCTCCTGGTTCGTGGACGACGTGG AGGTAAACACAGCCACGACGAAGCCGAGAGAGGAGCAGTTCAACAGCACCTACCGCGTGGTCAGCGCCCT GCGCATCCAGCACCAGGACTGGACTGGAGGAAAGGAGTTCAAGTGCAAGGTCCACAACGAAGGCCTCCCG GCCCCCATCGTGAGGACCATCTCCAGGACCAAAGGGCCGGCCCGGGAGCCGCAGGTGTATGTCCTGGCCC CACCCCAGGAAGAGCTCAGCAAAAGCACGGTCAGCCTCACCTGCATGGTCACCAGCTTCTACCCAGACTA CATCGCCGTGGAGTGGCAGAGAAACGGGCAGCCTGAGTCGGAGGACAAGTACGGCACGACCCCGCCCCAG CTGGACGCCGACAGCTCCTACTTCCTGTACAGCAAGCTCAGGGTGGACAGGAACAGCTGGCAGGAAGGAG ACACCTACACGTGTGTGGTGATGCACGAGGCCCTGCACAATCACTACACGCAGAAGTCCACCTCTAAGTC TGCGGGTAAA |

**[Table 1-5]**

| | | | MW:49kDa |
|---|---|---|---|
| SPOT No. | | | ⑥ |
| protein | Isoelectric point | Range | 6-12 |
| | | Preferably | 7-11.5 |
| | | More preferably | 8-11 |
| | Molecular weight | Range | 20-90 |
| | | preferably | 30-80 |
| | | More preferably | 40-60 |
| | Searched database | | Uniprot |
| | Biological species | | Cow (*Bos taurus*) |
| | accession No. | | F1N1N6 |
| | Protein name | | Perilipin |
| | Full-length sequence (SEQ ID NO: 10) | | MASVAAEPQLSVVTRVANLPLVSSTYDLVSSAYISRKDQYPYLKSLCEMAEKGMKTITSV AVTSALPIIQKLEPQIAVANTYACKGLDRIEEKLPILNQPTNQVVANAKGAMTGAKDAVT TTVTGAKDSVASTITGVVDRTKGAVTGSVEKTKSVVSGSINTVLRSRVMQLMSSGVENAL TKSELLVDQYLPLTKDELEKEAKKVEGFDMVQKPSYYVRLGSLSTKLRSRAYQQALCRVE EAKRKGQETISQLHSAFNLSELARKNVHNANQKIQDAQDKLYLSWLEWKRSIGYDDTDES HCAEHIESRTLAIARNLTQQLQTMCHTLLSNIQGLPQNIQDRANHLGVMAGDIYSVFRNA ASFKEVSDGLLASSKGQLQKMKESLDDVMDYLVNNTPLNWLVGPFYPQVTESESAQAPGT TRRPGRWSRKHPKPVPVSNAEGSQPDDSSS |
| DNA | Searched database | | GenBank |
| | accession No. | | XM_024995432.1 |
| | Full-length sequence (SEQ ID NO: 9) | | ATGGCATCTGTTGCAGCTGAACCACAACTGAGTGTGGTGACCAGGGTGGCCAACCTCCCCTTGGTGAGCT CCACGTATGATCTGGTCTCCTCGGCTTACATCAGTAGAAAGGATCAGTATCCTTACTTGAAGTCTCTGTG TGAGATGGCAGAGAAGGGCATGAAGACCATCACTTCTGTGGCTGTGACCAGCGCGCTGCCAATCATCCAA AAGTTAGAGCCACAAATTGCTGTTGCCAATACCTATGCGTGTAAGGGGCTAGACAGGATTGAGGAGAAGC TGCCTATTCTGAATCAGCCAACAAACCAGGTTGTGGCCAATGCCAAAGGGGCTATGACTGGGGCAAAAGA TGCTGTGACGACTACTGTGACCGGGGCCAAGGATTCTGTGGCCAGCACAATCACGGGGGTGGTGGACAGG ACCAAGGGAGCTGTGACTGGTAGTGTGGAGAAGACCAAGTCCGTGGTCAGTGGCAGCATTAACACAGTCC TACGAAGTCGGGTGATGCAGCTGATGAGCAGTGGAGTAGAAAATGCACTCACCAAATCAGAGCTGCTGGT AGACCAGTACCTCCCTCTTACCAAGGATGAACTAGAAAAAGAAGCCAAAAAAGTGGAAGGATTCGATATG GTTCAGAAGCCGAGTTACTATGTTAGACTGGGATCTCTGTCCACCAAGCTGCGCTCACGGGCCTACCAGC AGGCCCTTTGTAGGGTTGAAGAAGCTAAGCGAAAAGGCCAGGAGACCATTTCTCAGCTCCATTCCGCCTT CAACCTGAGTGAACTTGCCAGGAAGAATGTGCATAATGCCAACCAGAAAATTCAGGATGCTCAGGATAAG CTCTATCTGTCCTGGCTGGAGTGGAAGAGAAGCATCGGCTACGATGATACAGATGAATCCCACTGTGCTG AGCACATTGAGTCACGTACTCTTGCTATTGCCCGGAACCTGACTCAGCAGCTCCAGACCATGTGCCACAC CCTCCTGTCCAACATCCAGGGGTTACCACAGAACATTCAAGATCGGGCCAACCACTTGGGGGTGATGGCT GGTGACATCTACTCTGTGTTTCGCAATGCTGCCTCCTTTAAAGAAGTGTCTGATGGCCTCCTCGCTTCCA GCAAGGGGCAGCTGCAGAAAATGAAGGAGTCTTTAGATGATGTGATGGATTATCTTGTTAACAACACACC CCTCAACTGGCTGGTAGGTCCCTTTTATCCCCAGGTGACCGAGTCTGAGAGTGCTCAGGCCCCAGGTACA ACCAGGAGGCCTGGCAGGTGGAGTAGAAAACACCCTAAACCCGTCCCTGTCAGCAATGCAGAGGGCAGCC AGCCAGATGACAGCTCCTCT |

**[Table 1-6]**

| | | | MW:82kDa |
|---|---|---|---|
| SPOT No. | | | ⑦ |
| protein | Isoelectric point | Range | 5-12 |
| | | Preferably | 5.5-11 |
| | | More preferably | 6-10 |
| | Molecular weight | Range | 40-160 |
| | | Preferably | 50-110 |
| | | More preferably | 60-100 |
| | Searched database | | Uniprot |
| | Biological species | | Cow (*Bos taurus*) |
| | accession No. | | P81265 |
| | Protein name | | Polymeric immunoglobulin receptor |
| | Full-length sequence (SEQ ID NO: 12) | | KSPIFGPEEVTSVEGRSVSIKCYYPPTSVNRHTRKYWCRQGA QGRCTTLISSEGYVSDDYVGRANLTNFPESGTFVVDISHLTHKDSGRYKCGLGISSRGLN FDVSLEVSQDPAQASHAHVYTVDLGRTVTINCPFTRANSEKRKSLCKKTIQDCFQVVDST GYVSNSYKDRAHISILGTNTLVFSVVINRVKLSDAGMYVCQAGDDAKADKINIDLQVLEP EPELVYGDLRSSVTFDCSLGPEVANVPKFLCQKKNGGACNVVINTLGKKAQDFQGRIVSV PKDNGVFSVHITSLRKEDAGRYVCGAQPEGEPQDGWPVQAWQLFVNEETAIPASPSVVKG VRGGSVTVSCPYNPKDANSAKYWCHWEEAQNGRCPRLVESRGLIKEQYEGRLALLTEPGN GTYTVILNQLTDQDTGFYWCVTDGDTRWISTVELKVVQGEPSLKVPKNVTAWLGEPLKLS CHFPCKFYSFEKYWCKWSNRGCSALPTQNDGPSQAFVSCDQNSQVVSLNLDTVTKEDEGW YWCGVKEGPRYGETAAVYVAVESRVKGSQGAKQVKAAPAGAAIQSRAGEIQNKALLDPSF FAKESVKDAAGGPGAPADPGRPTGYSGSSKALVSTLVPLALVLVAGVVAIGVVRARHRKN VDRISIRSYRTDISMSDFENSRDFEGRDNMGASPEAQETSLGGKDEFATTTEDTVESKEP KKAKRSSKEEADEAFTTFLLQAKNLASAATQNGPTEA |
| | Searched database | | GenBank |
| | accession No. | | AAC41620.1 |
| DNA | Full-length sequence (SEQ ID NO: 11) | | AAGAGT CCCATCTTCGGTCCCGAGGAGGTGACCAGCGTGGAAGGCCGCTCAGTGTCCATCAAGTGC TACTACCCGCCCACCTCCGTCAACCGGCACACGCGCAAGTACTGGTGCCGGCAGGGAGCC CAGGGCCGCTGCACGACCCTCATCTCCTCGGAGGGCTACGTCTCCGACGACTACGTGGGC AGAGCCAACCTCACCAACTTCCCGGAGAGCGGCACGTTTGTGGTGGACATCAGCCATCTC ACCCATAAAGACTCAGGGCGCTACAAGTGTGGCCTGGGCATTAGCAGCCGTGGCCTTAAC TTCGATGTGAGCCTGGAGGTCAGCCAAGATCCTGCACAGGCAAGTCATGCCCACGTCTAC ACTGTAGACCTGGGCAGGACTGTGACCATCAACTGCCCTTTCACGCGTGCGAATTCTGAG AAGAGAAAATCCTTGTGCAAGAAGACAATCCAGGACTGTTTCCAAGTTGTCGACTCCACC GGGTATGTGAGCAACAGCTATAAAGACAGAGCACATATCAGTATCCTAGGTACCAACACA TTAGTGTTCAGCGTTGTCATCAACCGAGTCAAGCTCAGTGATGCTGGGATGTATGTCTGC CAGGCTGGGGACGATGCCAAAGCCGATAAAATCAACATTGACCTCCAGGTGCTGGAGCCT GAGCCTGAGCTGGTTTATGGAGACTTGAGGAGCTCGGTGACCTTTGACTGTTCCCTGGGC CCCGAGGTGGCAAATGTGCCCAAATTTCTGTGCCAGAAGAAGAATGGGGGAGCTTGCAAT GTGGTCATCAACACGTTGGGGAAGAAGGCTCAGGACTTCCAGGGCAGGATCGTGTCCGTG CCCAAGGACAATGGTGTCTTCAGTGTGCACATTACCAGCCTGAGGAAAGAGGACGCAGGG CGCTACGTGTGCGGGGCCCAGCCTGAGGGTGAGCCCCAGGACGGCTGGCCTGTGCAGGCC TGGCAACTCTTCGTCAATGAAGAGACGGCAATCCCCGCAAGCCCCTCCGTGGTGAAAGGT GTGAGGGGAGGCTCTGTGACCGTATCTTGCCCCTACAACCCGAAGGATGCCAACAGCGCG AAGTACTGGTGTCACTGGGAAGAGGCTCAAAACGGCCGCTGCCCGCGGCTGGTGGAGAGC CGGGGGCTGATAAAGGAGCAGTACGAGGGCAGGCTGGCGCTGCTTACCGAGCCGGGCAAC GGCACCTACACCGTCATCCTCAACCAGCTCACCGACCAGGACACCGGCTTCTACTGGTGC GTGACCGACGGCGACACGCGCTGGATCTCCACAGTGGAGCTCAAGGTTGTCCAAGGAGAA CCAAGCCTCAAGGTACCCAAGAACGTCACGGCTTGGCTGGGAGAGCCCTTAAAGCTCTCC TGCCACTTCCCCTGCAAATTCTACTCCTTTGAGAAGTACTGGTGTAAGTGGAGCAACAGA GGCTGCAGCGCCCTGCCCACCCAGAACGACGGCCCCAGCCAGGCCTTTGTGAGCTGTGAC CAGAACAGCCAGGTCGTCTCCCTGAACCTGGACACAGTCACCAAGGAGGATGAAGGCTGG TACTGGTGTGGAGTGAAGGAGGGCCCCCGATATGGGGAGACGGCGGCTGTCTACGTGGCA GTGGAGAGCAGGGTGAAGGGGTCCCAAGGCGCCAAGCAAGTGAAAGCTGCCCCTGCGGGG GCGGCAATACAGTCGAGGGCCGGGGAGATCCAGAACAAAGCCCTTCTGGACCCCAGCTTT TTCGCAAAGGAAAGTGTGAAGGACGCTGCTGGTGGACCCGGAGCACCTGCAGATCCTGGC CGCCCTACAGGATACAGCGGGAGCTCCAAAGCACTGGTCTCCACCCTGGTGCCCCTGGCC CTGGTCCTGGTCGCAGGGGTCGTGGCGATCGGGGTGGTCCGAGCCCGGCACAGGAAGAAC GTCGACCGGATTTCAATCAGGAGCTACCGGACAGATATCAGCATGTCAGACTTTGAGAAC TCCAGGGATTTTGAAGGACGTGACAACATGGGAGCCTCTCCAGAGGCCCAAGAGACGTCT CTCGGAGGGAAGGACGAGTTTGCCACCACTACCGAGGACACCGTGGAGAGCAAAGAACCC AAGAAGGCAAAGAGGTCGTCCAAGGAGGAAGCCGACGAGGCCTTCACCACCTTCCTCCTC CAGGCCAAAAACCTGGCCTCCGCCGCAACCCAGAACGGCCCGACAGAAGCC |

**[Table 1-7]**

| | | | MW:74kDa |
|---|---|---|---|
| SPOT No. | | | ⑧ |
| protein | Isoelectric point | Range | 6-12 |
| | | Preferably | 7-11.5 |
| | | More preferably | 8-11 |
| | Molecular weight | Range | 40-110 |
| | | Preferably | 50-100 |
| | | More preferably | 60-80 |
| | Searched database | | Uniprot |
| | Biological species | | Cow (*Bos taurus*) |
| | accession No. | | A0A0F6QNP7 |
| | Protein name | | C3-beta-c |
| | Full-length sequence (SEQ ID NO: 14) | | NPMYSMITPNILRVESEETVVLEAHGGQGTIQVSVTVH DFPAKKQVLSNENTQLNSNNGYLSTVTIKIPASKELKSDKGHKFVTVVATFGNVQVEKVV LISLQSGYLFIQTDKTIYTPGSTVLYRVFTVDHKLLPVGQTVFITIETPDGIPVKRDSKS SQNQFGILTLSWNIPELVNMGVWKIKAYYEDSPQQVFSAEFEVKEYVLPSFEVQLEPEEK FYYIDDPDGLKVNIIARFLYGEQVDGTAFVIFGVQDGDRRISLTHSLTRVPINDGNGEAI LKRQVLLNGVQPSRADALVGKSIYVSATVILQSGSDMVEAERTGIPIVTSPYQIHFTKTP KFFKPAMPFDLMVYVTNPDGSPARHIPVVTQGSNVQSLTQDDGVAKLSINTQNKRDPLTI TVRTKKDNIPEGRQATRTMQALPYNTQGNSNNYLHLSVPRVELKPGETLNVNFHLRTDPG EQAKIRYYTYMIMNKGKLLKVGRQYREPGQDLVVLPLTITSDFIPSFRLVAYYTLINAKG QREVVADSVWVDVKDSCMGTLVVKNGGKEEKHHRPGQQITLKIEADQGARVGLVAVDKGV FVLNKKNKLTQRKIWDVVEKADIGCTPGSGRNYAGVFTDAGLTLKTSQGLETQQRADPQC PQPATRRR |
| DNA | Searched database | | GenBank |
| | accession No. | | AKE14287.1 |
| | Full-length sequence (SEQ ID NO: 13) | | AACCCCATGTATTCCATGATCACCCCCAACATCCTGCGTGTGGAGAGCGAGGAG ACTGTGGTGCTGGAGGCCCACGGAGGACAAGGGACTATTCAGGTCTCGGTCACCGTCCAC GACTTCCCGGCCAAGAAACAAGTGCTGTCAAACGAAAACACCCAGCTGAACAGCAACAAT GGCTACCTGAGCACCGTCACCATCAAGATCCCGGCCAGCAAAGAGTTAAAATCCGACAAG GGGCACAAGTTCGTGACTGTCGTAGCGACCTTCGGGAATGTCCAGGTGGAGAAGGTGGTG CTGATCAGCCTTCAGAGCGGGTACCTCTTCATCCAGACGGACAAGACCATCTACACCCCA GGCTCCACGGTCCTCTATCGGGTCTTCACTGTTGACCACAAGCTGCTGCCCGTGGGCCAG ACGGTTTTCATCACCATCGAGACCCCTGATGGCATCCCTGTCAAGCGGGACTCCAAGTCT TCTCAGAACCAGTTTGGCATTTTGACCTTGTCCTGGAACATTCCAGAGCTAGTGAACATG GGGGTGTGGAAGATCAAAGCCTACTATGAAGATTCTCCGCAGCAGGTCTTCTCCGCTGAG TTTGAGGTGAAGGAATATGTGCTTCCCAGTTTTGAGGTCCAACTAGAGCCTGAAGAGAAA TTCTACTACATTGATGACCCAGATGGCCTGAAGGTCAACATCATTGCCAGGTTCTTGTAC GGGGAACAGGTGGACGGAACAGCTTTCGTCATCTTTGGGGTCCAGGATGGCGACCGGAGA ATTTCATTGACTCACTCCCTCACCCGTGTTCCGATCAACGATGGTAATGGGGAGGCCATA CTGAAACGTCAGGTTTTGCTGAATGGAGTACAGCCCTCCCGAGCTGATGCCCTGGTGGGC AAGTCCATATATGTGTCCGCCACCGTCATCTTGCAATCAGGCAGTGACATGGTGGAGGCA GAGCGCACCGGGATCCCCATTGTGACTTCCCCCTACCAGATCCATTTCACCAAGACCCCC AAGTTCTTCAAACCTGCCATGCCCTTCGACCTCATGGTGTACGTGACAAACCCCGACGGC TCCCCCGCTCGCCACATCCCGGTGGTGACCCAGGGCAGCAATGTGCAGTCCTTGACCCAG GATGATGGCGTGGCCAAGCTAAGCATCAACACACAGAACAAACGGGATCCCCTGACCATC ACCGTACGCACAAAAAAGGACAATATCCCCGAGGGGCGACAGGCCACCAGGACCATGCAG GCCCTACCCTACAACACCCAGGGGAACTCCAATAACTACCTGCACCTCTCTGTGCCCCGC GTGGAGCTCAAGCCTGGGGAGACTCTCAACGTCAACTTCCACCTGCGCACGGACCCCGGC GAGCAAGCCAAGATCCGCTACTACACCTACATGATCATGAACAAGGGGAAGCTGTTGAAG GTGGGACGCCAGTACCGAGAGCCCGGCCAGGACCTAGTGGTGTTGCCCTTGACCATCACG TCAGACTTCATCCCTTCCTTCCGCCTGGTGGCCTATTACACACTGATTAATGCCAAAGGC CAGAGGGAGGTGGTGGCCGACTCCGTGTGGGTGGATGTCAAGGACTCGTGTATGGGCACG CTGGTGGTGAAAAATGGTGGGAAGGAAGAGAAACACCATCGACCCGGGCAACAGATAACC CTGAAGATCGAGGCTGACCAGGGGGCCCGAGTGGGGCTCGTGGCCGTGGACAAGGGCGTG TTTGTGCTGAATAAGAAGAACAAATTGACCCAGAGAAAGATCTGGGACGTGGTGGAGAAA GCGGACATTGGCTGCACCCCAGGCAGTGGAAGAAACTATGCTGGCGTCTTCACGGATGCA GGACTCACTCTCAAGACCAGCCAGGGCCTGGAGACCCAGCAGAGGGCAGATCCGCAGTGC CCGCAACCAGCCACCCGCCGACGC |

**[Table 1-8]**

| | | | MW:25kDa |
|---|---|---|---|
| SPOT No. | | | ⑨ |
| protein | Isoelectric point | Range | 4-12 |
| | | preferably | 4.5-11 |
| | | More preferably | 5-10 |
| | Molecular weight | Range | 10-50 |
| | | Preferably | 15-40 |
| | | More preferably | 20-30 |
| | Searched database | | Uniprot |
| | Biological species | | Cow (*Bos taurus*) |
| | accession No. | | Q1RMN8 |
| | Protein name | | Immunoglobulin light chain. lambda gene cluster |
| | Full-length sequence (SEQ ID NO: 16) | | QAVLTQPSSVSGSLGQRVSITCSGSSSNIGRDNVGWYQQVP GSGLRTIIYSSSSRPSGVPDRFSGSKSGNTATLTISSLQAEDEADYFCVAYDSSSSTVFG SGTTLTVLGQPKSPPSVTLFPPSTEELNGNKATLVCLISDFYPGSVTVVWKADGSTITRN VETTRASKQSNSKYAASSYLSLTSSDWKSKGSYSCEVTHEGSTVTKTVKPSECS |
| DNA | Searched database | | GenBank |
| | accession No. | | AAI14802. 1 |
| | Full-length sequence (SEQ ID NO: 15) | | CAG GCTGTGCTGACTCAGCCGTCCTCCGTGTCCGGCTCCCTGGGCCAGAGGGTCTCCATCACC TGCTCTGGAAGCAGCAGCAACATCGGTAGAGATAATGTGGGCTGGTACCAACAGGTCCCA GGATCGGGCCTCAGAACCATCATCTATAGTAGTAGCAGTCGACCCTCGGGGGTCCCCGAC CGATTCTCCGGCTCCAAGTCTGGCAACACAGCCACCCTGACCATCAGCTCCCTCCAGGCT GAGGACGAGGCGGATTATTTCTGTGTAGCTTATGACAGTAGTAGTAGTACTGTTTTCGGC AGCGGGACCACACTGACCGTCCTGGGTCAGCCCAAGTCCCCACCCTCGGTCACCCTGTTC CCGCCCTCCACGGAGGAGCTCAACGGCAACAAGGCCACCCTGGTGTGTCTCATCAGCGAC TTCTACCCGGGTAGCGTGACCGTGGTCTGGAAGGCAGACGGCAGCACCATCACCCGCAAC GTGGAGACCACCCGGGCCTCCAAACAGAGCAACAGCAAGTACGCGGCCAGCAGCTACCTG AGCCTGACGAGCAGCGACTGGAAATCGAAAGGCAGTTACAGCTGCGAGGTCACGCACGAG GGGAGCACCGTGACGAAGACAGTGAAGCCCTCAGAGTGTTCT |

**[Table 1-9]**

| | | | MW:59kDa |
|---|---|---|---|
| SPOT No. | | | ⑩ |
| protein | Isoelectric point | Range | 1-7 |
| | | Preferably | 1.5-6 |
| | | More preferably | 2-5 |
| | Molecular weight | Range | 40-160 |
| | | Preferably | 45-110 |
| | | More preferably | 50-100 |
| | Searched database | | Uniprot |
| | Biological species | | Cow (*Bos taurus*) |
| | accession No. | | F1N726 |
| | Protein name | | Glycoprotein 2 |
| | Full-length sequence (SEQ ID NO: 18) | | EQQGYRNSTNTGSYEKDLDCGAPGTPEAQLCFDP CQNYTLLNEPFRSTENTEDIQGCDSDKHGWYRFVGDGGVRMPEDCVPTFRCQTSAPLWLN GTHPGLGEGIVNRTACAHWSGNCCLWKTEVLVKACPGPYYVYRLEGTPQCSLRYCTDPAT AEDKCDRTCRPEEECRLVSGTWGCFCRQDLNVSDVHSLQPQLDCGDTEIKVSLDKCLLGS LGFGDEVHAYLRDGNWNCSSLRQSEEENWISVTNPTQAGACGNILERNQTHAIYINTLSL VNDFIIRDTILSINFQCAYPLDMKVSLQMALQPIVSSLNITVDGEGEFTVRMALFQDQDY TSPYEGTAVMLSVESMLYVGTILERGDTSRFNLVLKNCYATPTEDKTDPVKYFIIRNSCP NQRDSTISVEENGVSAESRFSVQMFKFAGNYDLVFLHCEVSLCDFIKEECQPSCSRSQLR SEGVAIDPARVLDLGPITRKGAQSLGVMSGTPNTAGFLVAWPLVLLPVLLAGLF |
| DNA | Searched database | | GenBank |
| | accession No. | | NM 001075950.2 |
| | Full-length sequence (SEQ ID NO: 17) | | GAACAGCAAGGTTACAGAAACTCCACCAACACTGGTTCCTATGAAAAGGACTTGGATTGCGG AGCCCCTGGCACCCCAGAGGCCCAGCTCTGTTTTGACCCCTGCCAGAATTACACCCTCCTGAATGAACCC TTTCGAAGCACAGAGAATACAGAAGACATCCAAGGCTGTGACAGTGACAAACACGGCTGGTACCGATTTG TGGGGGACGGAGGAGTGAGGATGCCAGAGGACTGTGTCCCCACGTTCCGATGCCAGACGTCTGCACCCCT GTGGCTGAATGGCACCCATCCTGGCCTTGGGGAGGGCATCGTCAACCGCACTGCCTGTGCCCACTGGAGT GGCAACTGCTGCCTCTGGAAGACAGAGGTGCTGGTGAAAGCCTGCCCGGGCCCATACTACGTGTACCGGT TGGAGGGCACCCCTCAATGTAGTCTGAGATACTGCACAGACCCTGCCACCGCGGAGGACAAGTGTGACAG GACCTGCCGCCCCGAGGAGGAGTGTCGCCTCGTCAGTGGCACCTGGGGCTGCTTCTGCAGACAGGACCTC AACGTCTCCGATGTGCACAGTTTGCAGCCTCAGCTGGACTGTGGGGACACAGAGATCAAAGTGTCACTGG ACAAGTGTCTGCTGGGAAGTCTGGGTTTCGGAGATGAGGTCCATGCCTACCTGCGGGACGGGAACTGGAA CTGCAGCAGCCTCAGGCAGAGTGAAGAGGAGAACTGGATATCTGTGACCAATCCCACCCAGGCTGGTGCC TGCGGAAACATTCTGGAGAGAAACCAGACCCATGCCATCTACATAAACACGCTCTCCTTAGTCAACGATT TCATCATCAGAGACACCATCCTCAGCATCAACTTCCAGTGTGCCTACCCACTGGACATGAAAGTGAGCCT TCAAATGGCCCTGCAGCCCATCGTAAGTTCCCTGAACATCACTGTGGATGGGGAGGGAGAGTTCACTGTC AGGATGGCCCTCTTTCAAGACCAGGACTACACGTCTCCTTATGAAGGGACTGCAGTCATGCTGTCTGTTG AATCCATGCTCTATGTGGGCACCATCTTGGAGAGAGGGGACACGTCCCGATTTAACCTGGTGTTGAAGAA CTGCTACGCCACGCCCACTGAAGACAAGACTGACCCTGTGAAGTACTTCATCATCAGGAACAGCTGCCCA AATCAACGTGATTCCACCATCTCCGTGGAAGAGAATGGGGTGTCAGCAGAAAGCCGGTTCTCAGTTCAGA TGTTCAAGTTTGCTGGAAATTATGACCTAGTTTTCCTGCATTGTGAGGTTTCTCTCTGTGATTTCATTAA AGAAGAGTGCCAACCGTCTTGCTCAAGAAGTCAACTCCGCAGTGAAGGGGTGGCTATTGATCCAGCCCGA GTTCTAGATCTGGGACCCATCACTCGCAAAGGTGCCCAGTCTCTTGGTGTCATGAGTGGAACACCCAACA CTGCAGGGTTCCTGGTGGCCTGGCCCCTGGTTCTCCTGCCTGTCCTCCTGGCTGGGCTGTTC |

**[Table 1-10]**

| | | | MW:47kDa |
|---|---|---|---|
| SPOT No. | | | ⑪ |
| protein | Isoelectric point | Range | 4-11 |
| | | Preferably | 4.5-10 |
| | | More preferably | 5-9 |
| | Molecular weight | Range | 30-80 |
| | | Preferably | 35-70 |
| | | More preferably | 40-60 |
| | Searched database | | Uniprot |
| | Biological species | | Cow (*Bos taurus*) |
| | accession No. | | Q95114 |
| | Protein name | | Lactadherin |
| | Full-length sequence (SEQ ID NO: 20) | | ASGDFCDSSLCLHGGTCLLNEDRTPPFYCLCPEGFTGLLCNE TEHGPCFPNPCHNDAECQVTDDSHRGDVFIQYICKCPLGYVGIHCETTCTSPLGMQTGAI ADSQISASSMHLGFMGLQRWAPELARLHQTGIVNAWTSGNYDKNPWIQVNLMRKMWVTGV VTQGASRAGSAEYLKTFKVAYSTDGRQFQFIQVAGRSGDKIFIGNVNNSGLKINLFDTPL ETQYVRLVPIICHRGCTLRFELLGCELNGCTEPLGLKDNTIPNKQITASSYYKTWGLSAF SWFPYYARLDNQGKFNAWTAQTNSASEWLQIDLGSQKRVTGIITQGARDFGHIQYVAAYR VAYGDDGVTWTEYKDPGASESKIFPGNMDNNSHKKNIFETPFQARFVRIQPVAWHNRITL RVELLGC [?] |
| DNA | Searched database | | CenBank |
| | accession No. | | CAA62997.1 mutant |
| | Full-length sequence (SEQ ID NO: 19) | | GCCTCC GGTGACTTCTGTGACTCCAGCTTGTGCCTACATGGTGGGACCTGCTTGTTGAACGAGGAC AGAACCCCCCCTTCTACTGCCTCTGCCCCGAAGGCTTCACGGGCCTCCTATGCAACGAG ACAGAGCACGGTCCCTGTTTCCCAAACCCCTGCCACAATGATGCTGAGTGCCAGGTAACT GACGACTCCCACCGAGGGGATGTCTTCATCCAGTACATCTGCAAGTGCCCTCTTGGATAC GTGGGCATCCACTGTGAGACCACCTGCACCTCACCGCTGGGCATGCAGACGGGTGCCATT GCCGACTCCCAGATCTCTGCCTCGTCCATGCATTTGGGCTTCATGGGTTTGCAGCGCTGG GCCCCGGAGCTAGCCCGCCTGCACCAGACGGGCATCGTCAACGCTTGGACATCCGGCAAC TATGACAAAAACCCCTGGATCCAGGTGAACCTGATGCGGAAGATGTGGGTGACGGGTGTG GTGACCCAGGGGGCCAGCCGCGCAGGGAGTGCTGAGTACCTGAAGACTTTTAAAGTGGCC TACAGCACCGATGGGCGCCAATTCCAGTTCATCCAGGTTGCAGGGCGGTCAGGAGATAAG ATATTTATAGGTAATGTGAACAACAGCGGCCTGAAGATTAACCTGTTTGATACCCCCTTG GAGACGCAGTATGTGAGACTGGTACCCATCATCTGCCACCGGGGCTGCACCCTCCGCTTT GAACTCCTTGGCTGTGAGTTGAATGGATGCACTGAACCCCTAGGCCTGAAGGATAATACC ATCCCCAACAAGCAGATCACAGCCTCCAGCTACTACAAAACCTGGGGCCTGAGTGCCTTT AGCTGGTTTCCCTACTACGCACGACTGGATAATCAGGGCAAGTTCAACGCCTGGACCGCC CAGACCAACAGTGCCTCTGAGTGGCTGCAGATTGACCTGGGCTCCCAGAAGCGAGTCACG GGCATCATCACCCAGGGTGCCCGAGACTTTGGCCACATTCAATATGTGGCTGCCTACAGG GTGGCCTATGGTGATGATGGTGTGACCTGGACTGAGTACAAGGACCCGGGGGCCTCAGAA AGCAAGATTTTCCCTGGTAACATGGACAATAATTCCCACAAGAAGAACATATTTGAGACG CCGTTCCAGGCTCGCTTCGTGCGGATCCAGCCCGTGGCCTGGCACAACCGTATCACCCTG CGAGTGGAGCTGCTGGGCTGT |

**[Table 1-11]**

| MW:34kDa | | | |
|---|---|---|---|
| SPOT No. | | | ⑫ |
| protein | Isoelectric point | Range | 3-8 |
| | | preferably | 3.5-7 |
| | | More preferably | 4-6 |
| | Molecular weight | Range | 20-70 |
| | | Preferably | 25-60 |
| | | More preferably | 30-50 |
| | Searched | database | Uniprot |
| | Biological | species | Cow (*Bos taurus*) |
| | | accession No. | Q3ZCH5 |
| | Protein | name | Zinc-alpha-2-glycoprotein |
| | Full-length sequence (SEQ ID NO: 22) | | EETQAGNYSLSFLYTGLSKPREGFPSFQAVAYLNDQPFFHYNS EGRRAEPLAPWSQVEGMEDWEKESALQRAREDIFMETLSDIMDYYKDREGSHTFQGAFGC ELRNNESSGAFWGYAYDGQDFIKFDKEIPAWVPLDPAAQNTKRKWEAEAVYVQRAKAYLE EECPGMLRRYLPYSRTHLDRQESPSVSVTGHAAPGHKRTLKCLAYDFYPRSIGLHWTRAG DAQEAESGGDVLPSGNGTYQSWVVVGVPPEDQAPYSCHVEHRSLTRPLTVPWDPRQQAE |
| DNA | Searched | database | GenBank |
| | | accession No. | AAI02238.1 |
| | Full-length sequence (SEQ ID NO: 21) | | GAGGAGACC CAGGCTGGGAATTACTCTCTAAGCTTCCTCTACACCGGGCTGTCCAAGCCCCGTGAGGGC TTCCCCAGCTTCCAGGCCGTTGCCTACCTCAATGACCAGCCCTTCTTCCACTACAATAGT GAAGGCAGGAGGGCCGAGCCCCTGGCGCCATGGAGTCAGGTGGAAGGAATGGAGGACTGG GAGAAGGAGAGTGCCCTTCAGAGGGCCAGAGAGGACATCTTCATGGAGACCCTGAGCGAT ATCATGGACTACTACAAGGACAGAGAAGGGTCTCACACCTTTCAAGGAGCATTCGGCTGT GAGCTCCGGAATAACGAAAGTAGTGGAGCATTCTGGGGGTACGCCTACGATGGCCAGGAC TTCATCAAGTTCGACAAAGAAATCCCAGCCTGGGTCCCTCTGGACCCAGCAGCTCAGAAC ACCAAGCGGAAGTGGGAAGCAGAGGCAGTCTACGTGCAGCGGGCCAAGGCCTACCTGGAA GAGGAGTGCCCAGGGATGCTGCGGAGGTACCTGCCCTACAGCAGGACCCACCTGGACCGA CAAGAGTCTCCTTCTGTGTCGGTCACCGGGCACGCGGCTCCAGGGCACAAGAGGACACTC AAGTGCCTGGCCTACGACTTCTACCCACGAAGCATCGGGCTGCACTGGACTCGGGCTGGT GACGCCCAGGAGGCTGAGTCAGGGGGCGACGTGCTCCCCAGCGGCAACGGCACTTACCAG TCCTGGGTGGTGGTGGGGGTCCCCCCTGAGGACCAAGCCCCGTACTCCTGCCACGTGGAG CACAGAAGCCTGACCCGGCCCCTCACCGTCCCGTGGGACCCGCGGCAACAAGCCGAG |

**[Table 1-12]**

| | | | MW:17kDa |
|---|---|---|---|
| SPOT No. | | | ⑬ |
| protein | Isoelectric point | Range | 4-10 |
| | | Preferably | 4.5-9 |
| | | More preferably | 5-8 |
| | Molecular weight | Range | 5-30 |
| | | Preferably | 10-25 |
| | | More preferably | 15-20 |
| | Searched database | | Uniprot |
| | Biological species | | Cow (*Bos taurus*) |
| | accession No. | | P79345 |
| | Protein name | | intracellular cholesterol transporter 2 |
| | Full-length sequence (SEQ ID NO: 24) | | EPVKFKDCGSWVGVIKEVNVSPCPTQPCKLHRGQSYSVNVT FTSNTQSQSSKAVVHGIVMGIPVPFPIPESDGCKSGIRCPIEKDKTYNYVNKLPVKNEYP SIKVVVEWELTDDKNQRFFCWQIPIEVEA |
| DNA | Searched database | | GenBank |
| | accession No. | | CAA59794.1 |
| | Full-length sequence (SEQ ID NO: 23) | | GAG CCGGTGAAATTCAAGGACTGCGGTTCTTGGGTCGGAGTTATAAAGGAAGTGAATGTGAGC CCATGTCCCACCCAGCCTTGCAAACTGCACAGAGGACAGTCTTACAGTGTTAATGTCACG TTCACCAGTAATACTCAGTCTCAAAGTAGCAAGGCTGTGGTACATGGCATTGTGATGGGC ATCCCAGTTCCCTTTCCCATTCCTGAGTCTGATGGTTGTAAGTCTGGAATCAGATGCCCC ATCGAAAAAGATAAGACCTATAACTACGTGAACAAACTACCCGTGAAGAATGAATACCCC TCCATAAAAGTGGTGGTGGAGTGGGAACTCACGGATGACAAAAACCAACGTTTCTTCTGC TGGCAGATCCCAATAGAGGTTGAAGCC |

**[Table 1-13]**

| | | | MW:43kDa |
|---|---|---|---|
| SPOT No. | | | ⑭ |
| protein | Isoelectric point | Range | 3-8 |
| | | Preferably | 3.5-7 |
| | | More preferably | 4-6 |
| | Molecular weight | Range | 20-70 |
| | | Preferably | 25-60 |
| | | More preferably | 30-50 |
| | Searched database | | Uniprot |
| | Biological species | | Cow (*Bos taurus*) |
| | accession No. | | Q32PJ2 |
| | Protein name | | Apolipoprotein A-IV |
| | Full-length sequence (SEQ ID NO: 26) | | EVNADQVATVIWDYFSQLGNNAKKAVEHIQKSELTQQLNT LFQDKLGEVSTYTDDLQKKLVPFATELHERLTKDSEKLKEEIRKELEDLRARLLPHATEV SQKIGDNVRELQQRLGPYAEELRTQVDTQAQQLRRQLTPYVERMEKVMRQNLDQLQASLA PYAEELQATVNQRVEELKGRLTPYADQLQTKIEENVEELRRSLAPYAQDVQGKLNHQLEG LAFQMKKHAEELKAKISAKAEELRQGLVPLVNSVHGSQLGNAEDLQKSLAELSSRLDQQV EDFRRTVGPYGETFNKAMVQQLDTLRQKLGPLAGDVEDHLSFLEKDLRDKVSSFFNTLKE KESQAPALPAQEEMPVPLGG |
| DNA | Searched database | | GenBank |
| | accession No. | | AAI08097.1 |
| | Full-length sequence (SEQ ID NO: 25) | | GAGGTCAATGCCGACCAGGTGGCCACCGTGATTTGGGACTACTTCAGCCAGCTGGGCAAC AATGCCAAGAAGGCGGTGGAACATATCCAGAAGTCTGAGCTCACCCAGCAGCTCAACACC CTCTTCCAAGATAAACTTGGGGAAGTGAGCACCTACACGGACGACCTGCAGAAGAAGCTG GTGCCCTTTGCCACGGAACTGCATGAACGGCTGACCAAGGACTCCGAGAAGCTGAAGGAG GAGATTCGGAAGGAGCTGGAGGACCTGCGGGCCCGGCTGCTGCCCCACGCCACGGAGGTG AGCCAGAAGATCGGGGACAACGTGCGCGAGCTACAGCAGCGCCTGGGACCATACGCAGAG GAGCTGCGCACCCAGGTCGACACCCAGGCGCAGCAGCTGCGGCGCCAGCTGACGCCCTAT GTGGAGCGCATGGAGAAGGTGATGCGGCAGAACCTGGACCAGCTGCAGGCCTCGCTGGCG CCCTACGCCGAGGAGCTCCAGGCCACGGTCAACCAGCGCGTGGAAGAGCTCAAGGGGCGC CTGACGCCCTACGCGGACCAGCTCCAGACCAAGATCGAGGAGAACGTGGAGGAACTGCGC CGCAGCCTGGCCCCTTATGCTCAGGACGTCCAGGGGAAGCTCAACCACCAGCTGGAGGGC CTGGCCTTTCAGATGAAGAAGCATGCCGAGGAGCTAAAGGCCAAGATCTCAGCCAAAGCC GAGGAGCTGCGGCAGGGGCTGGTGCCCCTGGTCAACAGCGTGCATGGAAGTCAGCTGGGC AATGCCGAGGACTTGCAGAAGTCACTGGCCGAGCTGAGCAGCCGCCTGGACCAGCAGGTG GAGGACTTCCGCCGCACCGTGGGGCCCTACGGCGAGACCTTCAACAAAGCCATGGTGCAG CAGCTGGACACGCTCAGGCAGAAGCTGGGCCCCCTGGCGGGGGACGTGGAAGATCACCTG AGTTTCCTGGAGAAGGATCTGAGGGACAAGGTCAGCAGCTTCTTCAACACCCTCAAGGAG AAAGAGAGCCAGGCCCCCGCCCTCCCCGCGCAGGAGGAGATGCCCGTCCCATTGGGGGGC |

**[Table 1-14]**

| | | | | | | | | | | | | | | |
|---|---|---|---|---|---|---|---|---|---|---|---|---|---|---|
| | SPOT | ① | ②③ | ④ | ⑤ | ⑥ | ⑦ | ⑧ | ⑨ | ⑩ | ⑪ | ⑫ | ⑬ | ⑭ |
| | pe | NCBI | Uniprot | Uniprot | NCBI | Uniprot | Uniprot | Uniprot | Uniprot | Uniprot | Uniprot | Uniprot | Uniprot | Uniprot |
| | accession | AAN60017.1 | G3X0N3 | G3MXZ0 | S22080 | FINING | P81265 | A0A0F6QNP7 | Q1RMN8 | F1N728 | Q96114 | Q3ZCH5 | P79346 | Q32PJ2 |
| Grouping based on functions | protein name | immunoglobulin M heavy chain secretory form | Beta-1metal-binding globulin | Lactoperoxidase | Ig heavy chain precursor (B/MT.4A.17.H5.A5) | Perilipin | Polymeric immunoglobulin receptor | C3-beta-c | Immunoglobulin light chain, lambda gene glister | Glycoprotein 2 | Lactadharin | Zinc-alpha-2-glycoprotein | intracellular cholesterol transporter 2 | Apolipoprotein A-IV |
| Group 1 | Signal transduction | • | • | • | • | | | | | | | | | |
| Group 2 | Immuno system | • | | | • | | • | • | • | | | | | |
| Group 3 | glycoprotein | | | | | | | | | • | • | • | | |
| Group 4 | cholesterol binding | | | | | | | | | | | | • | • |

Spots 2 and 3 are derived from the same protein. The total of 14 different proteins (1) to (13) have been identified as new milk-derived antigens:
(1) immunoglobulin M heavy chain secretory form (spot 1);
(2) beta-1 metal-binding globulin (spots 2 and 3);
(3) lactoperoxidase (spot 4);
(4) Ig heavy chain precursor (B/MT.4A.17. H5. A5) (spot 5);
(5) perilipin (spot 6);
(6) polymeric immunoglobulin receptor (spot 7);
(7) C3-beta-c (spot 8);
(8) immunoglobulin light chain, lambda gene cluster (spot 9);
(9) glycoprotein 2 (spot 10);
(10) lactadherin (spot 11);
(11) zinc-alpha-2-glycoprotein (spot 12);
(12) intracellular cholesterol transporter 2 (spot 13); and
(13) apolipoprotein A-IV (spot 14).

As shown in Table 2, proteins (1) to (13) are classified under 4 groups, each sharing a function:
group 1(signal transduction function): proteins (1), (2), (3), (4), and (5);
group 2(immune system function): proteins (1), (4), (6), (7), and (8);
group 3 (glycoprotein function): proteins (9), (10), and (11); and
group 4 (cholesterol binding function): proteins (12) and (13).

The antigen of spot 1 in the invention is not limited and may be any of the following (1-a) to (1-f).
(1-a) A protein comprising the amino acid sequence set forth in SEQ ID NO: 2;
(1-b) a protein comprising an amino acid sequence with deletion, substitution, insertion or addition of one or several amino acids in SEQ ID NO: 2;
(1-c) a protein comprising an amino acid sequence having 70% or higher identity to the amino acid sequence set forth in SEQ ID NO: 2;
(1-d) a protein comprising an amino acid sequence encoded by a nucleotide sequence with deletion, substitution, insertion or addition of one or several nucleotides in SEQ ID NO: 1;
(1-e) a protein comprising an amino acid sequence encoded by a nucleotide sequence having 70% or higher identity to the nucleotide sequence set forth in SEQ ID NO: 1; and
(1-f) a protein comprising an amino acid sequence encoded by a nucleic acid that hybridizes under stringent conditions with a nucleic acid consisting of a nucleotide sequence complementary to the nucleotide sequence set forth in SEQ ID NO: 1.

The antigen of spot 2 or 3 in the invention is not limited and may be any of the following (2-a) to (2-f).
(2-a) A protein comprising the amino acid sequence set forth in SEQ ID NO: 4;
(2-b) a protein comprising an amino acid sequence with deletion, substitution, insertion or addition of one or several amino acids in SEQ ID NO: 4;
(2-c) a protein comprising an amino acid sequence having 70% or higher identity to the amino acid sequence set forth in SEQ ID NO: 4;
(2-d) a protein comprising an amino acid sequence encoded by a nucleotide sequence with deletion, substitution, insertion or addition of one or several nucleotides in SEQ ID NO: 3;
(2-e) a protein comprising an amino acid sequence encoded by a nucleotide sequence having 70% or higher identity to the nucleotide sequence set forth in SEQ ID NO: 3; and
(2-f) a protein comprising an amino acid sequence encoded by a nucleic acid that hybridizes under stringent conditions with a nucleic acid consisting of a nucleotide sequence complementary to the nucleotide sequence set forth in SEQ ID NO: 3.

The antigen of spot 4 in the invention is not limited and may be any of the following (3-a) to (3-f).
(3-a) A protein comprising the amino acid sequence set forth in SEQ ID NO: 6;
(3-b) a protein comprising an amino acid sequence with deletion, substitution, insertion or addition or one or several amino acids in SEQ ID NO: 6;
(3-c) a protein comprising an amino acid sequence having 70% or higher identity to the amino acid sequence set forth in SEQ ID NO: 6;
(3-d) a protein comprising an amino acid sequence encoded by a nucleotide sequence with deletion, substitution, insertion or addition of one or several nucleotides in SEQ ID NO: 5;
(3-e) a protein comprising an amino acid sequence encoded by a nucleotide sequence having 70% or higher identity to the nucleotide sequence set forth in SEQ ID NO: 5; and
(3-f) a protein comprising an amino acid sequence encoded by a nucleic acid that hybridizes under stringent conditions with a nucleic acid consisting of a nucleotide sequence complementary to the nucleotide sequence set forth in SEQ ID NO: 5.

The antigen of spot 5 in the invention is not limited and may be any of the following (4-a) to (4-f).
(4-a) A protein comprising the amino acid sequence set forth in SEQ ID NO: 8;
(4-b) a protein comprising an amino acid sequence with deletion, substitution, insertion or addition of one or several amino acids in SEQ ID NO: 8;
(4-c) a protein comprising an amino acid sequence having 70% or higher identity to the amino acid sequence set forth in SEQ ID NO: 8;
(4-d) a protein comprising an amino acid sequence encoded by a nucleotide sequence with deletion, substitution, insertion or addition of one or several nucleotides in SEQ ID NO: 7;
(4-e) a protein comprising an amino acid sequence encoded by a nucleotide sequence having 70% or higher identity to the nucleotide sequence set forth in SEQ ID NO: 7; and
(4-f) a protein comprising an amino acid sequence encoded by a nucleic acid that hybridizes under stringent conditions with a nucleic acid consisting of a nucleotide sequence complementary to the nucleotide sequence set forth in SEQ ID NO: 7.

The antigen of spot 6 in the invention is not limited and may be any of the following (5-a) to (5-f).
(5-a) A protein comprising the amino acid sequence set forth in SEQ ID NO: 10;
(5-b) a protein comprising an amino acid sequence with deletion, substitution, insertion or addition of one or several amino acids in SEQ ID NO: 10;
(5-c) a protein comprising an amino acid sequence having 70% or higher identity to the amino acid sequence set forth in SEQ ID NO: 10;
(5-d) a protein comprising an amino acid sequence encoded by the nucleotide sequence with deletion, substitution, insertion or addition of one or several nucleotides in SEQ ID NO: 9;
(5-e) a protein comprising an amino acid sequence encoded by a nucleotide sequence having 70% or higher identity to the nucleotide sequence set forth in SEQ ID NO: 9; and
(5-f) a protein comprising an amino acid sequence encoded by a nucleic acid that hybridizes under stringent conditions with a nucleic acid consisting of a nucleotide sequence complementary to the nucleotide sequence set forth in SEQ ID NO: 9.

The antigen of spot 7 in the invention is not limited and may be any of the following (6-a) to (6-f).
(6-a) A protein comprising the amino acid sequence set forth in SEQ ID NO: 12;
(6-b) a protein comprising an amino acid sequence with deletion, substitution, insertion or addition of one or several amino acids in SEQ ID NO: 12;
(6-c) a protein comprising an amino acid sequence having 70% or higher identity to the amino acid sequence set forth in SEQ ID NO: 12;
(6-d) a protein comprising an amino acid sequence encoded by a nucleotide sequence with deletion, substitution, insertion or addition of one or several nucleotides in SEQ ID NO: 11;
(6-e) a protein comprising an amino acid sequence encoded by a nucleotide sequence having 70% or higher identity to the nucleotide sequence set forth in SEQ ID NO: 11; and
(6-f) a protein comprising an amino acid sequence encoded by a nucleic acid that hybridizes under stringent conditions with a nucleic acid consisting of a nucleotide sequence complementary to the nucleotide sequence set forth in SEQ ID NO: 11.

The antigen of spot 8 in the invention is not limited and may be any of the following (7-a) to (7-f).
(7-a) A protein comprising the amino acid sequence set forth in SEQ ID NO: 14;
(7-b) a protein comprising the amino acid sequence with deletion, substitution, insertion or addition of one or several amino acids in SEQ ID NO: 14;
(7-c) a protein comprising an amino acid sequence having 70% or higher identity to the amino acid sequence set forth in SEQ ID NO: 14;
(7-d) a protein comprising an amino acid sequence encoded by the nucleotide sequence with deletion, substitution, insertion or addition of one or several nucleotides in SEQ ID NO: 13;
(7-e) a protein comprising an amino acid sequence encoded by a nucleotide sequence having 70% or higher identity to the nucleotide sequence set forth in SEQ ID NO: 13; and
(7-f) a protein comprising an amino acid sequence encoded by a nucleic acid that hybridizes under stringent conditions with a nucleic acid consisting of a nucleotide sequence complementary to the nucleotide sequence set forth in SEQ ID NO: 13.

The antigen of spot 9 in the invention is not limited and may be any of the following (8-a) to (8-f).
(8-a) A protein comprising the amino acid sequence set forth in SEQ ID NO: 16;
(8-b) a protein comprising an amino acid sequence with deletion, substitution, insertion or addition of one or several amino acids in SEQ ID NO: 16;
(8-c) a protein comprising an amino acid sequence having 70% or higher identity to the amino acid sequence set forth in SEQ ID NO: 16;
(8-d) a protein comprising an amino acid sequence encoded by a nucleotide sequence with deletion, substitution, insertion or addition of one or several nucleotides in SEQ ID NO: 15;
(8-e) a protein comprising an amino acid sequence encoded by a nucleotide sequence having 70% or higher identity to the nucleotide sequence set forth in SEQ ID NO: 15; and
(8-f) a protein comprising an amino acid sequence encoded by a nucleic acid that hybridizes under stringent conditions with a nucleic acid consisting of a nucleotide sequence complementary to the nucleotide sequence set forth in SEQ ID NO: 15.

The antigen of spot 10 in the invention is not limited and may be any of the following (9-a) to (9-f).
(9-a) A protein comprising the amino acid sequence set forth in SEQ ID NO: 18;
(9-b) a protein comprising an amino acid sequence with deletion, substitution, insertion or addition of one or several amino acids in SEQ ID NO: 18;
(9-c) a protein comprising an amino acid sequence having 70% or higher identity to the amino acid sequence set forth in SEQ ID NO: 18;
(9-d) a protein comprising an amino acid sequence encoded by the nucleotide sequence with deletion, substitution, insertion or addition of one or several nucleotides in SEQ ID NO: 17;
(9-e) a protein comprising an amino acid sequence encoded by a nucleotide sequence having 70% or higher identity to the nucleotide sequence set forth in SEQ ID NO: 17; and
(9-f) a protein comprising an amino acid sequence encoded by a nucleic acid that hybridizes under stringent conditions with a nucleic acid consisting of a nucleotide sequence complementary to the nucleotide sequence set forth in SEQ ID NO: 17.

The antigen of spot 11 in the invention is not limited and may be any of the following (10-a) to (10-f).
(10-a) A protein comprising the amino acid sequence set forth in SEQ ID NO: 20;
(10-b) a protein comprising an amino acid sequence with deletion, substitution, insertion or addition of one or several amino acids in SEQ ID NO: 20;
(10-c) a protein comprising an amino acid sequence having 70% or higher identity to the amino acid sequence set forth in SEQ ID NO: 20;
(10-d) a protein comprising an amino acid sequence encoded by the nucleotide sequence with deletion, substitution, insertion or addition of one or several nucleotides in SEQ ID NO: 19;
(10-e) a protein comprising an amino acid sequence encoded by a nucleotide sequence having 70% or higher identity to the nucleotide sequence set forth in SEQ ID NO: 19; and
(10-f) a protein comprising an amino acid sequence encoded by a nucleic acid that hybridizes under stringent conditions with a nucleic acid consisting of a nucleotide sequence complementary to the nucleotide sequence set forth in SEQ ID NO: 19.

The antigen of spot 12 in the invention is not limited and may be any of the following (11-a) to (11-f).
(11-a) A protein comprising the amino acid sequence set forth in SEQ ID NO: 22;
(11-b) a protein comprising an amino acid sequence with deletion, substitution, insertion or addition of one or several amino acids in SEQ ID NO: 22;
(11-c) a protein comprising an amino acid sequence having 70% or higher identity to the amino acid sequence set forth in SEQ ID NO: 22;
(11-d) a protein comprising an amino acid sequence encoded by the nucleotide sequence with deletion, substitution, insertion or addition of one or several nucleotides in SEQ ID NO: 21;
(11-e) a protein comprising an amino acid sequence encoded by a nucleotide sequence having 70% or higher identity to the nucleotide sequence set forth in SEQ ID NO: 21; and
(11-f) a protein comprising an amino acid sequence encoded by a nucleic acid that hybridizes under stringent conditions with a nucleic acid consisting of a nucleotide sequence complementary to the nucleotide sequence set forth in SEQ ID NO: 21.

The antigen of spot 13 in the invention is not limited and may be any of the following (12-a) to (12-f).
(12-a) A protein comprising the amino acid sequence set forth in SEQ ID NO: 24;
(12-b) a protein comprising an amino acid sequence with deletion, substitution, insertion or addition of one or several amino acids in SEQ ID NO: 24;
(12-c) a protein comprising an amino acid sequence having 70% or higher identity to the amino acid sequence set forth in SEQ ID NO: 24;
(12-d) a protein comprising an amino acid sequence encoded by the nucleotide sequence with deletion, substitution, insertion or addition of one or several nucleotides in SEQ ID NO: 23;
(12-e) a protein comprising an amino acid sequence encoded by a nucleotide sequence having 70% or higher identity to the nucleotide sequence set forth in SEQ ID NO: 23; and
(12-f) a protein comprising an amino acid sequence encoded by a nucleic acid that hybridizes under stringent conditions with a nucleic acid consisting of a nucleotide sequence complementary to the nucleotide sequence set forth in SEQ ID NO: 23.

The antigen of spot 14 in the invention is not limited and may be any of the following (13-a) to (13-f).
(13-a) A protein comprising the amino acid sequence set forth in SEQ ID NO: 26;
(13-b) a protein comprising an amino acid sequence with deletion, substitution, insertion or addition of one or several amino acids in SEQ ID NO: 26;
(13-c) a protein comprising an amino acid sequence having 70% or higher identity to the amino acid sequence set forth in SEQ ID NO: 26;
(13-d) a protein comprising an amino acid sequence encoded by the nucleotide sequence with deletion, substitution, insertion or addition of one or several nucleotides in SEQ ID NO: 25;
(13-e) a protein comprising an amino acid sequence encoded by a nucleotide sequence having 70% or higher identity to the nucleotide sequence set forth in SEQ ID NO: 25; and
(13-f) a protein comprising an amino acid sequence encoded by a nucleic acid that hybridizes under stringent conditions with a nucleic acid consisting of a nucleotide sequence complementary to the nucleotide sequence set forth in SEQ ID NO: 25.

In an embodiment, the above antigen proteins (1) to (13) and the below-described polypeptides (E1) to (E25) include the corresponding proteins or polypeptides, the amino acid residue(s) of which is modified by, for instance, phosphorylation, glycosylation, amino acylation, ring-opening, or deamidation.

Preferably, the above antigen proteins (1) to (13) and the below-described polypeptides (E1) to (E25) are allergy antigens.

As used herein, an amino acid sequence "with deletion, substitution, insertion or addition of one or several amino acids " means that the amino acid sequence of interest refers to an amino acid sequence in which one to several amino acids are deleted and/or substituted with other amino acid(s), other amino acid(s) are inserted, and/or other amino acid(s) are added. The number of "several amino acids" is not limited and means 200 or less, 100 or less, 50 or less, 30 or less, 20 or less, 15 or less, 12 or less, 10 or less, 8 or less, 6 or less, 4 or less, or 3 or less amino acids. Alternatively, the several amino acids mean 30% and preferably 25%, 20%, 15%, 10%, 5%, 3%, 2%, or 1% amino acids based on the full-length amino acid sequence.

The above substitution is preferably conservative substitution. In the conservative substitution, a certain amino acid residue(s) is replaced by a residue(s) having similar physicochemical characteristics. Any substitution is allowed unless the features of the original sequence structure are not substantially changed. For example, any substitution is allowed unless the substituted amino acid(s) destroys the helix present in the original sequence or destroys another type of the secondary structure characteristic of the original sequence. Hereinbelow, examples of a residue where the amino acid residue can be substituted by conservative substitution are illustrated while classified. However, the replaceable amino acid residue is not limited to those listed below.
Group A: leucine, isoleucine, valine, alanine, methionine, glycine, cysteine, or proline.
Group B: aspartic acid or glutamic acid.
Group C: asparagine or glutamine.
Group D: lysine or arginine.
Group E: serine or threonine.
Group F: phenylalanine, tyrosine, tryptophan, or histidine.

In the case of non-conservative substitution, a member of any one of the above groups may be replaced by a member of another group. For example, to exclude unwanted glycosylation, an amino acid(s) of the above group B, D, or E may be substituted by an amino acid(s) of any of the other groups. In addition, to prevent folding into a protein conformation, a cysteine may be deleted or substituted by another amino acid. In addition, to keep a hydrophilicity/hydrophobicity balance or to increase hydrophilicity to make synthesis easy, an amino acid(s) may be substituted in consideration of amino acid hydropathy index (J. Kyte and R. Doolittle, J. Mol. Biol., Vol. 157, p.105-132, 1982) as an indicator for amino acid hydrophilicity/hydrophobicity.

In another embodiment, an amino acid of interest may be substituted by an amino acid with less steric hindrance than the original amino acid. For example, an amino acid of group F may be substituted by an amino acid of any of group A, B, C, D, or E; or an amino acid with a charge may be substituted by an amino acid without a charge. For instance, a group B amino acid may be substituted by a group C amino acid. This may improve binding ability to IgE antibodies.

As used herein, the identity (%) between two amino acid sequences may be determined by a visual test or mathematical calculation. In addition, a computer program may be used to determine the identity (%). Examples of such a computer program include BLAST or ClustalW. Various conditions (parameters) of identity search by, in particular, BLAST program have been described by Altschul and colleagues (Nucl. Acids. Res., 25, p.3389-3402, 1997). The information (BLAST manual, Altschul, et al., NCB/NLM/NIH Bethesda, MD 20894; Altschul *et al.*) is publicly available from the website of NCBI or DNA Data Bank of Japan (DDBJ). In addition, a program such as genetic information processing software GENETYX Ver.7 (GENETYX), DNASIS Pro (Hitachi Software), or Vector NTI (Infomax) may be used for determination.

As used herein, a nucleotide sequence with "deletion, substitution, insertion or addition of one or several nucleotides" means that the nucleotide sequence of interest refers to a nucleotide sequence in which one or several nucleotides are deleted or substituted by other nucleotide(s); or other nucleotide(s) is inserted and/or added to give a nucleotide sequence. The number of "several nucleotides" is not limited and means 600 or less, 300 or less, 150 or less, 100 or less, 50 or less, 30 or less, 20 or less, 15 or less, 12 or less, 10 or less, 8 or less, 6 or less, 4 or less, or 3 or less nucleotides. Alternatively, the several nucleotides mean 30% or preferably 25%, 20%, 15%, 10%, 5%, 3%, 2%, or 1% of nucleotides based on the full-length nucleotide sequence. It is preferable that the above nucleotide deletion, substitution, insertion, or addition causes no frame shift in the amino acid-encoding sequence.

As used herein, the identity (%) between two nucleotide sequences may be determined by a visual test or mathematical calculation. In addition, a computer program may be used to determine the identity (%). Examples of such a sequence comparison computer program include BLASTN program, which is available from the website (https://blast.ncbi.nlm.nih.gov/Blast.cgi) of United States National Library of Medicine (Altschul et al. (1990) J. Mol. Biol. 215: 403-10): Ver. 2.2.7 or WU-BLAST 2.0 algorithm. Standard default parameters of WU-BLAST 2.0 may be set using those described in the following internet site: http://blast.wustl.edu.

As used herein, the wording "under stringent conditions" means to hybridize under moderately or highly stringent conditions. Specifically, the moderately stringent conditions may be easily determined, based on, for example, the DNA length, by those having ordinary skill. The essential conditions are disclosed by Sambrook et al., Molecular Cloning: A Laboratory Manual, 3rd ed., Chapters 6 to 7, Cold Spring Harbor Laboratory Press, 2001. The moderately stringent hybridization conditions are preferably conditions of 1 × SSC to 6 × SSC at 42°C to 55°C, more preferably conditions of 1 × SSC to 3 × SSC at 45°C to 50°C, and most preferably conditions of 2 × SSC at 50°C. When the hybridization solution contains, for instance, about 50% formamide, the temperature 5 to 15°C lower than the above temperature should be adopted. Examples of the washing conditions include 0.5 × SSC to 6 × SSC at 40°C to 60°C. During hybridization or washing, 0.05% to 0.2% SDS is routinely added, and about 0.1% SDS is preferably added. The highly stringent conditions may also be easily determined, based on, for example, the DNA length, by those skilled in the art. In general, the highly stringent conditions include hybridization and/or washing at a temperature higher than and/or a salt concentration lower than those under the moderately stringent conditions. The hybridization conditions are, for example, conditions of 0.1 × SSC to 2 × SSC at 55°C to 65°C, more preferably conditions of 0.1 × SSC to 1 × SSC at 60°C to 65°C, and most preferably conditions of 0.2 × SSC at 63°C. Washing conditions are, for example, 0.2 × SSC to 2 × SSC at 50°C to 68°C and more preferably 0.2 × SSC at 60 to 65°C.

The variants corresponding to items (b) to (f) of the above (1) to (13) preferably include, but are not limited to, the below-described epitope (variant) amino acid sequences. The epitope amino acid sequences included are not limited to one sequence, and preferably include all of the sequences of epitopes derived from each of the respective proteins. For example, (E1) and (E2) are epitopes derived from protein (1). (1-b) to (1-f) preferably include at least one of an (E1) or (E2) epitope amino acid sequence (including a variant).

Each antigen may be separated/purified from milk by combining protein purification procedures well-known to those skilled in the art. Alternatively, each antigen may be separated/purified by expressing the antigen as a recombinant protein by a gene recombinant technology well-known to those skilled in the art and then performing a protein purification procedure well-known to those skilled in the art.

Examples of the protein purification procedure include: solubility-based methods (e.g., salting out, solvent precipitation); methods using differences in molecular weight (e.g., dialysis, ultrafiltration, gel filtration, SDS-PAGE); charge-based methods (e.g., ion exchange chromatography, hydroxyapatite chromatography); specific affinity-based methods (e.g., affinity chromatography); methods using differences in hydrophobicity (e.g., reversed-phase high-performance liquid chromatography); or methods using differences in isoelectric point (e.g., isoelectric focusing).

Each protein may be prepared using a gene recombinant technology by constructing an expression vector comprising the antigen-coding nucleic acid, transfecting or transforming the expression vector in a suitable host cell, culturing the host cell under conditions fit for expressing a recombinant protein, and collecting the recombinant protein expressed in the host cell.

The "vector" is a nucleic acid capable of being used for introducing, into a host cell, nucleic acid linked to the vector. The "expression vector" is a vector capable of inducing expression of a protein encoded by the nucleic acid introduced in the vector. Examples of the vector include a plasmid vector or a viral vector. Those skilled in the art can select, depending on the type of host cell used, an expression vector suitable for expressing a recombinant protein.

The "host cell" is a cell transfected or transformed with a vector. The host cell can be selected, if appropriate, depending on a vector to be used, by those skilled in the art. The host cell may be derived from a prokaryote such as *E. coli.* When a prokaryotic cell such as *E. coli* is used as a host, an antigen of the invention may include an N-terminal methionine residue so as to easily express a recombinant protein in the prokaryotic cell. This N-terminal methionine may be cleaved from the recombinant protein after expression. Alternatively, the host cell may be a eukaryote-derived cell such as a unicellular eukaryote (e.g., yeast), a plant cell, or an animal cell (e.g., a human cell, a monkey cell, a hamster cell, a rat cell, a mouse cell, an insect cell), or a *Bombyx mori.*

Gene transfer or genetic transformation of an expression vector into a host cell may be carried out, if appropriate, using a known technique by those skilled in the art. In addition, those skilled in the art can select, if appropriate, conditions suitable for expressing a recombinant protein in accordance with the type of host cell and then culturing the host cell to express the recombinant protein. Then, the host cell expressing the recombinant protein may be homogenized. The resulting homogenate may be subjected to a suitable combination of the above-mentioned protein purification procedures. Finally, the antigen as expressed as the recombinant protein can be separated/purified. The above expression vector or synthesized double-stranded DNA, or mRNA transcribed from the DNA may be introduced into a cell-free protein synthesis system for expression. The expressed protein may be separated/purified to prepare the antigen.

Each antigen in the invention can specifically bind to an IgE antibody from an allergic patient.

### Diagnosis Kit/Diagnosis Method (1)

The invention provides a method for providing an indicator for diagnosing an allergy in a subject, comprising the steps of:
(i) contacting a sample obtained from the subject with an antigen, wherein the sample is a solution comprising an IgE antibody;
(ii) detecting binding between the IgE antibody present in the sample obtained from the subj ect and the antigen; and
(iii) when the binding between the IgE antibody in the subject and the antigen is detected, an indicator of the fact that the subject is allergic is provided,
   wherein the antigen is at least one protein specified as any of the antigens (1) to (13).

The "allergy" is not limited, and is, in one embodiment, an allergy to milk and preferably an allergy to cow's milk.

As used herein, the term "diagnosis" generally includes not only a (definitive) diagnosis by a physician, but also mere "detection," including the possibility. As used herein, the term "diagnosis" or "detection" refers to, in one embodiment, *in vivo, in vitro,* or *ex vivo* "diagnosis" or "detection. Preferred is *in vitro* "diagnosis" or "detection".

A sample obtained from a subject is a solution comprising an IgE antibody collected from the subject. Examples of such a solution include blood, saliva, sputum, snot, urine, sweat, or tears. Each sample obtained from the subject may be pretreated to increase the concentration of IgE antibody in the sample prior to contact with an antigen. The sample pretreatment may include, for example, obtaining serum or plasma from blood. In addition, a Fab portion, which is the portion of binding to an antigen, may be purified. In a particularly preferred embodiment, step (i) is carried out by bringing the antigen into contact with an IgE antibody in serum obtained from the subject.

The IgE antibody may be an IgE antibody itself or a mast cell to which the IgE antibody is bound.

The contact or binding between the sample obtained from the subject and the antigen may be detected by a known procedure. Examples of such a procedure include ELISA (Enzyme-Linked Immunosorbent Assay), sandwich immunoassay, immunoblotting, immunoprecipitation, or immunochromatography-mediated detection. In any of them, the technique includes: bringing an IgE antibody from a subject into contact with an antigen for binding; applying an enzyme-labeled secondary antibody to the IgE antibody specifically bound to the antigen; adding an enzyme substrate (usually a chromogenic or luminescent reagent) to detecting an enzymatic reaction product; and then detecting binding of the antigen to the IgE antibody from the subject.
Alternatively, a fluorescently labeled secondary antibody may be used for detection in the procedure. Otherwise, detection by surface plasmon resonance (SPR) or other measuring procedures that can be used to evaluate the binding of an antigen to an IgE antibody may be used. Multiple antigen-specific IgE antibodies may be mixed.

Each antigen may be in the form of an isolated antigen immobilized on or inside a carrier. In this case, ELISA, sandwich immunoassay, immunochromatography, surface plasmon resonance, etc. can be used in steps (i) and (ii), and in step (i), the sample obtained from the subject is brought into contact with a surface on which the antigen is immobilized. The isolated antigen may be obtained from an object of interest (e.g., a raw material, a processed product) by isolation/purification using a combination of protein purification procedures well-known to those skilled in the art, or by preparation using gene recombinant technology. The antibody may also be attached onto a surface.

The antigen is not necessarily immobilized on a carrier. In this case, flow cytometry, etc., can be used in steps (i) and (ii), and the presence of the antigen bound to an antibody can be confirmed by laser light. Examples include the basophil activation test (BAT). Other examples include the histamine release test (HRT), in which an antigen is further brought into contact with blood cells in a sample to determine whether or not histamine is released.

Each antigen may also be separated by 2D electrophoresis, transferred, and detected by immunoblotting. Here, 2D electrophoresis is a technique for separating protein samples by performing isoelectric focusing in the first dimension and SDS-PAGE in the second dimension. In this case, the conditions of 2D electrophoresis are not particularly limited as long as each antigen in the invention can be separated. For example, the conditions for 2D electrophoresis described in the above section "To Identify Antigen" can be used. Alternatively, the conditions for electrophoresis may be determined by referring to the descriptions in the above-mentioned PTLs 1 to 4. The 2D electrophoresis can be performed using conditions satisfying the following at least one condition selected from the group consisting of:
(A) when the length of a gel as a first-dimensional isoelectric focusing gel is in the range of 5 to 10 cm, the pH range of the gel is from 3 to 10, and the pH gradient of the gel in the electrophoresis direction is "a" for the gel length up to pH 5, "b" for the gel length between pH 5 and 7, and "c" for the gel length above pH 7, the relationships "a<b" and "b>c" are satisfied;
(B) in the case of (A) where the total length of the gel is set to 1, "a" is in the range 0.15-0.3, "b" is in the range 0.4-0.7, and "c" is in the range 0.15-0.3;
(C) in the first dimensional isoelectric focusing, a constant voltage step is performed by applying a constant voltage with a value in the range of 100 V to 600 V to each gel comprising the sample, and a voltage increase step is started to increase the voltage from the above constant voltage after an electrophoresis change per 30 min of running becomes within the range of 5 µA;
(D) in the case of (C), the final voltage of the voltage increase step is in the range of 3000 V to 6000 V;
(E) the longitudinal gel length of the first-dimensional isoelectric focusing gel is from 5 to 10 cm, and the gel concentration at the base end of the second-dimensional electrophoresis gel in the electrophoresis direction is from 3 to 6%; and
(F) in the case of (E), the gel concentration at the tip of the second dimensional electrophoresis gel in the electrophoresis direction is set to be higher than that at the base end in the electrophoresis direction.

The above antigens (1) to (13) are an antigen that can specifically bind to an IgE antibody of an allergic patient (e.g., a patient with milk allergy). This makes it possible to provide an indicator indicating that the subject has the allergy when the binding of the antigen to the IgE antibody from the subject is detected.

The invention also provides a kit for diagnosing an allergy comprising at least one of the above antigens (1) to (13). The diagnosis kit of the invention may be used in a method for providing an indicator for diagnosing an allergy as described above, or in a diagnosis method described below. The diagnosis kit of the invention may comprise at least one of the above antigens (1) to (13) as well as an enzyme-labeled anti-IgE antibody and a chromogenic or luminescent substrate that serves as a substrate for the enzyme. It is also possible to use a fluorescently labeled anti-IgE antibody. In the diagnosis kit of the invention, the antigen may be provided in a state where the antigen is immobilized on or inside a carrier. The diagnosis kit of the invention may also be provided with instructions on the procedure for diagnosis and a package comprising the instructions.

In another embodiment, the above diagnosis kit contains a companion diagnostic agent for allergy. The companion diagnostic agent may be used to identify a patient who is expected to benefit from a drug or who is at a risk of serious adverse effects from a drug, or to study the response of a drug to optimize treatment with the drug. Here, the treatment optimization includes, for example, determining a dosage regimen and a dose, deciding discontinuation of administration, and identifying which allergen component is used to produce immunological tolerance.

The invention also provides a composition for diagnosing an allergy comprising at least one of the above antigens (1) to (13). The diagnostic composition of the invention may be used in the following diagnosis method. The diagnostic composition of the invention may optionally contain a pharmaceutically acceptable carrier and/or an additive(s) commonly used with an antigen of the invention.

An embodiment of the invention provides a method for diagnosing an allergy in a subject, comprising the steps of:
(i) contacting a sample obtained from the subject with an antigen;
(ii) detecting binding of the antigen to an IgE antibody in the sample obtained from the subj ect; and
(iii) determining that the subject has the allergy when the binding of the antigen to the IgE antibody from the subject is detected,
   wherein the antigen is at least one protein specified as any of the antigens (1) to (13). Here, steps (i) and (ii) are performed as described for each step of the method of providing an indicator for diagnosing an allergy.

In another embodiment, the invention provides a method for diagnosing an allergy in a subject, comprising administering to the subject at least one of the above antigens (1) to (13). The method may be in the form of a skin test characterized by applying the antigen to the skin. Examples of the form of the skin test include: a prick test, in which a diagnostic composition is applied onto the skin, a small cut is given to a degree to which no bleeding occurs, and an antigen is permeated into the skin; a scratch test, in which a diagnostic composition is applied, and the skin is slightly scratched to observe the resulting reaction; a patch test, in which a diagnostic composition in the form of a cream or ointment is applied to the skin and the reaction is observed; or an intracutaneous test, in which an antigen is administered intracutaneously and the reaction is observed. In the case of occurrence of a skin reaction, such as skin swelling, in an antigen-applied site, the subject is diagnosed as having an allergy. Here, the amount of antigen applied to the skin may be, for example, a dose of 100 µg or less per application.

In the allergy diagnosis, a loading test is often performed to identify the antigen. At least one of the above antigens (1) to (13) may be used as an active ingredient for the loading test to diagnose an allergy. Here, each antigen protein used in the loading test may be an expressed and purified protein. Examples include pollen rice, in which the cedar pollen antigen gene is transformed into rice and the antigen protein is expressed in the rice. In this way, the antigen protein may be expressed in a raw material/processed product.

In one embodiment, the above diagnostic composition or diagnosis kit may be used for a prick test, scratch test, patch test, intracutaneous test, etc.

In addition, another embodiment of the invention provides at least one of the above antigens (1) to (13) for use in allergy diagnosis. Here included is providing at least one of the above antigens (1) to (13) in a mixture with a known antigen(s).

Still another embodiment of the invention provides use of at least one of the above antigens (1) to (13) in the manufacture of a composition for diagnosing an allergy.

### Composition/Treatment Method (1)

The invention provides a composition comprising at least one of the above antigens (1) to (13).

In one embodiment, the composition of the invention is a pharmaceutical composition. In one embodiment, the composition of the invention is a quasi-drug composition or a non-medicinal composition (e.g., a cosmetic composition, a food composition).

In one embodiment, the above composition is used to treat an allergy (e.g., an allergy to milk). As used herein, the "allergy treatment" means increasing the limit of the amount of antigen that does not cause any symptom when taken into the body, with the ultimate goal of achieving a state (remission) in which the disease does not develop with normal intake of the antigen.

The invention also provides a method for treating an allergy, comprising administering to a patient in need of allergy treatment at least one of the above antigens (1) to (13).

Another embodiment of the invention provides at least one of the above antigens (1) to (13) for use in allergy treatment. Still another embodiment of the invention provides use of at least one of the above antigens (1) to (13) in the manufacture of a drug for an allergy.

In the allergy treatment, desensitization therapy, which aims to induce immune tolerance by administering an antigen to a patient, is often used. At least one of the above antigens (1) to (13) may be used as an active ingredient for the allergy desensitization therapy. The antigenic protein used for the desensitization therapy may be an expressed and purified protein, or may be an antigen protein expressed in a raw material/processed product, such as pollen rice, in which the cedar pollen antigen gene is transformed into rice and the resulting antigen protein is expressed in the rice.

The composition of the invention may be administered by a regular administration route. Examples of the regular administration route include oral, sublingual, transdermal, intracutaneous, subcutaneous, intravascular, nasal, intramuscular, intraperitoneal, or intrarectal administration.

The composition of the invention may be used as a composition comprising a pharmaceutically acceptable adjuvant, an excipient, or various types of additive(s) (e.g., a stabilizer, a dissolution aid, an emulsifier, a buffer, a preservative, a colorant) generally used together with an antigen of the invention, if necessary. The dosage form of the composition can be selected, if appropriate, depending on the administration route by those skilled in the art. Examples of the form include tablets, capsules, pastilles, sublingual tablets, injections, nasal sprays, cataplasms, liquids, creams, lotions, or suppositories. The dosage, frequency of administration, and/or period of administration of the composition of the invention may be selected, if appropriate, by a physician in view of, for instance, the route of administration, symptoms, and patient characteristics such as the age and body weight. For example, in the case of adults, a dose of 100 µg or less per administration may be given. The dosing interval may be, for example, daily, once weekly, twice monthly, or once every three months. The administration period may be, for example, several weeks to several years. The dosage in the administration method may be increased in a stepwise manner over the course of the administration period.

### Tester Composition (1)

The invention provides a tester composition comprising an antibody against at least one of the above antigens (1) to (13).

The antibody can be produced by a conventional procedure. For example, the antibody may be produced by immunizing a mammal such as a rabbit with any of the antigen antigens (1) to (13). The antibody may be an Ig antibody, a polyclonal antibody, a monoclonal antibody, or an antigen-binding fragment thereof (e.g., a Fab, F(ab')₂, Fab').

In addition, in the tester composition described above, the antibody may be provided in a form immobilized or bound onto or inside a carrier. The carrier is not particularly limited as long as the carrier can be used for detection of antibody-antigen binding. Any carrier known to those skilled in the art can be utilized.

Examples of the method for determining the presence or absence of an antigen include:
-- a method comprising bringing a produced Ig antibody-comprising tester composition into contact with a sample prepared from a raw material/processed product, detecting biding of the antibody to an antigen in the sample by using, for instance, ELISA, and determining, when the binding of the Ig antibody to the antigen is detected, that the antigen is contained in the raw material/processed product of interest; or
-- a method comprising soaking a raw material/processed product into a filter paper and allowing a reaction with an antibody solution so as to detect an antigen contained therein.

Another embodiment of the invention includes a tester composition for determining the presence or absence of an allergic antigen in an object of interest, comprising a primer having a nucleotide sequence complementary to a portion of at least one of the nucleotide sequence set forth in SEQ ID NO: 1, 3, 5, 7, 9, 11, 13, 15, 17, 19, 21, 23, or 25. The above primer is not limited and, for example, has a nucleotide sequence complementary to, preferably, 12, 15, 20, or 25 nucleotides in a 3'-terminal portion or a center portion of at least one sequence of the nucleotide sequence set forth in SEQ ID NO: 1, 3, 5, 7, 9, 11, 13, 15, 17, 19, 21, 23, or 25. Particularly when the object of interest is mRNA, a primer complementary to the poly A tail is included. In a preferred embodiment, the above primer-comprising tester composition may further comprise a primer comprising a nucleotide sequence of a 5'-terminal portion, preferably a nucleotide sequence consisting of 12, 15, 20, or 25 nucleotides, of at least one sequence of the nucleotide sequence set forth in SEQ ID NO: 1, 3, 5, 7, 9, 11, 13, 15, 17, 19, 21, 23, or 25.

For example, using the above complementary primer and, as a template, DNA or mRNA obtained from milk, cDNA may be amplified by PCR (Polymerase Chain Reaction) including RT-PCR (Reverse Transcription-Polymerase Chain Reaction), and the sequence of the cDNA amplified may be compared to that of SEQ ID NO: 1, 3, 5, 7, 9, 11, 13, 15, 17, 19, 21, 23, or 25 to determine the presence or absence of the antigen. Examples of the PCR amplification method include RACE (Rapid Amplification of cDNA End) protocol. In this case, even if a point mutation encoding the same amino acid exists in comparison of the amplified cDNA with SEQ ID NO: 1, 3, 5, 7, 9, 11, 13, 15, 17, 19, 21, 23, or 25, or even if the nucleotide sequence of the amplified cDNA has insertion, deletion, substitution, or addition of nucleotides in the nucleotide sequence set forth in SEQ ID NO: 1, 3, 5, 7, 9, 11, 13, 15, 17, 19, 21, 23, or 25, the antigen is determined to be present when the amino acid sequence encoded by the amplified cDNA has 70% or higher, preferably 80, 90, 95, 98, or 99% or higher identity to the amino acid sequence set forth in SEQ ID NO: 2, 4, 6, 8, 10, 12, 14, 16, 18, 20, 22, 24, or 26.

In one embodiment, the tester composition described above is used to test whether or not an antigen is included in an object of interest, for example, in a foodstuff (milk) or in a food production line. The above-mentioned tester composition may be used for quality inspection of production lines and pre-shipment products by manufacturers, or for checking whether or not an antigen is included in raw materials and processed products of interest by consumers themselves. In addition, the tester composition can also be used for checking inclusion of the antigen by measuring a change in the protein peak(s) by mass spectroscopy.

### Method of Determining Presence or Absence of Antigen (1)

The invention encompasses a method for determining the presence or absence of any of the above antigens (1) to (13) in an object of interest, the method comprising bringing an antibody against at least one of the above antigens (1) to (13) into contact with a raw material/processed product (including a liquid).

The raw material may be a foodstuff or may be a cosmetic raw material, a pharmaceutical raw material, or the like. The processed product may be a processed food product or may be a cosmetic product, a pharmaceutical product, or the like.

The antibody, the method of producing the antibody, the method of bringing the antibody into contact with a raw material/processed product, and the binding of the antibody to an antigen are as described above in the "Tester Composition (1)".

### Antigen-Removed Food, etc. (1)

The invention provides a raw material or processed product characterized in that at least one of the above antigens (1) to (13) is eliminated or reduced. In one embodiment, the "raw material or processed product" may be a "milk or processed milk product" and may be a "beef or processed beef product".

The method of removing or reducing an antigen of the invention from a raw material or processed product is not limited. Any method may be carried out to remove or reduce the antigen as long as the antigen of the invention can be eliminated or reduced.

For example, a raw material (e.g., milk or beef) from which an antigen of the invention has been eliminated or reduced may be obtained from cows in which expression of the antigen of the invention has been altered using a genetic modification technology.

Any of techniques known to those skilled in the art may be used for the genetic modification technology. For instance, Oishi, et al. (Scientific Reports, Vol. 6, Article number: 23980, 2016, doi: 10.1038/srep23980) discloses that a genome editing technology CRISPER/Cas9 is applied to a chicken primordial germ cell to produce an ovomucoid gene-deleted individual. A similar technique may be used to obtain a cow in which expression of an antigen of the invention has been altered, and from such a cow, beef or milk from which an antigen of the invention has been eliminated or reduced may be obtained.

In addition, cross-breeding with a cow, in which the antigen is not expressed or the expression level is low, by using artificial insemination may allow generation of a cow in which the expression of the antigen of the invention has been eliminated or reduced, and from such a cow, beef or milk from which the antigen of the invention has been eliminated or reduced may be obtained. The cow may be artificially bred by a common procedure.

The processed product, from which an antigen of the invention has been eliminated or reduced, may be a processed product using, as a material, the raw material from which an antigen of the invention has been eliminated or reduced. In the case of using ordinary raw materials as materials, the antigen-removing or reducing treatment in the invention may be carried out before or after preparation of the processed product. Examples of the method of removing or reducing an antigen of the invention from a processed product using ordinary raw materials as materials include: a method for removing a protein component from a raw material/processed product by, for instance, high-pressure treatment, neutral salt solution-using elution, or high-temperature steaming; or a method for using thermal treatment- or acid treatment-mediated hydrolysis/denaturation/amino acid modification (e.g., chemical modification, elimination or the like of a side chain).

### Method of Producing Raw Material or Processed Product with Antigen Eliminated or Reduced (1)

The invention provides a method for producing a raw material or processed product in which an antigen is eliminated or reduced, the method comprising the step of confirming that the antigen has been eliminated or reduced in a production process of the raw material or the processed product, wherein the antigen is at least one of the above antigens (1) to (13).

The step of confirming that the antigen has been eliminated or reduced in a production process of the raw material or processed product in which the antigen is eliminated or reduced may be carried out by confirming the presence or absence of the antigen by a procedure described in the above section "Tester Composition (1)".

The milk or processed milk product or beef or processed beef product in which the antigen is eliminated or reduced may be produced by the procedure described in the above section "Antigen-Removed Food, etc. (1)".

### Epitopes

For the antigens identified as shown in Examples 1 to 3, the epitopes and the amino acids within the epitopes that are each important for binding to an IgE antibody from an allergic patient were identified as demonstrated in Example 4.

The results are summarized in Table 2. In Table 2, P1 to P25 are the patient numbers assigned to the respective 25 allergic patients.

**[Table 2-1]**

| E1 Spot No. 1 | | | | | | | |
|---|---|---|---|---|---|---|---|
| Protein name | immunoglobulin M heavy chain secretory form | | SEQ ID NO: | Cross-reactivity-confirmed food | Graph No. | Synthesized sequence | SEQ ID NO: |
| 15-residue sequence | QNVYTCQVEHNKETF | | 27 | | | | |
| P1 | Key | QNVYTCQVEHNKETF | 28 | | | | |
| | Key | SQNVYTC | 29 | | | | |
| | Key | TCQVEHN | 30 | | | | |
| | Key | HNKETFQ | 31 | | | | |
| P2 | Key | QNVYTCQVEHNKETF | 32 | | | | |
| | Key | LSQNVYTC | 33 | | | | |
| | Key | HNKE | 34 | | | | |
| P3 | Key | QNVYTCQVEHNKETF | 35 | | | | |
| | Key | HNKE | 36 | | | | |
| P4 | Preferable | QNVYTCQVEHNKETF | 37 | | | | |
| | Key | XXXYXCQXXXNKXXX | 38 | | | | |
| | Key | HNKE | 39 | | | | |
| P5 | Key | QNVYTCQVEHNKETF | 40 | | | | |
| | Key | DWLSQNVYTC | 41 | | | | |
| P6 | Key | QNVYTCQVEHNKETF | 42 | | | | |
| | Key | LSQNVYTC | 43 | | | | |
| | Key | NVYTCOVEHN | 44 | | | | |
| | Key | YTCOVEHNKE | 45 | | | | |
| | Key | HNKETF | 46 | | | | |
| P8 | Key | QNVYTCQVEHNKETF | 47 | | | | |
| P9 | Key | QNVYTCQVEHNKETF | 48 | | | | |
| P20 | Preferable | XXXYXXQXXXNKETF | 49 | | | | |
| | Key | XXXXXXXXXXNKEXF | 50 | | | | |
| | Preferable | YTCQVEHNKE | 51 | Sheep | 6 | YTCQVEHNKE | 51 |
| | Key | YXXQXXXNKE | 52 | Chicken | 6 | YLSQTEENKE | 1115 |
| | More preferable | HNKETF | 53 | | | | |
| | Preferable | XNKETF | 54 | | | | |
| | Key | XNKEXF | 55 | | | | |
| P21 | Key | HNKETF | 56 | | | | |
| P22 | Key | YTCOVEHNKE | 57 | | | | |
| | Key | HNKETF | 58 | | | | |

**[Table 2-2]**

| E2 Spot No. 1 | | | |
|---|---|---|---|
| Protein name | immunoglobulin M heavy chain secretory form | | SEQ ID NO: |
| 15-residue sequence | TKSKYVTSSPAPEPQ | | 59 |
| P1 | More preferable | TKSKYVTSSPAPEPQ | 60 |
| | Preferable | XKSKXXXSSPAPEPQ | 61 |
| | Key | XKXKXXXXXXXXXPQ | 62 |
| | Preferable | KSKYVTSSP | 63 |
| | Key | KSKXXXSSP | 64 |
| | Preferable | KSKYVTSS | 65 |
| | Key | KSKXXXSS | 66 |
| | Key | YVTS | 67 |
| P2 | Preferable | TKSKYVTSSPAPEPQ | 68 |
| | Key | XKXKXXXXSPAXEPQ | 69 |
| | Key | SKYVTS | 70 |
| | Key | KYVTS | 71 |
| | Preferable | PAPEPQDPSV | 72 |
| | Key | PAXEPQDPSV | 73 |
| P8 | Key | TKSKYVTSSPAPEPQ | 74 |
| | Key | SKYVTSS | 75 |
| P9 | Key | TKSKYVTSSPAPEPQ | 76 |
| | More preferable | KSKYVTSSP | 77 |
| | Preferable | KSKYVTSS | 78 |
| | Key | SKYVTS | 79 |
| | Key | YVTSSPAPEP | 80 |
| P12 | Preferable | TKSKYVTSSPAPEPQ | 81 |
| | Key | XKSKXXTSSPAXEPQ | 82 |
| | More preferable | KSKYVTSS | 83 |
| | Preferable | SKYVTS | 84 |
| | Key | SKXXTS | 85 |
| P16 | Key | KYVTS | 86 |
| P18 | Preferable | KYVTSS | 87 |
| | Key | YVTS | 88 |
| P20 | Key | SKYVTS | 89 |
| P21 | Preferable | KYVTSS | 90 |
| | Key | YVTS | 91 |
| P23 | Preferable | XKXKYXTSXPAPEPQ | 92 |
| | Key | XXXKYXTXXPAXXXX | 93 |
| | Still more preferable | KSKYVTSSP | 94 |
| | More preferable | KYVTSSPAPE | 95 |
| | Preferable | KYVTS | 96 |
| | Key | KYXTS | 97 |

**[Table 2-3]**

| E3 Spot No. 2, 3 | | | |
|---|---|---|---|
| Protein name | Beta-1 metal-binding globulin | | SEQ ID NO: |
| 15-residue sequence | CGDNTRKSVDNYQEC | | 98 |
| P2 | More preferable | CGDNTRKSVDNYQEC | 99 |
| | Preferable | CGDNTRKSVDNYXXX | 100 |
| | Key | XGDNTRKSXDNXXXX | 101 |
| | More preferable | KSVDNYQECY | 102 |
| | Preferable | KSVDNYXXXY | 103 |
| | Key | KSXDNXXXXY | 104 |
| | More preferable | DNYQECYLA | 105 |
| | Preferable | DNYXXXYLA | 106 |
| | Key | DNXXXXYLA | 107 |
| P3 | More preferable | CGDNTRKSVDNYQEC | 108 |
| | Preferable | XGDNXRKSXDNYXXX | 109 |
| | Key | XGXXXXKSXDNYXXX | 110 |
| | Preferable | KSVDNYQECY | 111 |
| | Key | KSXDNYXXXY | 112 |
| | Key | DNYQECYLA | 113 |
| P4 | Preferable | CGDNTRKSVDNYQEC | 114 |
| | Key | XXDXXRKSXDNXXXX | 115 |
| | Key | DNTR | 116 |
| | Preferable | TRKSVDNYQE | 117 |
| | Key | XRKSXDNXXX | 118 |
| | Preferable | KSVDNYQECY | 119 |
| | Key | KSXDNXXXXY | 120 |
| P8 Key | | CGDNTRKSVDNYQEC | 121 |
| P12 | More preferable | CGDNTRKSVDNYQEC | 122 |
| | Preferable | XGDNTRKXXDNYXXX | 123 |
| | Key | XGDNTRXXXXNXXXX | 124 |
| | Key | DNTR | 125 |
| | Preferable | KSVDNYQECY | 126 |
| | Key | KXXDNYXXXY | 127 |
| P17 | Key | XXDXXRKSXDNYXXX | 128 |
| P18 | Preferable | XGXXXRKSVDNYXXX | 129 |
| | Key | XXXXXXKXXDNYXXX | 130 |
| P21 | Preferable | XGDNTRKSVDNYQXX | 131 |
| | Key | XXXXXRKXXDNYXXX | 132 |
| P22 | Preferable | XXXXXRKSVDNYQXX | 133 |
| | Key | XXXXXXKXVDNYXXX | 134 |
| | Preferable | DNTRKSV | 135 |
| | Key | XXXRKSV | 136 |
| | More preferable | TRKSVDNYQE | 137 |
| | Preferable | XRKSVDNYQX | 138 |
| | Key | XXKXVDNYXX | 139 |
| P24 | Preferable | XGDNTRKXXDNXXXX | 140 |
| | Key | XXDNXRXXXXNXXXX | 141 |
| P25 | Preferable | XGXNTRKSXDNXQXX | 142 |
| | Key | XXXXXRKSXDNXXXX | 143 |
| | Key | DNTR | 144 |

**[Table 2-4]**

| E4 Spot No. 2, 3 | | | |
|---|---|---|---|
| Protein name | Beta-1 metal-binding globulin | | SEQ ID NO: |
| 15-residue sequence | SPHGKDLLFKDSADG | | 145 |
| P1 | Key | SPHGKDLLFKDSADG | 146 |
| | Key | SPHGKD | 147 |
| | Key | KDLLFKDS | 148 |
| P2 | Key | SPHGKDLLFKDSADG | 149 |
| | Key | QLFQSPHGKD | 150 |
| P3 | Preferable | SPHGKDLLFKDSADG | 151 |
| | Key | XXHGKDXXXKXXXXX | 152 |
| | Key | KDSADGFLK | 153 |
| P4 | Preferable | SPHGKDLLFKDSADG | 154 |
| | Key | XXHGKXXXXKDXAXX | 155 |
| | Preferable | QSPHGKDLL | 156 |
| | Key | QXXHGKXXX | 157 |
| | Key | KDSADG | 158 |
| P5 | Preferable | SPHGKDLLFKDSADG | 159 |
| | Key | XPHGKXXXXXXXXXX | 160 |
| | Preferable | QLFQSPHGKD | 161 |
| | Key | QLFQXPHGKX | 162 |
| | Key | GKDLL | 163 |
| | Key | KDSADGFL | 164 |
| P6 | Key | SPHGKDLLFKDSADG | 165 |
| | Key | KDSADGFL | 166 |
| P7 | Key | SPHGKDLLFKDSADG | 167 |
| | Key | QLFQSPHGKD | 168 |
| P8 | Key | SPHGKDLLFKDSADG | 169 |
| P9 | Preferable | SPHGKDLLFKDSADG | 170 |
| | Key | SXHXKDXXXXDXXDG | 171 |
| | Preferable | KDLLFK | 172 |
| | Key | DLLFK | 173 |
| | Preferable | FKDSADGFLK | 174 |
| | Key | XXDXXDGFLK | 175 |
| P12 | Key | SPHGKDLLFKDSADG | 176 |
| | Key | PHGKDLLFK | 177 |
| | Key | GKDLLFKDSA | 178 |
| | Key | KDSADG | 179 |
| P16 | Key | XXHXKXXXXKDSXXX | 180 |
| | Key | DLLFKDS | 181 |
| P17 | Key | GKDLL | 182 |
| P20 | Key | XXHXKXXLFKXXXXX | 183 |
| | Preferable | KDLLFK | 184 |
| | Key | DLLFK | 185 |
| P21 | Key | KDSADG | 186 |
| P22 | Preferable | XXHXKDXLFKDSXXX | 187 |
| | Key | XXHXKXXXXKDXXXX | 188 |
| | Preferable | KDLLFK | 189 |
| | Key | KDXLFK | 190 |
| P23 | Key | XXXXKXLLFKXXXXX | 191 |
| | Key | LLFK | 192 |
| P24 | Key | SPHGKD | 193 |
| | Preferable | FKDSADG | 194 |
| | Key | KDSA | 195 |

**[Table 2-5]**

| E5 Spot No. 2. 3 | | | | | | | |
|---|---|---|---|---|---|---|---|
| Protein name | Beta-1 metal-binding globulin | | SEQ ID NO: | Cross-reactivity-confirmed food | Graph No. | Synthesized sequence | SEQ ID NO: |
| 15-residue sequence | HSTVFDNLPNPEDRK | | 196 | | | | |
| P1 | Preferable | HSTVFDNLPNPEDRK | 197 | | | | |
| | Key | XXXXXXXXXNPEDRK | 198 | | | | |
| | Key | FVKHSTVFDN | 199 | | | | |
| | Key | VKHSTVFD | 200 | | | | |
| | Key | KHSTVFD | 201 | | | | |
| | Preferable | NPEDRK | 202 | | | | |
| | Key | NPEDR | 203 | Sheep | 3 | NPEDR | 203 |
| P2 | Key | HSTVFDNLPNPEDRK | 204 | | | | |
| | Preferable | KHSTVFD | 205 | | | | |
| | Key | HSTVFD | 206 | | | | |
| | Preferable | NPEDRK | 207 | | | | |
| | Key | NPEDR | 208 | | | | |
| P3 | Key | HSTVFDNLPNPEDRK | 209 | | | | |
| | More preferable | FVKHSTVFD | 210 | | | | |
| | Preferable | KHSTVFD | 211 | | | | |
| | Key | HSTVFD | 212 | | | | |
| | Key | NPEDR | 213 | | | | |
| P4 | Key | HSTVFDNLPNPEDRK | 214 | | | | |
| | Preferable | KHSTVFD | 215 | | | | |
| | Key | HSTVFD | 216 | | | | |
| | Key | NPEDR | 217 | | | | |
| P5 | Key | HSTVFDNLPNPEDRK | 218 | | | | |
| | Key | HSTVFD | 219 | | | | |
| | Key | NPEDR | 220 | Sheep | 4 | NPEDR | 220 |
| P6 | Key | HSTVFDNLPNPEDRK | 221 | | | | |
| P8 | Preferable | HSTVFDNLPNPEDRK | 222 | | | | |
| | Key | XXXXXXXXXXPEDRK | 223 | | | | |
| | Key | KHSTVFD | 224 | | | | |
| | Preferable | NPEDRK | 225 | | 5 | NPEDRK | 225 |
| | Key | XPEDRK | 226 | Sheep | 5 | HPEDRK | 1116 |
| P9 | Key | HSTVFDNLPNPEDRK | 227 | | | | |
| P11 | Key | HSTVFDNLPNPEDRK | 228 | | | | |
| P15 | Key | TVFD | 229 | | | | |
| | Key | NPEDR | 230 | | | | |
| P16 | Key | STVFD | 231 | | | | |
| | Preferable | NPEDRK | 232 | | | | |
| | Key | NPEDR | 233 | | | | |
| P17 | Key | STVFD | 234 | | | | |
| | Key | NPEDR | 235 | | | | |
| P18 | Preferable | HSTVFD | 236 | | | | |
| | Key | TVFD | 237 | | | | |
| | Key | NPEDR | 238 | | | | |
| P20 | Key | XSXXXXXXXXPEDRX | 239 | | | | |
| | More preferable | DNLPNPEDRK | 240 | | | | |
| | Preferable | NPEDR | 241 | Sheep | 6 | NPEDR | 241 |
| | Key | XPEDR | 242 | Chicken | 6 | KPEDR | 1117 |
| | | | | Sheep | 6 | VPEDR | 1118 |
| P21 | Key | NPEDRK | 243 | | | | |
| P22 | Key | HSTVFD | 244 | | | | |
| | Key | NPEDRK | 245 | | | | |
| P23 | Preferable | STVFD | 246 | | | | |
| | Key | TVFD | 247 | | | | |
| | Key | NPEDRK | 248 | | | | |
| P24 | More preferable | XXXXXXXXXNPEDRK | 249 | | | | |
| | Preferable | XXXXXXXXXXPEDRK | 250 | | | | |
| | Key | XXXXXXXXXXPEDRX | 251 | | | | |
| | More preferable | NPEDRK | 252 | | | | |
| | Preferable | NPEDR | 253 | | | | |
| | Key | XPEDR | 254 | | | | |

**[Table 2-6]**

| E6 | | | |
|---|---|---|---|
| Spot No. 2, 3 | | | |
| Protein name | Beta-1 metal-binding globulin | | SEQ ID NO: |
| 15-residue sequence | DCTSNFCLFQSNSKD | | 255 |
| P2 | Key | DCTSNFCLFQSNSKD | 256 |
| | Key | QSNSK | 257 |
| P3 | Preferable | DCTSNFCLFQSNSKD | 258 |
| | Key | XXXXXXXXFQSNSKD | 259 |
| | Preferable | CLFQSNSK | 260 |
| | Key | QSNSK | 261 |
| P4 | Preferable | DCTSNFCLFQSNSKD | 262 |
| | Key | XXXXXXXXFQSNSKD | 263 |
| | Key | QSNSK | 264 |
| P8 | Preferable | DCTSNFCLFQSNSKD | 265 |
| | Key | XXXXXFCLFQSXSKD | 266 |
| | More preferable | LFQSNSKD | 267 |
| | Preferable | FQSNSK | 268 |
| | Key | FQSXSK | 269 |
| | Key | QSNS | 270 |
| P10 | Preferable | DCTSNFCLFQSNSKD | 271 |
| | Key | XXXXXXCLFXXXXKX | 272 |
| | More preferable | FQSNSK | 273 |
| | Preferable | QSNSK | 274 |
| | Key | QSNS | 275 |
| P16 | Key | QSNSK | 276 |
| P17 | Key | XXXXXXXXFXXNSKD | 277 |
| | Key | QSNSK | 278 |
| P20 | Preferable | CLFQSNSK | 279 |
| | Key | QSNSK | 280 |
| P21 | Key | QSNSK | 281 |
| P22 | Key | XXXXXXCLFXXXSKD | 282 |
| | Preferable | CLFQSNSK | 283 |
| | Key | QSNSK | 284 |
| P23 | Preferable | QSNSK | 285 |
| | Key | QSNS | 286 |
| P25 | Key | XXXXXXXXFXXNSKD | 287 |
| | Key | QSNSK | 288 |

**[Table 2-7]**

| E7 | | | | | | | |
|---|---|---|---|---|---|---|---|
| Spot No. 2. 3 | | | | | | | |
| Protein name | | Beta-1 metal-binding globulin | SEQ ID NO: | Cross-reactivity-confirmed food | Graph No. | "sequence" | SEQ ID NO: |
| 15-residue sequence | | LAENYKTEGESCKNT | 289 | | | | |
| P2 | Preferable | LAENYKTEGESCKNT | 290 | | | | |
| | Key | XXXXXKXXXESXKXT | 291 | | | | |
| | Key | AEMYKTEG | 292 | | | | |
| | Preferable | YKTEGESCK | 293 | | | | |
| | Key | XKXXXESXK | 294 | | | | |
| | More preferable | ESCKNTPEK | 295 | | | | |
| | Preferable | ESCKNTPE | 296 | | | | |
| | Key | ESXKXTPE | 297 | | | | |
| P3 | Preferable | LAENYKTEGESCKNT | 298 | | | | |
| | Key | XXXXYKXXGESCKNT | 299 | | | | |
| | Preferable | YKTEGESCK | 300 | | | | |
| | Key | KTEGE | 301 | | | | |
| | Key | YKXXGESCK | 302 | | | | |
| | Key | ESCKNTPE | 303 | | | | |
| P4 | Preferable | LAENYKTEGESCKNT | 304 | | | | |
| | Key | XXEXXKXEGEXCKXX | 305 | | | | |
| | Preferable | GESCKNTPE | 306 | | | | |
| | Key | GEXCKXXPE | 307 | | | | |
| P8 | Preferable | LAENYKTEGESCKNT | 308 | | | | |
| | Key | XXXXXXXXXESXKNX | 309 | | | | |
| | Key | KTEGESCK | 310 | | | | |
| | Preferable | ESCKNTPEK | 311 | | | | |
| | Key | ESXKNXPEK | 312 | | | | |
| P9 | Preferable | LAENYKTEGESCKNT | 313 | | | | |
| | Key | XXXXXXXEGESXKXX | 314 | | | | |
| | Preferable | NYKTEGESCK | 315 | | | | |
| | Key | XXXXEGESXK | 316 | | | | |
| | Preferable | ESCKNTPEK | 317 | | | | |
| | Key | ESXKXXPEK | 318 | | | | |
| P16 | Preferable | XXXXYKXEGEXCKNT | 319 | | | | |
| | Key | XXXXXXXXGXXXKNT | 320 | | | | |
| | Preferable | YKTEGESCK | 321 | | | | |
| | Key | YKXEGEXCK | 322 | | | | |
| P20 | Key | XXEXYKXXGEXCKNT | 323 | | | | |
| | More preferable | YKTEGESCK | 324 | | | | |
| | Preferable | KTEGESC | 325 | | 6 | KTEGESC | 325 |
| | Key | KXXGEXC | 326 | Chicken | 6 | KRRGESC | 1119 |
| | | | | Sheep | 6 | KADGESC | 1120 |
| P21 | Key | XXENYKXXXXXCKNT | 327 | | | | |
| | Preferable | YKTEGESCK | 328 | | | | |
| | Key | KTEGES | 329 | | | | |
| P23 | Preferable | XXXXYXXXXESCKNT | 330 | | | | |
| | Key | XXXXXXXXXXSCKNT | 331 | | | | |
| | Key | KTEGES | 332 | | | | |
| P24 | Key | XXXXXKXXGESXKXX | 333 | | | | |

**[Table 2-8]**

| E8 | | | |
|---|---|---|---|
| Spot No. 2, 3 | | | |
| Protein name | Beta-1 metal-binding globulin | | SEQ ID NO: |
| 15-residue sequence | NHAVVSRKDKATCVE | | 334 |
| P2 | Key | NHAVVSRKDKATCVE | 335 |
| P3 | Key | NHAVVSRKDKATCVE | 336 |
| P7 | Key | NHAVVSRKDKATCVE | 337 |
| | Key | RGPNHAVVS | 338 |
| P8 | Preferable | NHAVVSRKDKATCVE | 339 |
| | Key | XXXXVXXKDXAXXXX | 340 |
| | Key | RKDKAT | 341 |
| P9 | Key | NHAVVSRKDKATCVE | 342 |
| P10 | Key | NHAVVSRKDKATCVE | 343 |
| | Key | RKDKAT | 344 |
| P16 | Key | XXXVVSRKDKAXXXX | 345 |
| | Key | RKDK | 346 |
| P21 | Key | XXXXVXRKXKATXXX | 347 |
| | Preferable | RKDKAT | 348 |
| | Key | RKXKAT | 349 |
| P22 | Key | XXXXVSXKXKAXXXX | 350 |
| P23 | Preferable | XXXVVSRKDKATXXX | 351 |
| | Key | XXXXVSRKDKXXXXX | 352 |

**[Table 2-9]**

| E9 | | | | | | | |
|---|---|---|---|---|---|---|---|
| Spot No. 4 | | | | | | | |
| Protein name | Lactoperoxidase | | SEQ ID NO: | Cross-reactivity-confirmed food | Graph No. | Synthesized sequence | SEQ ID NO: |
| 15-residue sequence | ETELGSNEHSKTQCE | | 353 | | | | |
| P2 | Key | ETELGSNEHSKTQCE | 354 | | | | |
| | Preferable | SKTQC | 355 | | | | |
| | Key | SKTQ | 356 | | | | |
| P3 | Key | ETELGSNEHSKTQCE | 357 | | | | |
| | Key | SKTQC | 358 | | | | |
| P4 | Key | ETELGSNEHSKTQCE | 359 | | | | |
| | Preferable | HSKTQC | 360 | | | | |
| | Key | SKTQC | 361 | | | | |
| P5 | Key | ETELGSNEHSKTQCE | 362 | | 4 | ETELGSNEHSKTQCE | 362 |
| | Key | FAPETELGS | 363 | | | | |
| | Preferable | SKTQC | 364 | | | | |
| | Key | SKTQ | 365 | | | | |
| P8 | Key | ETELGSNEHSKTQCE | 366 | | 5 | ETELGSNEHSKTQCE | 366 |
| P9 | Key | ETELGSNEHSKTQCE | 367 | | | | |
| P12 | Key | ETELGSNEHSKTOCE | 368 | | | | |
| | Preferable | EHSKTOC | 369 | | | | |
| | Key | SKTQ | 370 | | | | |
| P17 | Key | SKTO | 371 | | | | |
| P20 | Key | XXXXXXXEXXKTXXE | 372 | Chicken, Sheep | 6 | ETELGSNEHSKTQCE | 362 |
| | | | | Chicken | 6 | EWQNRLEEIKKTMFE | 1121 |
| | | | | Sheep | 6 | EKTFECGECGKTFWE | 1122 |
| | Key | SKTQ | 373 | | | | |
| P24 | Preferable | SKTQC | 374 | | | | |
| | Key | SKTQ | 375 | | | | |
| P25 | Key | SKTQ | 376 | | | | |

**[Table 2-10]**

| E10 | | | |
|---|---|---|---|
| Spot No. 4 | | | |
| Protein name | | Lactoperoxidase | SEQ ID NO: |
| 15-residue sequence | | LHTLFFNTWRIIKDG | 377 |
| P1 | Preferable | LHTLFFNTWRIIKDG | 378 |
| | Key | XXXXXXXXXXXIKDG | 379 |
| | Key | LPLHTLFFNT | 380 |
| P2 | Key | LHTLFFNTWRIIKDG | 381 |
| P3 | Key | LHTLFFNTWRIIKDG | 382 |
| P8 | Key | LHTLFFNTWRIIKDG | 383 |
| P23 | Key | RIIK | 384 |

**[Table 2-11]**

| E11 | | | |
|---|---|---|---|
| Spot No. 7 | | | |
| Protein name | Polymeric immunoglobulin receptor | | SEQ ID NO: |
| 15-residue sequence | SFEKYWCKWSNRGCS | | 385 |
| P2 | More preferable | SFEKYWCKWSNRGCS | 386 |
| | Preferable | XFEKYWCKWSNRGCS | 387 |
| | Key | XXEKYWCKWSNXXXX | 388 |
| | Preferable | FEKYW | 389 |
| | Key | XEKYW | 390 |
| | Preferable | YWCKWSNRGC | 391 |
| | Key | YWCKWSNXXX | 392 |
| P3 | More preferable | SFEKYWCKWSNRGCS | 393 |
| | Preferable | XFEKYWCKWSNRGCS | 394 |
| | Key | XXEKYWCKWSNRGCX | 395 |
| P4 | Still more preferable | SFEKYWCKWSNRGCS | 396 |
| | More preferable | XFEKYXCKWSNXXXX | 397 |
| | Preferable | XXEKYXCKWSNXXXX | 398 |
| | Key | XXEXXXXXWSNXXXX | 399 |
| P6 | Key | SFEKYWCKWSNRGCS | 400 |
| P8 | More preferable | SFEKYWCKWSNRGCS | 401 |
| | Preferable | XFEKYWCKWSNRGCS | 402 |
| | Key | XXEKYXCKWSNRXXX | 403 |
| P9 | Preferable | SFEKYWCKWSNRGCS | 404 |
| | Key | XXXKYWCXXSXRGXX | 405 |
| P10 | Preferable | SFEKYWCKWSNRGCS | 406 |
| | Key | XXXKYWCKWSNRXXX | 407 |
| P17 | Preferable | XFXKYWCKWSNRXXX | 408 |
| | Key | XXXKYXXKWSNXXXX | 409 |
| P19 | Key | XFXKYWCKWSNRGXX | 410 |
| P23 | Key | XXXXXXXXXXNRGCS | 411 |

**[Table 2-12]**

| E12 Spot No. 7 | | | |
|---|---|---|---|
| Protein name | Polymeric immunoglobulin receptor | | SEQ ID NO: |
| 15-residue sequence | NLDTVTKEDEGWYWC | | 412 |
| P2 | Key | NLDTVTKEDEGWYWC | 413 |
| | More preferable | SLNLDTVT | 414 |
| | Preferable | LNLDTVT | 415 |
| | Key | LDTVT | 416 |
| | Preferable | TKEDEGWYWC | 417 |
| | Key | EGWY | 418 |
| P3 | Key | NLDTVTKEDEGWYWC | 419 |
| | Preferable | VVSLNLDTVT | 420 |
| | Key | SLNLDTVT | 421 |
| | Preferable | VTKEDEGWYW | 422 |
| | Key | KEDEGWY | 423 |
| | Key | EGWYWCGVKE | 424 |
| P4 | Key | NLDTVTKEDEGWYWC | 425 |
| | Preferable | VVSLNLDTVT | 426 |
| | Key | VSLNLDTVT | 427 |
| | Preferable | TKEDEGWYWC | 428 |
| | Key | KEDEGWYWC | 429 |
| P8 | Preferable | NLDTVTKEDEGWYWC | 430 |
| | Key | XXXTVXKEXEXXXXX | 431 |
| | Preferable | VSLNLDTVT | 432 |
| | Key | VSLXXXTVX | 433 |
| | More preferable | TKEDEGWYWC | 434 |
| | Preferable | TKEDEGWYW | 435 |
| | Key | KEDEGWYW | 436 |
| P20 | Key | LDTVTKEDEG | 437 |
| | Key | KEDEGWY | 438 |
| P22 | Preferable | XXXXVXKEDEXXXXX | 439 |
| | Key | XXXXXXKEDEXXXXX | 440 |
| | More preferable | VTKEDEGWYW | 441 |
| | Preferable | TKEDEGWYW | 442 |
| | Key | XKEDEXXXX | 443 |
| P23 | Key | NLDTVT | 444 |
| | Preferable | TKEDEGWYW | 445 |
| | Key | KEDEGWY | 446 |
| P25 | Key | XXXTVTKEDEXXXXX | 447 |
| | More preferable | KEDEGWYWC | 448 |
| | Preferable | KEDEG | 449 |
| | Key | KEDEX | 450 |

**[Table 2-13]**

| E13 Spot No. 8 | | | | | | | |
|---|---|---|---|---|---|---|---|
| Protein name | C3-beta-c | | SEQ ID NO: | Cross-reactivity-confirmed food | Graph No. | Synthesized sequence | SEQ ID NO: |
| 15-residue sequence | VKNGGKEEKHHRPGQ | | 451 | | | | |
| P1 | Key | VKNGGKEEKHHRPGQ | 452 | | | | |
| | Key | KNGGKEE | 453 | | | | |
| | Key | GGKE | 454 | | | | |
| P2 | Preferable | VKNGGKEEKHHRPGQ | 455 | | | | |
| | Key | XXNXXXEEXXXXPGQ | 456 | | | | |
| | Preferable | KNGGKEEK | 457 | | | | |
| | Key | KNGGKE | 458 | | | | |
| | Preferable | EKHHRPGQQI | 459 | | | | |
| | Key | EXXXXPGQQI | 460 | | | | |
| P3 | Key | VKNGGKEEKHHRPGQ | 461 | | | | |
| | Key | TLVVKNGGKE | 462 | | | | |
| | Preferable | NGGKEEK | 463 | | | | |
| | Key | GGKEEK | 464 | | | | |
| | Key | EKHHRPGQQI | 465 | | | | |
| P5 | Preferable | VKNGGKEEKHHRPGQ | 466 | | | | |
| | Key | XXXGXKEEXXXXXGQ | 467 | | | | |
| | Most preferable | KNGGKEEKHH | 468 | | | | |
| | Still more preferable | KNGGKEEKH | 469 | | | | |
| | More preferable | KNGGKEEKH | 470 | | | | |
| | Preferable | NGGKEEK | 471 | Sheep | 4 | NGGKEEK | 471 |
| | Key | XGXKEEX | 472 | Chicken | 4 | TGGKEEK | 1123 |
| | Preferable | KEEKHHRPGQ | 473 | Sheep | 4 | KEEKHHRPGQ | 473 |
| | Key | KEEXXXXXGQ | 474 | Chicken | 4 | KEESVPL VGQ | 1124 |
| | Preferable | EKHHRPGQQI | 475 | | | | |
| | Key | EXXXXXGQQI | 476 | | | | |
| P8 | Key | VKNGGKEEKHHRPGQ | 477 | | | | |
| | Preferable | VKNGGKEEKH | 478 | | | | |
| | Key | KNGGKEEK | 479 | | | | |
| | Key | KEEKHHRP | 480 | | | | |
| | Key | EKHHRPGQQI | 481 | | | | |
| P9 | Key | VKNGGKEEKHHRPGQ | 482 | | | | |
| | Key | TLVVKNGGK | 483 | | | | |
| | Key | KEEK | 484 | | | | |
| | Key | KHHRPGQQI | 485 | | | | |
| P10 | Key | VKNGGKEEKHHRPGQ | 486 | | | | |
| | Preferable | TLVVKNGGKE | 487 | | | | |
| | Key | TLVVKNGGK | 488 | | | | |
| P16 | Key | XXXXXXEEXHHXPGQ | 489 | | | | |
| | More preferable | KNGGKEEK | 490 | | | | |
| | Preferable | GKEEKHHRP | 491 | | | | |
| | Key | KEEK | 492 | | | | |
| P17 | More preferable | KNGGKEEK | 493 | | | | |
| | Preferable | GGKEEK | 494 | | | | |
| | Key | GKEEK | 495 | | | | |
| P20 | More preferable | KNGGKEEK | 496 | | | | |
| | Preferable | GGKEEKHHRP | 497 | | | | |
| | Key | KEEK | 498 | | | | |
| P21 | Key | GGKEEK | 499 | | | | |
| P22 | Key | XXXGXXEEKXXXXXQ | 500 | | | | |

**[Table 2-14]**

| E14 Spot No. 5 | | | | | | | |
|---|---|---|---|---|---|---|---|
| Protein name | **Ig heavy chain precursor (B/MT. 4A. 17. H5. A5)** | | **SEQ ID NO:** | Cross-reactivity-confirmed food | Graph No. | Synthesized sequence | **SEQ ID NO:** |
| **15-residue sequence** | **ESEDKYGTTPPQLDA** | | 501 | | | | |
| | More preferable | ESEDKYGTTPPOLDA | 502 | | | | |
| | Preferable | XXEDKYXXXPXXXXX | 503 | | | | |
| | Key | XXEDKYXXXXXXXXX | 501 | | | | |
| P1 | Preferable | DKYGTTP | 505 | | 3 | DKYGTTP | 505 |
| | Key | DKYXXXP | 506 | Sheep | 3 | DKYELPP | 1125 |
| | Key | YGTTPPOLDA | 507 | | | | |
| | Preferable | ESEDKYGTTPPDLDA | 508 | | | | |
| | Key | XSXDKYXXXXXXXXX | 509 | | | | |
| | Preferable | EDKYGTT | 510 | | | | |
| P2 | Key | EDKYGT | 511 | | | | |
| | Preferable | YGTTPPOLDA | 512 | | | | |
| | Key | YGTTPPOLD | 513 | | | | |
| | Preferable | ESEOKYGTTPPOLDA | 514 | | | | |
| | Key | XSEDKYXXXXXXXXX | 515 | | | | |
| P3 | Key | EDKY | 516 | | | | |
| | Key | YGTTPPOLDA | 517 | | | | |
| | Still more preferable | ESEDKYGTTPPOLDA | 518 | | | | |
| | More preferable | XSEDKYGXTPPOLXX | 519 | | | | |
| | Preferable | XXEDKYGXXXPDXXX | 520 | | | | |
| | Key | XXXDKYGXXXPOXXX | 521 | | | | |
| P5 | Preferable | DKYGT | 522 | | | | |
| | Key | DKYGX | 523 | | | | |
| | More preferable | YGTTPPQLDA | 524 | | | | |
| | Preferable | GTTPPQLDA | 515 | | | | |
| | Key | GXTPPOLXX | 518 | | | | |
| | Preferable | EKSEDKYGTTPPQLDA | 517 | | | | |
| | Key | XSXDKYXXTXPXXXX | 528 | | | | |
| | More preferable | SEDKYGTTPP | 529 | | | | |
| P7 | Preferable | EDKYGTT | 530 | | | | |
| | Key | XDKYXXT | 531 | | | | |
| | Preferable | XGTTPPQLDA | 532 | | | | |
| | Key | YGTTPPOLD | 533 | | | | |
| | Preferable | ESEOKTGTTPPOLDA | 534 | | | | |
| | Key | XSEDKYGXTXXXXXX | 535 | | | | |
| | Still more preferable | SEDKYGTTP | 536 | | | | |
| P8 | More preferable | EDKYGTTP | 577 | | | | |
| | Preferable | EDKYGTT | 538 | | | | |
| | key | EDKYGXT | 539 | | | | |
| | Key | ESEDKYGTTPPOLDA | 540 | | | | |
| | Preferable | EDKYGT | 541 | | | | |
| P12 | Key | EDKY | 542 | | | | |
| | Key | XGTTPPQLDA | 543 | | | | |
| | More preferable | XSEDKYXXTXPXXXX | 544 | | | | |
| | Preferable | XSXDKYXXTXPXXXX | 545 | | | | |
| | Key | XSXDKYXXXXXXXXX | 546 | | | | |
| P14 | Preferable | EDKYG | 547 | | | | |
| | Key | EDKY | 548 | | | | |
| | Preferable | XGTTPPQLDA | 549 | | | | |
| | Key | YGTTPPOLD | 550 | | | | |
| | Preferable | XXXDKYXXTPPXLDA | 551 | | | | |
| | Key | XXXDKYXXTXXXXXX | 552 | | | | |
| | Still more preferable | SEDKYGTTPP | 553 | | | | |
| | More preferable | EDKYGTTP | 551 | | | | |
| P16 | Preferable | XDKYXXTP | 555 | | | | |
| | Key | XDKYXXTX | 556 | | | | |
| | More preferable | YGTTPPOLDA | 557 | | | | |
| | Preferable | GTTPPOLD | 558 | | | | |
| | Key | XXTPPXLD | 559 | | | | |
| | Preferable | XSXDKYXXTPPOXXX | 560 | | | | |
| | Key | XXXDKYXXXXXOXXX | 561 | | | | |
| | Preferable | SEDKYGTTP | 561 | | | | |
| P17 | Key | EDKY | 563 | | | | |
| | More preferable | XGTTPPQLDA | 564 | | | | |
| | Preferable | TTPPOLD | 565 | | | | |
| | Key | XTPPOXX | 566 | | | | |
| | Preferable | XSXDKYXXTPPOXXX | 567 | | | | |
| | Key | XXXDKYXXXXPXXXX | 568 | | | | |
| P1B | More preferable | EDKYGTTP | 589 | | | | |
| | Preferable | YGTTPPOLDA | 570 | | | | |
| | Key | YGTTP | 571 | | | | |
| P20 | Preferable | XXEDKYXXTPPOXDA | 572 | | | | |
| | Key | XXEDKYXXXXXXXXA | 573 | | | | |
| | More preferable | EDKYGTTPP | 574 | | 6 | EDKYGTTP | 574 |
| | Preferable | EDKYXXTPP | 575 | | | | |
| | Key | EDKYXXXXX | 576 | Chicken | 6 | EDKYRKIN | 1126 |
| | | | | Sheep | 6 | EDKYGTTT | 1127 |
| | More preferable | XGTTPPQLDA | 577 | | | | |
| | Preferable | YGTTPPOLD | 578 | | | | |
| | Key | YXXTPPOXD | 579 | | | | |
| | More preferable | XSXDKYXXTPPOLDA | 580 | | | | |
| | Preferable | XXXDKYXXTXPQLXA | 581 | | | | |
| | Key | XXXDKYXXTXPDXXX | 581 | | | | |
| | More preferable | SEDKYGTTP | 583 | | | | |
| | Preferable | SEDKY | 584 | | | | |
| P21 | Key | SXDKY | 585 | | | | |
| | Most preferable | XGTTPPQLD | 586 | | | | |
| | Still more preferable | YGTPPQL | 587 | | | | |
| | More preferable | YXXTPPQL | 588 | | | | |
| | Preferable | YXXTXPQL | 589 | | | | |
| | Key | YXXTXPQX | 590 | | | | |
| | More preferable | XSXXKYXTTXPOLDX | 591 | | | | |
| | Preferable | XXXXKYXTTXPOLXX | 592 | | | | |
| | Key | XXXXKYXXTXPQXXX | 593 | | | | |
| P23 | Still more preferable | YGTTPPOLD | 594 | | | | |
| | More preferable | YGTTPPQL | 595 | | | | |
| | Preferable | YXTTXPQL | 596 | | | | |
| | Key | YXXTXPQX | 597 | | | | |
| | Preferable | XXEDKYXXXXPXXXX | 598 | | | | |
| | Key | XXEDKYXXXXXXXXX | 599 | | | | |
| | More preferable | EDKYGTTP | 600 | | | | |
| P24 | Preferable | EDKYGTT | 601 | | | | |
| | Key | EDKYXXX | 602 | | | | |
| | Preferable | YGTTPPOLDA | 603 | | | | |
| | Key | GTTPPQLD | 604 | | | | |

**[Table 2-15]**

| E15 Spot No. 5 | | | | | | | |
|---|---|---|---|---|---|---|---|
| Protein name | Ig heavy chain precursor (B./MT. 4A. 17. H5. A5) | | SEQ ID NO: | Cross-reactivity-confirmed food | Graph No. | Synthesized sequence | SEQ ID NO: |
| 15-residue sequence | TKENSKSQVSLSVSS | | 605 | | | | |
| P1 | Still more preferable | TKENSKSQVSLSVSS | 606 | | | | |
| | More preferable | XKXNSKSQVSLSVSX | 607 | | | | |
| | Preferable | XKXXSKSQXSLSXSX | 608 | | | | |
| | Key | XKXXSKSQXSXSXXX | 609 | | | | |
| | Preferable | KENSK | 610 | | 3 | KENSK | 610 |
| | Key | KXNSK | 611 | Sheep | 3 | KINSK | 1128 |
| | Key | ENSK | 612 | | | | |
| P2 | Key | TKENSKSQVSLSVSS | 613 | | | | |
| P3 | Key | TKENSKSQVSLSVSS | 614 | | | | |
| | Key | VSLSVSSVTP | 615 | | | | |
| P4 | More preferable | TKENSKSQVSLSVSS | 616 | | | | |
| | Preferable | XXENSKSQXXXXXXX | 617 | | | | |
| | Key | XXXNSKSXXXXXXXX | 618 | | | | |
| P5 | Preferable | TKENSKSQVSLSVSS | 619 | | | | |
| | Key | XKENSKSQXXXXXXX | 620 | | | | |
| P8 | Key | TKENSKSQVSLSVSS | 621 | | | | |
| | Key | SLSVSSVTP | 622 | | | | |
| P9 | Key | TKENSKSQVSLSVSS | 623 | | | | |
| P10 | Key | TKENSKSQVSLSVSS | 624 | | | | |
| P16 | Key | XKXXSKXXXXXXXSS | 625 | | | | |
| | Key | NSKS | 626 | | | | |
| P17 | Key | XXENSKXXXXXXXXX | 627 | | | | |
| P21 | Key | XXXNSKSXXXXXXXX | 628 | | | | |
| P23 | Preferable | XXEXSKXQXXLSVSS | 629 | | | | |
| | Key | XXEXSKXXXXLXVSS | 630 | | | | |
| P25 | Key | TXENSKXXXXXXXXX | 631 | | | | |
| | Key | ENSK | 632 | | | | |

**[Table 2-16]**

| E16 | | | |
|---|---|---|---|
| Spot No. 6 | | | |
| Protein name | Perilipin | | SEQ ID NO: |
| 15-residue sequence | TTVTGAKDSVASTIT | | 633 |
| P2 | Preferable | TTVTGAKDSVASTIT | 634 |
| | Key | XXXXXXKDSXXSXXX | 635 |
| | Preferable | AVTTTVTGAK | 636 |
| | Key | AVTTTVTGA | 637 |
| | Key | KDSV | 638 |
| P5 | More preferable | TTVTGAKDSVASTIT | 639 |
| | Preferable | TTVTGXKDXVAXXXX | 640 |
| | Key | TXVXGXKXXVXXXXX | 641 |
| | Most preferable | DAVTTTVTGA | 642 |
| | Still more preferable | AVTTTVTGA | 643 |
| | More preferable | TTTVTGA | 644 |
| | Preferable | TTTVTGX | 645 |
| | Key | TTXVXGX | 646 |
| | More preferable | TGAKDSVAS | 647 |
| | Preferable | DSVAS | 648 |
| | Key | DSVA | 649 |
| P8 | Key | TTVTGAKDSVASTIT | 650 |
| | Key | AVTTTVTGA | 651 |
| | Key | DSVAST | 652 |
| P9 | Key | TTVTGAKDSVASTIT | 653 |
| P10 | Key | TTVTGAKDSVASTIT | 654 |
| | Preferable | AVTTTVTGAK | 655 |
| | Key | TTTVTGA | 656 |
| | Key | TVTGAKDSVA | 657 |
| | Preferable | KDSVASTI | 658 |
| | Key | DSVAST | 659 |
| P13 | Preferable | TTVTGAKDSVASTIT | 660 |
| | Key | XXXXXXKXXVASXIX | 661 |
| | Key | AVTTTVTGA | 662 |
| | Preferable | KDSVASTI | 663 |
| | Key | KXXVASXI | 664 |
| P16 | More preferable | XXXXGXKDSVAXXXX | 665 |
| | Preferable | XXXXXXKDSVAXXXX | 666 |
| | Key | XXXXXXKDSXAXXXX | 667 |
| | Preferable | KDSVA | 668 |
| | Key | KDSV | 669 |
| P17 | Preferable | TXXTGAKXSXXXXXX | 670 |
| | Key | TXXTGXKXSXXXXXX | 671 |
| | Preferable | GAKDSVA | 672 |
| | Key | GAKXSXX | 673 |

**[Table 2-17]**

| E17 | | | |
|---|---|---|---|
| Spot No. 6 | | | |
| Protein name | Perilipin | | SEQ ID NO: |
| 15-residue sequence | NQKIQDAQDKLYLSW | | 674 |
| P2 | Key | NQKIQDAQDKLYLSW | 675 |
| | Key | AQDKLYLSWL | 676 |
| P3 | Key | NQKIQDAQDKLYLSW | 677 |
| P5 | More preferable | NQKIQDAQDKLYLSW | 678 |
| | Preferable | XQKXXXXXXKLYLSW | 679 |
| | Key | XQKXXXXXXXLYLSW | 680 |
| | More preferable | AQDKLYLSWL | 681 |
| | Preferable | XXXKLYLSWL | 682 |
| | Key | XXXXLYLSWL | 683 |
| P8 | Key | NQKIQDAQDKLYLSW | 684 |
| P9 | Key | NQKIQDAQDKLYLSW | 685 |
| | Key | DKLYLSWLEW | 686 |
| P16 | Key | NQKIQXXXXXXXXXX | 687 |
| P21 | More preferable | NQKIQXXQXKLYLSX | 688 |
| | Preferable | XQKIQXXXXKLYLSX | 689 |
| | Key | XQKIQXXXXXXYLXX | 690 |
| | More preferable | QDKLYLSW | 691 |
| | Preferable | QXKLYLSX | 692 |
| | Key | XXKLYLSX | 693 |

**[Table 2-18]**

| E18 | | | | | | | |
|---|---|---|---|---|---|---|---|
| Spot No. 12 | | | | | | | |
| Protein name | Zinc-alpha-2-glycoprotein | | SEQ ID NO: | Cross-reactivity-confirmed food | Graph No. | Synthesized sequence | SEQ ID NO: |
| 15-residue sequence | EDWEKESALQRARED | | 694 | | | | |
| P1 | More preferable | EDWEKESALQRARED | 695 | | | | |
| | Preferable | XXWEKEXXLQXXXED | 696 | | | | |
| | Key | XXWEKEXXXXXXXXX | 697 | | | | |
| | More preferable | WEKESAL | 698 | | 3 | WEKESAL | 698 |
| | Preferable | WEKEXXL | 699 | Sheep | 3 | WEKEMKL | 1129 |
| | Key | WEKEXXX | 700 | | | | |
| | Preferable | EKESAL | 701 | | | | |
| | Key | EKEXXL | 702 | | | | |
| P2 | Preferable | EDWEKESALQRARED | 703 | | | | |
| | Key | XXWEKEXXXXXXXXX | 704 | | | | |
| | Most preferable | MEDWEKESAL | 705 | | | | |
| | Still more preferable | DWEKESALQR | 706 | | | | |
| | More preferable | DWEKESAL | 707 | | | | |
| | Preferable | DWEKESA | 708 | | | | |
| | Key | XWEKEXX | 709 | | | | |
| P3 | Key | EDWEKESALQRARED | 710 | | | | |
| | Key | WEKE | 711 | | | | |
| P4 | Key | EDWEKESALQRARED | 712 | | | | |
| | Still more preferable | DWEKESAL | 713 | | | | |
| | More preferable | DWEKESAL | 714 | | | | |
| | Preferable | WEKES | 715 | | | | |
| | Key | WEKE | 716 | | | | |
| P5 | Preferable | EDWEKESALQRARED | 717 | | | | |
| | Key | XXXXKEXALXRXRXD | 718 | | | | |
| | Preferable | WEKESAL | 719 | | | | |
| | Key | WEKES | 720 | | | | |
| | Preferable | KESALQRARE | 721 | | | | |
| | Key | KEXALXRXRX | 722 | | | | |
| P8 | Key | EDWEKESALQRARED | 723 | | | | |
| | Preferable | WEKESA | 724 | | | | |
| | Key | WEKE | 725 | | | | |
| | Key | KESALQRARE | 726 | | | | |
| P10 | Key | EDWEKESALQRARED | 727 | | | | |
| | Key | MEDWEKE | 728 | | | | |
| | Key | KESALQR | 729 | | | | |
| | Key | LGRAREDIFM | 730 | | | | |
| P12 | Key | EDWEKESALQRARED | 731 | | | | |
| | Key | WEKE | 732 | | | | |
| P16 | Key | XXXXKEXAXXRXRXD | 733 | | | | |
| | More preferable | DWEKESALQR | 734 | | | | |
| | Preferable | WEKESALQR | 735 | | | | |
| | Key | WEKESAL | 736 | | | | |
| | Preferable | KESALQRARE | 737 | | | | |
| | Key | KEXAXXRXRX | 738 | | | | |
| P17 | Preferable | WEKESAL | 739 | | | | |
| | Key | EKESAL | 740 | | | | |
| P20 | Preferable | XXWEKEXXXXXXRED | 741 | | | | |
| | Key | XXXXKXXXXXXXRED | 742 | | | | |
| | Preferable | WEKESALQR | 743 | | | | |
| | Key | KESAL | 744 | | | | |
| P22 | Key | XXWEKEXXXXXXRXX | 745 | | | | |
| | Preferable | DWEKESALQR | 746 | | | | |
| | Key | XWEKEXXXXX | 747 | | | | |
| P23 | Key | DWEKESALQR | 748 | | | | |
| | Key | ESALQRAR | 749 | | | | |
| P24 | Preferable | WEKES | 750 | | | | |
| | Key | WEKE | 751 | | | | |
| P25 | Preferable | XXWEKEXAXXXXRXX | 752 | | | | |
| | Key | XXWEKEXXXXXXXXX | 753 | | | | |
| | Still more preferable | WEKESALQR | 754 | | | | |
| | More preferable | WEKESAL | 755 | | | | |
| | Preferable | WEKEXAX | 756 | | | | |
| | Key | WEKEXXX | 757 | | | | |

**[Table 2-19]**

| E19 | | | |
|---|---|---|---|
| Spot No. 12 | | | |
| Protein name | Zinc-alpha-2-glycoprotein | | SEQ ID NO: |
| 15-residue sequence | LSDIMDYYKDREGSH | | 758 |
| P1 | Key | LSDIMDYYKDREGSH | 759 |
| | Key | KDREGS | 760 |
| P2 | More preferable | LSDIMDYYKDREGSH | 761 |
| | Preferable | XXXXXXXXKXREGSH | 762 |
| | Key | XXXXXXXXKXXEGSH | 763 |
| | More preferable | KDREGS | 764 |
| | Preferable | KDREG | 765 |
| | Key | KXREG | 766 |
| | Key | DREG | 767 |
| P3 | Preferable | LSDIMDYYKDREGSH | 768 |
| | Key | XXXXXXXXKXREGXX | 769 |
| | Preferable | KDREG | 770 |
| | Key | KXREG | 771 |
| | Key | DREG | 772 |
| P4 | Key | LSDIMDYYKDREGSH | 773 |
| | Key | KDREGS | 774 |
| P8 | Key | LSDIMDYYKDREGSH | 775 |
| | Key | DREG | 776 |
| P16 | More preferable | KDREGS | 777 |
| | Preferable | KDREG | 778 |
| | Key | DREG | 779 |
| P17 | Preferable | KDREGS | 780 |
| | Key | KDRE | 781 |
| P20 | Key | KDREG | 782 |
| P21 | Key | XXXXXXYYKDXXXXX | 783 |
| P22 | Key | KDREG | 784 |
| P23 | Preferable | XXXXXXXXKDREGSH | 785 |
| | Key | XXXXXXXXKXXEGSH | 786 |
| | More preferable | KDREGS | 787 |
| | Preferable | KDREG | 788 |
| | Key | DREG | 789 |
| P24 | Key | XXXXXXXXKDXEGXX | 790 |
| | Key | DREG | 791 |

**[Table 2-20]**

| E20 Spot No. 10 | | | | | | | |
|---|---|---|---|---|---|---|---|
| Protein name | Glycoprotein 2 | | SEQ ID NO | Cross-reactivity-confirmed food | Graph No. | Synthesized sequence | SEQ ID NO: |
| 15-residue sequence | DPATAEDKCDRTCRP | | 792 | | | | |
| P1 | Preferable | DPATAEDKCDRTCRP | 793 | | | | |
| | Key | XXATAEDKCDRTCRP | 794 | | | | |
| | Key | ATAEDKCDRT | 795 | | | | |
| | Key | AEDKCDRTCR | 796 | | | | |
| P2 | Still more preferable | DPATAEDKCDRTCRP | 797 | | | | |
| | More preferable | DPATAEDKCDRTXRP | 798 | | | | |
| | Preferable | XXATAEDKCDRXXXP | 799 | | | | |
| | Key | XXATAEDKCDRXXXX | 800 | | | | |
| | Key | EDKC | 801 | | | | |
| P3 | More preferable | DPATAEDKCDRTCRP | 802 | | | | |
| | Preferable | XXATXEDKCDRTXXX | 803 | | | | |
| | Key | XXXXXXDKXDRXXXX | 804 | | | | |
| P4 | More preferable | DPATAEDKCDRTCRP | 805 | | | | |
| | Preferable | DPATXXDKCDRTCRP | 806 | | | | |
| | Key | XPATXXDKCDRTCRP | 807 | | | | |
| | More preferable | AEDKCDRT | 808 | | | | |
| | Preferable | AEDKCDRT | 809 | | | | |
| | Key | EDKCDR | 810 | | | | |
| P5 | Still more preferable | DPATAEDKCDRTCRP | 811 | | | | |
| | More preferable | DPATAEDKCDRTXRP | 812 | | | | |
| | Preferable | XPATAEDKCDRTXXX | 813 | | | | |
| | Key | XPATXXDKCDRXXXX | 814 | | | | |
| | More preferable | RYCTDPATAE | 815 | | | | |
| | Preferable | RYCTXPATAE | 816 | | | | |
| | Key | RYCTXPATXX | 817 | | | | |
| | Preferable | AEDKCDRTCR | 818 | | 4 | AEDKCDRTCR | 81a |
| | Key | XXDKCDRXXX | 819 | Sheep | 4 | LNDKCDRLRK | 1130 |
| P8 | More preferable | DPATAEDKCDRTCRP | 820 | | | | |
| | Preferable | XXXTXEDKXDRTXXX | 821 | | | | |
| | Key | XXXXXXDKXDRXXXX | 822 | | | | |
| | Key | EDKCD | 823 | | | | |
| P10 | Key | DPATAEDKCDRTCRP | 824 | | | | |
| | Key | YCTDPATAE | 825 | | | | |
| | Key | TDPATAEDKC | 826 | | | | |
| | Key | ATAEDKCDR | 827 | | | | |
| P12 | Preferable | DPATAEDKCDRTCRP | 828 | | | | |
| | Key | XXXXXXDKXDXTCXX | 829 | | | | |
| | Key | EDKCD | 830 | | | | |
| P16 | Preferable | XXXTAEDKCDRTXXX | 831 | | | | |
| | Key | XXXXXXDKCDRXXXX | 832 | | | | |
| | Key | AEDKC | 833 | | | | |
| P17 | Preferable | DXXTAXDKCDRXXXX | 834 | | | | |
| | Key | XXXTXXDKXXRXXXX | 835 | | | | |
| | Preferable | DPATAEDKCD | 836 | | | | |
| | Key | DXXTAXDKCD | 837 | | | | |
| | More preferable | AEDKCDRTC | 838 | | | | |
| | Preferable | AEDKCDRT | 839 | | | | |
| | Key | AXDKCDRX | 840 | | | | |
| P18 | Preferable | XXXXXXDKCDRTXRP | 841 | | | | |
| | Key | XXXXXXDKCBRTXXX | 842 | | | | |
| | Still more preferable | ATAEDKCDR | 843 | | | | |
| | More preferable | EDKCDRTCR | 844 | | | | |
| | Preferable | EDKCDR | 845 | | | | |
| | Key | XDKCDR | 846 | | | | |
| P20 | Preferable | XPATAEDKXXXXXXX | 847 | | | | |
| | Key | XPAXXXDKXXXXXXX | 848 | | | | |
| P21 | Preferable | XXXTAXXXCDRXXXX | 849 | | | | |
| | Key | XXXTAXXXCDRXXXX | 850 | | | | |
| | Preferable | AEDKCDRTC | 851 | | | | |
| | Key | AEDKCD | 852 | | | | |
| P23 | More preferable | XXXXAXDKCDRXXXX | 853 | | | | |
| | Preferable | XXXTAXXXCDRXXXX | 854 | | | | |
| | Key | XXXXXXDKXDRXXXX | 855 | | | | |
| | Still more preferable | AEDKCDR | 856 | | | | |
| | More preferable | AEDKCD | 857 | | | | |
| | Preferable | AXDKCD | 858 | | | | |
| | Key | XXDKCD | 859 | | | | |
| P25 | More preferable | DXXTAEDKCDRTCRP | 860 | | | | |
| | Preferable | XXXTXEDKCDRTXRP | 861 | | | | |
| | Key | XXXTXXDKCDRTXRP | 862 | | | | |
| | Most preferable | PATAEDKCDR | 863 | | | | |
| | Even more preferable | AEDKCDRTC | 864 | | | | |
| | Still more preferable | AEDKCDR | 865 | | | | |
| | More preferable | AEDKCD | 866 | | | | |
| | Preferable | XEDKCD | 867 | | | | |
| | Key | XXDKCD | 868 | | | | |

**[Table 2-21]**

| E21 Spot No. 10 | | | |
|---|---|---|---|
| Protein name | Glycoprotein 2 | | SEQ ID NO: |
| 15-residue sequence | TEDIQGCDSDKHGWY | | 869 |
| P1 | More preferable | TEDIQGCDSDKHGWY | 870 |
| | Preferable | TXDIQXXDXDKHXXY | 871 |
| | Key | TXDIQXXDXDKXXXY | 872 |
| | More preferable | IQGCDSDKHG | 873 |
| | Preferable | IQXXDXDKHX | 874 |
| | Key | IQXXDXDKXX | 875 |
| | More preferable | DSDKHGWYR | 876 |
| | Preferable | DXDKHXXYR | 877 |
| | Key | DXDKXXXYR | 878 |
| | More preferable | DKHGWYRFVG | 879 |
| | Preferable | DKHXXYRFVG | 880 |
| | Key | DKXXXYRFVG | 881 |
| P2 | Preferable | TEDIQGCDSDKHGWY | 882 |
| | Key | TXDIQXXXXXXXXXX | 883 |
| | Preferable | ENTEDIQG | 884 |
| | Key | ENTXDIQX | 885 |
| | Key | IQGCDSDKHG | 886 |
| | Key | DKHGWY | 887 |
| P3 | Key | TEDIQGCDSDKHGWY | 888 |
| | Key | ENTEDIQGCD | 889 |
| P4 | Key | TEDIQGCDSDKHGWY | 890 |
| | Key | DSDK | 891 |
| | Key | DKHGWYRFVG | 892 |
| P8 | Key | TEDIQGCDSDKHGWY | 893 |
| | Key | NIEDIQG | 894 |
| P9 | Key | TEDIQGCDSDKHGWY | 895 |
| | Key | STENTEDIQG | 896 |
| | More preferable | EDIQGCDSDK | 897 |
| | Preferable | IQGCDSDKHG | 898 |
| | Key | IQGCDSDK | 899 |
| | Key | DKHGWYR | 900 |
| | Key | DKHGWY | 901 |
| P10 | Key | TEDIQGCDSDKHGWY | 902 |
| | Key | IQGCDSDKHG | 903 |
| | Key | DKHGWY | 904 |
| P12 | Key | TEDIQGCDSDKHGWY | 905 |
| P13 | More preferable | TEDIQGCDSDKHGWY | 906 |
| | Preferable | XXDXQXXXXXKXGWY | 907 |
| | Key | XXXXQXXXXXXXGWY | 908 |
| | Preferable | DKHGWY | 909 |
| | Key | XKXGWY | 910 |
| P16 | Key | XXDIQXXXXXKXXXX | 911 |
| | Preferable | IQGCDSDKHG | 912 |
| | Key | IQGCDSDKH | 913 |
| | Key | DKHGWY | 914 |
| P17 | Preferable | DKHGWY | 915 |
| | Key | DKHG | 916 |
| P20 | More preferable | EDIQGCDSDK | 917 |
| | Preferable | IQGCDSDKHG | 918 |
| | Key | IQGCDSDK | 919 |
| | Key | DKHGWY | 920 |
| P23 | More preferable | IQGCDSDKHG | 921 |
| | Preferable | SDKHGWY | 922 |
| | Key | SDKH | 923 |
| P25 | Preferable | XXDIQXCDXDKHGWY | 924 |
| | Key | XXDXQXXXXDKXXXX | 925 |
| | Preferable | IQGCDSDKHG | 926 |
| | Key | DKHGWY | 927 |

**[Table 2-22]**

| E22 Spot No. 11 | | | |
|---|---|---|---|
| Protein name | Lactadher in | | SEQ ID NO: |
| 15-residue sequence | SESKIFPGNMDNNSH | | 928 |
| P1 | Key | SESKIFPGNMDNNSH | 929 |
| | Key | ESKIF | 930 |
| | Preferable | NMDNNSHKKN | 931 |
| | Key | MDNNSHKK | 932 |
| P2 | Key | SESKIFPGNMDNNSH | 933 |
| | Preferable | ESKIFPGNM | 934 |
| | Key | ESKIFPGN | 935 |
| | More preferable | NMDNNSHKK | 936 |
| | Preferable | MDNNSHKK | 937 |
| | Key | MDNNS | 938 |
| P3 | Key | SESKIFPGNMDNNSH | 939 |
| | Key | SKIF | 940 |
| | Key | NMDNNSHKKN | 941 |
| P4 | Preferable | SESKIFPGNMDNNSH | 942 |
| | Key | SESKXXXXXXXXNXX | 943 |
| | Key | SESKIF | 944 |
| | Key | SKIFPGNMDN | 945 |
| | More preferable | MDNNSHK | 946 |
| | Preferable | MDNNSH | 947 |
| | Key | MDNNS | 948 |
| P5 | Preferable | SESKIFPGNMDNNSH | 949 |
| | Key | XXXXXFPGNMDNNSH | 950 |
| | Preferable | ESKIFPGNM | 951 |
| | Key | XXXXFPGNM | 952 |
| | Key | FPGNMDNNSH | 953 |
| | Preferable | NMDNNSHKK | 954 |
| | Key | MDNNSH | 955 |
| P8 | Key | SESKIFPGNMDNNSH | 956 |
| | Preferable | ESKIF | 957 |
| | Key | SKIF | 958 |
| P10 | Key | SESKIFPGNMDNNSH | 959 |
| P11 | Key | SESKIFPGNMDNNSH | 960 |
| | Key | ASESKIF | 961 |
| | Key | MDNNS | 962 |
| P12 | Key | SESKIFPGNMDNNSH | 963 |
| | Key | ESKIF | 964 |
| P14 | Key | SESKIFPXXXXXXXX | 965 |
| | Key | ESKIF | 966 |
| P16 | Preferable | ESKIFPGN | 967 |
| | Key | ESKIF | 968 |
| P17 | Key | XEXKXXXXXXXXXSH | 969 |
| | Preferable | ESKIFPG | 970 |
| | Key | ESKIF | 971 |
| | Preferable | MDNNSH | 972 |
| | Key | MDNNS | 973 |
| P19 | Key | XXXXXFXGNMDNNSH | 974 |
| P20 | Key | ESKIF | 975 |
| | Key | MDNNSH | 976 |
| P21 | Preferable | XXXXXXXXNMDNNSH | 977 |
| | Key | XXXXXXXXNMDXNSH | 978 |
| | Key | SESKIFPGNM | 979 |
| | Preferable | SKIFPGNMDN | 980 |
| | Key | XXXXXXNMDN | 981 |
| | Preferable | MDNNS | 982 |
| | Key | MDXNS | 983 |
| P23 | More preferable | ESKIFPGNM | 984 |
| | Preferable | SKIFPGNM | 985 |
| | Key | SKIF | 986 |
| | Key | MDNNS | 987 |
| P24 | Preferable | ESKIFPG | 988 |
| | Key | ESKIF | 989 |
| P25 | Preferable | SESKIFPXXXXXXSX | 990 |
| | Key | SXXKIFXXXXXXXXX | 991 |
| | More preferable | SESKIFPGNM | 992 |
| | Preferable | SESKIFPXXX | 993 |
| | Key | SXXKIFXXXX | 994 |
| | Preferable | SKIFPGNMDN | 995 |
| | Key | SKIFPXXXXX | 996 |

**[Table 2-23]**

| E23 Spot No. 9 | | | |
|---|---|---|---|
| Protein name | Immunoglobulin light chain, lambda gene cluster | | SEQ ID NO: |
| 15-residue sequence | QPKSPPSVTLFPPST | | 997 |
| P2 | Still more preferable | QPKSPPSVTLFPPST | 998 |
| | More preferable | XXKSXXSXTLFXPST | 999 |
| | Preferable | XXKXXXSXTLFXPSX | 1000 |
| | Key | XXKXXXXXXLFXPSX | 1001 |
| | Preferable | KSPPSV | 1002 |
| | Key | SPPSV | 1003 |
| | Still more preferable | SVTLFPPSTE | 1004 |
| | More preferable | SXTLFXPSTE | 1005 |
| | Preferable | SXTLFXPSXE | 1006 |
| | Key | XXXLFXPSXE | 1007 |
| P3 | Key | QPKSPPSVTLFPPST | 1008 |
| | Key | VLGQPKSPPS | 1009 |
| | Key | GQPKSPPSVT | 1010 |
| | Preferable | KSPPSVTLF | 1011 |
| | Key | SPPS | 1012 |
| | Key | LFPPSTE | 1013 |
| P4 | Preferable | QPKSPPSVTLFPPST | 1014 |
| | Key | QXKSXPSXXXXXXXX | 1015 |
| | Preferable | KSPPS | 1016 |
| | Key | KSXPS | 1017 |
| P10 | More preferable | QPKSPPSVTLFPPST | 1018 |
| | Preferable | XXKSXXSVXLFXXXX | 1019 |
| | Key | XXKSXXXVXLFXXXX | 1020 |
| | Key | TVLGQPKSPP | 1021 |
| | Key | SPPSV | 1022 |
| | Key | SVTLFPPSTE | 1023 |
| | Key | LFPPSTEELN | 1024 |
| P12 | Key | QPKSPPSVTLFPPST | 1025 |
| | Key | VLGQPKSPP | 1026 |
| | More preferable | KSPPSVTL | 1027 |
| | Preferable | KSPPSVT | 1028 |
| | Key | KSPP | 1029 |
| | Preferable | LFPPSTEELN | 1030 |
| | Key | LFPPSTEE | 1031 |
| P21 | Preferable | QPKSXXXXXXFPPST | 1032 |
| | Key | XXKSXXXXXXXXPST | 1033 |
| | Key | SPPS | 1034 |
| P23 | Key | XXKSXXSXXXFXXXX | 1035 |
| | Key | SPPS | 1036 |
| | Key | SVTLFPPST | 1037 |

**[Table 2-24]**

| E24 Spot No. 13 | | | |
|---|---|---|---|
| Protein name | intracellular cholesterol transporter 2 | | SEQ ID NO: |
| 15-residue sequence | GIPVPFPIPESDGCK | | 1038 |
| P2 | More preferable | GIPVPFPIPESDGCK | 1039 |
| | Preferable | XXPVPFPIXESDGXK | 1040 |
| | Key | XXXVPFPXXESDXXX | 1041 |
| | Preferable | ESDGCK | 1042 |
| | Key | ESDGXK | 1043 |
| P4 | Key | GIPVPFPIPESDGCK | 1044 |
| | Key | ESDGCK | 1045 |
| P10 | Key | GIPVPFPIPESDGCK | 1046 |
| | Preferable | IVMGIPVPF | 1047 |
| | Key | IPVPF | 1048 |
| | Preferable | ESDGCK | 1049 |
| | Key | ESDG | 1050 |
| P12 | Key | GIPVPFPIPESDGCK | 1051 |
| | Key | ESDGCK | 1052 |
| P13 | Preferable | GIPVPFPIPESDGCK | 1053 |
| | Key | XXXXXXXXXESDGXK | 1054 |
| | Preferable | ESDGCK | 1055 |
| | Key | ESDGXK | 1056 |
| P21 | Preferable | GXXXXFPIPESXXXK | 1057 |
| | Key | XXXXXFXXPESXXXK | 1058 |
| | More preferable | IPVPFPIPES | 1059 |
| | Preferable | XXXXFPIPES | 1060 |
| | Key | XXXXFXXPES | 1061 |
| | Preferable | ESDGCK | 1062 |
| | Key | ESDG | 1063 |
| P23 | More preferable | GIPVPFPIP | 1064 |
| | Preferable | IPVPFPIP | 1065 |
| | Key | IPVPFPI | 1066 |
| | Key | FPIPESDGCK | 1067 |
| P24 | Preferable | XXXXXXXXXXSDGCK | 1068 |
| | Key | XXXXXXXXXXSDGXK | 1069 |
| | More preferable | ESDGCK | 1070 |
| | Preferable | XSDGCK | 1071 |
| | Key | XSDGXK | 1072 |
| P25 | Key | XXXXXXXXXESDXCK | 1073 |
| | Preferable | ESDGCK | 1074 |
| | Key | ESDXCK | 1075 |

**[Table 2-25]**

| E25 Spot No. 14 | | | | | | | |
|---|---|---|---|---|---|---|---|
| Protein name | Apol ipoprotein A-IV | | SEQ ID NO: | Cross-reactivity-confirmed food | Graph No. | Synthesized sequence | SEQ ID NO: |
| 15-residue sequence | LTKDSEKLKEEIRKE | | 1076 | | | | |
| P1 | Preferable | LTKDSEKLKEEIRKE | 1077 | | | | |
| | Key | XXXXXXKLKEEXRXE | 1078 | | | | |
| | Key | KEEI | 1079 | | | | |
| P2 | Key | LTKDSEKLKEEIRKE | 1080 | | | | |
| | Key | KDSE | 1081 | | | | |
| | Key | KLKEEIRKE | 1082 | | | | |
| | Key | KEEIRKELED | 1083 | | | | |
| P3 | Preferable | LTKDSEKLKEEIRKE | 1084 | | | | |
| | Key | XXXDXEXXKXXXRXE | 1085 | | | | |
| P8 | Preferable | LTKDSEKLKEEIRKE | 1086 | | | | |
| | Key | XXXXXEKLKXXXRKE | 1087 | | | | |
| | Key | ERLTKDSE | 1088 | | | | |
| | Preferable | LTKDSEKLKE | 1089 | | | | |
| | Key | XXXXXEKLKX | 1090 | | | | |
| | Preferable | LKEEIRK | 1091 | | | | |
| | Key | LKXXXRK | 1092 | | | | |
| P9 | Key | LTKDSEKLKEEIRKE | 1093 | | | | |
| | Key | RLTKDSEKLK | 1094 | | | | |
| | Key | KLKEEI | 1095 | | | | |
| P16 | Preferable | XXKDSEKLKEEXXXX | 1096 | | | | |
| | Key | XXKDXXXLKEEXXXX | 1097 | | | | |
| | Key | KDSE | 1098 | | | | |
| | More preferable | KLKEEIRKE | 1099 | | | | |
| | Preferable | KLKEEXXXX | 1100 | | | | |
| | Key | XLKEEXXXX | 1101 | | | | |
| P17 | Key | LTKDSE | 1102 | | | | |
| | More preferable | KDSEKLKEEI | 1103 | | | | |
| | Preferable | KLKEEIRKE | 1104 | | | | |
| | Key | KLKEEI | 1105 | | | | |
| P20 | Preferable | XTXDXEKLKEEXRKE | 1106 | | | | |
| | Key | XXXDXXKLKEXXRKX | 1107 | | | | |
| | Still more preferable | EKLKEEIR | 1108 | | | | |
| | More preferable | EKLKEE | 1109 | | | | |
| | Preferable | KLKEE | 1110 | Sheep | 6 | KLKEE | 1110 |
| | Key | KLKEX | 1111 | Chicken | 6 | KLKER | 1131 |
| P22 | Key | LTXXSXXXKEEIXXE | 1112 | | | | |
| | Preferable | KEEIR | 1113 | | | | |
| | Key | KEEI | 1114 | | | | |

### [Table 2-26]

**Table 2-26**

| | Food with cross-reactivity (BLAST target food) | | | | | |
|---|---|---|---|---|---|---|
| Patient No. | Chicken egg | Wheat | Peanut | Shrimp | Buckwheat | Sheep milk |
| 1 | | | | | | ● |
| 2 | ● | ● | ● | ● | ● | ● |
| 3 | ● | ● | ● | | ● | ● |
| 4 | ● | ● | | | | ● |
| 5 | ● | | | | | ● |
| 6 | ● | | | | | ● |
| 7 | ● | | | | | ● |
| 8 | | | | | | ● |
| 9 | | | | | | ● |
| 10 | ● | | | | | ● |
| 11 | ● | | | | | ● |
| 12 | | | | | | ● |
| 13 | ● | | | | | ● |
| 14 | ● | | ● | ● | ● | ● |
| 15 | | | ● | | | ● |
| 16 | ● | | | | | ● |
| 17 | ● | | | | | ● |
| 18 | | ● | | | | ● |
| 19 | ● | ● | | | | ● |
| 20 | ● | | | | | ● |
| 21 | ● | ● | | ● | | ● |
| 22 | | | | | | ● |
| 23 | | ● | | | | ● |
| 24 | | | | | | ● |
| 25 | ● | | | | | ● |

| | | | | | | |
|---|---|---|---|---|---|---|
| ● : Allergic food | | | | | | |

Table 2-1 to Table 2-25 show the specific sequences identified as epitopes that each bind to an IgE antibody in Example 4, and the sequences for which epitope cross-reactivity was confirmed in Example 5. Table 2-26 shows an example of foodstuffs to which 25 patients are allergic, and is a table summarizing the cases analyzed for epitope cross-reactivity in Example 5.

The invention provides a polypeptide comprising each amino acid sequence of (E1) to (E25) which comprises or consists of each of amino acid sequences of SEQ ID NOs: 27 to 1131 listed in Table 2 as a polypeptide comprising an amino acid sequence that specifically binds to an IgE antibody from an allergic patient. (E1) to (E25) are each an amino acid sequence that binds to an IgE antibody and is derived from one of the proteins listed in "Protein name" in Table 2 (hereinafter, sometimes referred to as an "epitope").
(1) (E1), (E2): derived from an immunoglobulin M heavy chain secretory form protein;
(2) (E3), (E4), (E5), (E6), (E7), (E8) derived from a beta-1 metal-binding globulin protein;
(3) (E9), (E10): derived from a lactoperoxidase protein;
(4) (E14), (E15): derived from a an Ig heavy chain precursor (B/MT.4A.17. H5. A5) protein;
(5) (E16), (E17): derived from a perilipin protein;
(6) (E11), (E12): derived from a polymeric immunoglobulin receptor protein;
(7) (E13): derived from a protein: (7) C3-beta-c;
(8) (E23): derived from an immunoglobulin light chain, lambda gene cluster protein;
(9) (E20), (E21): derived from a glycoprotein 2 protein;
(10) (E22): derived from a lactadherin protein;
(11) (E18), (E19): derived from a zinc-alpha-2-glycoprotein protein;
(12) (E24): derived from an intracellular cholesterol transporter 2 protein; and
(13) (E25): derived from an apolipoprotein A-IV protein.

The epitope antigen of the invention is not limited, and preferably includes at least one of the following polypeptides.
(E1) A polypeptide comprising at least one amino acid sequence selected from amino acid sequences set forth in SEQ ID NOs: 27 to 58 and 1115;
(E2) a polypeptide comprising at least one amino acid sequence selected from amino acid sequences set forth in SEQ ID NOs: 59 to 97;
(E3) a polypeptide comprising at least one amino acid sequence selected from amino acid sequences set forth in SEQ ID NOs: 98 to 144;
(E4) a polypeptide comprising at least one amino acid sequence selected from amino acid sequences set forth in SEQ ID NOs: 145 to 195;
(E5) a polypeptide comprising at least one amino acid sequence selected from amino acid sequences set forth in SEQ ID NOs: 196 to 254 and 1116 to 1118;
(E6) a polypeptide comprising at least one amino acid sequence selected from amino acid sequences set forth in SEQ ID NOs: 255 to 288;
(E7) a polypeptide comprising at least one amino acid sequence selected from amino acid sequences set forth in SEQ ID NOs: 289 to 333 and 1119 to 1120;
(E8) a polypeptide comprising at least one amino acid sequence selected from amino acid sequences set forth in SEQ ID NOs: 334 to 352;
(E9) a polypeptide comprising at least one amino acid sequence selected from amino acid sequences set forth in SEQ ID NOs: 353 to 376 and 1121 to 1122;
(E10) a polypeptide comprising at least one amino acid sequence selected from amino acid sequences set forth in SEQ ID NOs: 377 to 384;
(E11) a polypeptide comprising at least one amino acid sequence selected from amino acid sequences set forth in SEQ ID NOs:385 to 411;
(E12) a polypeptide comprising at least one amino acid sequence selected from amino acid sequences set forth in SEQ ID NOs: 412 to 450;
(E13) a polypeptide comprising at least one amino acid sequence selected from amino acid sequences set forth in SEQ ID NOs: 451 to 500 and 1123 to 1124;
(E14) a polypeptide comprising at least one amino acid sequence selected from amino acid sequences set forth in SEQ ID NOs: 501 to 604 and 1125 to 1127;
(E15) a polypeptide comprising at least one amino acid sequence selected from amino acid sequences set forth in SEQ ID NOs: 605 to 632 and 1128;
(E16) a polypeptide comprising at least one amino acid sequence selected from amino acid sequences set forth in SEQ ID NOs: 633 to 673;
(E17) a polypeptide comprising at least one amino acid sequence selected from amino acid sequences set forth in SEQ ID NOs: 674 to 693;
(E18) a polypeptide comprising at least one amino acid sequence selected from amino acid sequences set forth in SEQ ID NOs: 694 to 757 and 1129;
(E19) a polypeptide comprising at least one amino acid sequence selected from amino acid sequences set forth in SEQ ID NOs: 758 to 791;
(E20) a polypeptide comprising at least one amino acid sequence selected from amino acid sequences set forth in SEQ ID NOs: 792 to 868 and 1130;
(E21) a polypeptide comprising at least one amino acid sequence selected from amino acid sequences set forth in SEQ ID NOs: 869 to 927;
(E22) a polypeptide comprising at least one amino acid sequence selected from amino acid sequences set forth in SEQ ID NOs: 928 to 996;
(E23) a polypeptide comprising at least one amino acid sequence selected from amino acid sequences set forth in SEQ ID NOs: 997 to 1037;
(E24) a polypeptide comprising at least one amino acid sequence selected from amino acid sequences set forth in SEQ ID NOs: 1038 to 1075; or
(E25) a polypeptide comprising at least one amino acid sequence selected from amino acid sequences set forth in SEQ ID NOs:1076 to 1114 and 1131.

Polypeptides (E1) to (E25), which have been described as a preferred embodiment, have each a specific sequence identified as an epitope that binds to an IgE in Examples herein as listed in Table 2. The epitope antigens of the invention may include not only the polypeptides (E1) to (E25) of the above-described preferred embodiment, but also variants, etc., described below. Hereinbelow, forms (variants) that can be included in the epitope antigens of the invention will be described.

Here, 25 different sequences including SEQ ID NO:27, 59, 98, 145, 196, 255, 289, 334, 353, 377, 385, 412, 451, 501, 605, 633, 674, 694, 758, 792, 869, 928, 997, 1038, or 1076, which have been described in "15-residue sequence" of Table 2, are 15-common-amino-acid-residue sequences each identified, as an epitope that binds to an IgE antibody, in epitopes (E1) to (E-25), by epitope mapping based on overlapping. One embodiment of the invention provides polypeptides comprising or consisting of these amino acid sequences. The epitope sequence included in the polypeptide of the invention may be the entire or portion of the 15 common amino acid residues in the epitope. Each epitope sequence has 4 amino acid residues or more, 5 amino acid residues or more, 6 amino acid residues or more, 7 amino acid residues or more, 8 amino acid residues or more, 9 amino acid residues or more, 10 amino acid residues or more, 11 amino acid residues or more, 12 amino acid residues or more, 13 amino acid residues or more, or 14 amino acid residues or more.

In the Examples herein, for example, a large number of polypeptides consisting of four amino acid residues (e.g., SEQ ID NO: 34, 67, 116, 195, 229, 275) have been identified as an epitope that binds to an IgE antibody. In addition, the binding to an IgE antibody was confirmed in the case of five or more amino acid residues. Thus, the presence of at least four amino acid residues is useful as an epitope sequence.

According to an embodiment of the invention, examples of a polypeptide variant include a polypeptide comprising four or more amino acid residues in the amino acid sequence specifically described in any of the above (E1) to (E25). For instance, the (E1) variant may include a "polypeptide comprising four or more amino acid residues in at least one amino acid sequence among the amino acid sequences set forth in SEQ ID NOs: 27 to 58 and 1115". Preferred are 4 amino acid residues or more, 5 amino acid residues or more, 6 amino acid residues or more, 7 amino acid residues or more, 8 amino acid residues or more, 9 amino acid residues or more, 10 amino acid residues or more, 11 amino acid residues or more, 12 amino acid residues or more, 13 amino acid residues or more, or 14 amino acid residues or more in at least one amino acid sequence among the amino acid sequences set forth in SEQ ID NOs: 27 to 58 and 1115. The same applies to (E2) to (E25).

The "Preferable" sequence in Table 2 refers to a shorter partial sequence that can function as an epitope in the "15-residue sequence". The "More preferable" sequence refers to a sequence more preferable than the above shorter partial sequence in view of improving the binding to an IgE antibody. The "Key" sequence refers to a sequence that seems to be critical in the "15-residue sequence". In the "Key" sequence, the amino acid sequence denoted by "X" is an amino acid residue confirmed to have a binding ability to IgE antibodies which remains even after the amino acid residue is replaced with any alanine (glycine if the original amino acid residue is alanine) by alanine/glycine scanning. Thus, X can be any amino acid residue, preferably alanine (or glycine). Note that each sequence including no "X" denoted in the "Key" sequence is a case where no amino acid residues, the change of which still preserves the binding to an IgE antibody, have been found by alanine/glycine scanning.

For each of the epitopes listed in Table 2, each amino acid residue denoted by X is an amino acid residue, the change of which has been found to maintain the binding ability to an IgE antibody. The invention preferably allows one or more of the amino acid residues denoted as X in the "key" sequence corresponding to each "Preferable" sequence to be replaced by a given amino acid residue(s). For example, in one embodiment, in the preferable sequence SEQ ID NO: 27, there are multiple corresponding key sequences such as SEQ ID NOs: 28, 34, etc. Hereinafter, the same applies to the other "Preferable" sequences in (E1), the "key" sequences, or (E2) to (E25).

The number of amino acid residues that may be substituted is not limited, and is preferably 6 or less, 5 or less, 4 or less, 3 or less, 2 or less, or 1 or less. Hereinafter, the same applies to (E2) to (E25).

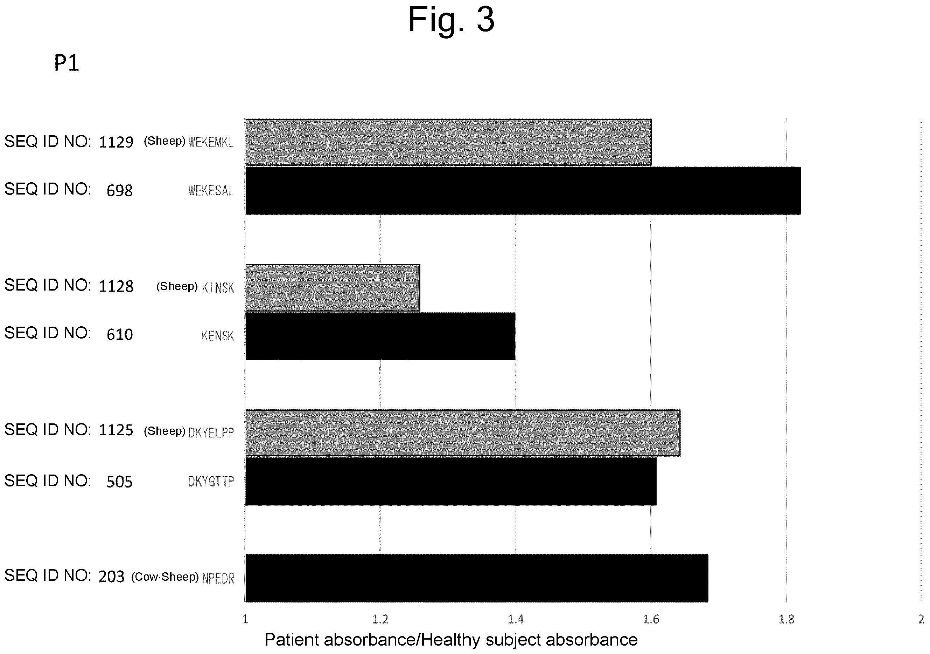

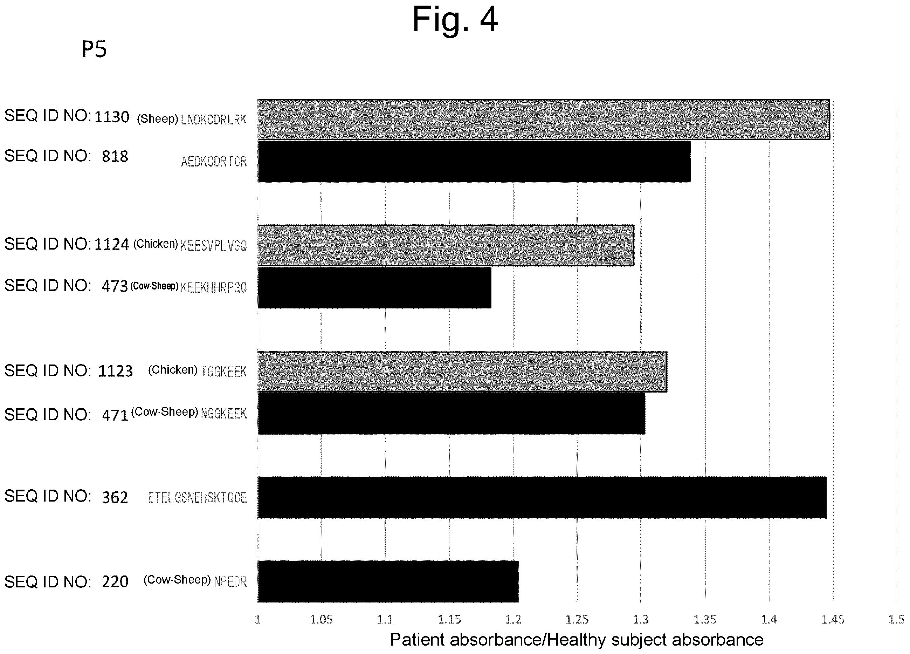

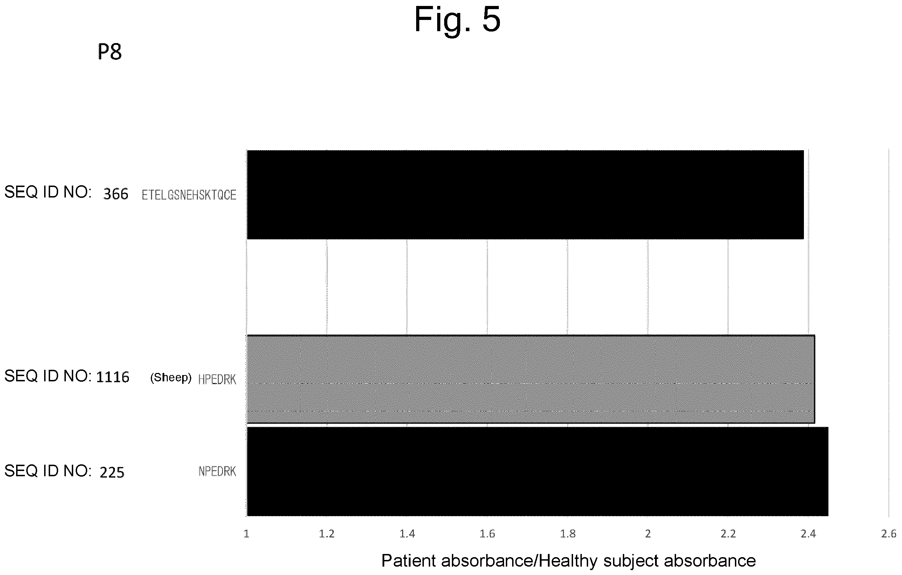

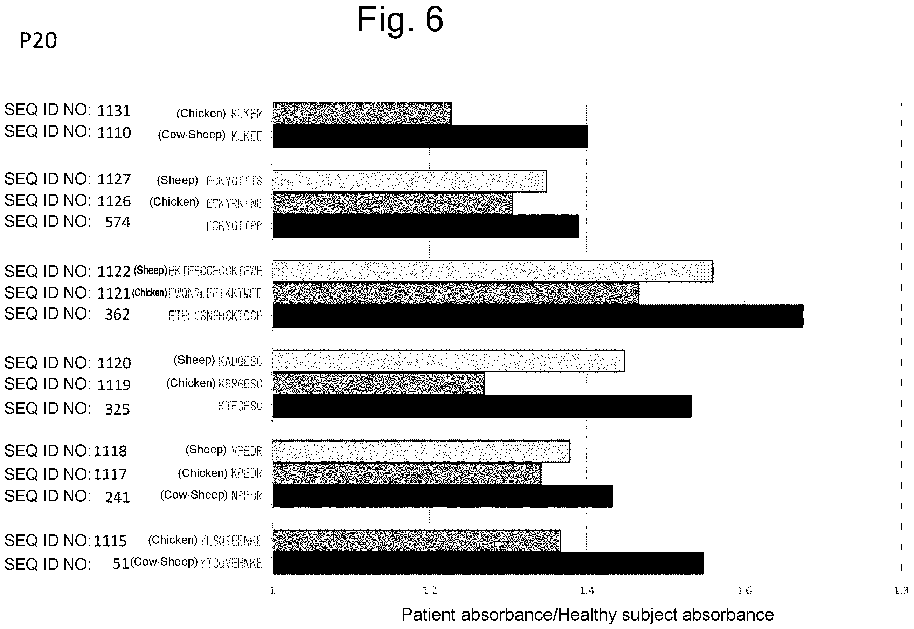

Each sequence listed in the "Synthesized sequence" and/or "SEQ ID NO:" column to the right of the Graph No. in Table 2 is the sequence for which the epitope cross-reactivity was found in Example 5. The wording "epitope cross-reactivity was found" means that an IgE antibody in serum of each allergic patient showed higher binding ability than IgE antibodies in serum of a non-allergic subject (specifically, larger than "1" in Figs. 3 to 6). Thus, in one embodiment, the polypeptides of the invention include these amino acid sequences, or polypeptides comprising these amino acid sequences. In one embodiment, the IgE antibody in serum of each allergic patient binds to the polypeptide in the invention by 1.05-fold, 1.10-fold, 1.15-fold, 1.20-fold, or 1.25-fold more than the IgE antibodies in serum of a non-allergic subject.

As used herein, the "polypeptide comprising any of the amino acid sequences of (E1) to (E25)" includes a polypeptide comprising or consisting of any of the amino acid sequences of the polypeptides (E1) to (E25) described above as a preferred embodiment, as well as each form (variant) in which an amino acid residue(s) is substituted as described above. The wording "comprising any of the amino acid sequences set forth in SEQ ID NOs: ..." means that any other amino acid sequence may be included as long as it does not affect the binding ability (i.e., the function as each epitope) of any of the amino acid sequences set forth in SEQ ID NOs: .... (including the above substituted forms) to an IgE antibody. A "polypeptide comprising any of the amino acid sequences set forth in (E1) to (E25)" may be a polypeptide in which two or more polypeptides comprising any of the amino acid sequences set forth in (E1) to (E25) are linked together with or without a spacer. The type of spacer is not particularly limited, and those routinely used by those skilled in the art for linking multiple peptides can be used. The spacer may be a polypeptide such as a hydrocarbon chain (e.g., Acp(6)-OH) or an amino acid chain.

Amino acid residues in the polypeptide other than those specifically identified can be optionally selected as long as they do not affect the binding to an IgE antibody (i.e., the function as an epitope). The amino acid residue(s) is not limited, and is preferably selected, if appropriate, from the sequence of the respective corresponding underlying epitope or the sequence of the underlying protein. For example, in SEQ ID NO: 34, only "HNKE" is specified, but if other amino acid residue(s) is to be added, they should be selected, if appropriate, from the base sequence SEQ ID NO: 27. Then, for the sequence listed in Table 2-1 as (E1), the amino acid residues of the sequence derived from the base protein (1) (corresponding to spot (1)) should be added.

The polypeptide comprising any of the amino acid sequences (E1)-(E25) above may be prepared by a chemical synthesis procedure such as solid phase peptide synthesis. Alternatively, each epitope-comprising polypeptide may be separated/produced by expressing the polypeptide as a recombinant polypeptide by a gene recombinant technology well-known to those skilled in the art and then performing a protein production procedure well-known to those skilled in the art. A polypeptide may be obtained by combining and linking two or more polypeptides, or may be obtained by repeatedly linking a single epitope. In this case, the binding ability to an Ig antibody is generally improved.

The length of the polypeptide comprising any of the amino acid sequences (E1) to (E25) is not limited. In a preferred embodiment, the length of the polypeptide comprising any of the amino acid sequences (E1) to (E25) may be 500 amino acids or less, 300 amino acids or less, 200 amino acids or less, 100 amino acids or less, 50 amino acids or less, 30 amino acids or less, 20 amino acids or less, 15 amino acids or less, 10 amino acids or less, or 5 amino acids or less. If the polypeptide is obtained by repeatedly linking one or more of the above amino acid sequences (E1) to (E25) one or two or more times, the length of the amino acid sequence portion may preferably be 1000 amino acids or less, 750 amino acids or less, 500 amino acids or less, 250 amino acids or less, 100 amino acids or less, 75 amino acids or less, 50 amino acids or less, 30 amino acids or less, 15 amino acids or less, 10 amino acids or less, or 5 amino acids or less. The number of amino acid residues listed as the length of the above polypeptide in a preferred embodiment is the sum of the lengths of the sequences (excluding the spacer) before and after the spacer.

Each antigen in the invention can specifically bind to an IgE antibody from an allergic patient.

### Diagnosis Kit/Diagnosis Method (2)

The invention provides a method for providing an indicator for diagnosing an allergy in a subject, comprising the steps of:
(i) contacting a sample obtained from the subject with an antigen, wherein the sample is a solution comprising an IgE antibody;
(ii) detecting binding between the IgE antibody present in the sample obtained from the subject and the antigen; and
(iii) when the binding between the IgE antibody in the subject and the antigen is detected, an indicator of the fact that the subject is allergic is provided,
   wherein the antigen is a polypeptide which is at least one polypeptide comprising any of the amino acid sequences set forth in (E1) to (E25) or a polypeptide in which two or more polypeptides comprising any of the amino acid sequences set forth in (E1) to (E25) are linked together with or without a spacer.

The "at least one polypeptide comprising any of the amino acid sequences set forth in (E1) to (E25)" or "the polypeptide in which two or more polypeptides comprising any of the amino acid sequences set forth in (E1) to (E25) are linked together with or without a spacer" may be herein referred to as the "antigen comprising any of the above (E1) to (E25)." The type of spacer is not particularly limited, and those routinely used by those skilled in the art for linking multiple peptides can be used. The spacer may be a hydrocarbon chain such as Acp(6)-OH or a polypeptide such as an amino acid chain.

In the case where the polypeptides comprising any of the above amino acid sequences (E1) to (E25) are linked together with or without a spacer, the number of polypeptides linked is not particularly limited. In one embodiment, the number is 2 or more, 3 or more, 4 or more, 5 or more, 6 or more, 8 or more, 10 or more, or 15 or more. In one embodiment, the number is 30 or less, 20 or less, 15 or less, 10 or less, 8 or less, 6 or less, 5 or less, 3 or less, or 2 or less.

In the polypeptide comprising any of the above amino acid sequences (E1) to (E25), the same unit may be repeated, or different units may be linked. In this way, when multiple polypeptides comprising any of the above amino acid sequences (E1) to (E25) are linked, the resulting polypeptide of the invention is applicable to the method, the kit, or the composition of the invention.

The sample obtained from a subject is as described in the above section "Diagnosis Kit and Diagnosis Method (1)".

Detection of contact and the binding between a sample obtained from a subject and the polypeptide may be performed by the known procedure described in the above section "Diagnosis Kit and Diagnosis Method (1)". Examples of the procedure that can be performed include ELISA (Enzyme-Linked Immunosorbent Assay), sandwich immunoassay, immunoblotting, immunoprecipitation, or immunochromatography.

The polypeptide comprising any of the above amino acid sequences (E1) to (E25) may be immobilized on or inside a carrier. In this case, steps (i) and (ii), can be performed using ELISA, sandwich immunoassay, immunochromatography, surface plasmon resonance, etc., and step (i) mentioned above can be performed by bringing the sample obtained from the subject into contact with a surface on which the polypeptide comprising any of the above amino acid sequences (E1) to (E25) is immobilized. In addition, the IgE antibody from the subject may be immobilized on or inside a carrier and the resulting material may then be used. The binding to the polypeptide comprising any of the amino acid sequences (E1)-(E25) may be detected by this technique. To establish binding onto a carrier or space from the carrier, or to facilitate contact of a polypeptide with the antibody, a spacer or a tag such as biotin may be added to the N-terminus or C-terminus of the polypeptide. In the case of binding to biotin, the carrier preferably has avidin.

The polypeptide comprising any of the above amino acid sequences (E1) to (E25) may not be necessarily immobilized on or inside a carrier. In this case, flow cytometry, etc., can be used in steps (i) and (ii), and the presence of the IgE-bound polypeptide comprising the amino acid sequences (E1) to (E25) can be confirmed with laser light. Examples of this procedure include the basophil activation test (BAT), which detects a surface antigen CD203c that appears when basophils are activated by contact with the polypeptide comprising any of the amino acid sequences (E1) to (E25). Other examples include the histamine release test (HRT), in which the polypeptide comprising any of the above amino acid sequences (E1) to (E25) is further brought into contact with blood cells in a sample to examine whether or not histamine is released.

The polypeptide comprising any of the amino acid sequences (E1)-(E25) is an antigen that specifically binds to an IgE antibody in an allergic patient. Therefore, when binding between an IgE antibody from a subject and the antigen is detected, an indicator indicating that the subject is allergic (including an indicator for cross-reactivity) is provided. To facilitate the synthesis of the polypeptide comprising any of the above amino acid sequences (E1) to (E25) using, for example, *E. coli,* a sequence may be added before or after the epitope so that the sequence length is made longer. In such a case, when the binding of the IgE antibody from a subject to any of the amino acid sequences (E1) to (E25) is detected, an indicator indicating that the subject is allergic (including an indicator for cross-reactivity) is provided. Thus, any sequence may be added before or after the epitope, namely any of the amino acid sequences (E1) to (E25).

The invention also provides a kit for diagnosing an allergy comprising at least one polypeptide comprising any of the above amino acid sequences (E1) to (E25). The diagnosis kit of the invention may be used in a method for providing an indicator for diagnosing an allergy as described above, or in a diagnosis method described below. The diagnosis kit of the invention may comprise at least one polypeptide comprising any of the above amino acid sequences (E1) to (E25), as well as an enzyme-labeled anti-IgE antibody and a chromogenic or luminescent substrate that serves as a substrate for the enzyme. It is also possible to use a fluorescently labeled anti-IgE antibody. In the diagnosis kit of the invention, the polypeptide comprising any of the above amino acid sequences (E1) to (E25) may be immobilized on or inside a carrier. The diagnosis kit of the invention may also be provided with instructions on the procedure for diagnosis and a package comprising the instructions.

In another embodiment, the above diagnosis kit contains a companion diagnostic agent for allergy. The companion diagnostic agent may be used to identify a patient who is expected to benefit from a drug or who is at a risk of severe adverse effects from a drug, or to study the reactivity of a drug to optimize treatment with the drug. Here, the treatment optimization includes, for example, determining a dosage regimen and a dose, deciding discontinuation of administration, and confirming an allergen component to be use for immunological tolerance.

The invention also provides a composition for diagnosing an allergy comprising at least one polypeptide comprising any of the above amino acid sequences (E1) to (E25). The diagnostic composition of the invention may be used in the following diagnosis method. The diagnostic composition of the invention may optionally contain a pharmaceutically acceptable carrier and/or an additive(s) commonly used with the polypeptide in the invention.

An embodiment of the invention provides a method for diagnosing an allergy in a subject, comprising the steps of:
(i) contacting a sample obtained from the subject with an antigen;
(ii) detecting binding of the antigen to an IgE antibody in the sample obtained from the subj ect; and
(iii) determining that the subject has the allergy when the binding of the antigen to the IgE antibody from the subject is detected,
   wherein the antigen is at least one polypeptide specified as a polypeptide comprising any of the above amino acid sequences (E1) to (E25). Here, steps (i) and (ii) are performed as described for each step of the method of providing an indicator for diagnosing an allergy.

In another embodiment, the invention provides a method for diagnosing an allergy in a subject, comprising administering to the subject at least one polypeptide comprising any of the above amino acid sequences (E1) to (E25). The method may be in the form of a skin test characterized by applying to the skin the polypeptide comprising any of the above amino acid sequences (E1) to (E25). Examples of the form of the skin test include: a prick test, in which a diagnostic composition is applied onto the skin, a small cut is given to a degree to which no bleeding occurs, to allow the polypeptide comprising any of the above amino acid sequences (E1) to (E25) to penetrate into the skin, thereby observing a skin reaction; a scratch test, in which a diagnostic composition is applied, and the skin is slightly scratched to observe the resulting reaction; a patch test, in which a diagnostic composition in the form of a cream or ointment is applied to the skin and the reaction is observed; or an intracutaneous test, in which the polypeptide comprising any of the above amino acid sequences (E1) to (E25) is administered intracutaneously and the reaction is observed. In the case of occurrence of a skin reaction, such as skin swelling, at the site where the polypeptide comprising any of the above amino acid sequences (E1) to (E25) has been applied, the subject is diagnosed as having an allergy. Here, the amount of polypeptide to be applied to the skin may be, for example, a dose of 100 µg or less per application.

In the allergy diagnosis, an oral loading test is often performed to identify the antigen and to examine the degree of antigen intake and symptoms. At least one polypeptide comprising any of the above amino acid sequences (E1) to (E25) may be used as an active ingredient for the oral loading test to diagnose an allergy. Here, the polypeptide to be used for the oral loading test may be an expressed and purified polypeptide, or may be a polypeptide expressed in a raw material/processed product, such as pollen rice expressing pollen allergens which are obtained by transforming rice with a gene of a cedar pollen antigen gene and expressing the polypeptide in the rice.

In one embodiment, the above diagnostic composition or diagnosis kit may be used for a prick test, scratch test, patch test, intracutaneous test, etc.

In addition, another embodiment of the invention also provides at least one polypeptide comprising any of the above amino acid sequences (E1) to (E25) for use in allergy diagnosis.

Still another embodiment of the invention provides use of at least one polypeptide comprising any of the above amino acid sequences (E1) to (E25) in the manufacture of an allergy diagnostic agent.

In this section, the allergy to be diagnosed may be an allergy to the polypeptide comprising any of the above amino acid sequences (E1) to (E25). That is, allergy diagnosis including detecting an allergy and providing a diagnostic indicator is a diagnosis of not only an allergy to a single polypeptide comprising any of the above amino acid sequences (E1) to (E25), but also an allergy including cross-reactivity.

### Composition/Treatment Method (2)

The invention also provides a composition comprising at least one polypeptide comprising any of the above amino acid sequences (E1) to (E25).

In one embodiment, the composition of the invention is a pharmaceutical composition. In one embodiment, the composition of the invention is a quasi-drug composition or a non-medicinal composition (e.g., a cosmetic composition, a food composition).

In one embodiment, the above composition is used to treat an allergy. The allergy treatment means increasing the limit amount of a polypeptide in which the polypeptide does not cause any symptom even when taken into the body, and the treatment is ultimately aimed at achieving a state (remission) in which the disease does not develop with normal intake of the polypeptide.

The invention also provides a method for treating an allergy, comprising administering to a patient in need of allergy treatment at least one polypeptide comprising any of the above amino acid sequences (E1) to (E25).

Another embodiment of the invention also provides at least one polypeptide comprising any of the above amino acid sequences (E1) to (E25) for use in allergy treatment. Still another embodiment of the invention provides use of at least one polypeptide comprising any of the above amino acid sequences (E1) to (E25) in the manufacture of an allergy therapeutic agent.

In the allergy treatment, hyposensitization therapy, which aims to induce immunological tolerance by administering an antigen to a patient, is often used. At least one polypeptide comprising any of the above amino acid sequences (E1) to (E25) may be used as an active ingredient for hyposensitization therapy for an allergy. Here, each antigen to be used in the hyposensitization therapy may be an expressed and purified polypeptide, or may be a polypeptide expressed in a raw material/processed product, such as pollen rice.

The administration route, the dosage, the administration frequency, and/or the administration period of the composition of the invention as well as other component(s) contained in the composition and the dosage form may be as described in the above section "Composition/Treatment Method (1)". The dose in the case of using the polypeptide comprising any of the above amino acid sequences (E1) to (E25) may be, for instance, a dose of 100 µg or less per dosing in the case of adults.

In this section, the allergy of treatment interest may be an allergy to the polypeptide comprising any of the above amino acid sequences (E1) to (E25). Specifically, in the allergy treatment, not only an allergy to a single polypeptide comprising any of the above amino acid sequences (E1) to (E25), but also an allergy including cross-reactivity should be treated.

### Tester Composition (2)

The invention provides a tester composition comprising an antibody against at least one polypeptide comprising any of the above amino acid sequences (E1) to (E25).

The antibody can be produced by a conventional procedure. For example, the antibody may be produced by immunizing a mammal such as a rabbit with the polypeptide comprising any of the above amino acid sequences (E1) to (E25). The antibody may be an Ig antibody, a polyclonal antibody, a monoclonal antibody, or an antigen-binding fragment thereof (e.g., a Fab, F(ab')₂, Fab').

In addition, in the tester composition described above, the antibody may be provided in a form immobilized or bound onto or inside a carrier. The carrier is not particularly limited as long as the carrier can be used for detecting the binding between the antibody and the polypeptide comprising any of the above amino acid sequences (E1) to (E25). Any carrier known to those skilled in the art can be utilized. It is also preferable that the antibody against the polypeptide comprising any of the above amino acid sequences (E1) to (E25) be an antibody against any of the epitopes described in the above section "Epitopes" or polypeptides having an amino acid sequence in which the critical amino acid is identical thereto. This makes it possible to provide a tester composition also capable of detecting cross-reactivity.

Examples of the method for determining the presence or absence of the polypeptide comprising any of the above amino acid sequences (E1) to (E25) include the following method.
-- A method comprising bringing a produced antibody-comprising tester composition into contact with a sample prepared from a raw material/processed product, detecting biding of the antibody to a polypeptide comprising any of the above amino acid sequences (E1) to (E25) in the sample by using, for instance, ELISA, and determining, when the binding of the antibody to the polypeptide is detected, that the polypeptide is contained in the raw material/processed product of interest (the "method for determining the presence or absence of the polypeptide" comprises determining that, when the binding of the antibody to the polypeptide is decreased, the polypeptide has been eliminated or reduced).
-- A method comprising soaking a raw material/processed product into a filter paper and allowing a reaction with an antibody solution so as to detect, therein, a polypeptide comprising any of the above amino acid sequences (E1) to (E25).

Another embodiment of the invention encompasses a tester composition for determining the presence or absence of a polypeptide comprising any of the above amino acid sequences (E1) to (E25) in an object of interest, the tester composition comprising a primer corresponding to an epitope or a polypeptide having an amino acid sequence in which the critical amino acid is identical thereto. The above primer is not limited, and may be designed so as to include a portion of the nucleotide sequence, or a complementary strand thereof, of a nucleic acid encoding, for instance, the amino acid sequence specified in any of the above (E1) to (E25). Alternatively, the primer may be designed so that the primer is a nucleotide sequence of a region upstream of a portion encoding an epitope having an amino acid sequence specified in any of the above (E1) to (E25), or a polypeptide having an amino acid sequence in which the critical amino acid is identical thereto, or a nucleotide sequence of a complementary strand of a region downstream of the portion encoding the epitope or a polypeptide having an amino acid sequences in which the critical amino acid is identical thereto, in nucleic acids encoding proteins comprising the epitope and the polypeptide having the amino acid sequence in which the critical amino acid is identical thereto. Examples of such a primer include a primer which is a portion of at least one nucleotide sequence selected from the group consisting of SEQ ID NOs: 1, 3, 5, 7, 9, 11, 13, 15, 17, 19, 21, 23, and 25 and/or a primer which is a portion of a sequence complementary to at least one nucleotide sequence selected from the group consisting of SEQ ID NOs: 1, 3, 5, 7, 9, 11, 13, 15, 17, 19, 21, 23, and 25. Here, the position of the epitope in the full-length sequence of an antigen is as specified in Table 2 based on the results of the Examples. In addition, particularly when mRNA is of interest, a primer complementary to the poly A tail may be contained.

For example, DNA or mRNA obtained from a sample is used as a template, and the DNA is amplified by PCR (Polymerase Chain Reaction) including RT-PCR using the primer described above. The presence or absence of an antigen comprising any of the above (E1) to (E25) is then determined by determining the presence or absence of a nucleic acid encoding the amino acid sequence specified in any of the above (E1) to (E25) in the sequence of the amplified DNA sequence. Examples of the methods of PCR amplification for mRNA of interest include RACE method. When one of the amino acid sequences encoded by three possible open reading frames in the amplified DNA contains the amino acid sequence specified in any of the above (E1) to (E25), it is determined that the antigen is present. When no DNA is amplification, it is determined that no antigen is included.

In one embodiment, the tester composition described above is used to determine the presence or absence of a polypeptide comprising any of the above amino acid sequences (E1) to (E25) in an object of interest, for example, in a raw material or in a processed product production line. The raw material may be a foodstuff or may be a cosmetic raw material, a pharmaceutical raw material, or the like. The processed product may be a processed food product or may be a cosmetic, a pharmaceutical product, or the like. The above-mentioned tester composition may be used for searching for a biological species contained as a raw material, may be used for quality inspection of production lines and pre-shipment products by manufacturers, or for checking whether or not an antigen in raw materials and processed products of interest by consumers or users themselves. In addition, the tester composition may be used for checking inclusion of the polypeptide by measuring a change in the polypeptide peak(s) by mass spectroscopy.

### Method of Determining Presence or Absence of Polypeptide (2)

The invention also encompasses a method for determining the presence or absence of a polypeptide comprising any of the above amino acid sequences (E1) to (E25) in a raw material/processed product. This method comprises detecting a polypeptide having the whole or a portion of the amino acid sequence of a polypeptide comprising any of the above amino acid sequences (E1) to (E25) in a raw material/processed product.

In one embodiment, the method of the invention comprises a step of bringing an antibody against at least one polypeptide comprising any of the above amino acid sequences (E1) to (E25) into contact with a raw material/processed product (including a liquid) to determine the presence or absence of the polypeptide in an object of interest.

The antibody, the definition of the raw material/processed product, the method of producing the antibody, the method of bringing the antibody into contact with a raw material/processed product, and the binding of the antibody to the antigen, for example, are as described above in the "Tester Composition (2)".

Alternatively, in an embodiment, the method of determining the presence or absence of an antigen comprises detecting a portion of an epitope in the polypeptide comprising any of the above amino acid sequences (E1) to (E25) contained in an antigen. The "portion of an epitope" preferably has 4 amino acid residues or more, 6 amino acid residues or more, or 8 amino acid residues or more. The portion of epitope may be detected by a known procedure for detecting a specific amino acid sequence that is a portion of the polypeptide. For instance, it is possible to consider a method comprising cleaving proteins in a raw material/processed product (e.g., foodstuff) of interest with a digestive enzyme for removing an antigen, separating the digested raw material/processed product by HPLC or the like, and measuring whether or not the peak(s) assigned to a given epitope peptide is reduced by the antigen-removing treatment. Alternatively, an antibody recognizing a portion of a polypeptide comprising any of the above amino acid sequences (E1) to (E25) may be used to determine the presence or absence of the polypeptide-comprising antigen in an object of interest.

### Antigen-Removed Raw Material, etc. (2)

The invention provides a raw material or processed product characterized in that at least one polypeptide comprising any of the above amino acid sequences (E1) to (E25) is eliminated or reduced.

The method of removing or reducing the antigen of the invention from a raw material or processed product is not limited. Any method of removing or reducing an antigen may be implemented as long as the polypeptide comprising any of the above amino acid sequences (E1) to (E25) can be eliminated or reduced. For instance, it is possible to use technique described in the above section "Antigen-Removed Food, etc. (1)".

Removal or reduction of at least one polypeptide comprising any of the above amino acid sequences (E1) to (E25) may be achieved by removing or reducing the whole amino acid sequence or by cleaving or eliminating a portion having an amino acid sequence of the above (E1) to (E25) from an antigen protein. The term "eliminating" includes deleting or modifying the whole or a portion of the portion having the sequence specified by any of the above (E1) to (E25).

For example, the raw material, from which the polypeptide comprising any of the above amino acid sequences (E1) to (E25) has been eliminated or reduced, may be prepared using a raw material in which the polypeptide comprising any of the above amino acid sequences (E1) to (E25) is not any more expressed by using a gene modification technology. Any of techniques known to those skilled in the art may be used for the gene modification knockout technology.

The processed product, from which the polypeptide comprising any of the above amino acid sequences (E1) to (E25) has been eliminated or reduced, may be a processed product using a raw material, from which the polypeptide comprising any of the above amino acid sequences (E1) to (E25) has been eliminated or reduced, such as powdered milk using digested proteins as a raw material. In the case of using a regular raw material, treatment for removing or reducing the polypeptide comprising any of the above amino acid sequences (E1) to (E25) should be carried out before, during, or after preparation of the processed product. The "preparation of the processed product" means, for instance, preparing a processed food product from a food raw material (e.g., milk or beef). For instance, the wording "preparation of a processed milk product" means preparing a milk product obtained by processing a main raw material milk or preparing a food comprising the milk product as a raw material. Examples include, but are not limited to, preparation of a milk product such as cream, butter, butter oil, cheese, whey, whey concentrate, ice cream, milk concentrate, skimmed milk concentrate, unsweetened condensed milk, unsweetened skimmed condensed milk, sweetened condensed milk, sweetened skimmed condensed milk, whole milk powder, skimmed milk powder, cream powder, whey powder, protein-enriched whey powder, buttermilk powder, sweetened milk powder, prepared milk powder, prepared liquid milk, fermented milk such as yogurt, a lactobacillus beverage, a milk drink, which are produced by processing milk or a cake, pastry, custard pudding, milk agar, biscuit, cookie, snack, chocolate, bread, white sauce, potage, cream stew, gratin, curry roux, or stew roux, which contain the above material(s) as a raw material. In addition, the wording "preparation of a processed beef product" means preparing a product obtained by processing a main raw material beef or preparing a food comprising the product as a raw material. Examples of the processed meat product include, but are not limited to, preparation of ham, sausage, bacon, bouillon, consomme, steak, grilled meat, roast beef, tartar steak, hamburger steak, hamburger, meatballs, or nuggets.

It is possible to use techniques described in the above section "Antigen-Removed Food, etc. (1)" as methods of removing or reducing a polypeptide comprising any of the above amino acid sequences (E1) to (E25) in a processed product using ordinary raw materials. Examples of the method for cleaving a polypeptide comprising any of the above amino acid sequences (E1) to (E25) include a method for treating the peptide by cleavage with a specific digestive enzyme.

### Method of Producing Raw Material or Processed Product with Antigen Eliminated or Reduced (2)

The invention provides a method for producing a raw material or processed product with at least one polypeptide comprising any of the amino acid sequences (E1) to (E25) eliminated or reduced, the method comprising the step of confirming that the antigen has been eliminated or reduced in a production process of the processed product.

In the production method, removal or reduction of the polypeptide comprising any of the amino acid sequences (E1) to (E25) means that at least one polypeptide comprising any of the above amino acid sequences (E1) to (E25) has been eliminated or reduced, or a portion of a sequence specified in any of the above (E1) to (E25) has been cleaved or eliminated from the antigen.

The technique for confirming that the polypeptide has been eliminated or reduced in a production process of a raw material or processed product is not particularly limited. Any technique may be used that can detect at least one polypeptide comprising any of the above amino acid sequences (E1) to (E25). For instance, the presence or absence of the polypeptides in a raw material or processed product may be confirmed based on the binding ability of an antibody against at least one polypeptide comprising any of the above amino acid sequences (E1) to (E25) to a sample comprising the raw material or a material occurring in the production process of the processed product. The details of such a method are as described in the above section "Diagnosis Kit and Diagnosis Method (2)". That is, in the above production method, the "IgE antibody from a subject" in the above section "Diagnosis Kit and Diagnosis Method (2)" may be replaced with the "antibody against at least one polypeptide comprising any of the above amino acid sequences (E1) to (E25)", and the "antigen" or "polypeptide" in the above section "Diagnosis Kit and Diagnosis Method (2)" may be replaced with the "sample comprising a material occurring in the production process of a processed product". Then, techniques described in the above section "Diagnosis Kit and Diagnosis Method (2)" can be used to confirm that the antigen has been eliminated or reduced in the production process of a processed product. In addition, the tester composition described in the above section "Tester Composition (2)" can be used.

The present invention also pertains to: use of a kit in a method for diagnosing an allergy; use of a kit for diagnosing an allergy and/or providing an indicator for diagnosis; a composition for use in a method for diagnosing an allergy; use of a composition for diagnosing an allergy and/or for providing an indicator for diagnosis; a method for diagnosing an allergy and/or providing an indicator for diagnosis; use of an antigen (protein antigen or epitope antigen) in a method for detecting the presence or absence of an IgE antibody in a sample obtained from a subject (a living body such as a human); an antigen (protein antigen or epitope antigen) for use in treatment of an allergy; a kit or composition for detecting binding between an antigen (protein antigen or epitope antigen) and an IgE antibody in a sample obtained from a subject (a living body such as a human), the kit or composition comprising the antigen (protein antigen or epitope antigen); or use of a kit or composition for detecting binding between an antigen (protein antigen or epitope antigen) and an IgE antibody in a sample obtained from a subject (a living body such as a human), the kit or composition comprising the antigen (protein antigen or epitope antigen). Each term such as the "antigen" is as described above.

Examples of the invention will be described below. The technical scope of the invention, however, is not limited to these Examples.

### Example 1: Confirmation of Protein Pattern

The below-described 2D electrophoresis was used to examine proteins contained in cow milk *(Bos taurus-derived* milk).

### Extraction of Proteins

Proteins contained in milk were extracted and purified as follows. A urea buffer was added to milk to extract proteins. The composition of the urea buffer was the following:
30 mM Tris
2 M thiourea
7 M urea
4% (w/v) CHAPS:
   3-[(3-cholamidopropyl)dimethylammonio]propanesulfonate
   Suitable amount of dilute hydrochloric acid

Distilled water was added to adjust the total volume to 100 mL. The pH was 8.5. Thereafter, the precipitation operation was repeated twice using the 2D-CleanUP kit (manufactured by GE Company). The first precipitation operation was performed by adding TCA (trichloroacetic acid) to the above collected protein extract liquid to collect the resulting precipitate (TCA precipitate) occurring in this operation. The second precipitation operation was performed by adding acetone to the above collected TCA precipitate to collect the resulting precipitate (sample) obtained by this operation.

### To Prepare Sample Solution

Part of the resulting sample (100 µg as a protein weight basis) was solubilized in 150 µl of DeStreak Rehydration Solution (manufactured by GE Company), which is a swelling buffer for a first-dimensional isoelectric focusing gel, to prepare a first-dimensional isoelectric focusing sample solution (sample swelling solution). The composition of DeStreak Rehydration Solution was the following:
7 M thiourea
2 M urea
4% (w/v) CHAPS
0.5% (v/v) IPG buffer; manufactured by GE Company
Appropriate amount of BPB (bromophenol blue)

### Permeation of Sample into First-dimensional Isoelectric Focusing Gel

A first-dimensional isoelectric focusing gel (IPG gel Immobiline Drystrip (pH 3-10 NL); manufactured by GE Company) was immersed in 140 µl of ID isoelectric focusing sample solution (sample swelling solution), and allowed to permeate the solution overnight at room temperature.

In this Example, an IPGphor, manufactured by GE Company, was used as an electrophoresis apparatus.

An electrophoresis tray was filled with silicon oil. Filter paper moistened with water was positioned at both ends of the gel impregnated with the sample, and the gel was set in the electrophoresis tray so as to be covered with silicon oil. Each electrode was set such that the filter paper was interposed between the gel and the electrode.

The upper limit of current value in the isoelectric focusing apparatus was set to 75 µA per gel. First-dimensional isoelectric focusing was performed using the voltage program set as follows: (1) a constant voltage step was performed at a constant voltage of 300 V until 750 Vhr (a current change range during 30-min electrophoresis before the end of this step was 5 µA); (2) the voltage was gradually increased up to 1000 V over 300 Vhr; (3) the voltage was further increased gradually up to 5000 V over 4500 Vhr; and then (4) the voltage was kept at a constant voltage of 5000 V until the total Vhr reached 12000.

### SDS Equilibration in Isoelectric Focusing Gel

After the above first-dimensional isoelectric focusing was performed, the gel was removed from the isoelectric focusing apparatus. Then, the gel was immersed in a reducing agent-comprising equilibration buffer, and was shaken at room temperature for 15 min. The composition of the reducing agent-comprising equilibration buffer was as follows:
100 mM Tris-HCl (pH 8.0)
6 M urea
30% (v/v) glycerol
2% (w/v) SDS
1% (w/v) DTT

Next, the reducing agent-comprising equilibration buffer was removed. The gel was then immersed in an alkylating agent-comprising equilibration buffer, and shaken at room temperature for 15 min to obtain an SDS-equilibrated gel. The composition of the alkylating agent-comprising equilibration buffer was as follows:
100 mM Tris-HCl (pH 8.0)
6 M urea
30% (v/v) glycerol
2% (w/v) SDS
2.5% (w/v) iodoacetamide

### Second-dimensional SDS-PAGE

In this Example, the electrophoresis apparatus used was a XCell SureLock Mini-Cell, manufactured by Life Technologies, Inc. The gel used for second-dimensional electrophoresis was NuPAGE 4-12% Bis-Tris Gels, manufactured by Life Technologies. In addition, an electrophoresis buffer having the following composition was prepared and used.
50 mM MOPS
50 mM Tris base
0.1% (w/v) SDS
1 mM EDTA

Also, in this Example, an agarose solution for adhesion was used in which 0.5% (w/v) agarose S (manufactured by Nippon Gene) and an appropriate amount of BPB (bromophenol blue) were dissolved in the electrophoresis buffer.

A SDS-PAGE well was washed adequately with the electrophoresis buffer, and the buffer used for the washing was removed. Next, a thoroughly dissolved agarose solution for adhesion was added to the well. Then, the SDS-equilibrated gel was immersed in the agarose. Thereafter, the SDS-equilibrated gel was adhered closely to the second-dimensional electrophoresis gel with forceps. Subsequently, it was confirmed that the agarose was sufficiently solidified in a state where the two gels were closely adhered. Finally, electrophoresis was conducted at a constant voltage of 200 V for about 45 min.

### Fluorescence Staining of Gel

Fluorescence staining of the gel was performed using SYPRO Ruby (Life Technologies, Inc.).

First, a sealed container to be used was thoroughly washed with 98% (v/v) ethanol beforehand. The electrophoresed second dimensional electrophoresis gel was removed from the SDS-PAGE apparatus and was placed in the washed sealed container. The gel was twice treated by immersion in an aqueous solution comprising 50% (v/v) methanol and 7% (v/v) acetic acid for 30 min. Next, the aqueous solution was replaced by water, followed by immersion for 10 min. Then, the second-dimensional electrophoresis gel was immersed in 40 ml of SYPRO Ruby, and shaken overnight at room temperature. Subsequently, the SYPRO Ruby was removed. After washed with water, the second-dimensional electrophoresis gel was shaken in an aqueous solution comprising 10% (v/v) methanol and 7% (v/v) acetic acid for 30 min. Further, the aqueous solution was replaced by water, followed by shaking for 30 min or longer.

### Analysis

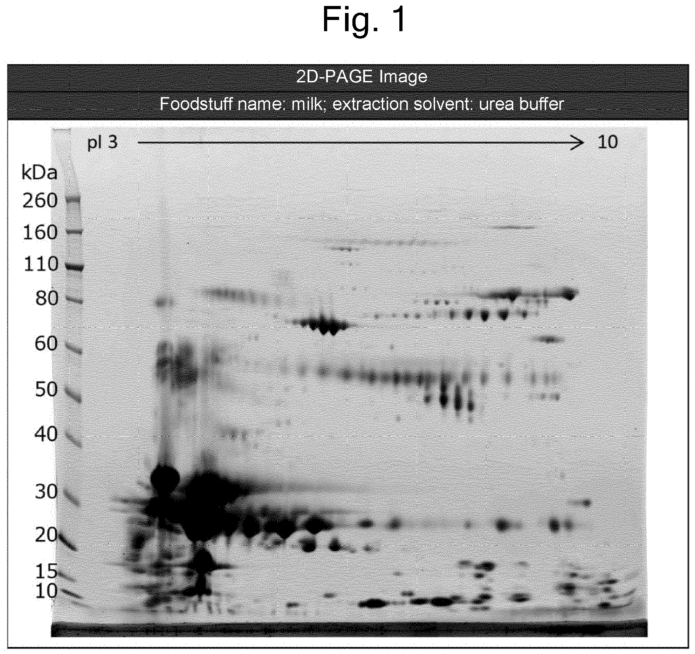

The second-dimensional electrophoresis gel, which underwent the series of the foregoing treatments, was subjected to fluorescence image scanning using a Typhoon 9500 (manufactured by GE Company). Fig. 1 shows the results of second-dimensional electrophoresis of proteins contained in milk. On the left side of the gel photograph in each figure, molecular weight marker bands can be seen, and the position of each band indicates a specific molecular weight (KDa).

### Example 2: Identification of Antigen by Immunoblotting

Each antigen was identified by immunoblotting as follows. The procedure described in Example 1 was performed until the "2D SDS-PAGE", and then, the following operations "Membrane Transfer", "Immunoblotting", and "Analysis" were carried out.

### Transfer to Membrane

Transfer to membrane was conducted using the following transfer apparatus and transfer buffer.

### Transfer apparatus: XCell SureLock Mini-Cell and XCell II Blotting Module (manufactured by Life Technologies)

Transfer buffer: NuPAGE transfer buffer (×20) (manufactured by Life Technologies) diluted 20-fold with milliQ water was used.

Specifically, proteins in the second-dimensional electrophoresis gel were transferred to a membrane (PVDF membrane) according to the following procedure.
(1) A PVDF membrane was immersed in 100% methanol and then milliQ water, and then placed in a transfer buffer for hydrophilic treatment of the PVDF membrane.
(2) A sponge, a filter paper, the gel after the second dimensional SDS-PAGE, the hydrophilized PVDF membrane, a filter paper, and a sponge were set in this order, and a constant voltage was applied thereto in the transfer apparatus for 1 h.

### Immunoblotting

Immunoblotting of the membrane was conducted using, as a primary antibody, serum from a patient with milk allergy or serum from a subject without milk allergy.

The immunoblotting of the membrane was performed according to the following procedure.
(1) The transferred membrane was shaken in Pierce (registered trademark) Fast Blocking Buffer (manufactured by Thermo, Inc.) (hereinafter referred to as "Fast Block") for 1 h at room temperature.
(2) 10% serum/Fast Block solution was added as a primary antibody, and the mixture was allowed to stand for 1 h at room temperature.
(3) The membrane was washed with PBST solution (PBS buffer comprising 0.1% nonionic surfactant Tween (registered trademark) 20) (5 min × 3 times).
(4) Anti-human IgE-HRP (horseradish peroxidase) was diluted 5000-fold with Fast Block solution to give a secondary antibody, and the membrane was shaken in the resulting solution for 1 h at room temperature.
(5) The membrane was washed with PBST solution (5 min × 3 times).
(6) In Pierce Western Blotting Substrate Plus (manufactured by Thermo, Inc.), the membrane was allowed to stand for 5 min.

### Analysis

The membrane, which underwent the foregoing series of treatments, was subjected to fluorescence image scanning using a Typhoon 9500 (manufactured by GE Company)

The immunoblot obtained by using serum from each patient with milk allergy was compared with the immunoblot obtained by using serum from a control, a subject without milk allergy. The immunoblot obtained by using serum from a patient with milk allergy was different, in terms of proteins contained in milk, from that in the case of using serum from a subject without milk allergy. Here, 14 spots, which differ from those of known milk allergen proteins, were detected (Fig. 2). As a result of analyzing the amino acid sequences in Example 3, spots 2 and 3 were found to be derived from the same protein. The isoelectric point of each spot was listed in Table 1.

### Example 3: Mass Spectrometry and Antigen Identification

The amino acid sequences of the antigens which generate the spots described above were identified by mass spectroscopy.

Specifically, the following procedure was used for protein extraction and mass spectrometry.
(1) Milk was subjected to protein extraction, second-dimensional electrophoresis, and transfer to membrane according to the procedures of Examples 1 and 2, and the membrane was stained with 0.008% Direct blue/40% ethanol and 10% acetic acid under shaking.
(2) The membrane was destained by treatment with 40% ethanol and 10% acetic acid for 5 min three times, washed with water for 5 min, and air-dried.
(3) Each spot of interest was cut out with a clean cutter blade and placed in a centrifuge tube. After hydrophilic treatment of the membrane with 50 µL of methanol, the membrane was washed twice with 100 µL of water, and centrifuged to remove water. Then, 20 µL of 20 mM NH₄HCO₃ and 50% acetonitrile was added.
(4) 1 µL of 1 pmol/µL lysyl endopeptidase (WAKO) was added, and the mixture was allowed to stand at 37°C for 60 min. The resulting solution was collected in a new centrifuge tube. Subsequently, 20 µL of 20 mM NH₄HCO₃/70% acetonitrile was added to the membrane, which was immersed for 10 min at room temperature and the resulting solution was further collected. The material was dissolved in 10 µL of 0.1% formic acid and 4% acetonitrile, and then transferred into the tube.
(5) The collected solution was dried under reduced pressure, dissolved in 15 µl of solution A (0.1% formic acid and 4% acetonitrile solution), and subjected to mass spectrometry (ESI-TOF6600, manufactured by AB Sciex, Inc.).
(6) Protein identification based on the mass data obtained from the mass spectrometer was performed by searching Uniprot and NCBI.

### Results

The amino acid sequence of each spot was detected. The mass data of each spot as obtained using a mass spectrometer was further analyzed using Uniprot, NCBI, and each spot was identified as each protein listed in Table 1.

Spots 2 and 3 are derived from the same protein. These spots were detected as different spots in the immunoblot of Example 2. This is because although the amino acid sequence is identical, the isoelectric point and/or the molecular weight were changed due to post-translational modifications such as glycosylation and phosphorylation. Although the post-translational modifications were different, the binding to an IgE antibody was still detected. This indicates that there was no influence of the post-translational modifications.

### Example 4: Identification of Epitopes

### Epitopes of allergenic components in milk

Epitopes of allergen components in milk were identified using the following procedures.

### (A) Milk Epitope Mapping (1)

Epitope mapping was performed using a library of overlapping peptides (length: 15 amino acids) corresponding to amino acid sequences identified as allergic components in milk. Specifically, a library of overlapping peptides was prepared based on the amino acid sequences of SEQ ID NOs: 2, 4, 6, 8, 10, 12, 14, 16, 18, 20, 22, 24, and 26.

Each peptides to be synthesized was shifted by 10 amino acids. That is, each peptide has a 5-amino-acid overlap with the previous and subsequent peptides.

For the peptide array preparation, the Intavis CelluSpots (trademark) technology was used. Specifically, the procedure was as follows: (1) peptides of interest were synthesized on amino-modified cellulose discs using an automated synthesizer (Intavis MultiPep RS); (2) each amino-modified cellulose disc was dissolved to obtain a cellulose-bound peptide solution; and (3) each cellulose-bound peptide was spotted on a coated glass slide. The details of each step were as follows.

### (1) Synthesis of Peptides

Each peptide was synthesized stepwise on an amino-modified cellulose disc in a 384-well synthesis plate by using 9-fluorenylmethoxycarbonyl (Fmoc) chemical reaction. Specifically, the amino acid with an amino group bound to the Fmoc group was activated in a solution comprising N,N'-diisopropylcarbodiimide (DIC) and 1-hydroxybenzotriazole (HOBt) in dimethylformamide (DMF). The mixture was added dropwise onto the cellulose disc so that the Fmoc-bound amino acid was bound to an amino group on the cellulose disc (coupling). Unreacted amino groups were capped with acetic anhydride, and the disc was washed with DMF. The Fmoc groups were eliminated from the amino groups of the amino acids bound to the amino groups on the cellulose discs by treating with piperidine and washing with DMF. The amino acids bound to the amino group on the cellulose disc was repeatedly subjected to the above coupling, capping, and Fmoc group, thereby extending the amino terminus for peptide synthesis.

### (2) Dissolution of Amino-Modified Cellulose Disc

The cellulose disc, on which a peptide of interest as obtained in the above "(1) To Synthesize Peptide" was bound, was transferred to a 96-well plate, and treated with a side chain deprotection mixture comprising trifluoroacetic acid (TFA), dichloromethane, triisopropylsilane (TIPS), and water for the amino acid side chain deprotection. Next, the deprotected cellulose-bound peptide was dissolved in a mixed solution of TFA, perfluoromethanesulfonic acid (TFMSA), TIPS, and water. The peptide was precipitated in tetrabutyl methyl ether (TBME), resuspend in dimethylsulfoxide (DMSO), and mixed with a mixed solution of NaCl, Na citrate, and water to prepare a peptide solution for slide spotting.

### (3) Spotting of Cellulose-bound Peptide Solution

The peptide solution for slide spotting as obtained in the above "(2) To Dissolve Amino-Modified Cellulose Disc" was spotted on an Intavis CelluSpots (trademark) slide by using an Intavis slide spotting robot, and the slide was dried to prepare a peptide array.

The peptide array was used to measure whether or not each peptide fragment was bound to an IgE antibody in serum from each patient with milk allergy through an antigen/antibody reaction. The measurement was conducted according to the following procedure.
(1) Peptides in Pierce Protein-Free (PBS) Blocking Buffer (manufactured by Thermo, Inc.) were shaken for 1 h at room temperature.
(2) The array was shaken overnight at 4°C in 2% serum/Pierce Protein-Free (PBS) Blocking Buffer (manufactured by Thermo, Inc.).
(3) The array was washed with PBST (PBS buffer comprising 3% nonionic surfactant Tween (registered trademark) 20) for 5 min (× 3 times).
(4) Anti-human IgE antibody-HRP (1:20,000, Pierce Protein-Free (PBS) Blocking Buffer (manufactured by Thermo, Inc.)) was added, and the mixture was shaken for 1 h at room temperature.
(5) The array was washed with PBST for 5 min (× 3 times).
(6) A Pierce ECL Plus Western Blotting Substrate (manufactured by Thermo, Inc.) was added, and the array was shaken for 5 min at room temperature.
(7) The chemiluminescence of each peptide treated in (1) to (6) above was measured using Amersham Imager 600.

ImageQuant TL (GE Healthcare, Inc.) was used to quantify and the results were expressed numerically, the level of chemiluminescence in an image obtained during the above measurement (7). The second highest value among the numeric values for the respective images obtained from the results using serum from each of 6 subjects without milk allergy was defined as The N2nd value. The N2nd value of each peptide was subtracted from numeric value obtained from images obtained from the results using serum from each of 25 patients. It was determined that a peptide having the difference of 800,000 or higher was a peptide bound to an IgE antibody in a patient-specific manner.

The peptides (SEQ ID NOs: 27, 59, 98, 145, 196, 255, 289, 334, 353, 377, 385, 412, 451, 501, 605, 633, 674, 694, 758, 792, 869, 928, 997, 1038, and 1076) derived from spots 1 to 14 (spots 2 and 3 are derived from the same protein), in which known epitopes were not included, were each found to be bound to an IgE antibody in a patient-specific manner.

### (B) Milk Epitope Mapping (2): Overlapping

As described in (A), on the basis of the sequences (SEQ ID NOs: 27, 59, 98, 145, 196, 255, 289, 334, 353, 377, 385, 412, 451, 501, 605, 633, 674, 694, 758, 792, 869, 928, 997, 1038, and 1076) of the peptides bound to an IgE antibody in serum in a patient-specific manner, a library of overlapping peptide fragments (the length: 10 amino acids) was constructed using in each peptide sequence or an allergic component amino acid sequence comprising the peptide sequence with sequences before and after the peptide being added. Then, epitope mapping was performed.

Each peptide to be synthesized was shifted by one amino acid. That is, each peptide has a 9-amino-acid overlap with the previous and subsequent peptides.

The library was constructed by substantially the same procedure as in (A). The same technique as above was used to evaluate each peptide fragment for binding to an IgE antibody in a patient's serum. The image obtained as the results of using the patient's serum was quantified to be represented numerically. This value was compared with a value obtained using each overlapping-based peptide. The peptide having lost or markedly decreased binding ability to an IgE antibody from a patient as compared with the peptide shifted one amino acid was determined as a peptide having no binding ability to the IgE antibody.

The level of chemiluminescence in an image obtained by measurement in the same manner as in (A) was converted into a numeric value. Among the values determined from the images obtained from the results of using a patient's serum, the value obtained using an overlapping base sequence (SEQ ID NO: 27, 59, 98, 145, 196, 255, 289, 334, 353, 377, 385, 412, 451, 501, 605, 633, 674, 694, 758, 792, 869, 928, 997, 1038, or 1076) was defined as 100%. In comparison with these peptides, the binding ability of other peptides are judged as follows: the value of less than 30% indicated no binding ability to the IgE antibody; the value of 30% or more and less than 50% indicated poor but some binding ability to the IgE antibody; the value of 50% or more and less than 70% indicated binding ability, though somewhat poor, to the IgE antibody; and the value of 70% or more indicated no difference in binding ability to the IgE antibody or indicated sufficient binding ability to the IgE antibody. In this way, the peptide was determined to be a peptide having remaining binding ability to the IgE antibody.

This analysis has revealed a critical region for binding to IgE antibody from patients, in each overlapping base sequence.

### (C) Milk Epitope Mapping (3): Alanine/Glycine Scanning

The amino acid sequences identified in (A) were used to construct, using substantially the same technique as above, a library of peptide fragments in which the amino acids were substituted from the amino-terminal side with alanine (glycine if the original amino acid was alanine) one by one by using a technique called alanine/glycine scanning (NPL 1). The same technique as above was used to evaluate each peptide fragment for binding to an IgE antibody in a patient's serum. The amino acid at a position where the binding ability to a patient's IgE antibody was lost or markedly decreased by alanine/glycine substitution was determined to be an amino acid critical for exertion of original antigenicity or an amino acid affecting exertion of original antigenicity. The amino acid, the substitution of which did not result in a loss or a marked decrease in the binding ability to the patient's IgE antibody, was determined as a replaceable amino acid that is not critical for exertion of original antigenicity.

The level of chemiluminescence in an image obtained by measurement in the same manner as in (A) was converted into a numeric value. Among the values determined from the images obtained from the results of using sera from 25 patients, the value obtained using each alanine/glycine scanning base sequence (SEQ ID NO: 27, 59, 98, 145, 196, 255, 289, 334, 353, 377, 385, 412, 451, 501, 605, 633, 674, 694, 758, 792, 869, 928, 997, 1038, or 1076) was defined as 100%. In comparison with these peptides, the binding ability of other peptides are judged as follows: the value of less than 30% indicated no binding ability to the IgE antibody; the value of 30% or more and less than 50% indicated poor but some binding ability to the IgE antibody; the value of 50% or more and less than 70% indicated binding ability, though somewhat poor, to the IgE antibody but indicated binding to the IgE antibody; and the value of 70% or more indicated no difference in binding ability to the IgE antibody or indicated sufficient binding ability to the IgE antibody. In this way, the peptide was determined to be a peptide having remaining binding ability to the IgE antibody.

The results of (A) to (C) have been analyzed to find a consensus sequence important for exertion of original antigenicity in regions important for binding to IgE antibody from patients, in the alanine/glycine scanning base sequence. The sequences for all 25 different epitopes were identified, and the results were summarized in Table 2.

### Example 5: Confirmation of Epitope Cross-Reactivity

Using a key sequence in which amino acids other than amino acids important for maintaining binding to IgE antibodies, in each of the epitope sequences found in the milk proteins in Table 2 were defined as any amino acid (X), proteins having the key sequence in allergen foodstuff in common among patients listed in Table 2-26 were searched by BLAST. Then, BLAST was used to search for proteins having the same key sequence in foodstuffs allergic to each patient at the same time as listed in Table 2-26. As a result, each amino acid sequence was identified which was included in sequences of each food listed in, for example, the column "Cross-reactivity-checked food" in Table 2.

Each peptide comprising an amino acid sequence listed in the column "Synthesized sequence" of Table 2 was evaluated, by ELISA, for binding to an IgE antibody from a patient allergic to each food listed in the column "Cross-reactivity-confirmed food" of Table 2. The peptide was synthesized by Fmoc method so that the peptide was N-terminally biotinylated.

The ELISA was performed according to the following procedure.
(1) The concentrations of the biotinylated peptides were adjusted to 10 µg/mL with PBST (0.1% Tween (registered trademark) 20).
(2) 20 µL of each peptide solution was added to each well of a streptavidin-coated 384-well plate and shaken for 1 h at room temperature. The solution was collected, and the well was then washed five times with PBS.
(3) 80 µL of Pierce Protein-Free (PBS) Blocking Buffer (manufactured by Thermo, Inc.) was added, and the mixture was shaken for 1 h at room temperature. The solution was removed, and the plate was then washed three times with PBST.
(4) 40 µL of 2% serum/Canget Signal Solution I (manufactured by TOYOBO, Inc.) was added, and the mixture was shaken for 1 h at room temperature. The solution was removed, and the plate was then washed five times with PBST.
(5) 40 µL of diluted secondary antibody solution (1:10000, Canget Signal Solution II (manufactured by TOYOBO, Inc.)) was added, and the mixture was shaken for 1 h at room temperature. The solution was removed, and the plate was then washed three times with PBST.
(6) 40 µL of 1-Step Ultra TMB-ELISA (manufactured by Thermo, Inc.) was added, and the mixture was shaken for 15 min at room temperature.
(7) 40 µL of 2 M H₂SO₄ was added. The absorbance at 450 nm was then measured.

Peptides having these amino acid sequences were prepared according to substantially the same procedure as in (A) of Example 4, and whether an IgE antibody in serum from an allergic patient or a non-allergic subject could bind to the peptides was measured. Serum from each of two non-allergic subjects was used for measurement. The results were averaged. Then, values divided by the average was used.

Figs. 3 to 6 show the results. P1, P5, P8, and P20 each represent the patient number. Bar graphs in each figure show absorbance of allergic patients/absorbance of non-allergic patients (healthy subjects). The origin of foodstuff as designated in each graph is the origin of foodstuff comprising a polypeptide including a base amino acid sequence of each synthetic sequence used. If not indicated, the origin of foodstuff is "Cow".

As clearly demonstrated in Figs. 3 to 6, all the polypeptides with each amino acid sequence designated in each figure exhibited higher (more than "1") binding ability to an IgE antibody from each allergic patient than an IgE antibody in serum from each non-allergic subject. Thus, the cross-reactivity of each polypeptide was demonstrated. This indicates that these epitopes can be utilized to detect the cross-reactivity to an antigen other than that from cow milk. Further, it has been supported that the site "X" can be any amino acid residue.

## Claims

1. A kit for diagnosing an allergy, comprising at least one of the following polypeptides (E1) to (E25):
(E1) a polypeptide comprising at least one amino acid sequence selected from amino acid sequences set forth in SEQ ID NOs: 27 to 58 and 1115;
(E2) a polypeptide comprising at least one amino acid sequence selected from amino acid sequences set forth in SEQ ID NOs: 59 to 97;
(E3) a polypeptide comprising at least one amino acid sequence selected from amino acid sequences set forth in SEQ ID NOs: 98 to 144;
(E4) a polypeptide comprising at least one amino acid sequence selected from amino acid sequences set forth in SEQ ID NOs: 145 to 195;
(E5) a polypeptide comprising at least one amino acid sequence selected from amino acid sequences set forth in SEQ ID NOs: 196 to 254 and 1116 to 1118;
(E6) a polypeptide comprising at least one amino acid sequence selected from amino acid sequences set forth in SEQ ID NOs: 255 to 288;
(E7) a polypeptide comprising at least one amino acid sequence selected from amino acid sequences set forth in SEQ ID NOs: 289 to 333 and 1119 to 1120;
(E8) a polypeptide comprising at least one amino acid sequence selected from amino acid sequences set forth in SEQ ID NOs: 334 to 352;
(E9) a polypeptide comprising at least one amino acid sequence selected from amino acid sequences set forth in SEQ ID NOs: 353 to 376 and 1121 to 1122;
(E10) a polypeptide comprising at least one amino acid sequence selected from amino acid sequences set forth in SEQ ID NOs: 377 to 384;
(E11) a polypeptide comprising at least one amino acid sequence selected from amino acid sequences set forth in SEQ ID NOs:385 to 411;
(E12) a polypeptide comprising at least one amino acid sequence selected from amino acid sequences set forth in SEQ ID NOs: 412 to 450;
(E13) a polypeptide comprising at least one amino acid sequence selected from amino acid sequences set forth in SEQ ID NOs: 451 to 500 and 1123 to 1124;
(E14) a polypeptide comprising at least one amino acid sequence selected from amino acid sequences set forth in SEQ ID NOs: 501 to 604 and 1125 to 1127;
(E15) a polypeptide comprising at least one amino acid sequence selected from amino acid sequences set forth in SEQ ID NOs: 605 to 632 and 1128;
(E16) a polypeptide comprising at least one amino acid sequence selected from amino acid sequences set forth in SEQ ID NOs: 633 to 673;
(E17) a polypeptide comprising at least one amino acid sequence selected from amino acid sequences set forth in SEQ ID NOs: 674 to 693;
(E18) a polypeptide comprising at least one amino acid sequence selected from amino acid sequences set forth in SEQ ID NOs: 694 to 757 and 1129;
(E19) a polypeptide comprising at least one amino acid sequence selected from amino acid sequences set forth in SEQ ID NOs: 758 to 791;
(E20) a polypeptide comprising at least one amino acid sequence selected from amino acid sequences set forth in SEQ ID NOs: 792 to 868 and 1130;
(E21) a polypeptide comprising at least one amino acid sequence selected from amino acid sequences set forth in SEQ ID NOs: 869 to 927;
(E22) a polypeptide comprising at least one amino acid sequence selected from amino acid sequences set forth in SEQ ID NOs: 928 to 996;
(E23) a polypeptide comprising at least one amino acid sequence selected from amino acid sequences set forth in SEQ ID NOs: 997 to 1037;
(E24) a polypeptide comprising at least one amino acid sequence selected from amino acid sequences set forth in SEQ ID NOs: 1038 to 1075; or
(E25) a polypeptide comprising at least one amino acid sequence selected from amino acid sequences set forth in SEQ ID NOs:1076 to 1114 and 1131.

2. A composition for diagnosing an allergy, the composition comprising at least one polypeptide as defined in any of (E1) to (E25) of claim 1.

3. A method for providing an indicator for diagnosing an allergy in a subject, the method comprising the steps of:
(i) contacting a sample obtained from the subject with an antigen, wherein the sample is a solution comprising an IgE antibody;
(ii) detecting binding between the IgE antibody present in the sample obtained from the subject and the antigen; and
(iii) when the binding between the IgE antibody in the subject and the antigen is detected, an indicator of the fact that the subject is allergic is provided,
wherein the antigen is at least one polypeptide as defined in any of (E1) to (E25) of claim 1.

4. An antigen, which is at least one polypeptide as defined in any of (E1) to (E25) of claim 1 and is causative of an allergy.

5. A composition comprising at least one antigen according to claim 4.

6. The composition according to claim 5wherein the composition is intended for the treatment of an allergy.

7. A tester composition for determining the presence or absence of an antigen in an object of interest, the tester composition comprising an antibody that binds to at least one polypeptide as defined in any of (E1) to (E25) of claim 1.

8. A tester composition for determining the presence or absence of an antigen in an object of interest, the tester composition comprising at least one primer comprising a portion of a nucleotide sequence, and/or a portion of a complementary strand thereof, of a nucleic acid encoding a polypeptide as defined in any of (E1) to (E25) of claim 1.

9. A tester composition for determining the presence or absence of an IgE antibody in a subject, the tester composition comprising a polypeptide as defined in any of (E1) to (E25) of claim 1.

10. A method for determining the presence or absence of a polypeptide as defined in any of (E1) to (E25) of claim 1 in a raw material or processed product, the method comprising detecting the polypeptide as defined in any of (E1) to (E25) of claim 1 in the raw material or processed product.

11. A raw material or processed product in which an antigen is eliminated or reduced, wherein the antigen is at least one polypeptide as defined in any of (E1) to (E25) of claim 1.

12. A method for producing a raw material or processed product in which an antigen is eliminated or reduced, the method comprising the step of confirming that the antigen has been eliminated or reduced in a production process of the raw material or the processed product, wherein the antigen is at least one polypeptide as defined in any of (E1) to (E25) of claim 1.


SEQUENCE LISTING

<110>  FUJITA ACADEMY
       HOYU CO., LTD.

<120>  Antigens that causes milk allergy

<130>  FA1621-21339

<150>  JP2021-061611
<151>  2021-03-31

<160>  1131

<170>  PatentIn version 3.5

<210>  1
<211>  1743
<212>  DNA
<213>  Bos taurus

<400>  1
tcccaggtgc agctgcggga gtcgggcccc agcctggtga agccctcaca gaccctctcc      60

ctcacctgca cggtctctgg attctcatta agcgattatc gtgtaggctg ggtccgccag     120

gctccaggaa aggcgctgga gtggctcggt gtaatgtata atgacggaac cactgcgtat     180

aggccagccc tgctatcccg gctccgaatc accagggaca actccaagaa ccaagtcgcc     240

ctgtcactaa gcagcgtaac aactgaggac acggccacat actactgtac aagaataatt     300

cctgtgtgta cttggcctta tggtgactat ggctgttatg acatcttggg ccaaggactc     360

ctggtcaccg tctcctcaga aggtgaatca cacccgagag tcttccccct ggtgtcctgc     420

gtgagctccc catccgatga gagcacggtg gccctgggct gcctggcccg ggacttcgtg     480

cccaattcag tcagcttctc ctggaagttc aacaacagca cagtcagcag cgagagattc     540

tggaccttcc ccgaagtcct gagggacggc ttgtggtcgg cctcctctca ggtggtcctg     600

ccctcctcaa gcgcctttca agggccggat gactacctgg tgtgcgaagt ccagcacccc     660

aagggaggaa agaccgtcgg caccgtgagg gtggtcgcta caaaggcgga agtgctgtcc     720

ccagtcgtga gtgtctttgt cccgcctcgc aacagcctct ctggtgacgg caatagcaag     780

tccagcctca tctgccaggc cacggacttc agccccaaac agatctcctt gtcctggttt     840

cgtgatggaa agcggatagt gtctggcatt tctgaaggcc aggtggagac tgtgcagtcc     900

tcacccgtaa ctttcagggc ctacagcatg ctgaccatca cggagagaga ctggctcagc     960

cagaacgtgt acacctgcca ggtagaacac aacaaggaaa ccttccagaa gaacgtgtcc    1020

tcctcatgtg atgttgcacc accatctccc atcggggtct tcaccatccc cccatccttc    1080

gccgacatct tcctcacgaa gtcagccaag ctgtcctgtc tggtcacaaa cctggcctcc    1140

tatgatggcc tgaacatcag ctggtcccgt cagaacggca aggccctgga gacccacacg    1200

tattttgagc gacacctcaa cgacaccttc agcgcccggg gtgaggcctc ggtctgctcg    1260

gaggactggg agtccggaga ggagttcacg tgcacagtgg cccactcgga cctgcccttc 1320

ccagaaaaga acaccgtctc caagcccaaa gatgtcgcca tgaaaccgcc gtccgtgtac 1380

ctgctgcctc caacgcggga acagctgagc ctgcgggagt cggcctccgt cacctgcctg 1440

gtgaagggct tcgcgcccgc ggacgtgttc gtgcagtggc tgcagagggg ggagcccgtg 1500

accaagagca agtacgtgac cagcagcccg gcgcccgagc cccaggaccc cagcgtgtac 1560

tttgtgcaca gcatcctgac ggtggccgag gaggactgga gcaaagggga gacctacacc 1620

tgcgtcgtgg gccacgaggc cctgccccac atggtcaccg agcggaccgt ggacaagtcc 1680

accggtaaac ccaccctgta caacgtgtcc ctggtcctgt ctgacacagc cagcacctgc 1740

tac 1743

<210> 2
<211> 581
<212> PRT
<213> Bos taurus

<400> 2

Ser Gln Val Gln Leu Arg Glu Ser Gly Pro Ser Leu Val Lys Pro Ser
1               5                   10                  15

Gln Thr Leu Ser Leu Thr Cys Thr Val Ser Gly Phe Ser Leu Ser Asp
            20                  25                  30

Tyr Arg Val Gly Trp Val Arg Gln Ala Pro Gly Lys Ala Leu Glu Trp
        35                  40                  45

Leu Gly Val Met Tyr Asn Asp Gly Thr Thr Ala Tyr Arg Pro Ala Leu
    50                  55                  60

Leu Ser Arg Leu Arg Ile Thr Arg Asp Asn Ser Lys Asn Gln Val Ala
65                  70                  75                  80

Leu Ser Leu Ser Ser Val Thr Thr Glu Asp Thr Ala Thr Tyr Tyr Cys
                85                  90                  95

Thr Arg Ile Ile Pro Val Cys Thr Trp Pro Tyr Gly Asp Tyr Gly Cys
            100                 105                 110

Tyr Asp Ile Leu Gly Gln Gly Leu Leu Val Thr Val Ser Ser Glu Gly
        115                 120                 125

Glu Ser His Pro Arg Val Phe Pro Leu Val Ser Cys Val Ser Ser Pro
    130                 135                 140

Ser Asp Glu Ser Thr Val Ala Leu Gly Cys Leu Ala Arg Asp Phe Val
145                 150                 155                 160

Pro Asn Ser Val Ser Phe Ser Trp Lys Phe Asn Asn Ser Thr Val Ser
                165                 170                 175

Ser Glu Arg Phe Trp Thr Phe Pro Glu Val Leu Arg Asp Gly Leu Trp
            180                 185                 190

Ser Ala Ser Ser Gln Val Val Leu Pro Ser Ser Ser Ala Phe Gln Gly
        195                 200                 205

Pro Asp Asp Tyr Leu Val Cys Glu Val Gln His Pro Lys Gly Gly Lys
    210                 215                 220

Thr Val Gly Thr Val Arg Val Val Ala Thr Lys Ala Glu Val Leu Ser
225                 230                 235                 240

Pro Val Val Ser Val Phe Val Pro Pro Arg Asn Ser Leu Ser Gly Asp
                245                 250                 255

Gly Asn Ser Lys Ser Ser Leu Ile Cys Gln Ala Thr Asp Phe Ser Pro
            260                 265                 270

Lys Gln Ile Ser Leu Ser Trp Phe Arg Asp Gly Lys Arg Ile Val Ser
        275                 280                 285

Gly Ile Ser Glu Gly Gln Val Glu Thr Val Gln Ser Ser Pro Val Thr
    290                 295                 300

Phe Arg Ala Tyr Ser Met Leu Thr Ile Thr Glu Arg Asp Trp Leu Ser
305                 310                 315                 320

Gln Asn Val Tyr Thr Cys Gln Val Glu His Asn Lys Glu Thr Phe Gln
                325                 330                 335

Lys Asn Val Ser Ser Ser Cys Asp Val Ala Pro Pro Ser Pro Ile Gly
            340                 345                 350

Val Phe Thr Ile Pro Pro Ser Phe Ala Asp Ile Phe Leu Thr Lys Ser
        355                 360                 365

Ala Lys Leu Ser Cys Leu Val Thr Asn Leu Ala Ser Tyr Asp Gly Leu
    370                 375                 380

Asn Ile Ser Trp Ser Arg Gln Asn Gly Lys Ala Leu Glu Thr His Thr
385                 390                 395                 400

Tyr Phe Glu Arg His Leu Asn Asp Thr Phe Ser Ala Arg Gly Glu Ala
                405                 410                 415

Ser Val Cys Ser Glu Asp Trp Glu Ser Gly Glu Glu Phe Thr Cys Thr
            420                 425                 430

Val Ala His Ser Asp Leu Pro Phe Pro Glu Lys Asn Thr Val Ser Lys
        435                 440                 445

Pro Lys Asp Val Ala Met Lys Pro Pro Ser Val Tyr Leu Leu Pro Pro
    450                 455                 460

Thr Arg Glu Gln Leu Ser Leu Arg Glu Ser Ala Ser Val Thr Cys Leu
465                 470                 475                 480

Val Lys Gly Phe Ala Pro Ala Asp Val Phe Val Gln Trp Leu Gln Arg
                485                 490                 495

Gly Glu Pro Val Thr Lys Ser Lys Tyr Val Thr Ser Ser Pro Ala Pro
            500                 505                 510

Glu Pro Gln Asp Pro Ser Val Tyr Phe Val His Ser Ile Leu Thr Val
        515                 520                 525

Ala Glu Glu Asp Trp Ser Lys Gly Glu Thr Tyr Thr Cys Val Val Gly
    530                 535                 540

His Glu Ala Leu Pro His Met Val Thr Glu Arg Thr Val Asp Lys Ser
545                 550                 555                 560

Thr Gly Lys Pro Thr Leu Tyr Asn Val Ser Leu Val Leu Ser Asp Thr
                565                 570                 575

Ala Ser Thr Cys Tyr
            580

<210>  3
<211>  2055
<212>  DNA
<213>  Bos taurus

<400>  3
gaccctgaga gaactgtgag atggtgcacc atttcaactc atgaggccaa taagtgtgcc      60

agttttcgtg aaaatgtgct tcgtatcttg gagagtggtc cttttgtctc ctgtgtgaag     120

aaaacctcac acatggattg catcaaggct atctcgaata acgaagcaga tgctgtgacg     180

ttggatggag gtttggtgta tgaggcaggc ctgaagccca acaacctgaa gcctgtggtg     240

gcagagttcc atgggacaaa agacaaccca caaactcact attatgctgt ggcggtggtg 300

aagaaggaca ctgacttcaa gctgaatgag ctcagaggca agaagtcctg ccacacaggc 360

ctcggcaggt ccgctgggtg gaacatcccc atgggcaaac tttataagga attgcctgat 420

ccacaggaat caattcagag agctgcggcc aacttcttct ctgccagctg tgtcccctgt 480

gcggatcaat catcatttcc caaactctgt caactgtgtg cggggaaagg gacagacaag 540

tgtgcctgct ccaaccacga accatacttc ggctactcag gggcctttaa atgtctgatg 600

gagggcgctg gggatgtggc ctttgtcaag cactcaacgg tattcgacaa cctgcctaac 660

ccagaagaca ggaaaaacta tgagctgctc tgcggggaca acacccggaa gtctgtagat 720

aattaccaag aatgctactt ggcaatggtt ccttcccatg cggttgtggc tcgaactgtg 780

ggcggcaagg aggatgtgat ctgggaactt ctcaatcacg cccaggaaca ttttggcaaa 840

gataaaccag acaatttcca gcttttccaa tcccctcatg ggaaggacct gctgtttaag 900

gactctgctg atgggttttt aaagattcct tctaagatgg attttgagct gtacttggga 960

tatgaatatg tcactgctct tcagaatcta agagaaagta aacccccgga ctcctcaaag 1020

gatgagtgca tggtgaagtg gtgtgcaatt ggtcaccagg agaggacaaa gtgtgatcgg 1080

tggagtgggt tcagcggcgg ggcaatagag tgtgaaacag cagagaacac tgaagagtgc 1140

atcgccaaga tcatgaaagg agaagctgat gccatgagct tggacggagg ctacctctac 1200

atagcaggca aatgtggcct ggtgcctgtc ctggcagaga actacaaaac tgagggtgaa 1260

agttgcaaga acacaccaga aaaagggtat cttgctgtag ctgtggttaa aacatcagat 1320

gctaatatca actggaacaa tctgaaagac aagaagtcct gccacactgc agtagacaga 1380

accgctggct ggaacatccc catgggtttg ctctacagca agattaataa ctgtaaattt 1440

gatgaatttt tcagcgcagg ttgtgcacct ggatctccgc gaaattccag tctctgtgct 1500

ctgtgcattg gctcagagaa gggtacagga aaggagtgtg ttcccaacag caatgaaaga 1560

tactatggct atacaggggc tttcaggtgt ctggtggaga agggagacgt ggcctttgtg 1620

aaggaccaga ctgtcataca gaacactgac ggaaataata atgaagcatg ggcaaaaaat 1680

ctgaagaagg aaaattttga agtactatgc aaagatggca ccaggaaacc tgtgacagat 1740

gctgagaact gccacctggc ccgaggcccg aatcatgctg tggtctcacg gaaagataag 1800

gcaacttgtg tggagaaaat attaaacaaa cagcaggatg attttggaaa atctgtaacc 1860

gactgcacga gcaatttttg tttattccaa tcaaattcca aggaccttct gttcagggat 1920

gacactaaat gtttggcttc aattgcgaaa aaaacatatg actcctactt aggggatgac 1980

tacgtcagag ctatgaccaa cctgagacaa tgctcaacct caaaactcct ggaagcatgc 2040

actttccaca aacct 2055

<210>  4
<211>  685
<212>  PRT
<213>  Bos taurus

<400>  4

Asp Pro Glu Arg Thr Val Arg Trp Cys Thr Ile Ser Thr His Glu Ala
1               5                   10                  15

Asn Lys Cys Ala Ser Phe Arg Glu Asn Val Leu Arg Ile Leu Glu Ser
            20                  25                  30

Gly Pro Phe Val Ser Cys Val Lys Lys Thr Ser His Met Asp Cys Ile
        35                  40                  45

Lys Ala Ile Ser Asn Asn Glu Ala Asp Ala Val Thr Leu Asp Gly Gly
    50                  55                  60

Leu Val Tyr Glu Ala Gly Leu Lys Pro Asn Asn Leu Lys Pro Val Val
65                  70                  75                  80

Ala Glu Phe His Gly Thr Lys Asp Asn Pro Gln Thr His Tyr Tyr Ala
                85                  90                  95

Val Ala Val Val Lys Lys Asp Thr Asp Phe Lys Leu Asn Glu Leu Arg
            100                 105                 110

Gly Lys Lys Ser Cys His Thr Gly Leu Gly Arg Ser Ala Gly Trp Asn
        115                 120                 125

Ile Pro Met Gly Lys Leu Tyr Lys Glu Leu Pro Asp Pro Gln Glu Ser
    130                 135                 140

Ile Gln Arg Ala Ala Ala Asn Phe Phe Ser Ala Ser Cys Val Pro Cys
145                 150                 155                 160

Ala Asp Gln Ser Ser Phe Pro Lys Leu Cys Gln Leu Cys Ala Gly Lys
                165                 170                 175

Gly Thr Asp Lys Cys Ala Cys Ser Asn His Glu Pro Tyr Phe Gly Tyr
            180                 185                 190

Ser Gly Ala Phe Lys Cys Leu Met Glu Gly Ala Gly Asp Val Ala Phe
        195                 200                 205

Val Lys His Ser Thr Val Phe Asp Asn Leu Pro Asn Pro Glu Asp Arg
    210                 215                 220

Lys Asn Tyr Glu Leu Leu Cys Gly Asp Asn Thr Arg Lys Ser Val Asp
225                 230                 235                 240

Asn Tyr Gln Glu Cys Tyr Leu Ala Met Val Pro Ser His Ala Val Val
                245                 250                 255

Ala Arg Thr Val Gly Gly Lys Glu Asp Val Ile Trp Glu Leu Leu Asn
            260                 265                 270

His Ala Gln Glu His Phe Gly Lys Asp Lys Pro Asp Asn Phe Gln Leu
        275                 280                 285

Phe Gln Ser Pro His Gly Lys Asp Leu Leu Phe Lys Asp Ser Ala Asp
    290                 295                 300

Gly Phe Leu Lys Ile Pro Ser Lys Met Asp Phe Glu Leu Tyr Leu Gly
305                 310                 315                 320

Tyr Glu Tyr Val Thr Ala Leu Gln Asn Leu Arg Glu Ser Lys Pro Pro
                325                 330                 335

Asp Ser Ser Lys Asp Glu Cys Met Val Lys Trp Cys Ala Ile Gly His
            340                 345                 350

Gln Glu Arg Thr Lys Cys Asp Arg Trp Ser Gly Phe Ser Gly Gly Ala
        355                 360                 365

Ile Glu Cys Glu Thr Ala Glu Asn Thr Glu Glu Cys Ile Ala Lys Ile
    370                 375                 380

Met Lys Gly Glu Ala Asp Ala Met Ser Leu Asp Gly Gly Tyr Leu Tyr
385                 390                 395                 400

Ile Ala Gly Lys Cys Gly Leu Val Pro Val Leu Ala Glu Asn Tyr Lys
                405                 410                 415

Thr Glu Gly Glu Ser Cys Lys Asn Thr Pro Glu Lys Gly Tyr Leu Ala
            420                 425                 430

Val Ala Val Val Lys Thr Ser Asp Ala Asn Ile Asn Trp Asn Asn Leu
        435                 440                 445

Lys Asp Lys Lys Ser Cys His Thr Ala Val Asp Arg Thr Ala Gly Trp
    450                 455                 460

Asn Ile Pro Met Gly Leu Leu Tyr Ser Lys Ile Asn Asn Cys Lys Phe
465                 470                 475                 480

Asp Glu Phe Phe Ser Ala Gly Cys Ala Pro Gly Ser Pro Arg Asn Ser
                485                 490                 495

Ser Leu Cys Ala Leu Cys Ile Gly Ser Glu Lys Gly Thr Gly Lys Glu
            500                 505                 510

Cys Val Pro Asn Ser Asn Glu Arg Tyr Tyr Gly Tyr Thr Gly Ala Phe
        515                 520                 525

Arg Cys Leu Val Glu Lys Gly Asp Val Ala Phe Val Lys Asp Gln Thr
    530                 535                 540

Val Ile Gln Asn Thr Asp Gly Asn Asn Asn Glu Ala Trp Ala Lys Asn
545                 550                 555                 560

Leu Lys Lys Glu Asn Phe Glu Val Leu Cys Lys Asp Gly Thr Arg Lys
                565                 570                 575

Pro Val Thr Asp Ala Glu Asn Cys His Leu Ala Arg Gly Pro Asn His
            580                 585                 590

Ala Val Val Ser Arg Lys Asp Lys Ala Thr Cys Val Glu Lys Ile Leu
        595                 600                 605

Asn Lys Gln Gln Asp Asp Phe Gly Lys Ser Val Thr Asp Cys Thr Ser
    610                 615                 620

Asn Phe Cys Leu Phe Gln Ser Asn Ser Lys Asp Leu Leu Phe Arg Asp
625                 630                 635                 640

Asp Thr Lys Cys Leu Ala Ser Ile Ala Lys Lys Thr Tyr Asp Ser Tyr
                645                 650                 655

Leu Gly Asp Asp Tyr Val Arg Ala Met Thr Asn Leu Arg Gln Cys Ser
            660                 665                 670

Thr Ser Lys Leu Leu Glu Ala Cys Thr Phe His Lys Pro
        675                 680                 685

<210>  5
<211>  2070
<212>  DNA
<213>  Bos taurus

<400>  5
gacacaattg cacaggccgc cagcaccacc accatctctg atgctgtgag taaggtcaag      60

atccaggtca acaaggcctt cctggattcc cggaccaggc tgaagacgac cttgagctct     120

gaggcaccca ccacccaaca gctctcagag tacttcaagc acgcaaaggg ccggacccgc 180

acggccattc gcaacgggca ggtgtgggag gagtccttaa agaggctgag gcgggacaca 240

accctgacca acgtcacaga ccctagcctg gacttgactg cactctcctg ggaggtgggc 300

tgcggtgccc cggttcctct ggtgaaatgt gatgaaaaca gcccttaccg caccatcacg 360

ggagactgta ataacaggag gagccccgca ctgggcgccg ccaacagggc gctggcgcgc 420

tggctgccgg cggagtacga ggacgggctc gccctgccct tcggctggac gcagaggaag 480

acgcgcaacg gcttccgcgt cccgctggcc cgggaggtat ccaacaaaat tgtaggctac 540

ctggacgaag agggtgttct ggaccaaaac aggtccctgc tcttcatgca gtggggtcag 600

attgtggacc acgacctgga cttcgccccg gaaacggaac tggggagcaa cgagcactct 660

aaaacccagt gtgaggagta ctgtatccag ggagacaact gcttccccat catgttcccg 720

aaaaatgatc ccaagttgaa gactcaaggg aaatgcatgc ctttcttccg agccgggttt 780

gtctgcccca ctccacctta ccagtcgttg gcccgagaac agatcaatgc tgtgacctcc 840

ttcctggacg ccagcttagt gtacggctct gagcccagtc tggccagccg tctccggaac 900

ctcagcagcc cgctgggcct catggctgtc aaccaagaag cctgggacca cgggctggcc 960

tacctgccct ttaacaacaa gaagccgagc ccctgtgagt tcatcaacac caccgcccgc 1020

gtgccctgtt tcctggcggg agattttcga gcctcagagc agattctgct ggccactgcc 1080

cacaccctcc ttctccggga gcacaaccgg ctggccagag aactaaagaa actcaaccct 1140

cactggaatg gagagaagct ctaccaggaa gcccggaaaa tcctgggagc cttcatacag 1200

atcatcacct ttagggacta cctacccatt gtgctaggta gtgagatgca gaagtggatc 1260

ccgccctacc aaggctataa taactctgtg gatccccgaa tttccaatgt cttcaccttt 1320

gccttccgct ttggccacat ggaggttccc tccactgtgt cccgcctgga tgagaattac 1380

cagccatggg gtccggaagc agagctcccc ctacacaccc tcttcttcaa cacctggagg 1440

ataatcaaag acggtggaat tgaccctctg gtgcggggtc tgctggccaa gaagtccaaa 1500

ctgatgaatc aggataaaat ggtgacgagt gagctgcgca acaagctttt ccagcccact 1560

cacaagatcc acggctttga cctggctgct atcaacttac agcgttgccg agaccatggg 1620

atgcctgggt acaactcctg gaggggcttc tgtggcctct cacagcccaa gacactgaag 1680

gggctgcaga ctgtgctgaa gaacaagata ctggctaaga agttaatgga tctctataag 1740

acccccgaca acattgacat ctggattgga ggcaacgctg agcccatggt agaaaggggc 1800

cgggtggggc cactcctggc ctgcctccta gggaggcaat tccagcagat acgtgatggg 1860

gacaggttct ggtgggagaa ccctggggtc ttcactgaga agcagcggga ctctctacag 1920

aaagtgtcct tctcacgcct catctgtgac aacacccaca tcacgaaggt cccgctgcat 1980

gccttccagg ccaacaacta cccacatgac tttgtggatt gctcaaccgt tgataagctg 2040

gatctctcac cctgggcctc cagggagaat                                    2070

<210>  6
<211>  690
<212>  PRT
<213>  Bos taurus

<400>  6

Asp Thr Ile Ala Gln Ala Ala Ser Thr Thr Thr Ile Ser Asp Ala Val
1               5                   10                  15

Ser Lys Val Lys Ile Gln Val Asn Lys Ala Phe Leu Asp Ser Arg Thr
            20                  25                  30

Arg Leu Lys Thr Thr Leu Ser Ser Glu Ala Pro Thr Thr Gln Gln Leu
        35                  40                  45

Ser Glu Tyr Phe Lys His Ala Lys Gly Arg Thr Arg Thr Ala Ile Arg
    50                  55                  60

Asn Gly Gln Val Trp Glu Glu Ser Leu Lys Arg Leu Arg Arg Asp Thr
65                  70                  75                  80

Thr Leu Thr Asn Val Thr Asp Pro Ser Leu Asp Leu Thr Ala Leu Ser
                85                  90                  95

Trp Glu Val Gly Cys Gly Ala Pro Val Pro Leu Val Lys Cys Asp Glu
            100                 105                 110

Asn Ser Pro Tyr Arg Thr Ile Thr Gly Asp Cys Asn Asn Arg Arg Ser
        115                 120                 125

Pro Ala Leu Gly Ala Ala Asn Arg Ala Leu Ala Arg Trp Leu Pro Ala
    130                 135                 140

Glu Tyr Glu Asp Gly Leu Ala Leu Pro Phe Gly Trp Thr Gln Arg Lys
145                 150                 155                 160

Thr Arg Asn Gly Phe Arg Val Pro Leu Ala Arg Glu Val Ser Asn Lys
                165                 170                 175

Ile Val Gly Tyr Leu Asp Glu Glu Gly Val Leu Asp Gln Asn Arg Ser
            180                 185                 190

Leu Leu Phe Met Gln Trp Gly Gln Ile Val Asp His Asp Leu Asp Phe
        195                 200                 205

Ala Pro Glu Thr Glu Leu Gly Ser Asn Glu His Ser Lys Thr Gln Cys
    210                 215                 220

Glu Glu Tyr Cys Ile Gln Gly Asp Asn Cys Phe Pro Ile Met Phe Pro
225                 230                 235                 240

Lys Asn Asp Pro Lys Leu Lys Thr Gln Gly Lys Cys Met Pro Phe Phe
                245                 250                 255

Arg Ala Gly Phe Val Cys Pro Thr Pro Pro Tyr Gln Ser Leu Ala Arg
            260                 265                 270

Glu Gln Ile Asn Ala Val Thr Ser Phe Leu Asp Ala Ser Leu Val Tyr
        275                 280                 285

Gly Ser Glu Pro Ser Leu Ala Ser Arg Leu Arg Asn Leu Ser Ser Pro
    290                 295                 300

Leu Gly Leu Met Ala Val Asn Gln Glu Ala Trp Asp His Gly Leu Ala
305                 310                 315                 320

Tyr Leu Pro Phe Asn Asn Lys Lys Pro Ser Pro Cys Glu Phe Ile Asn
                325                 330                 335

Thr Thr Ala Arg Val Pro Cys Phe Leu Ala Gly Asp Phe Arg Ala Ser
            340                 345                 350

Glu Gln Ile Leu Leu Ala Thr Ala His Thr Leu Leu Leu Arg Glu His
        355                 360                 365

Asn Arg Leu Ala Arg Glu Leu Lys Lys Leu Asn Pro His Trp Asn Gly
    370                 375                 380

Glu Lys Leu Tyr Gln Glu Ala Arg Lys Ile Leu Gly Ala Phe Ile Gln
385                 390                 395                 400

Ile Ile Thr Phe Arg Asp Tyr Leu Pro Ile Val Leu Gly Ser Glu Met
                405                 410                 415

Gln Lys Trp Ile Pro Pro Tyr Gln Gly Tyr Asn Asn Ser Val Asp Pro
            420                 425                 430

Arg Ile Ser Asn Val Phe Thr Phe Ala Phe Arg Phe Gly His Met Glu
        435                 440                 445

Val Pro Ser Thr Val Ser Arg Leu Asp Glu Asn Tyr Gln Pro Trp Gly
    450                 455                 460

Pro Glu Ala Glu Leu Pro Leu His Thr Leu Phe Phe Asn Thr Trp Arg
465                 470                 475                 480

Ile Ile Lys Asp Gly Gly Ile Asp Pro Leu Val Arg Gly Leu Leu Ala
                485                 490                 495

Lys Lys Ser Lys Leu Met Asn Gln Asp Lys Met Val Thr Ser Glu Leu
            500                 505                 510

Arg Asn Lys Leu Phe Gln Pro Thr His Lys Ile His Gly Phe Asp Leu
        515                 520                 525

Ala Ala Ile Asn Leu Gln Arg Cys Arg Asp His Gly Met Pro Gly Tyr
    530                 535                 540

Asn Ser Trp Arg Gly Phe Cys Gly Leu Ser Gln Pro Lys Thr Leu Lys
545                 550                 555                 560

Gly Leu Gln Thr Val Leu Lys Asn Lys Ile Leu Ala Lys Lys Leu Met
                565                 570                 575

Asp Leu Tyr Lys Thr Pro Asp Asn Ile Asp Ile Trp Ile Gly Gly Asn
            580                 585                 590

Ala Glu Pro Met Val Glu Arg Gly Arg Val Gly Pro Leu Leu Ala Cys
        595                 600                 605

Leu Leu Gly Arg Gln Phe Gln Gln Ile Arg Asp Gly Asp Arg Phe Trp
    610                 615                 620

Trp Glu Asn Pro Gly Val Phe Thr Glu Lys Gln Arg Asp Ser Leu Gln
625                 630                 635                 640

Lys Val Ser Phe Ser Arg Leu Ile Cys Asp Asn Thr His Ile Thr Lys
                645                 650                 655

Val Pro Leu His Ala Phe Gln Ala Asn Asn Tyr Pro His Asp Phe Val
            660                 665                 670

Asp Cys Ser Thr Val Asp Lys Leu Asp Leu Ser Pro Trp Ala Ser Arg
        675                 680                 685

Glu Asn
    690

<210>  7
<211>  1356

<212>  DNA
<213>  Bos taurus

<400>  7
tcccaggtgc agctgcggga gtcgggcccc agcctggtga agccctcaca gaccctctcc      60

ctcacgtgca cggtgtctgg attctcattg agcagctatg ctctaacctg ggtccgccag     120

gctccaggga aggcgctgga gtgggttggt ggtataacca gtggtggaac cacatactat     180

aatccagccc tgaaatcccg gctcagcatc accaaggaga actccaagag ccaagtctct     240

ctgtcagtga gcagcgtgac acctgaggac acagccacat actactgtgc aagaagtact     300

tatggtgagg ttggtgatgg tgccatcgcc gatgcctggg gccaaggact cctggtcacc     360

gtctcctcag cctccaccac agccccgaaa gtctaccctc tgagttcttg ctgcggggac     420

aagtccagct ccaccgtgac cctgggctgc ctggtctcca gctacatgcc cgagccggtg     480

accgtgacct ggaactcggg tgccctgaag agcggcgtgc acaccttccc ggctgtcctt     540

cagtcctccg ggctgtactc tctcagcagc atggtgaccg tgcccggcag cacctcagga     600

cagaccttca cctgcaacgt agcccacccg gccagcagca ccaaggtgga caaggctgtt     660

gatcccacat gcaaaccatc accctgtgac tgttgcccac cccctgagct ccccggagga     720

ccctctgtct tcatcttccc accgaaaccc aaggacaccc tcacaatctc gggaacgccc     780

gaggtcacgt gtgtggtggt ggacgtgggc cacgatgacc ccgaggtgaa gttctcctgg     840

ttcgtggacg acgtggaggt aaacacagcc acgacgaagc cgagagagga gcagttcaac     900

agcacctacc gcgtggtcag cgccctgcgc atccagcacc aggactggac tggaggaaag     960

gagttcaagt gcaaggtcca caacgaaggc ctcccggccc ccatcgtgag gaccatctcc    1020

aggaccaaag ggccggcccg ggagccgcag gtgtatgtcc tggccccacc ccaggaagag    1080

ctcagcaaaa gcacggtcag cctcacctgc atggtcacca gcttctaccc agactacatc    1140

gccgtggagt ggcagagaaa cgggcagcct gagtcggagg acaagtacgg cacgaccccg    1200

ccccagctgg acgccgacag ctcctacttc ctgtacagca agctcagggt ggacaggaac    1260

agctggcagg aaggagacac ctacacgtgt gtggtgatgc acgaggccct gcacaatcac    1320

tacacgcaga agtccacctc taagtctgcg ggtaaa                              1356


<210>  8
<211>  452
<212>  PRT
<213>  Bos taurus

<400>  8

Ser Gln Val Gln Leu Arg Glu Ser Gly Pro Ser Leu Val Lys Pro Ser
1               5                   10                  15


Gln Thr Leu Ser Leu Thr Cys Thr Val Ser Gly Phe Ser Leu Ser Ser

20                          25                          30

Tyr Ala Leu Thr Trp Val Arg Gln Ala Pro Gly Lys Ala Leu Glu Trp
            35                          40                          45

Val Gly Gly Ile Thr Ser Gly Gly Thr Thr Tyr Tyr Asn Pro Ala Leu
    50                          55                          60

Lys Ser Arg Leu Ser Ile Thr Lys Glu Asn Ser Lys Ser Gln Val Ser
65                          70                          75                          80

Leu Ser Val Ser Ser Val Thr Pro Glu Asp Thr Ala Thr Tyr Tyr Cys
                        85                          90                          95

Ala Arg Ser Thr Tyr Gly Glu Val Gly Asp Gly Ala Ile Ala Asp Ala
                100                         105                         110

Trp Gly Gln Gly Leu Leu Val Thr Val Ser Ser Ala Ser Thr Thr Ala
            115                         120                         125

Pro Lys Val Tyr Pro Leu Ser Ser Cys Cys Gly Asp Lys Ser Ser Ser
    130                         135                         140

Thr Val Thr Leu Gly Cys Leu Val Ser Ser Tyr Met Pro Glu Pro Val
145                         150                         155                         160

Thr Val Thr Trp Asn Ser Gly Ala Leu Lys Ser Gly Val His Thr Phe
                        165                         170                         175

Pro Ala Val Leu Gln Ser Ser Gly Leu Tyr Ser Leu Ser Ser Met Val
                180                         185                         190

Thr Val Pro Gly Ser Thr Ser Gly Gln Thr Phe Thr Cys Asn Val Ala
            195                         200                         205

His Pro Ala Ser Ser Thr Lys Val Asp Lys Ala Val Asp Pro Thr Cys
    210                         215                         220

Lys Pro Ser Pro Cys Asp Cys Cys Pro Pro Pro Glu Leu Pro Gly Gly
225                         230                         235                         240

Pro Ser Val Phe Ile Phe Pro Pro Lys Pro Lys Asp Thr Leu Thr Ile
                        245                         250                         255

Ser Gly Thr Pro Glu Val Thr Cys Val Val Val Asp Val Gly His Asp
                260                         265                         270

Asp Pro Glu Val Lys Phe Ser Trp Phe Val Asp Asp Val Glu Val Asn
        275                 280                 285

Thr Ala Thr Thr Lys Pro Arg Glu Glu Gln Phe Asn Ser Thr Tyr Arg
    290                 295                 300

Val Val Ser Ala Leu Arg Ile Gln His Gln Asp Trp Thr Gly Gly Lys
305                 310                 315                 320

Glu Phe Lys Cys Lys Val His Asn Glu Gly Leu Pro Ala Pro Ile Val
                325                 330                 335

Arg Thr Ile Ser Arg Thr Lys Gly Pro Ala Arg Glu Pro Gln Val Tyr
            340                 345                 350

Val Leu Ala Pro Pro Gln Glu Glu Leu Ser Lys Ser Thr Val Ser Leu
        355                 360                 365

Thr Cys Met Val Thr Ser Phe Tyr Pro Asp Tyr Ile Ala Val Glu Trp
    370                 375                 380

Gln Arg Asn Gly Gln Pro Glu Ser Glu Asp Lys Tyr Gly Thr Thr Pro
385                 390                 395                 400

Pro Gln Leu Asp Ala Asp Ser Ser Tyr Phe Leu Tyr Ser Lys Leu Arg
                405                 410                 415

Val Asp Arg Asn Ser Trp Gln Glu Gly Asp Thr Tyr Thr Cys Val Val
            420                 425                 430

Met His Glu Ala Leu His Asn His Tyr Thr Gln Lys Ser Thr Ser Lys
        435                 440                 445

Ser Ala Gly Lys
    450

<210>  9
<211>  1350
<212>  DNA
<213>  Bos taurus

<400>  9
atggcatctg ttgcagctga accacaactg agtgtggtga ccagggtggc caacctcccc      60

ttggtgagct ccacgtatga tctggtctcc tcggcttaca tcagtagaaa ggatcagtat     120

ccttacttga agtctctgtg tgagatggca gagaagggca tgaagaccat cacttctgtg     180

gctgtgacca gcgcgctgcc aatcatccaa aagttagagc cacaaattgc tgttgccaat     240

acctatgcgt gtaaggggct agacaggatt gaggagaagc tgcctattct gaatcagcca     300

acaaaccagg ttgtggccaa tgccaaaggg gctatgactg gggcaaaaga tgctgtgacg 360

actactgtga ccggggccaa ggattctgtg gccagcacaa tcacgggggt ggtggacagg 420

accaagggag ctgtgactgg tagtgtggag aagaccaagt ccgtggtcag tggcagcatt 480

aacacagtcc tacgaagtcg ggtgatgcag ctgatgagca gtggagtaga aaatgcactc 540

accaaatcag agctgctggt agaccagtac ctccctctta ccaaggatga actagaaaaa 600

gaagccaaaa aagtggaagg attcgatatg gttcagaagc cgagttacta tgttagactg 660

ggatctctgt ccaccaagct gcgctcacgg gcctaccagc aggccctttg tagggttgaa 720

gaagctaagc gaaaaggcca ggagaccatt tctcagctcc attccgcctt caacctgagt 780

gaacttgcca ggaagaatgt gcataatgcc aaccagaaaa ttcaggatgc tcaggataag 840

ctctatctgt cctggctgga gtggaagaga agcatcggct acgatgatac agatgaatcc 900

cactgtgctg agcacattga gtcacgtact cttgctattg cccggaacct gactcagcag 960

ctccagacca tgtgccacac cctcctgtcc aacatccagg ggttaccaca gaacattcaa 1020

gatcgggcca accacttggg ggtgatggct ggtgacatct actctgtgtt tcgcaatgct 1080

gcctccttta aagaagtgtc tgatggcctc ctcgcttcca gcaaggggca gctgcagaaa 1140

atgaaggagt ctttagatga tgtgatggat tatcttgtta acaacacacc cctcaactgg 1200

ctggtaggtc ccttttatcc ccaggtgacc gagtctgaga gtgctcaggc cccaggtaca 1260

accaggaggc ctggcaggtg gagtagaaaa caccctaaac ccgtccctgt cagcaatgca 1320

gagggcagcc agccagatga cagctcctct 1350

<210> 10
<211> 450
<212> PRT
<213> Bos taurus

<400> 10

Met Ala Ser Val Ala Ala Glu Pro Gln Leu Ser Val Val Thr Arg Val
1               5                   10                  15

Ala Asn Leu Pro Leu Val Ser Ser Thr Tyr Asp Leu Val Ser Ser Ala
            20                  25                  30

Tyr Ile Ser Arg Lys Asp Gln Tyr Pro Tyr Leu Lys Ser Leu Cys Glu
        35                  40                  45

Met Ala Glu Lys Gly Met Lys Thr Ile Thr Ser Val Ala Val Thr Ser
    50                  55                  60

Ala Leu Pro Ile Ile Gln Lys Leu Glu Pro Gln Ile Ala Val Ala Asn
65                  70                  75                  80

Thr Tyr Ala Cys Lys Gly Leu Asp Arg Ile Glu Glu Lys Leu Pro Ile
                85                  90                  95

Leu Asn Gln Pro Thr Asn Gln Val Val Ala Asn Ala Lys Gly Ala Met
            100                 105                 110

Thr Gly Ala Lys Asp Ala Val Thr Thr Thr Val Thr Gly Ala Lys Asp
        115                 120                 125

Ser Val Ala Ser Thr Ile Thr Gly Val Val Asp Arg Thr Lys Gly Ala
    130                 135                 140

Val Thr Gly Ser Val Glu Lys Thr Lys Ser Val Val Ser Gly Ser Ile
145                 150                 155                 160

Asn Thr Val Leu Arg Ser Arg Val Met Gln Leu Met Ser Ser Gly Val
                165                 170                 175

Glu Asn Ala Leu Thr Lys Ser Glu Leu Leu Val Asp Gln Tyr Leu Pro
            180                 185                 190

Leu Thr Lys Asp Glu Leu Glu Lys Glu Ala Lys Lys Val Glu Gly Phe
        195                 200                 205

Asp Met Val Gln Lys Pro Ser Tyr Tyr Val Arg Leu Gly Ser Leu Ser
    210                 215                 220

Thr Lys Leu Arg Ser Arg Ala Tyr Gln Gln Ala Leu Cys Arg Val Glu
225                 230                 235                 240

Glu Ala Lys Arg Lys Gly Gln Glu Thr Ile Ser Gln Leu His Ser Ala
                245                 250                 255

Phe Asn Leu Ser Glu Leu Ala Arg Lys Asn Val His Asn Ala Asn Gln
            260                 265                 270

Lys Ile Gln Asp Ala Gln Asp Lys Leu Tyr Leu Ser Trp Leu Glu Trp
        275                 280                 285

Lys Arg Ser Ile Gly Tyr Asp Asp Thr Asp Glu Ser His Cys Ala Glu
    290                 295                 300

His Ile Glu Ser Arg Thr Leu Ala Ile Ala Arg Asn Leu Thr Gln Gln
305                 310                 315                 320

Leu Gln Thr Met Cys His Thr Leu Leu Ser Asn Ile Gln Gly Leu Pro

325                 330                 335

Gln Asn Ile Gln Asp Arg Ala Asn His Leu Gly Val Met Ala Gly Asp
            340                 345                 350

Ile Tyr Ser Val Phe Arg Asn Ala Ala Ser Phe Lys Glu Val Ser Asp
        355                 360                 365

Gly Leu Leu Ala Ser Ser Lys Gly Gln Leu Gln Lys Met Lys Glu Ser
    370                 375                 380

Leu Asp Asp Val Met Asp Tyr Leu Val Asn Asn Thr Pro Leu Asn Trp
385                 390                 395                 400

Leu Val Gly Pro Phe Tyr Pro Gln Val Thr Glu Ser Glu Ser Ala Gln
                405                 410                 415

Ala Pro Gly Thr Thr Arg Arg Pro Gly Arg Trp Ser Arg Lys His Pro
            420                 425                 430

Lys Pro Val Pro Val Ser Asn Ala Glu Gly Ser Gln Pro Asp Asp Ser
        435                 440                 445

Ser Ser
    450

<210>  11
<211>  2217
<212>  DNA
<213>  Bos taurus

<400>  11
aagagtccca tcttcggtcc cgaggaggtg accagcgtgg aaggccgctc agtgtccatc      60

aagtgctact acccgcccac ctccgtcaac cggcacacgc gcaagtactg gtgccggcag     120

ggagcccagg gccgctgcac gaccctcatc tcctcggagg gctacgtctc cgacgactac     180

gtgggcagag ccaacctcac caacttcccg gagagcggca cgtttgtggt ggacatcagc     240

catctcaccc ataaagactc agggcgctac aagtgtggcc tgggcattag cagccgtggc     300

cttaacttcg atgtgagcct ggaggtcagc caagatcctg cacaggcaag tcatgcccac     360

gtctacactg tagacctggg caggactgtg accatcaact gccctttcac gcgtgcgaat     420

tctgagaaga gaaaatcctt gtgcaagaag acaatccagg actgtttcca agttgtcgac     480

tccaccgggt atgtgagcaa cagctataaa gacagagcac atatcagtat cctaggtacc     540

aacacattag tgttcagcgt tgtcatcaac cgagtcaagc tcagtgatgc tgggatgtat     600

gtctgccagg ctggggacga tgccaaagcc gataaaatca acattgacct ccaggtgctg     660

gagcctgagc ctgagctggt ttatggagac ttgaggagct cggtgacctt tgactgttcc 720

ctgggccccg aggtggcaaa tgtgcccaaa tttctgtgcc agaagaagaa tgggggagct 780

tgcaatgtgg tcatcaacac gttggggaag aaggctcagg acttccaggg caggatcgtg 840

tccgtgccca aggacaatgg tgtcttcagt gtgcacatta ccagcctgag gaaagaggac 900

gcagggcgct acgtgtgcgg ggcccagcct gagggtgagc cccaggacgg ctggcctgtg 960

caggcctggc aactcttcgt caatgaagag acggcaatcc ccgcaagccc ctccgtggtg 1020

aaaggtgtga ggggaggctc tgtgaccgta tcttgcccct acaacccgaa ggatgccaac 1080

agcgcgaagt actggtgtca ctgggaagag gctcaaaacg gccgctgccc gcggctggtg 1140

gagagccggg ggctgataaa ggagcagtac gagggcaggc tggcgctgct taccgagccg 1200

ggcaacggca cctacaccgt catcctcaac cagctcaccg accaggacac cggcttctac 1260

tggtgcgtga ccgacggcga cacgcgctgg atctccacag tggagctcaa ggttgtccaa 1320

ggagaaccaa gcctcaaggt acccaagaac gtcacggctt ggctgggaga gcccttaaag 1380

ctctcctgcc acttcccctg caaattctac tcctttgaga agtactggtg taagtggagc 1440

aacagaggct gcagcgccct gcccacccag aacgacggcc ccagccaggc ctttgtgagc 1500

tgtgaccaga acagccaggt cgtctccctg aacctggaca cagtcaccaa ggaggatgaa 1560

ggctggtact ggtgtggagt gaaggagggc ccccgatatg gggagacggc ggctgtctac 1620

gtggcagtgg agagcagggt gaaggggtcc caaggcgcca agcaagtgaa agctgcccct 1680

gcgggggcgg caatacagtc gagggccggg gagatccaga acaaagccct tctggacccc 1740

agctttttcg caaaggaaag tgtgaaggac gctgctggtg gacccggagc acctgcagat 1800

cctggccgcc ctacaggata cagcgggagc tccaaagcac tggtctccac cctggtgccc 1860

ctggccctgg tcctggtcgc aggggtcgtg gcgatcgggg tggtccgagc ccggcacagg 1920

aagaacgtcg accggatttc aatcaggagc taccggacag atatcagcat gtcagacttt 1980

gagaactcca gggattttga aggacgtgac aacatgggag cctctccaga ggcccaagag 2040

acgtctctcg gagggaagga cgagtttgcc accactaccg aggacaccgt ggagagcaaa 2100

gaacccaaga aggcaaagag gtcgtccaag gaggaagccg acgaggcctt caccaccttc 2160

ctcctccagg ccaaaaacct ggcctccgcc gcaacccaga acggcccgac agaagcc 2217

<210> 12
<211> 739
<212> PRT
<213> Bos taurus

<400> 12

Lys Ser Pro Ile Phe Gly Pro Glu Glu Val Thr Ser Val Glu Gly Arg
1               5                   10                  15

Ser Val Ser Ile Lys Cys Tyr Tyr Pro Pro Thr Ser Val Asn Arg His
            20                  25                  30

Thr Arg Lys Tyr Trp Cys Arg Gln Gly Ala Gln Gly Arg Cys Thr Thr
        35                  40                  45

Leu Ile Ser Ser Glu Gly Tyr Val Ser Asp Asp Tyr Val Gly Arg Ala
    50                  55                  60

Asn Leu Thr Asn Phe Pro Glu Ser Gly Thr Phe Val Val Asp Ile Ser
65                  70                  75                  80

His Leu Thr His Lys Asp Ser Gly Arg Tyr Lys Cys Gly Leu Gly Ile
                85                  90                  95

Ser Ser Arg Gly Leu Asn Phe Asp Val Ser Leu Glu Val Ser Gln Asp
            100                 105                 110

Pro Ala Gln Ala Ser His Ala His Val Tyr Thr Val Asp Leu Gly Arg
        115                 120                 125

Thr Val Thr Ile Asn Cys Pro Phe Thr Arg Ala Asn Ser Glu Lys Arg
    130                 135                 140

Lys Ser Leu Cys Lys Lys Thr Ile Gln Asp Cys Phe Gln Val Val Asp
145                 150                 155                 160

Ser Thr Gly Tyr Val Ser Asn Ser Tyr Lys Asp Arg Ala His Ile Ser
                165                 170                 175

Ile Leu Gly Thr Asn Thr Leu Val Phe Ser Val Val Ile Asn Arg Val
            180                 185                 190

Lys Leu Ser Asp Ala Gly Met Tyr Val Cys Gln Ala Gly Asp Asp Ala
        195                 200                 205

Lys Ala Asp Lys Ile Asn Ile Asp Leu Gln Val Leu Glu Pro Glu Pro
    210                 215                 220

Glu Leu Val Tyr Gly Asp Leu Arg Ser Ser Val Thr Phe Asp Cys Ser
225                 230                 235                 240

Leu Gly Pro Glu Val Ala Asn Val Pro Lys Phe Leu Cys Gln Lys Lys
                245                 250                 255

Asn Gly Gly Ala Cys Asn Val Val Ile Asn Thr Leu Gly Lys Lys Ala
            260                 265                 270

Gln Asp Phe Gln Gly Arg Ile Val Ser Val Pro Lys Asp Asn Gly Val
        275                 280                 285

Phe Ser Val His Ile Thr Ser Leu Arg Lys Glu Asp Ala Gly Arg Tyr
    290                 295                 300

Val Cys Gly Ala Gln Pro Glu Gly Glu Pro Gln Asp Gly Trp Pro Val
305                 310                 315                 320

Gln Ala Trp Gln Leu Phe Val Asn Glu Glu Thr Ala Ile Pro Ala Ser
                325                 330                 335

Pro Ser Val Val Lys Gly Val Arg Gly Gly Ser Val Thr Val Ser Cys
            340                 345                 350

Pro Tyr Asn Pro Lys Asp Ala Asn Ser Ala Lys Tyr Trp Cys His Trp
        355                 360                 365

Glu Glu Ala Gln Asn Gly Arg Cys Pro Arg Leu Val Glu Ser Arg Gly
    370                 375                 380

Leu Ile Lys Glu Gln Tyr Glu Gly Arg Leu Ala Leu Leu Thr Glu Pro
385                 390                 395                 400

Gly Asn Gly Thr Tyr Thr Val Ile Leu Asn Gln Leu Thr Asp Gln Asp
                405                 410                 415

Thr Gly Phe Tyr Trp Cys Val Thr Asp Gly Asp Thr Arg Trp Ile Ser
            420                 425                 430

Thr Val Glu Leu Lys Val Val Gln Gly Glu Pro Ser Leu Lys Val Pro
        435                 440                 445

Lys Asn Val Thr Ala Trp Leu Gly Glu Pro Leu Lys Leu Ser Cys His
    450                 455                 460

Phe Pro Cys Lys Phe Tyr Ser Phe Glu Lys Tyr Trp Cys Lys Trp Ser
465                 470                 475                 480

Asn Arg Gly Cys Ser Ala Leu Pro Thr Gln Asn Asp Gly Pro Ser Gln
                485                 490                 495

Ala Phe Val Ser Cys Asp Gln Asn Ser Gln Val Val Ser Leu Asn Leu
            500                 505                 510

Asp Thr Val Thr Lys Glu Asp Glu Gly Trp Tyr Trp Cys Gly Val Lys

        515                 520                 525

Glu Gly Pro Arg Tyr Gly Glu Thr Ala Ala Val Tyr Val Ala Val Glu
    530                 535                 540

Ser Arg Val Lys Gly Ser Gln Gly Ala Lys Gln Val Lys Ala Ala Pro
545                 550                 555                 560

Ala Gly Ala Ala Ile Gln Ser Arg Ala Gly Glu Ile Gln Asn Lys Ala
                565                 570                 575

Leu Leu Asp Pro Ser Phe Phe Ala Lys Glu Ser Val Lys Asp Ala Ala
            580                 585                 590

Gly Gly Pro Gly Ala Pro Ala Asp Pro Gly Arg Pro Thr Gly Tyr Ser
        595                 600                 605

Gly Ser Ser Lys Ala Leu Val Ser Thr Leu Val Pro Leu Ala Leu Val
    610                 615                 620

Leu Val Ala Gly Val Val Ala Ile Gly Val Val Arg Ala Arg His Arg
625                 630                 635                 640

Lys Asn Val Asp Arg Ile Ser Ile Arg Ser Tyr Arg Thr Asp Ile Ser
                645                 650                 655

Met Ser Asp Phe Glu Asn Ser Arg Asp Phe Glu Gly Arg Asp Asn Met
            660                 665                 670

Gly Ala Ser Pro Glu Ala Gln Glu Thr Ser Leu Gly Gly Lys Asp Glu
        675                 680                 685

Phe Ala Thr Thr Thr Glu Asp Thr Val Glu Ser Lys Glu Pro Lys Lys
    690                 695                 700

Ala Lys Arg Ser Ser Lys Glu Glu Ala Asp Glu Ala Phe Thr Thr Phe
705                 710                 715                 720

Leu Leu Gln Ala Lys Asn Leu Ala Ser Ala Ala Thr Gln Asn Gly Pro
                725                 730                 735

Thr Glu Ala

<210> 13
<211> 1938
<212> DNA
<213> Bos taurus

<400> 13
aaccccatgt attccatgat cacccccaac atcctgcgtg tggagagcga ggagactgtg 60
gtgctggagg cccacggagg acaagggact attcaggtct cggtcaccgt ccacgacttc 120
ccggccaaga aacaagtgct gtcaaacgaa aacacccagc tgaacagcaa caatggctac 180
ctgagcaccg tcaccatcaa gatcccggcc agcaaagagt taaaatccga caaggggcac 240
aagttcgtga ctgtcgtagc gaccttcggg aatgtccagg tggagaaggt ggtgctgatc 300
agccttcaga gcgggtacct cttcatccag acggacaaga ccatctacac cccaggctcc 360
acggtcctct atcgggtctt cactgttgac cacaagctgc tgcccgtggg ccagacggtt 420
ttcatcacca tcgagacccc tgatggcatc cctgtcaagc gggactccaa gtcttctcag 480
aaccagtttg gcattttgac cttgtcctgg aacattccag agctagtgaa catgggggtg 540
tggaagatca aagcctacta tgaagattct ccgcagcagg tcttctccgc tgagtttgag 600
gtgaaggaat atgtgcttcc cagttttgag gtccaactag agcctgaaga gaaattctac 660
tacattgatg acccagatgg cctgaaggtc aacatcattg ccaggttctt gtacggggaa 720
caggtggacg gaacagcttt cgtcatcttt ggggtccagg atggcgaccg gagaatttca 780
ttgactcact ccctcacccg tgttccgatc aacgatggta atggggaggc catactgaaa 840
cgtcaggttt tgctgaatgg agtacagccc tcccgagctg atgccctggt gggcaagtcc 900
atatatgtgt ccgccaccgt catcttgcaa tcaggcagtg acatggtgga ggcagagcgc 960
accgggatcc ccattgtgac ttccccctac cagatccatt tcaccaagac ccccaagttc 1020
ttcaaacctg ccatgccctt cgacctcatg gtgtacgtga caaaccccga cggctccccc 1080
gctcgccaca tcccggtggt gacccagggc agcaatgtgc agtccttgac ccaggatgat 1140
ggcgtggcca agctaagcat caacacacag aacaaacggg atcccctgac catcaccgta 1200
cgcacaaaaa aggacaatat ccccgagggg cgacaggcca ccaggaccat gcaggcccta 1260
ccctacaaca cccaggggaa ctccaataac tacctgcacc tctctgtgcc ccgcgtggag 1320
ctcaagcctg ggagactctc caacgtcaac ttccacctgc gcacggaccc cggcgagcaa 1380
gccaagatcc gctactacac ctacatgatc atgaacaagg ggaagctgtt gaaggtggga 1440
cgccagtacc gagagcccgg ccaggaccta gtggtgttgc ccttgaccat cacgtcagac 1500
ttcatccctt ccttccgcct ggtggcctat tacacactga ttaatgccaa ggccagaggg 1560
gaggtggtgg ccgactccgt gtgggtggat gtcaaggact cgtgtatggg cacgctggtg 1620
gtgaaaaatg gtgggaagga agagaaacac catcgacccg ggcaacagat aaccctgaag 1680
atcgaggctg accagggggc ccgagtgggg ctcgtggccg tggacaaggg cgtgtttgtg 1740
ctgaataaga agaacaaatt gacccagaga aagatctggg acgtggtgga gaaagcggac 1800
attggctgca ccccaggcag tggaagaaac tatgctggcg tcttcacgga tgcaggactc 1860

actctcaaga ccagccaggg cctggagacc cagcagaggg cagatccgca gtgcccgcaa 1920

ccagccaccc gccgacgc 1938

<210> 14
<211> 646
<212> PRT
<213> Bos taurus

<400> 14

Asn Pro Met Tyr Ser Met Ile Thr Pro Asn Ile Leu Arg Val Glu Ser
1               5                   10                  15

Glu Glu Thr Val Val Leu Glu Ala His Gly Gly Gln Gly Thr Ile Gln
            20                  25                  30

Val Ser Val Thr Val His Asp Phe Pro Ala Lys Lys Gln Val Leu Ser
        35                  40                  45

Asn Glu Asn Thr Gln Leu Asn Ser Asn Asn Gly Tyr Leu Ser Thr Val
    50                  55                  60

Thr Ile Lys Ile Pro Ala Ser Lys Glu Leu Lys Ser Asp Lys Gly His
65                  70                  75                  80

Lys Phe Val Thr Val Val Ala Thr Phe Gly Asn Val Gln Val Glu Lys
                85                  90                  95

Val Val Leu Ile Ser Leu Gln Ser Gly Tyr Leu Phe Ile Gln Thr Asp
            100                 105                 110

Lys Thr Ile Tyr Thr Pro Gly Ser Thr Val Leu Tyr Arg Val Phe Thr
        115                 120                 125

Val Asp His Lys Leu Leu Pro Val Gly Gln Thr Val Phe Ile Thr Ile
    130                 135                 140

Glu Thr Pro Asp Gly Ile Pro Val Lys Arg Asp Ser Lys Ser Ser Gln
145                 150                 155                 160

Asn Gln Phe Gly Ile Leu Thr Leu Ser Trp Asn Ile Pro Glu Leu Val
                165                 170                 175

Asn Met Gly Val Trp Lys Ile Lys Ala Tyr Tyr Glu Asp Ser Pro Gln
            180                 185                 190

Gln Val Phe Ser Ala Glu Phe Glu Val Lys Glu Tyr Val Leu Pro Ser
        195                 200                 205

Phe Glu Val Gln Leu Glu Pro Glu Glu Lys Phe Tyr Tyr Ile Asp Asp
    210                 215                 220

Pro Asp Gly Leu Lys Val Asn Ile Ile Ala Arg Phe Leu Tyr Gly Glu
225                 230                 235                 240

Gln Val Asp Gly Thr Ala Phe Val Ile Phe Gly Val Gln Asp Gly Asp
                245                 250                 255

Arg Arg Ile Ser Leu Thr His Ser Leu Thr Arg Val Pro Ile Asn Asp
            260                 265                 270

Gly Asn Gly Glu Ala Ile Leu Lys Arg Gln Val Leu Leu Asn Gly Val
        275                 280                 285

Gln Pro Ser Arg Ala Asp Ala Leu Val Gly Lys Ser Ile Tyr Val Ser
    290                 295                 300

Ala Thr Val Ile Leu Gln Ser Gly Ser Asp Met Val Glu Ala Glu Arg
305                 310                 315                 320

Thr Gly Ile Pro Ile Val Thr Ser Pro Tyr Gln Ile His Phe Thr Lys
                325                 330                 335

Thr Pro Lys Phe Phe Lys Pro Ala Met Pro Phe Asp Leu Met Val Tyr
            340                 345                 350

Val Thr Asn Pro Asp Gly Ser Pro Ala Arg His Ile Pro Val Val Thr
        355                 360                 365

Gln Gly Ser Asn Val Gln Ser Leu Thr Gln Asp Asp Gly Val Ala Lys
    370                 375                 380

Leu Ser Ile Asn Thr Gln Asn Lys Arg Asp Pro Leu Thr Ile Thr Val
385                 390                 395                 400

Arg Thr Lys Lys Asp Asn Ile Pro Glu Gly Arg Gln Ala Thr Arg Thr
                405                 410                 415

Met Gln Ala Leu Pro Tyr Asn Thr Gln Gly Asn Ser Asn Asn Tyr Leu
            420                 425                 430

His Leu Ser Val Pro Arg Val Glu Leu Lys Pro Gly Glu Thr Leu Asn
        435                 440                 445

Val Asn Phe His Leu Arg Thr Asp Pro Gly Glu Gln Ala Lys Ile Arg

450                 455                 460

Tyr Tyr Thr Tyr Met Ile Met Asn Lys Gly Lys Leu Leu Lys Val Gly
465                 470                 475                 480

Arg Gln Tyr Arg Glu Pro Gly Gln Asp Leu Val Val Leu Pro Leu Thr
                485                 490                 495

Ile Thr Ser Asp Phe Ile Pro Ser Phe Arg Leu Val Ala Tyr Tyr Thr
            500                 505                 510

Leu Ile Asn Ala Lys Gly Gln Arg Glu Val Val Ala Asp Ser Val Trp
        515                 520                 525

Val Asp Val Lys Asp Ser Cys Met Gly Thr Leu Val Val Lys Asn Gly
    530                 535                 540

Gly Lys Glu Glu Lys His His Arg Pro Gly Gln Gln Ile Thr Leu Lys
545                 550                 555                 560

Ile Glu Ala Asp Gln Gly Ala Arg Val Gly Leu Val Ala Val Asp Lys
                565                 570                 575

Gly Val Phe Val Leu Asn Lys Lys Asn Lys Leu Thr Gln Arg Lys Ile
            580                 585                 590

Trp Asp Val Val Glu Lys Ala Asp Ile Gly Cys Thr Pro Gly Ser Gly
        595                 600                 605

Arg Asn Tyr Ala Gly Val Phe Thr Asp Ala Gly Leu Thr Leu Lys Thr
    610                 615                 620

Ser Gln Gly Leu Glu Thr Gln Gln Arg Ala Asp Pro Gln Cys Pro Gln
625                 630                 635                 640

Pro Ala Thr Arg Arg Arg
                645

<210>  15
<211>  645
<212>  DNA
<213>  Bos taurus

<400>  15
caggctgtgc tgactcagcc gtcctccgtg tccggctccc tgggccagag ggtctccatc      60

acctgctctg gaagcagcag caacatcggt agagataatg tgggctggta ccaacaggtc     120

ccaggatcgg gcctcagaac catcatctat agtagtagca gtcgaccctc gggggtcccc     180

gaccgattct ccggctccaa gtctggcaac acagccaccc tgaccatcag ctccctccag     240

gctgaggacg aggcggatta tttctgtgta gcttatgaca gtagtagtag tactgttttc     300

ggcagcggga ccacactgac cgtcctgggt cagcccaagt ccccaccctc ggtcaccctg     360

ttcccgccct ccacggagga gctcaacggc aacaaggcca ccctggtgtg tctcatcagc     420

gacttctacc cgggtagcgt gaccgtggtc tggaaggcag acggcagcac catcacccgc     480

aacgtggaga ccacccgggc ctccaaacag agcaacagca agtacgcggc cagcagctac     540

ctgagcctga cgagcagcga ctggaaatcg aaaggcagtt acagctgcga ggtcacgcac     600

gaggggagca ccgtgacgaa gacagtgaag ccctcagagt gttct                     645

<210>  16
<211>  215
<212>  PRT
<213>  Bos taurus

<400>  16

Gln Ala Val Leu Thr Gln Pro Ser Ser Val Ser Gly Ser Leu Gly Gln
1               5                   10                  15

Arg Val Ser Ile Thr Cys Ser Gly Ser Ser Ser Asn Ile Gly Arg Asp
            20                  25                  30

Asn Val Gly Trp Tyr Gln Gln Val Pro Gly Ser Gly Leu Arg Thr Ile
        35                  40                  45

Ile Tyr Ser Ser Ser Ser Arg Pro Ser Gly Val Pro Asp Arg Phe Ser
    50                  55                  60

Gly Ser Lys Ser Gly Asn Thr Ala Thr Leu Thr Ile Ser Ser Leu Gln
65                  70                  75                  80

Ala Glu Asp Glu Ala Asp Tyr Phe Cys Val Ala Tyr Asp Ser Ser Ser
                85                  90                  95

Ser Thr Val Phe Gly Ser Gly Thr Thr Leu Thr Val Leu Gly Gln Pro
            100                 105                 110

Lys Ser Pro Pro Ser Val Thr Leu Phe Pro Pro Ser Thr Glu Glu Leu
        115                 120                 125

Asn Gly Asn Lys Ala Thr Leu Val Cys Leu Ile Ser Asp Phe Tyr Pro
    130                 135                 140

Gly Ser Val Thr Val Val Trp Lys Ala Asp Gly Ser Thr Ile Thr Arg
145                 150                 155                 160

Asn Val Glu Thr Thr Arg Ala Ser Lys Gln Ser Asn Ser Lys Tyr Ala
                165                 170                 175

Ala Ser Ser Tyr Leu Ser Leu Thr Ser Ser Asp Trp Lys Ser Lys Gly
            180                 185                 190

Ser Tyr Ser Cys Glu Val Thr His Glu Gly Ser Thr Val Thr Lys Thr
        195                 200                 205

Val Lys Pro Ser Glu Cys Ser
    210                 215

<210>  17
<211>  1524
<212>  DNA
<213>  Bos taurus

<400>  17
gaacagcaag gttacagaaa ctccaccaac actggttcct atgaaaagga cttggattgc      60

ggagcccctg gcaccccaga ggcccagctc tgttttgacc cctgccagaa ttacaccctc     120

ctgaatgaac cctttcgaag cacagagaat acagaagaca tccaaggctg tgacagtgac     180

aaacacggct ggtaccgatt tgtgggggac ggaggagtga ggatgccaga ggactgtgtc     240

cccacgttcc gatgccagac gtctgcaccc ctgtggctga atggcaccca tcctggcctt     300

ggggagggca tcgtcaaccg cactgcctgt gcccactgga gtggcaactg ctgcctctgg     360

aagacagagg tgctggtgaa agcctgcccg ggcccatact acgtgtaccg gttggagggc     420

acccctcaat gtagtctgag atactgcaca gaccctgcca ccgcggagga caagtgtgac     480

aggacctgcc gccccgagga ggagtgtcgc ctcgtcagtg gcacctgggg ctgcttctgc     540

agacaggacc tcaacgtctc cgatgtgcac agtttgcagc ctcagctgga ctgtggggac     600

acagagatca aagtgtcact ggacaagtgt ctgctgggaa gtctgggttt cggagatgag     660

gtccatgcct acctgcggga cgggaactgg aactgcagca gcctcaggca gagtgaagag     720

gagaactgga tatctgtgac caatcccacc caggctggtg cctgcggaaa cattctggag     780

agaaaccaga cccatgccat ctacataaac acgctctcct tagtcaacga tttcatcatc     840

agagacacca tcctcagcat caacttccag tgtgcctacc cactggacat gaaagtgagc     900

cttcaaatgg ccctgcagcc catcgtaagt tccctgaaca tcactgtgga tggggaggga     960

gagttcactg tcaggatggc cctctttcaa gaccaggact acacgtctcc ttatgaaggg    1020

actgcagtca tgctgtctgt tgaatccatg ctctatgtgg gcaccatctt ggagagaggg    1080

gacacgtccc gatttaacct ggtgttgaag aactgctacg ccacgcccac tgaagacaag    1140

actgaccctg tgaagtactt catcatcagg aacagctgcc caaatcaacg tgattccacc    1200

atctccgtgg aagagaatgg ggtgtcagca gaaagccggt tctcagttca gatgttcaag 1260

tttgctggaa attatgacct agttttcctg cattgtgagg tttctctctg tgatttcatt 1320

aaagaagagt gccaaccgtc ttgctcaaga agtcaactcc gcagtgaagg ggtggctatt 1380

gatccagccc gagttctaga tctgggaccc atcactcgca aaggtgccca gtctcttggt 1440

gtcatgagtg gaacacccaa cactgcaggg ttcctggtgg cctggcccct ggttctcctg 1500

cctgtcctcc tggctgggct gttc 1524

<210> 18
<211> 508
<212> PRT
<213> Bos taurus

<400> 18

Glu Gln Gln Gly Tyr Arg Asn Ser Thr Asn Thr Gly Ser Tyr Glu Lys
1               5                   10                  15

Asp Leu Asp Cys Gly Ala Pro Gly Thr Pro Glu Ala Gln Leu Cys Phe
            20                  25                  30

Asp Pro Cys Gln Asn Tyr Thr Leu Leu Asn Glu Pro Phe Arg Ser Thr
        35                  40                  45

Glu Asn Thr Glu Asp Ile Gln Gly Cys Asp Ser Asp Lys His Gly Trp
    50                  55                  60

Tyr Arg Phe Val Gly Asp Gly Gly Val Arg Met Pro Glu Asp Cys Val
65                  70                  75                  80

Pro Thr Phe Arg Cys Gln Thr Ser Ala Pro Leu Trp Leu Asn Gly Thr
                85                  90                  95

His Pro Gly Leu Gly Glu Gly Ile Val Asn Arg Thr Ala Cys Ala His
            100                 105                 110

Trp Ser Gly Asn Cys Cys Leu Trp Lys Thr Glu Val Leu Val Lys Ala
        115                 120                 125

Cys Pro Gly Pro Tyr Tyr Val Tyr Arg Leu Glu Gly Thr Pro Gln Cys
    130                 135                 140

Ser Leu Arg Tyr Cys Thr Asp Pro Ala Thr Ala Glu Asp Lys Cys Asp
145                 150                 155                 160

Arg Thr Cys Arg Pro Glu Glu Glu Cys Arg Leu Val Ser Gly Thr Trp
                165                 170                 175

Gly Cys Phe Cys Arg Gln Asp Leu Asn Val Ser Asp Val His Ser Leu
        180                 185                 190

Gln Pro Gln Leu Asp Cys Gly Asp Thr Glu Ile Lys Val Ser Leu Asp
    195                 200                 205

Lys Cys Leu Leu Gly Ser Leu Gly Phe Gly Asp Glu Val His Ala Tyr
    210                 215                 220

Leu Arg Asp Gly Asn Trp Asn Cys Ser Ser Leu Arg Gln Ser Glu Glu
225                 230                 235                 240

Glu Asn Trp Ile Ser Val Thr Asn Pro Thr Gln Ala Gly Ala Cys Gly
                245                 250                 255

Asn Ile Leu Glu Arg Asn Gln Thr His Ala Ile Tyr Ile Asn Thr Leu
            260                 265                 270

Ser Leu Val Asn Asp Phe Ile Ile Arg Asp Thr Ile Leu Ser Ile Asn
        275                 280                 285

Phe Gln Cys Ala Tyr Pro Leu Asp Met Lys Val Ser Leu Gln Met Ala
    290                 295                 300

Leu Gln Pro Ile Val Ser Ser Leu Asn Ile Thr Val Asp Gly Glu Gly
305                 310                 315                 320

Glu Phe Thr Val Arg Met Ala Leu Phe Gln Asp Gln Asp Tyr Thr Ser
                325                 330                 335

Pro Tyr Glu Gly Thr Ala Val Met Leu Ser Val Glu Ser Met Leu Tyr
            340                 345                 350

Val Gly Thr Ile Leu Glu Arg Gly Asp Thr Ser Arg Phe Asn Leu Val
        355                 360                 365

Leu Lys Asn Cys Tyr Ala Thr Pro Thr Glu Asp Lys Thr Asp Pro Val
    370                 375                 380

Lys Tyr Phe Ile Ile Arg Asn Ser Cys Pro Asn Gln Arg Asp Ser Thr
385                 390                 395                 400

Ile Ser Val Glu Glu Asn Gly Val Ser Ala Glu Ser Arg Phe Ser Val
                405                 410                 415

Gln Met Phe Lys Phe Ala Gly Asn Tyr Asp Leu Val Phe Leu His Cys
            420                 425                 430

Glu Val Ser Leu Cys Asp Phe Ile Lys Glu Glu Cys Gln Pro Ser Cys
        435                 440                 445

Ser Arg Ser Gln Leu Arg Ser Glu Gly Val Ala Ile Asp Pro Ala Arg
    450                 455                 460

Val Leu Asp Leu Gly Pro Ile Thr Arg Lys Gly Ala Gln Ser Leu Gly
465                 470                 475                 480

Val Met Ser Gly Thr Pro Asn Thr Ala Gly Phe Leu Val Ala Trp Pro
                485                 490                 495

Leu Val Leu Leu Pro Val Leu Leu Ala Gly Leu Phe
            500                 505

<210>  19
<211>  1227
<212>  DNA
<213>  Bos taurus

<400>  19
gcctccggtg acttctgtga ctccagcttg tgcctacatg gtgggacctg cttgttgaac      60

gaggacagaa cccccccctt ctactgcctc tgccccgaag gcttcacggg cctcctatgc     120

aacgagacag agcacggtcc ctgtttccca aacccctgcc acaatgatgc tgagtgccag     180

gtaactgacg actcccaccg aggggatgtc ttcatccagt acatctgcaa gtgccctctt     240

ggatacgtgg gcatccactg tgagaccacc tgcacctcac cgctgggcat gcagacgggt     300

gccattgccg actcccagat ctctgcctcg tccatgcatt tgggcttcat gggtttgcag     360

cgctgggccc cggagctagc ccgcctgcac cagacgggca tcgtcaacgc ttggacatcc     420

ggcaactatg acaaaaaccc ctggatccag gtgaacctga tgcggaagat gtgggtgacg     480

ggtgtggtga cccagggggc cagccgcgca gggagtgctg agtacctgaa gacttttaaa     540

gtggcctaca gcaccgatgg gcgccaattc cagttcatcc aggttgcagg gcggtcagga     600

gataagatat ttataggtaa tgtgaacaac agcggcctga agattaacct gtttgatacc     660

cccttggaga cgcagtatgt gagactggta cccatcatct gccaccgggg ctgcaccctc     720

cgctttgaac tccttggctg tgagttgaat ggatgcactg aacccctagg cctgaaggat     780

aataccatcc ccaacaagca gatcacagcc tccagctact acaaaacctg gggcctgagt     840

gcctttagct ggtttcccta ctacgcacga ctggataatc agggcaagtt caacgcctgg     900

accgcccaga ccaacagtgc ctctgagtgg ctgcagattg acctgggctc ccagaagcga     960

gtcacgggca tcatcaccca gggtgcccga gactttggcc acattcaata tgtggctgcc    1020

tacagggtgg cctatggtga tgatggtgtg acctggactg agtacaagga cccgggggcc    1080

tcagaaagca agattttccc tggtaacatg gacaataatt cccacaagaa gaacatattt 1140

gagacgccgt tccaggctcg cttcgtgcgg atccagcccg tggcctggca caaccgtatc 1200

accctgcgag tggagctgct gggctgt 1227

<210> 20
<211> 409
<212> PRT
<213> Bos taurus

<400> 20

Ala Ser Gly Asp Phe Cys Asp Ser Ser Leu Cys Leu His Gly Gly Thr
1               5                   10                  15

Cys Leu Leu Asn Glu Asp Arg Thr Pro Pro Phe Tyr Cys Leu Cys Pro
            20                  25                  30

Glu Gly Phe Thr Gly Leu Leu Cys Asn Glu Thr Glu His Gly Pro Cys
        35                  40                  45

Phe Pro Asn Pro Cys His Asn Asp Ala Glu Cys Gln Val Thr Asp Asp
    50                  55                  60

Ser His Arg Gly Asp Val Phe Ile Gln Tyr Ile Cys Lys Cys Pro Leu
65                  70                  75                  80

Gly Tyr Val Gly Ile His Cys Glu Thr Thr Cys Thr Ser Pro Leu Gly
                85                  90                  95

Met Gln Thr Gly Ala Ile Ala Asp Ser Gln Ile Ser Ala Ser Ser Met
            100                 105                 110

His Leu Gly Phe Met Gly Leu Gln Arg Trp Ala Pro Glu Leu Ala Arg
        115                 120                 125

Leu His Gln Thr Gly Ile Val Asn Ala Trp Thr Ser Gly Asn Tyr Asp
    130                 135                 140

Lys Asn Pro Trp Ile Gln Val Asn Leu Met Arg Lys Met Trp Val Thr
145                 150                 155                 160

Gly Val Val Thr Gln Gly Ala Ser Arg Ala Gly Ser Ala Glu Tyr Leu
                165                 170                 175

Lys Thr Phe Lys Val Ala Tyr Ser Thr Asp Gly Arg Gln Phe Gln Phe
            180                 185                 190

Ile Gln Val Ala Gly Arg Ser Gly Asp Lys Ile Phe Ile Gly Asn Val
        195                 200                 205

Asn Asn Ser Gly Leu Lys Ile Asn Leu Phe Asp Thr Pro Leu Glu Thr
    210                 215                 220

Gln Tyr Val Arg Leu Val Pro Ile Ile Cys His Arg Gly Cys Thr Leu
225                 230                 235                 240

Arg Phe Glu Leu Leu Gly Cys Glu Leu Asn Gly Cys Thr Glu Pro Leu
                245                 250                 255

Gly Leu Lys Asp Asn Thr Ile Pro Asn Lys Gln Ile Thr Ala Ser Ser
            260                 265                 270

Tyr Tyr Lys Thr Trp Gly Leu Ser Ala Phe Ser Trp Phe Pro Tyr Tyr
        275                 280                 285

Ala Arg Leu Asp Asn Gln Gly Lys Phe Asn Ala Trp Thr Ala Gln Thr
    290                 295                 300

Asn Ser Ala Ser Glu Trp Leu Gln Ile Asp Leu Gly Ser Gln Lys Arg
305                 310                 315                 320

Val Thr Gly Ile Ile Thr Gln Gly Ala Arg Asp Phe Gly His Ile Gln
                325                 330                 335

Tyr Val Ala Ala Tyr Arg Val Ala Tyr Gly Asp Asp Gly Val Thr Trp
            340                 345                 350

Thr Glu Tyr Lys Asp Pro Gly Ala Ser Glu Ser Lys Ile Phe Pro Gly
        355                 360                 365

Asn Met Asp Asn Asn Ser His Lys Lys Asn Ile Phe Glu Thr Pro Phe
    370                 375                 380

Gln Ala Arg Phe Val Arg Ile Gln Pro Val Ala Trp His Asn Arg Ile
385                 390                 395                 400

Thr Leu Arg Val Glu Leu Leu Gly Cys
                405

<210>  21
<211>  846
<212>  DNA
<213>  Bos taurus

<400>  21
gaggagaccc aggctgggaa ttactctcta agcttcctct acaccgggct gtccaagccc      60

cgtgagggct tccccagctt ccaggccgtt gcctacctca atgaccagcc cttcttccac 120

tacaatagtg aaggcaggag ggccgagccc ctggcgccat ggagtcaggt ggaaggaatg 180

gaggactggg agaaggagag tgcccttcag agggccagag aggacatctt catggagacc 240

ctgagcgata tcatggacta ctacaaggac agagaagggt ctcacacctt tcaaggagca 300

ttcggctgtg agctccggaa taacgaaagt agtggagcat tctgggggta cgcctacgat 360

ggccaggact tcatcaagtt cgacaaagaa atcccagcct gggtccctct ggacccagca 420

gctcagaaca ccaagcggaa gtgggaagca gaggcagtct acgtgcagcg ggccaaggcc 480

tacctggaag aggagtgccc agggatgctg cggaggtacc tgccctacag caggacccac 540

ctggaccgac aagagtctcc ttctgtgtcg gtcaccgggc acgcggctcc agggcacaag 600

aggacactca agtgcctggc ctacgacttc tacccacgaa gcatcgggct gcactggact 660

cgggctggtg acgcccagga ggctgagtca gggggcgacg tgctccccag cggcaacggc 720

acttaccagt cctgggtggt ggtgggggtc ccccctgagg accaagcccc gtactcctgc 780

cacgtggagc acagaagcct gacccggccc ctcaccgtcc cgtgggaccc gcggcaacaa 840

gccgag 846

<210> 22
<211> 282
<212> PRT
<213> Bos taurus

<400> 22

Glu Glu Thr Gln Ala Gly Asn Tyr Ser Leu Ser Phe Leu Tyr Thr Gly
1               5                   10                  15

Leu Ser Lys Pro Arg Glu Gly Phe Pro Ser Phe Gln Ala Val Ala Tyr
            20                  25                  30

Leu Asn Asp Gln Pro Phe Phe His Tyr Asn Ser Glu Gly Arg Arg Ala
        35                  40                  45

Glu Pro Leu Ala Pro Trp Ser Gln Val Glu Gly Met Glu Asp Trp Glu
    50                  55                  60

Lys Glu Ser Ala Leu Gln Arg Ala Arg Glu Asp Ile Phe Met Glu Thr
65                  70                  75                  80

Leu Ser Asp Ile Met Asp Tyr Tyr Lys Asp Arg Glu Gly Ser His Thr
                85                  90                  95

Phe Gln Gly Ala Phe Gly Cys Glu Leu Arg Asn Asn Glu Ser Ser Gly
            100                 105                 110

Ala Phe Trp Gly Tyr Ala Tyr Asp Gly Gln Asp Phe Ile Lys Phe Asp
        115                 120                 125

Lys Glu Ile Pro Ala Trp Val Pro Leu Asp Pro Ala Ala Gln Asn Thr
    130                 135                 140

Lys Arg Lys Trp Glu Ala Glu Ala Val Tyr Val Gln Arg Ala Lys Ala
145                 150                 155                 160

Tyr Leu Glu Glu Glu Cys Pro Gly Met Leu Arg Arg Tyr Leu Pro Tyr
                165                 170                 175

Ser Arg Thr His Leu Asp Arg Gln Glu Ser Pro Ser Val Ser Val Thr
            180                 185                 190

Gly His Ala Ala Pro Gly His Lys Arg Thr Leu Lys Cys Leu Ala Tyr
        195                 200                 205

Asp Phe Tyr Pro Arg Ser Ile Gly Leu His Trp Thr Arg Ala Gly Asp
    210                 215                 220

Ala Gln Glu Ala Glu Ser Gly Gly Asp Val Leu Pro Ser Gly Asn Gly
225                 230                 235                 240

Thr Tyr Gln Ser Trp Val Val Val Gly Val Pro Pro Glu Asp Gln Ala
                245                 250                 255

Pro Tyr Ser Cys His Val Glu His Arg Ser Leu Thr Arg Pro Leu Thr
            260                 265                 270

Val Pro Trp Asp Pro Arg Gln Gln Ala Glu
        275                 280

<210>  23
<211>  390
<212>  DNA
<213>  Bos taurus

<400>  23
gagccggtga aattcaagga ctgcggttct tgggtcggag ttataaagga agtgaatgtg      60

agcccatgtc ccacccagcc ttgcaaactg cacagaggac agtcttacag tgttaatgtc     120

acgttcacca gtaatactca gtctcaaagt agcaaggctg tggtacatgg cattgtgatg     180

ggcatcccag ttccctttcc cattcctgag tctgatggtt gtaagtctgg aatcagatgc     240

cccatcgaaa aagataagac ctataactac gtgaacaaac tacccgtgaa gaatgaatac     300

ccctccataa aagtggtggt ggagtgggaa ctcacggatg acaaaaacca acgtttcttc     360

tgctggcaga tcccaataga ggttgaagcc                                    390

<210>  24
<211>  130
<212>  PRT
<213>  Bos taurus

<400>  24

Glu Pro Val Lys Phe Lys Asp Cys Gly Ser Trp Val Gly Val Ile Lys
1               5                   10                  15

Glu Val Asn Val Ser Pro Cys Pro Thr Gln Pro Cys Lys Leu His Arg
            20                  25                  30

Gly Gln Ser Tyr Ser Val Asn Val Thr Phe Thr Ser Asn Thr Gln Ser
        35                  40                  45

Gln Ser Ser Lys Ala Val Val His Gly Ile Val Met Gly Ile Pro Val
    50                  55                  60

Pro Phe Pro Ile Pro Glu Ser Asp Gly Cys Lys Ser Gly Ile Arg Cys
65                  70                  75                  80

Pro Ile Glu Lys Asp Lys Thr Tyr Asn Tyr Val Asn Lys Leu Pro Val
                85                  90                  95

Lys Asn Glu Tyr Pro Ser Ile Lys Val Val Val Glu Trp Glu Leu Thr
            100                 105                 110

Asp Asp Lys Asn Gln Arg Phe Phe Cys Trp Gln Ile Pro Ile Glu Val
        115                 120                 125

Glu Ala
    130

<210>  25
<211>  1080
<212>  DNA
<213>  Bos taurus

<400>  25
gaggtcaatg ccgaccaggt ggccaccgtg atttgggact acttcagcca gctgggcaac      60

aatgccaaga aggcggtgga acatatccag aagtctgagc tcacccagca gctcaacacc     120

ctcttccaag ataaacttgg ggaagtgagc acctacacgg acgacctgca gaagaagctg     180

gtgccctttg ccacggaact gcatgaacgg ctgaccaagg actccgagaa gctgaaggag     240

gagattcgga aggagctgga ggacctgcgg gcccggctgc tgccccacgc cacggaggtg     300

agccagaaga tcggggacaa cgtgcgcgag ctacagcagc gcctgggacc atacgcagag 360

gagctgcgca cccaggtcga cacccaggcg cagcagctgc ggcgccagct gacgccctat 420

gtggagcgca tggagaaggt gatgcggcag aacctggacc agctgcaggc ctcgctggcg 480

ccctacgccg aggagctcca ggccacggtc aaccagcgcg tggaagagct caaggggcgc 540

ctgacgccct acgcggacca gctccagacc aagatcgagg agaacgtgga ggaactgcgc 600

cgcagcctgg ccccttatgc tcaggacgtc caggggaagc tcaaccacca gctggagggc 660

ctggcctttc agatgaagaa gcatgccgag gagctaaagg ccaagatctc agccaaagcc 720

gaggagctgc ggcaggggct ggtgcccctg gtcaacagcg tgcatggaag tcagctgggc 780

aatgccgagg acttgcagaa gtcactggcc gagctgagca gccgcctgga ccagcaggtg 840

gaggacttcc gccgcaccgt ggggccctac ggcgagacct tcaacaaagc catggtgcag 900

cagctggaca cgctcaggca gaagctgggc cccctggcgg gggacgtgga agatcacctg 960

agtttcctgg agaaggatct gagggacaag gtcagcagct tcttcaacac cctcaaggag 1020

aaagagagcc aggcccccgc cctccccgcg caggaggaga tgcccgtccc attggggggc 1080

<210> 26
<211> 360
<212> PRT
<213> Bos taurus

<400> 26

Glu Val Asn Ala Asp Gln Val Ala Thr Val Ile Trp Asp Tyr Phe Ser
1               5                   10                  15

Gln Leu Gly Asn Asn Ala Lys Lys Ala Val Glu His Ile Gln Lys Ser
            20                  25                  30

Glu Leu Thr Gln Gln Leu Asn Thr Leu Phe Gln Asp Lys Leu Gly Glu
        35                  40                  45

Val Ser Thr Tyr Thr Asp Asp Leu Gln Lys Lys Leu Val Pro Phe Ala
    50                  55                  60

Thr Glu Leu His Glu Arg Leu Thr Lys Asp Ser Glu Lys Leu Lys Glu
65                  70                  75                  80

Glu Ile Arg Lys Glu Leu Glu Asp Leu Arg Ala Arg Leu Leu Pro His
                85                  90                  95

Ala Thr Glu Val Ser Gln Lys Ile Gly Asp Asn Val Arg Glu Leu Gln
            100                 105                 110

Gln Arg Leu Gly Pro Tyr Ala Glu Glu Leu Arg Thr Gln Val Asp Thr

115                 120                 125

Gln Ala Gln Gln Leu Arg Arg Gln Leu Thr Pro Tyr Val Glu Arg Met
    130                 135                 140

Glu Lys Val Met Arg Gln Asn Leu Asp Gln Leu Gln Ala Ser Leu Ala
145                 150                 155                 160

Pro Tyr Ala Glu Glu Leu Gln Ala Thr Val Asn Gln Arg Val Glu Glu
                165                 170                 175

Leu Lys Gly Arg Leu Thr Pro Tyr Ala Asp Gln Leu Gln Thr Lys Ile
            180                 185                 190

Glu Glu Asn Val Glu Glu Leu Arg Arg Ser Leu Ala Pro Tyr Ala Gln
        195                 200                 205

Asp Val Gln Gly Lys Leu Asn His Gln Leu Glu Gly Leu Ala Phe Gln
    210                 215                 220

Met Lys Lys His Ala Glu Glu Leu Lys Ala Lys Ile Ser Ala Lys Ala
225                 230                 235                 240

Glu Glu Leu Arg Gln Gly Leu Val Pro Leu Val Asn Ser Val His Gly
                245                 250                 255

Ser Gln Leu Gly Asn Ala Glu Asp Leu Gln Lys Ser Leu Ala Glu Leu
            260                 265                 270

Ser Ser Arg Leu Asp Gln Gln Val Glu Asp Phe Arg Arg Thr Val Gly
        275                 280                 285

Pro Tyr Gly Glu Thr Phe Asn Lys Ala Met Val Gln Gln Leu Asp Thr
    290                 295                 300

Leu Arg Gln Lys Leu Gly Pro Leu Ala Gly Asp Val Glu Asp His Leu
305                 310                 315                 320

Ser Phe Leu Glu Lys Asp Leu Arg Asp Lys Val Ser Ser Phe Phe Asn
                325                 330                 335

Thr Leu Lys Glu Lys Glu Ser Gln Ala Pro Ala Leu Pro Ala Gln Glu
            340                 345                 350

Glu Met Pro Val Pro Leu Gly Gly
        355                 360

<210>  27
<211>  15
<212>  PRT
<213>  Artificial Sequence

<220>
<223>  epitope

<400>  27

Gln Asn Val Tyr Thr Cys Gln Val Glu His Asn Lys Glu Thr Phe
1               5                   10                  15


<210>  28
<211>  15
<212>  PRT
<213>  Artificial Sequence

<220>
<223>  epitope

<400>  28

Gln Asn Val Tyr Thr Cys Gln Val Glu His Asn Lys Glu Thr Phe
1               5                   10                  15


<210>  29
<211>  7
<212>  PRT
<213>  Artificial Sequence

<220>
<223>  epitope

<400>  29

Ser Gln Asn Val Tyr Thr Cys
1               5


<210>  30
<211>  7
<212>  PRT
<213>  Artificial Sequence

<220>
<223>  epitope

<400>  30

Thr Cys Gln Val Glu His Asn
1               5


<210>  31
<211>  7
<212>  PRT
<213>  Artificial Sequence

<220>
<223>  epitope

<400>  31

His Asn Lys Glu Thr Phe Gln
1               5

<210>  32
<211>  15
<212>  PRT
<213>  Artificial Sequence

<220>
<223>  epitope

<400>  32

Gln Asn Val Tyr Thr Cys Gln Val Glu His Asn Lys Glu Thr Phe
1               5                   10                  15

<210>  33
<211>  8
<212>  PRT
<213>  Artificial Sequence

<220>
<223>  epitope

<400>  33

Leu Ser Gln Asn Val Tyr Thr Cys
1               5

<210>  34
<211>  4
<212>  PRT
<213>  Artificial Sequence

<220>
<223>  epitope

<400>  34

His Asn Lys Glu
1

<210>  35
<211>  15
<212>  PRT
<213>  Artificial Sequence

<220>
<223>  epitope

<400>  35

Gln Asn Val Tyr Thr Cys Gln Val Glu His Asn Lys Glu Thr Phe
1               5                   10                  15

<210>  36
<211>  4
<212>  PRT
<213>  Artificial Sequence

<220>
<223>  epitope

<400>  36

His Asn Lys Glu
1


<210>  37
<211>  15
<212>  PRT
<213>  Artificial Sequence

<220>
<223>  epitope

<400>  37

Gln Asn Val Tyr Thr Cys Gln Val Glu His Asn Lys Glu Thr Phe
1               5                   10                  15


<210>  38
<211>  15
<212>  PRT
<213>  Artificial Sequence

<220>
<223>  epitope


<220>
<221>  misc_feature
<222>  (1)..(3)
<223>  Xaa can be any naturally occurring amino acid

<220>
<221>  misc_feature
<222>  (5)..(5)
<223>  Xaa can be any naturally occurring amino acid

<220>
<221>  misc_feature
<222>  (8)..(10)
<223>  Xaa can be any naturally occurring amino acid

<220>
<221>  misc_feature
<222>  (13)..(15)
<223>  Xaa can be any naturally occurring amino acid

<400>  38

Xaa Xaa Xaa Tyr Xaa Cys Gln Xaa Xaa Xaa Asn Lys Xaa Xaa Xaa
1               5                   10                  15

<210>  39
<211>  4
<212>  PRT
<213>  Artificial Sequence

<220>
<223>  epitope

<400>  39

His Asn Lys Glu
1


<210>  40
<211>  15
<212>  PRT
<213>  Artificial Sequence

<220>
<223>  epitope

<400>  40

Gln Asn Val Tyr Thr Cys Gln Val Glu His Asn Lys Glu Thr Phe
1               5                   10                  15


<210>  41
<211>  10
<212>  PRT
<213>  Artificial Sequence

<220>
<223>  epitope

<400>  41

Asp Trp Leu Ser Gln Asn Val Tyr Thr Cys
1               5                   10


<210>  42
<211>  15
<212>  PRT
<213>  Artificial Sequence

<220>
<223>  epitope

<400>  42

Gln Asn Val Tyr Thr Cys Gln Val Glu His Asn Lys Glu Thr Phe
1               5                   10                  15


<210>  43
<211>  8
<212>  PRT
<213>  Artificial Sequence

<220>
<223>  epitope

<400>  43

Leu Ser Gln Asn Val Tyr Thr Cys
1               5



<210>  44
<211>  10
<212>  PRT
<213>  Artificial Sequence

<220>
<223>  epitope

<400>  44

Asn Val Tyr Thr Cys Gln Val Glu His Asn
1               5                   10



<210>  45
<211>  10
<212>  PRT
<213>  Artificial Sequence

<220>
<223>  epitope

<400>  45

Tyr Thr Cys Gln Val Glu His Asn Lys Glu
1               5                   10



<210>  46
<211>  6
<212>  PRT
<213>  Artificial Sequence

<220>
<223>  epitope

<400>  46

His Asn Lys Glu Thr Phe
1               5



<210>  47
<211>  15
<212>  PRT
<213>  Artificial Sequence

<220>
<223>  epitope

<400>  47

Gln Asn Val Tyr Thr Cys Gln Val Glu His Asn Lys Glu Thr Phe
1               5                   10                  15

<210>  48
<211>  15
<212>  PRT
<213>  Artificial Sequence

<220>
<223>  epitope

<400>  48

Gln Asn Val Tyr Thr Cys Gln Val Glu His Asn Lys Glu Thr Phe
1               5                   10                  15


<210>  49
<211>  15
<212>  PRT
<213>  Artificial Sequence

<220>
<223>  epitope


<220>
<221>  misc_feature
<222>  (1)..(3)
<223>  Xaa can be any naturally occurring amino acid

<220>
<221>  misc_feature
<222>  (5)..(6)
<223>  Xaa can be any naturally occurring amino acid

<220>
<221>  misc_feature
<222>  (8)..(10)
<223>  Xaa can be any naturally occurring amino acid

<400>  49

Xaa Xaa Xaa Tyr Xaa Xaa Gln Xaa Xaa Xaa Asn Lys Glu Thr Phe
1               5                   10                  15


<210>  50
<211>  15
<212>  PRT
<213>  Artificial Sequence

<220>
<223>  epitope


<220>
<221>  misc_feature
<222>  (1)..(10)
<223>  Xaa can be any naturally occurring amino acid

<220>
<221>  misc_feature
<222>  (14)..(14)
<223>  Xaa can be any naturally occurring amino acid

<400>  50

Xaa Xaa Xaa Xaa Xaa Xaa Xaa Xaa Xaa Xaa Asn Lys Glu Xaa Phe
1               5                   10                  15


<210>  51
<211>  10
<212>  PRT
<213>  Artificial Sequence

<220>
<223>  epitope

<400>  51

Tyr Thr Cys Gln Val Glu His Asn Lys Glu
1               5                   10


<210>  52
<211>  10
<212>  PRT
<213>  Artificial Sequence

<220>
<223>  epitope


<220>
<221>  misc_feature
<222>  (2)..(3)
<223>  Xaa can be any naturally occurring amino acid

<220>
<221>  misc_feature
<222>  (5)..(7)
<223>  Xaa can be any naturally occurring amino acid

<400>  52

Tyr Xaa Xaa Gln Xaa Xaa Xaa Asn Lys Glu
1               5                   10


<210>  53
<211>  6
<212>  PRT
<213>  Artificial Sequence

<220>
<223>  epitope

<400>  53

His Asn Lys Glu Thr Phe
1               5


<210>  54
<211>  6
<212>  PRT
<213>  Artificial Sequence

<220>
<223>  epitope

<220>
<221>  misc_feature
<222>  (1)..(1)
<223>  Xaa can be any naturally occurring amino acid

<400>  54

Xaa Asn Lys Glu Thr Phe
1               5

<210>  55
<211>  6
<212>  PRT
<213>  Artificial Sequence

<220>
<223>  epitope

<220>
<221>  misc_feature
<222>  (1)..(1)
<223>  Xaa can be any naturally occurring amino acid

<220>
<221>  misc_feature
<222>  (5)..(5)
<223>  Xaa can be any naturally occurring amino acid

<400>  55

Xaa Asn Lys Glu Xaa Phe
1               5

<210>  56
<211>  6
<212>  PRT
<213>  Artificial Sequence

<220>
<223>  epitope

<400>  56

His Asn Lys Glu Thr Phe
1               5

<210>  57
<211>  10
<212>  PRT
<213>  Artificial Sequence

<220>
<223>  epitope

<400>  57

Tyr Thr Cys Gln Val Glu His Asn Lys Glu
1               5                   10


<210>  58
<211>  6
<212>  PRT
<213>  Artificial Sequence

<220>
<223>  epitope

<400>  58

His Asn Lys Glu Thr Phe
1               5


<210>  59
<211>  15
<212>  PRT
<213>  Artificial Sequence

<220>
<223>  epitope

<400>  59

Thr Lys Ser Lys Tyr Val Thr Ser Ser Pro Ala Pro Glu Pro Gln
1               5                   10                  15


<210>  60
<211>  15
<212>  PRT
<213>  Artificial Sequence

<220>
<223>  epitope

<400>  60

Thr Lys Ser Lys Tyr Val Thr Ser Ser Pro Ala Pro Glu Pro Gln
1               5                   10                  15


<210>  61
<211>  15
<212>  PRT
<213>  Artificial Sequence

<220>
<223>  epitope


<220>
<221>  misc_feature
<222>  (1)..(1)
<223>  Xaa can be any naturally occurring amino acid

<220>

<221>  misc_feature
<222>  (5)..(7)
<223>  Xaa can be any naturally occurring amino acid

<400>  61

Xaa Lys Ser Lys Xaa Xaa Xaa Ser Ser Pro Ala Pro Glu Pro Gln
1               5                   10                  15


<210>  62
<211>  15
<212>  PRT
<213>  Artificial Sequence

<220>
<223>  epitope


<220>
<221>  misc_feature
<222>  (1)..(1)
<223>  Xaa can be any naturally occurring amino acid

<220>
<221>  misc_feature
<222>  (3)..(3)
<223>  Xaa can be any naturally occurring amino acid

<220>
<221>  misc_feature
<222>  (5)..(13)
<223>  Xaa can be any naturally occurring amino acid

<400>  62

Xaa Lys Xaa Lys Xaa Xaa Xaa Xaa Xaa Xaa Xaa Xaa Xaa Pro Gln
1               5                   10                  15


<210>  63
<211>  9
<212>  PRT
<213>  Artificial Sequence

<220>
<223>  epitope

<400>  63

Lys Ser Lys Tyr Val Thr Ser Ser Pro
1               5


<210>  64
<211>  9
<212>  PRT
<213>  Artificial Sequence

<220>
<223>  epitope

<220>
<221>  misc_feature
<222>  (4)..(6)
<223>  Xaa can be any naturally occurring amino acid

<400>  64

Lys Ser Lys Xaa Xaa Xaa Ser Ser Pro
1               5


<210>  65
<211>  8
<212>  PRT
<213>  Artificial Sequence

<220>
<223>  epitope

<400>  65

Lys Ser Lys Tyr Val Thr Ser Ser
1               5


<210>  66
<211>  8
<212>  PRT
<213>  Artificial Sequence

<220>
<223>  epitope


<220>
<221>  misc_feature
<222>  (4)..(6)
<223>  Xaa can be any naturally occurring amino acid

<400>  66

Lys Ser Lys Xaa Xaa Xaa Ser Ser
1               5


<210>  67
<211>  4
<212>  PRT
<213>  Artificial Sequence

<220>
<223>  epitope

<400>  67

Tyr Val Thr Ser
1


<210>  68
<211>  15
<212>  PRT
<213>  Artificial Sequence

<220>
<223>  epitope

<400>  68

Thr Lys Ser Lys Tyr Val Thr Ser Ser Pro Ala Pro Glu Pro Gln
1               5                   10                  15


<210>  69
<211>  15
<212>  PRT
<213>  Artificial Sequence

<220>
<223>  epitope


<220>
<221>  misc_feature
<222>  (1)..(1)
<223>  Xaa can be any naturally occurring amino acid

<220>
<221>  misc_feature
<222>  (3)..(3)
<223>  Xaa can be any naturally occurring amino acid

<220>
<221>  misc_feature
<222>  (5)..(8)
<223>  Xaa can be any naturally occurring amino acid

<220>
<221>  misc_feature
<222>  (12)..(12)
<223>  Xaa can be any naturally occurring amino acid

<400>  69

Xaa Lys Xaa Lys Xaa Xaa Xaa Xaa Ser Pro Ala Xaa Glu Pro Gln
1               5                   10                  15


<210>  70
<211>  6
<212>  PRT
<213>  Artificial Sequence

<220>
<223>  epitope

<400>  70

Ser Lys Tyr Val Thr Ser
1               5


<210>  71
<211>  5
<212>  PRT
<213>  Artificial Sequence

<220>
<223>  epitope

<400>  71

Lys Tyr Val Thr Ser
1               5


<210>  72
<211>  10
<212>  PRT
<213>  Artificial Sequence

<220>
<223>  epitope

<400>  72

Pro Ala Pro Glu Pro Gln Asp Pro Ser Val
1               5                   10


<210>  73
<211>  10
<212>  PRT
<213>  Artificial Sequence

<220>
<223>  epitope


<220>
<221>  misc_feature
<222>  (3)..(3)
<223>  Xaa can be any naturally occurring amino acid

<400>  73

Pro Ala Xaa Glu Pro Gln Asp Pro Ser Val
1               5                   10


<210>  74
<211>  15
<212>  PRT
<213>  Artificial Sequence

<220>
<223>  epitope

<400>  74

Thr Lys Ser Lys Tyr Val Thr Ser Ser Pro Ala Pro Glu Pro Gln
1               5                   10                  15


<210>  75
<211>  7
<212>  PRT
<213>  Artificial Sequence

<220>
<223>  epitope

<400>  75

Ser Lys Tyr Val Thr Ser Ser
1               5


<210>  76
<211>  15
<212>  PRT
<213>  Artificial Sequence

<220>
<223>  epitope

<400>  76

Thr Lys Ser Lys Tyr Val Thr Ser Ser Pro Ala Pro Glu Pro Gln
1               5                   10                  15


<210>  77
<211>  9
<212>  PRT
<213>  Artificial Sequence

<220>
<223>  epitope

<400>  77

Lys Ser Lys Tyr Val Thr Ser Ser Pro
1               5


<210>  78
<211>  8
<212>  PRT
<213>  Artificial Sequence

<220>
<223>  epitope

<400>  78

Lys Ser Lys Tyr Val Thr Ser Ser
1               5


<210>  79
<211>  6
<212>  PRT
<213>  Artificial Sequence

<220>
<223>  epitope

<400>  79

Ser Lys Tyr Val Thr Ser
1               5

<210>  80
<211>  10
<212>  PRT
<213>  Artificial Sequence

<220>
<223>  epitope

<400>  80

Tyr Val Thr Ser Ser Pro Ala Pro Glu Pro
1               5                   10


<210>  81
<211>  15
<212>  PRT
<213>  Artificial Sequence

<220>
<223>  epitope

<400>  81

Thr Lys Ser Lys Tyr Val Thr Ser Ser Pro Ala Pro Glu Pro Gln
1               5                   10                  15


<210>  82
<211>  15
<212>  PRT
<213>  Artificial Sequence

<220>
<223>  epitope


<220>
<221>  misc_feature
<222>  (1)..(1)
<223>  Xaa can be any naturally occurring amino acid

<220>
<221>  misc_feature
<222>  (5)..(6)
<223>  Xaa can be any naturally occurring amino acid

<220>
<221>  misc_feature
<222>  (12)..(12)
<223>  Xaa can be any naturally occurring amino acid

<400>  82

Xaa Lys Ser Lys Xaa Xaa Thr Ser Ser Pro Ala Xaa Glu Pro Gln
1               5                   10                  15


<210>  83
<211>  8
<212>  PRT

<213>  Artificial Sequence

<220>
<223>  epitope

<400>  83

Lys Ser Lys Tyr Val Thr Ser Ser
1               5


<210>  84
<211>  6
<212>  PRT
<213>  Artificial Sequence

<220>
<223>  epitope

<400>  84

Ser Lys Tyr Val Thr Ser
1               5


<210>  85
<211>  6
<212>  PRT
<213>  Artificial Sequence

<220>
<223>  epitope


<220>
<221>  misc_feature
<222>  (3)..(4)
<223>  Xaa can be any naturally occurring amino acid

<400>  85

Ser Lys Xaa Xaa Thr Ser
1               5


<210>  86
<211>  5
<212>  PRT
<213>  Artificial Sequence

<220>
<223>  epitope

<400>  86

Lys Tyr Val Thr Ser
1               5


<210>  87
<211>  6
<212>  PRT
<213>  Artificial Sequence

<220>
<223>  epitope

<400>  87

Lys Tyr Val Thr Ser Ser
1               5



<210>  88
<211>  4
<212>  PRT
<213>  Artificial Sequence

<220>
<223>  epitope

<400>  88

Tyr Val Thr Ser
1



<210>  89
<211>  6
<212>  PRT
<213>  Artificial Sequence

<220>
<223>  epitope

<400>  89

Ser Lys Tyr Val Thr Ser
1               5



<210>  90
<211>  6
<212>  PRT
<213>  Artificial Sequence

<220>
<223>  epitope

<400>  90

Lys Tyr Val Thr Ser Ser
1               5



<210>  91
<211>  4
<212>  PRT
<213>  Artificial Sequence

<220>
<223>  epitope

<400>  91

Tyr Val Thr Ser

1


<210>  92
<211>  15
<212>  PRT
<213>  Artificial Sequence

<220>
<223>  epitope



<220>
<221>  misc_feature
<222>  (1)..(1)
<223>  Xaa can be any naturally occurring amino acid

<220>
<221>  misc_feature
<222>  (3)..(3)
<223>  Xaa can be any naturally occurring amino acid

<220>
<221>  misc_feature
<222>  (6)..(6)
<223>  Xaa can be any naturally occurring amino acid

<220>
<221>  misc_feature
<222>  (9)..(9)
<223>  Xaa can be any naturally occurring amino acid

<400>  92

Xaa Lys Xaa Lys Tyr Xaa Thr Ser Xaa Pro Ala Pro Glu Pro Gln
1               5                   10                  15


<210>  93
<211>  15
<212>  PRT
<213>  Artificial Sequence

<220>
<223>  epitope



<220>
<221>  misc_feature
<222>  (1)..(3)
<223>  Xaa can be any naturally occurring amino acid

<220>
<221>  misc_feature
<222>  (6)..(6)
<223>  Xaa can be any naturally occurring amino acid

<220>
<221>  misc_feature
<222>  (8)..(9)
<223>  Xaa can be any naturally occurring amino acid

<220>

<221>  misc_feature
<222>  (12)..(15)
<223>  Xaa can be any naturally occurring amino acid

<400>  93

Xaa Xaa Xaa Lys Tyr Xaa Thr Xaa Xaa Pro Ala Xaa Xaa Xaa Xaa
1               5                   10                  15


<210>  94
<211>  9
<212>  PRT
<213>  Artificial Sequence

<220>
<223>  epitope

<400>  94

Lys Ser Lys Tyr Val Thr Ser Ser Pro
1               5


<210>  95
<211>  10
<212>  PRT
<213>  Artificial Sequence

<220>
<223>  epitope

<400>  95

Lys Tyr Val Thr Ser Ser Pro Ala Pro Glu
1               5                   10


<210>  96
<211>  5
<212>  PRT
<213>  Artificial Sequence

<220>
<223>  epitope

<400>  96

Lys Tyr Val Thr Ser
1               5


<210>  97
<211>  5
<212>  PRT
<213>  Artificial Sequence

<220>
<223>  epitope


<220>
<221>  misc_feature

<222>  (3)..(3)
<223>  Xaa can be any naturally occurring amino acid

<400>  97

Lys Tyr Xaa Thr Ser
1               5


<210>  98
<211>  15
<212>  PRT
<213>  Artificial Sequence

<220>
<223>  epitope

<400>  98

Cys Gly Asp Asn Thr Arg Lys Ser Val Asp Asn Tyr Gln Glu Cys
1               5                   10                  15


<210>  99
<211>  15
<212>  PRT
<213>  Artificial Sequence

<220>
<223>  epitope

<400>  99

Cys Gly Asp Asn Thr Arg Lys Ser Val Asp Asn Tyr Gln Glu Cys
1               5                   10                  15


<210>  100
<211>  15
<212>  PRT
<213>  Artificial Sequence

<220>
<223>  epitope


<220>
<221>  misc_feature
<222>  (13)..(15)
<223>  Xaa can be any naturally occurring amino acid

<400>  100

Cys Gly Asp Asn Thr Arg Lys Ser Val Asp Asn Tyr Xaa Xaa Xaa
1               5                   10                  15


<210>  101
<211>  15
<212>  PRT
<213>  Artificial Sequence

<220>

<223>  epitope

<220>
<221>  misc_feature
<222>  (1)..(1)
<223>  Xaa can be any naturally occurring amino acid

<220>
<221>  misc_feature
<222>  (9)..(9)
<223>  Xaa can be any naturally occurring amino acid

<220>
<221>  misc_feature
<222>  (12)..(15)
<223>  Xaa can be any naturally occurring amino acid

<400>  101

Xaa Gly Asp Asn Thr Arg Lys Ser Xaa Asp Asn Xaa Xaa Xaa Xaa
1               5                   10                  15

<210>  102
<211>  10
<212>  PRT
<213>  Artificial Sequence

<220>
<223>  epitope

<400>  102

Lys Ser Val Asp Asn Tyr Gln Glu Cys Tyr
1               5                   10

<210>  103
<211>  10
<212>  PRT
<213>  Artificial Sequence

<220>
<223>  epitope

<220>
<221>  misc_feature
<222>  (7)..(9)
<223>  Xaa can be any naturally occurring amino acid

<400>  103

Lys Ser Val Asp Asn Tyr Xaa Xaa Xaa Tyr
1               5                   10

<210>  104
<211>  10
<212>  PRT
<213>  Artificial Sequence

<220>
<223>  epitope


<220>
<221>  misc_feature
<222>  (3)..(3)
<223>  Xaa can be any naturally occurring amino acid

<220>
<221>  misc_feature
<222>  (6)..(9)
<223>  Xaa can be any naturally occurring amino acid

<400>  104

Lys Ser Xaa Asp Asn Xaa Xaa Xaa Xaa Tyr
1               5                   10


<210>  105
<211>  9
<212>  PRT
<213>  Artificial Sequence

<220>
<223>  epitope

<400>  105

Asp Asn Tyr Gln Glu Cys Tyr Leu Ala
1               5


<210>  106
<211>  9
<212>  PRT
<213>  Artificial Sequence

<220>
<223>  epitope


<220>
<221>  misc_feature
<222>  (4)..(6)
<223>  Xaa can be any naturally occurring amino acid

<400>  106

Asp Asn Tyr Xaa Xaa Xaa Tyr Leu Ala
1               5


<210>  107
<211>  9
<212>  PRT
<213>  Artificial Sequence

<220>
<223>  epitope

<220>
<221>  misc_feature
<222>  (3)..(6)
<223>  Xaa can be any naturally occurring amino acid

<400>  107

Asp Asn Xaa Xaa Xaa Xaa Tyr Leu Ala
1               5


<210>  108
<211>  15
<212>  PRT
<213>  Artificial Sequence

<220>
<223>  epitope

<400>  108

Cys Gly Asp Asn Thr Arg Lys Ser Val Asp Asn Tyr Gln Glu Cys
1               5                   10                  15


<210>  109
<211>  15
<212>  PRT
<213>  Artificial Sequence

<220>
<223>  epitope


<220>
<221>  misc_feature
<222>  (1)..(1)
<223>  Xaa can be any naturally occurring amino acid

<220>
<221>  misc_feature
<222>  (5)..(5)
<223>  Xaa can be any naturally occurring amino acid

<220>
<221>  misc_feature
<222>  (9)..(9)
<223>  Xaa can be any naturally occurring amino acid

<220>
<221>  misc_feature
<222>  (13)..(15)
<223>  Xaa can be any naturally occurring amino acid

<400>  109

Xaa Gly Asp Asn Xaa Arg Lys Ser Xaa Asp Asn Tyr Xaa Xaa Xaa
1               5                   10                  15


<210>  110
<211>  15
<212>  PRT

<213>  Artificial Sequence

<220>
<223>  epitope


<220>
<221>  misc_feature
<222>  (1)..(1)
<223>  Xaa can be any naturally occurring amino acid

<220>
<221>  misc_feature
<222>  (3)..(6)
<223>  Xaa can be any naturally occurring amino acid

<220>
<221>  misc_feature
<222>  (9)..(9)
<223>  Xaa can be any naturally occurring amino acid

<220>
<221>  misc_feature
<222>  (13)..(15)
<223>  Xaa can be any naturally occurring amino acid

<400>  110

Xaa Gly Xaa Xaa Xaa Xaa Lys Ser Xaa Asp Asn Tyr Xaa Xaa Xaa
1               5                   10                  15


<210>  111
<211>  10
<212>  PRT
<213>  Artificial Sequence

<220>
<223>  epitope

<400>  111

Lys Ser Val Asp Asn Tyr Gln Glu Cys Tyr
1               5                   10


<210>  112
<211>  10
<212>  PRT
<213>  Artificial Sequence

<220>
<223>  epitope


<220>
<221>  misc_feature
<222>  (3)..(3)
<223>  Xaa can be any naturally occurring amino acid

<220>
<221>  misc_feature
<222>  (7)..(9)

<223>  Xaa can be any naturally occurring amino acid

<400>  112

Lys Ser Xaa Asp Asn Tyr Xaa Xaa Xaa Tyr
1               5                   10


<210>  113
<211>  9
<212>  PRT
<213>  Artificial Sequence

<220>
<223>  epitope

<400>  113

Asp Asn Tyr Gln Glu Cys Tyr Leu Ala
1               5


<210>  114
<211>  15
<212>  PRT
<213>  Artificial Sequence

<220>
<223>  epitope

<400>  114

Cys Gly Asp Asn Thr Arg Lys Ser Val Asp Asn Tyr Gln Glu Cys
1               5                   10                  15


<210>  115
<211>  15
<212>  PRT
<213>  Artificial Sequence

<220>
<223>  epitope


<220>
<221>  misc_feature
<222>  (1)..(2)
<223>  Xaa can be any naturally occurring amino acid

<220>
<221>  misc_feature
<222>  (4)..(5)
<223>  Xaa can be any naturally occurring amino acid

<220>
<221>  misc_feature
<222>  (9)..(9)
<223>  Xaa can be any naturally occurring amino acid

<220>
<221>  misc_feature
<222>  (12)..(15)

<223>  Xaa can be any naturally occurring amino acid

<400>  115

Xaa Xaa Asp Xaa Xaa Arg Lys Ser Xaa Asp Asn Xaa Xaa Xaa Xaa
1               5                   10                  15


<210>  116
<211>  4
<212>  PRT
<213>  Artificial Sequence

<220>
<223>  epitope

<400>  116

Asp Asn Thr Arg
1


<210>  117
<211>  10
<212>  PRT
<213>  Artificial Sequence

<220>
<223>  epitope

<400>  117

Thr Arg Lys Ser Val Asp Asn Tyr Gln Glu
1               5                   10


<210>  118
<211>  10
<212>  PRT
<213>  Artificial Sequence

<220>
<223>  epitope


<220>
<221>  misc_feature
<222>  (1)..(1)
<223>  Xaa can be any naturally occurring amino acid

<220>
<221>  misc_feature
<222>  (5)..(5)
<223>  Xaa can be any naturally occurring amino acid

<220>
<221>  misc_feature
<222>  (8)..(10)
<223>  Xaa can be any naturally occurring amino acid

<400>  118

Xaa Arg Lys Ser Xaa Asp Asn Xaa Xaa Xaa

1               5                   10


<210>  119
<211>  10
<212>  PRT
<213>  Artificial Sequence

<220>
<223>  epitope

<400>  119

Lys Ser Val Asp Asn Tyr Gln Glu Cys Tyr
1               5                   10


<210>  120
<211>  10
<212>  PRT
<213>  Artificial Sequence

<220>
<223>  epitope


<220>
<221>  misc_feature
<222>  (3)..(3)
<223>  Xaa can be any naturally occurring amino acid

<220>
<221>  misc_feature
<222>  (6)..(9)
<223>  Xaa can be any naturally occurring amino acid

<400>  120

Lys Ser Xaa Asp Asn Xaa Xaa Xaa Xaa Tyr
1               5                   10


<210>  121
<211>  15
<212>  PRT
<213>  Artificial Sequence

<220>
<223>  epitope

<400>  121

Cys Gly Asp Asn Thr Arg Lys Ser Val Asp Asn Tyr Gln Glu Cys
1               5                   10                  15


<210>  122
<211>  15
<212>  PRT
<213>  Artificial Sequence

<220>
<223>  epitope

<400>  122

Cys Gly Asp Asn Thr Arg Lys Ser Val Asp Asn Tyr Gln Glu Cys
1               5                   10                  15


<210>  123
<211>  15
<212>  PRT
<213>  Artificial Sequence

<220>
<223>  epitope


<220>
<221>  misc_feature
<222>  (1)..(1)
<223>  Xaa can be any naturally occurring amino acid

<220>
<221>  misc_feature
<222>  (8)..(9)
<223>  Xaa can be any naturally occurring amino acid

<220>
<221>  misc_feature
<222>  (13)..(15)
<223>  Xaa can be any naturally occurring amino acid

<400>  123

Xaa Gly Asp Asn Thr Arg Lys Xaa Xaa Asp Asn Tyr Xaa Xaa Xaa
1               5                   10                  15


<210>  124
<211>  15
<212>  PRT
<213>  Artificial Sequence

<220>
<223>  epitope


<220>
<221>  misc_feature
<222>  (1)..(1)
<223>  Xaa can be any naturally occurring amino acid

<220>
<221>  misc_feature
<222>  (7)..(10)
<223>  Xaa can be any naturally occurring amino acid

<220>
<221>  misc_feature
<222>  (12)..(15)
<223>  Xaa can be any naturally occurring amino acid

<400>  124

Xaa Gly Asp Asn Thr Arg Xaa Xaa Xaa Xaa Asn Xaa Xaa Xaa Xaa
1               5                   10                  15

<210>  125
<211>  4
<212>  PRT
<213>  Artificial Sequence

<220>
<223>  epitope

<400>  125

Asp Asn Thr Arg
1

<210>  126
<211>  10
<212>  PRT
<213>  Artificial Sequence

<220>
<223>  epitope

<400>  126

Lys Ser Val Asp Asn Tyr Gln Glu Cys Tyr
1               5                   10

<210>  127
<211>  10
<212>  PRT
<213>  Artificial Sequence

<220>
<223>  epitope

<220>
<221>  misc_feature
<222>  (2)..(3)
<223>  Xaa can be any naturally occurring amino acid

<220>
<221>  misc_feature
<222>  (7)..(9)
<223>  Xaa can be any naturally occurring amino acid

<400>  127

Lys Xaa Xaa Asp Asn Tyr Xaa Xaa Xaa Tyr
1               5                   10

<210>  128
<211>  15
<212>  PRT
<213>  Artificial Sequence

<220>

<223>  epitope

<220>
<221>  misc_feature
<222>  (1)..(2)
<223>  Xaa can be any naturally occurring amino acid

<220>
<221>  misc_feature
<222>  (4)..(5)
<223>  Xaa can be any naturally occurring amino acid

<220>
<221>  misc_feature
<222>  (9)..(9)
<223>  Xaa can be any naturally occurring amino acid

<220>
<221>  misc_feature
<222>  (13)..(15)
<223>  Xaa can be any naturally occurring amino acid

<400>  128

Xaa Xaa Asp Xaa Xaa Arg Lys Ser Xaa Asp Asn Tyr Xaa Xaa Xaa
1               5                   10                  15

<210>  129
<211>  15
<212>  PRT
<213>  Artificial Sequence

<220>
<223>  epitope

<220>
<221>  misc_feature
<222>  (1)..(1)
<223>  Xaa can be any naturally occurring amino acid

<220>
<221>  misc_feature
<222>  (3)..(5)
<223>  Xaa can be any naturally occurring amino acid

<220>
<221>  misc_feature
<222>  (13)..(15)
<223>  Xaa can be any naturally occurring amino acid

<400>  129

Xaa Gly Xaa Xaa Xaa Arg Lys Ser Val Asp Asn Tyr Xaa Xaa Xaa
1               5                   10                  15

<210>  130
<211>  15
<212>  PRT
<213>  Artificial Sequence

<220>
<223>  epitope


<220>
<221>  misc_feature
<222>  (1)..(6)
<223>  Xaa can be any naturally occurring amino acid

<220>
<221>  misc_feature
<222>  (8)..(9)
<223>  Xaa can be any naturally occurring amino acid

<220>
<221>  misc_feature
<222>  (13)..(15)
<223>  Xaa can be any naturally occurring amino acid

<400>  130

Xaa Xaa Xaa Xaa Xaa Xaa Lys Xaa Xaa Asp Asn Tyr Xaa Xaa Xaa
1               5                   10                  15


<210>  131
<211>  15
<212>  PRT
<213>  Artificial Sequence

<220>
<223>  epitope


<220>
<221>  misc_feature
<222>  (1)..(1)
<223>  Xaa can be any naturally occurring amino acid

<220>
<221>  misc_feature
<222>  (14)..(15)
<223>  Xaa can be any naturally occurring amino acid

<400>  131

Xaa Gly Asp Asn Thr Arg Lys Ser Val Asp Asn Tyr Gln Xaa Xaa
1               5                   10                  15


<210>  132
<211>  15
<212>  PRT
<213>  Artificial Sequence

<220>
<223>  epitope


<220>
<221>  misc_feature
<222>  (1)..(5)

<223>  Xaa can be any naturally occurring amino acid

<220>
<221>  misc_feature
<222>  (8)..(9)
<223>  Xaa can be any naturally occurring amino acid

<220>
<221>  misc_feature
<222>  (13)..(15)
<223>  Xaa can be any naturally occurring amino acid

<400>  132

Xaa Xaa Xaa Xaa Xaa Arg Lys Xaa Xaa Asp Asn Tyr Xaa Xaa Xaa
1               5                   10                  15


<210>  133
<211>  15
<212>  PRT
<213>  Artificial Sequence

<220>
<223>  epitope


<220>
<221>  misc_feature
<222>  (1)..(5)
<223>  Xaa can be any naturally occurring amino acid

<220>
<221>  misc_feature
<222>  (14)..(15)
<223>  Xaa can be any naturally occurring amino acid

<400>  133

Xaa Xaa Xaa Xaa Xaa Arg Lys Ser Val Asp Asn Tyr Gln Xaa Xaa
1               5                   10                  15


<210>  134
<211>  15
<212>  PRT
<213>  Artificial Sequence

<220>
<223>  epitope


<220>
<221>  misc_feature
<222>  (1)..(6)
<223>  Xaa can be any naturally occurring amino acid

<220>
<221>  misc_feature
<222>  (8)..(8)
<223>  Xaa can be any naturally occurring amino acid

<220>

<221>  misc_feature
<222>  (13)..(15)
<223>  Xaa can be any naturally occurring amino acid

<400>  134

Xaa Xaa Xaa Xaa Xaa Xaa Lys Xaa Val Asp Asn Tyr Xaa Xaa Xaa
1               5                   10                  15


<210>  135
<211>  7
<212>  PRT
<213>  Artificial Sequence

<220>
<223>  epitope

<400>  135

Asp Asn Thr Arg Lys Ser Val
1               5


<210>  136
<211>  7
<212>  PRT
<213>  Artificial Sequence

<220>
<223>  epitope


<220>
<221>  misc_feature
<222>  (1)..(3)
<223>  Xaa can be any naturally occurring amino acid

<400>  136

Xaa Xaa Xaa Arg Lys Ser Val
1               5


<210>  137
<211>  10
<212>  PRT
<213>  Artificial Sequence

<220>
<223>  epitope

<400>  137

Thr Arg Lys Ser Val Asp Asn Tyr Gln Glu
1               5                   10


<210>  138
<211>  10
<212>  PRT
<213>  Artificial Sequence

<220>
<223>  epitope


<220>
<221>  misc_feature
<222>  (1)..(1)
<223>  Xaa can be any naturally occurring amino acid

<220>
<221>  misc_feature
<222>  (10)..(10)
<223>  Xaa can be any naturally occurring amino acid

<400>  138

Xaa Arg Lys Ser Val Asp Asn Tyr Gln Xaa
1               5                   10


<210>  139
<211>  10
<212>  PRT
<213>  Artificial Sequence

<220>
<223>  epitope


<220>
<221>  misc_feature
<222>  (1)..(2)
<223>  Xaa can be any naturally occurring amino acid

<220>
<221>  misc_feature
<222>  (4)..(4)
<223>  Xaa can be any naturally occurring amino acid

<220>
<221>  misc_feature
<222>  (9)..(10)
<223>  Xaa can be any naturally occurring amino acid

<400>  139

Xaa Xaa Lys Xaa Val Asp Asn Tyr Xaa Xaa
1               5                   10


<210>  140
<211>  15
<212>  PRT
<213>  Artificial Sequence

<220>
<223>  epitope


<220>
<221>  misc_feature
<222>  (1)..(1)
<223>  Xaa can be any naturally occurring amino acid

<220>
<221>  misc_feature
<222>  (8)..(9)
<223>  Xaa can be any naturally occurring amino acid

<220>
<221>  misc_feature
<222>  (12)..(15)
<223>  Xaa can be any naturally occurring amino acid

<400>  140

Xaa Gly Asp Asn Thr Arg Lys Xaa Xaa Asp Asn Xaa Xaa Xaa Xaa
1               5                   10                  15


<210>  141
<211>  15
<212>  PRT
<213>  Artificial Sequence

<220>
<223>  epitope


<220>
<221>  misc_feature
<222>  (1)..(2)
<223>  Xaa can be any naturally occurring amino acid

<220>
<221>  misc_feature
<222>  (5)..(5)
<223>  Xaa can be any naturally occurring amino acid

<220>
<221>  misc_feature
<222>  (7)..(10)
<223>  Xaa can be any naturally occurring amino acid

<220>
<221>  misc_feature
<222>  (12)..(15)
<223>  Xaa can be any naturally occurring amino acid

<400>  141

Xaa Xaa Asp Asn Xaa Arg Xaa Xaa Xaa Xaa Asn Xaa Xaa Xaa Xaa
1               5                   10                  15


<210>  142
<211>  15
<212>  PRT
<213>  Artificial Sequence

<220>
<223>  epitope


<220>
<221>  misc_feature

<222>  (1)..(1)
<223>  Xaa can be any naturally occurring amino acid

<220>
<221>  misc_feature
<222>  (3)..(3)
<223>  Xaa can be any naturally occurring amino acid

<220>
<221>  misc_feature
<222>  (9)..(9)
<223>  Xaa can be any naturally occurring amino acid

<220>
<221>  misc_feature
<222>  (12)..(12)
<223>  Xaa can be any naturally occurring amino acid

<220>
<221>  misc_feature
<222>  (14)..(15)
<223>  Xaa can be any naturally occurring amino acid

<400>  142

Xaa Gly Xaa Asn Thr Arg Lys Ser Xaa Asp Asn Xaa Gln Xaa Xaa
1               5                   10                  15


<210>  143
<211>  15
<212>  PRT
<213>  Artificial Sequence

<220>
<223>  epitope


<220>
<221>  misc_feature
<222>  (1)..(5)
<223>  Xaa can be any naturally occurring amino acid

<220>
<221>  misc_feature
<222>  (9)..(9)
<223>  Xaa can be any naturally occurring amino acid

<220>
<221>  misc_feature
<222>  (12)..(15)
<223>  Xaa can be any naturally occurring amino acid

<400>  143

Xaa Xaa Xaa Xaa Xaa Arg Lys Ser Xaa Asp Asn Xaa Xaa Xaa Xaa
1               5                   10                  15


<210>  144
<211>  4
<212>  PRT
<213>  Artificial Sequence

<220>
<223>  epitope

<400>  144

Asp Asn Thr Arg
1


<210>  145
<211>  15
<212>  PRT
<213>  Artificial Sequence

<220>
<223>  epitope

<400>  145

Ser Pro His Gly Lys Asp Leu Leu Phe Lys Asp Ser Ala Asp Gly
1               5                   10                  15


<210>  146
<211>  15
<212>  PRT
<213>  Artificial Sequence

<220>
<223>  epitope

<400>  146

Ser Pro His Gly Lys Asp Leu Leu Phe Lys Asp Ser Ala Asp Gly
1               5                   10                  15


<210>  147
<211>  6
<212>  PRT
<213>  Artificial Sequence

<220>
<223>  epitope

<400>  147

Ser Pro His Gly Lys Asp
1               5


<210>  148
<211>  8
<212>  PRT
<213>  Artificial Sequence

<220>
<223>  epitope

<400>  148

Lys Asp Leu Leu Phe Lys Asp Ser

1               5


<210>  149
<211>  15
<212>  PRT
<213>  Artificial Sequence

<220>
<223>  epitope

<400>  149

Ser Pro His Gly Lys Asp Leu Leu Phe Lys Asp Ser Ala Asp Gly
1               5                   10                  15


<210>  150
<211>  10
<212>  PRT
<213>  Artificial Sequence

<220>
<223>  epitope

<400>  150

Gln Leu Phe Gln Ser Pro His Gly Lys Asp
1               5                   10


<210>  151
<211>  15
<212>  PRT
<213>  Artificial Sequence

<220>
<223>  epitope

<400>  151

Ser Pro His Gly Lys Asp Leu Leu Phe Lys Asp Ser Ala Asp Gly
1               5                   10                  15


<210>  152
<211>  15
<212>  PRT
<213>  Artificial Sequence

<220>
<223>  epitope


<220>
<221>  misc_feature
<222>  (1)..(2)
<223>  Xaa can be any naturally occurring amino acid

<220>
<221>  misc_feature
<222>  (7)..(9)
<223>  Xaa can be any naturally occurring amino acid

<220>
<221>  misc_feature
<222>  (11)..(15)
<223>  Xaa can be any naturally occurring amino acid

<400>  152

Xaa Xaa His Gly Lys Asp Xaa Xaa Xaa Lys Xaa Xaa Xaa Xaa Xaa
1               5                   10                  15


<210>  153
<211>  9
<212>  PRT
<213>  Artificial Sequence

<220>
<223>  epitope

<400>  153

Lys Asp Ser Ala Asp Gly Phe Leu Lys
1               5


<210>  154
<211>  15
<212>  PRT
<213>  Artificial Sequence

<220>
<223>  epitope

<400>  154

Ser Pro His Gly Lys Asp Leu Leu Phe Lys Asp Ser Ala Asp Gly
1               5                   10                  15


<210>  155
<211>  15
<212>  PRT
<213>  Artificial Sequence

<220>
<223>  epitope


<220>
<221>  misc_feature
<222>  (1)..(2)
<223>  Xaa can be any naturally occurring amino acid

<220>
<221>  misc_feature
<222>  (6)..(9)
<223>  Xaa can be any naturally occurring amino acid

<220>
<221>  misc_feature
<222>  (12)..(12)
<223>  Xaa can be any naturally occurring amino acid

<220>
<221>  misc_feature
<222>  (14)..(15)
<223>  Xaa can be any naturally occurring amino acid

<400>  155

Xaa Xaa His Gly Lys Xaa Xaa Xaa Xaa Lys Asp Xaa Ala Xaa Xaa
1               5                   10                  15


<210>  156
<211>  9
<212>  PRT
<213>  Artificial Sequence

<220>
<223>  epitope

<400>  156

Gln Ser Pro His Gly Lys Asp Leu Leu
1               5


<210>  157
<211>  9
<212>  PRT
<213>  Artificial Sequence

<220>
<223>  epitope


<220>
<221>  misc_feature
<222>  (2)..(3)
<223>  Xaa can be any naturally occurring amino acid

<220>
<221>  misc_feature
<222>  (7)..(9)
<223>  Xaa can be any naturally occurring amino acid

<400>  157

Gln Xaa Xaa His Gly Lys Xaa Xaa Xaa
1               5


<210>  158
<211>  6
<212>  PRT
<213>  Artificial Sequence

<220>
<223>  epitope

<400>  158

Lys Asp Ser Ala Asp Gly
1               5

<210>  159
<211>  15
<212>  PRT
<213>  Artificial Sequence

<220>
<223>  epitope

<400>  159

Ser Pro His Gly Lys Asp Leu Leu Phe Lys Asp Ser Ala Asp Gly
1               5                   10                  15


<210>  160
<211>  15
<212>  PRT
<213>  Artificial Sequence

<220>
<223>  epitope


<220>
<221>  misc_feature
<222>  (1)..(1)
<223>  Xaa can be any naturally occurring amino acid

<220>
<221>  misc_feature
<222>  (6)..(15)
<223>  Xaa can be any naturally occurring amino acid

<400>  160

Xaa Pro His Gly Lys Xaa Xaa Xaa Xaa Xaa Xaa Xaa Xaa Xaa Xaa
1               5                   10                  15


<210>  161
<211>  10
<212>  PRT
<213>  Artificial Sequence

<220>
<223>  epitope

<400>  161

Gln Leu Phe Gln Ser Pro His Gly Lys Asp
1               5                   10


<210>  162
<211>  10
<212>  PRT
<213>  Artificial Sequence

<220>
<223>  epitope

<220>
<221>  misc_feature
<222>  (5)..(5)
<223>  Xaa can be any naturally occurring amino acid

<220>
<221>  misc_feature
<222>  (10)..(10)
<223>  Xaa can be any naturally occurring amino acid

<400>  162

Gln Leu Phe Gln Xaa Pro His Gly Lys Xaa
1               5                   10


<210>  163
<211>  5
<212>  PRT
<213>  Artificial Sequence

<220>
<223>  epitope

<400>  163

Gly Lys Asp Leu Leu
1               5


<210>  164
<211>  8
<212>  PRT
<213>  Artificial Sequence

<220>
<223>  epitope

<400>  164

Lys Asp Ser Ala Asp Gly Phe Leu
1               5


<210>  165
<211>  15
<212>  PRT
<213>  Artificial Sequence

<220>
<223>  epitope

<400>  165

Ser Pro His Gly Lys Asp Leu Leu Phe Lys Asp Ser Ala Asp Gly
1               5                   10                  15


<210>  166
<211>  8
<212>  PRT
<213>  Artificial Sequence

<220>
<223>  epitope

<400>  166

Lys Asp Ser Ala Asp Gly Phe Leu
1               5


<210>  167
<211>  15
<212>  PRT
<213>  Artificial Sequence

<220>
<223>  epitope

<400>  167

Ser Pro His Gly Lys Asp Leu Leu Phe Lys Asp Ser Ala Asp Gly
1               5                   10                  15


<210>  168
<211>  10
<212>  PRT
<213>  Artificial Sequence

<220>
<223>  epitope

<400>  168

Gln Leu Phe Gln Ser Pro His Gly Lys Asp
1               5                   10


<210>  169
<211>  15
<212>  PRT
<213>  Artificial Sequence

<220>
<223>  epitope

<400>  169

Ser Pro His Gly Lys Asp Leu Leu Phe Lys Asp Ser Ala Asp Gly
1               5                   10                  15


<210>  170
<211>  15
<212>  PRT
<213>  Artificial Sequence

<220>
<223>  epitope

<400>  170

Ser Pro His Gly Lys Asp Leu Leu Phe Lys Asp Ser Ala Asp Gly

1               5                   10                  15


<210>  171
<211>  15
<212>  PRT
<213>  Artificial Sequence

<220>
<223>  epitope


<220>
<221>  misc_feature
<222>  (2)..(2)
<223>  Xaa can be any naturally occurring amino acid

<220>
<221>  misc_feature
<222>  (4)..(4)
<223>  Xaa can be any naturally occurring amino acid

<220>
<221>  misc_feature
<222>  (7)..(10)
<223>  Xaa can be any naturally occurring amino acid

<220>
<221>  misc_feature
<222>  (12)..(13)
<223>  Xaa can be any naturally occurring amino acid

<400>  171

Ser Xaa His Xaa Lys Asp Xaa Xaa Xaa Xaa Asp Xaa Xaa Asp Gly
1               5                   10                  15


<210>  172
<211>  6
<212>  PRT
<213>  Artificial Sequence

<220>
<223>  epitope

<400>  172

Lys Asp Leu Leu Phe Lys
1               5


<210>  173
<211>  5
<212>  PRT
<213>  Artificial Sequence

<220>
<223>  epitope

<400>  173

Asp Leu Leu Phe Lys

1               5


<210>  174
<211>  10
<212>  PRT
<213>  Artificial Sequence

<220>
<223>  epitope

<400>  174

Phe Lys Asp Ser Ala Asp Gly Phe Leu Lys
1               5                   10


<210>  175
<211>  10
<212>  PRT
<213>  Artificial Sequence

<220>
<223>  epitope


<220>
<221>  misc_feature
<222>  (1)..(2)
<223>  Xaa can be any naturally occurring amino acid

<220>
<221>  misc_feature
<222>  (4)..(5)
<223>  Xaa can be any naturally occurring amino acid

<400>  175

Xaa Xaa Asp Xaa Xaa Asp Gly Phe Leu Lys
1               5                   10


<210>  176
<211>  15
<212>  PRT
<213>  Artificial Sequence

<220>
<223>  epitope

<400>  176

Ser Pro His Gly Lys Asp Leu Leu Phe Lys Asp Ser Ala Asp Gly
1               5                   10                  15


<210>  177
<211>  9
<212>  PRT
<213>  Artificial Sequence

<220>
<223>  epitope

<400>  177

Pro His Gly Lys Asp Leu Leu Phe Lys
1               5


<210>  178
<211>  10
<212>  PRT
<213>  Artificial Sequence

<220>
<223>  epitope

<400>  178

Gly Lys Asp Leu Leu Phe Lys Asp Ser Ala
1               5                   10


<210>  179
<211>  6
<212>  PRT
<213>  Artificial Sequence

<220>
<223>  epitope

<400>  179

Lys Asp Ser Ala Asp Gly
1               5


<210>  180
<211>  15
<212>  PRT
<213>  Artificial Sequence

<220>
<223>  epitope


<220>
<221>  misc_feature
<222>  (1)..(2)
<223>  Xaa can be any naturally occurring amino acid

<220>
<221>  misc_feature
<222>  (4)..(4)
<223>  Xaa can be any naturally occurring amino acid

<220>
<221>  misc_feature
<222>  (6)..(9)
<223>  Xaa can be any naturally occurring amino acid

<220>
<221>  misc_feature
<222>  (13)..(15)
<223>  Xaa can be any naturally occurring amino acid

<400>  180

Xaa Xaa His Xaa Lys Xaa Xaa Xaa Xaa Lys Asp Ser Xaa Xaa Xaa
1               5                   10                  15


<210>  181
<211>  7
<212>  PRT
<213>  Artificial Sequence

<220>
<223>  epitope

<400>  181

Asp Leu Leu Phe Lys Asp Ser
1               5


<210>  182
<211>  5
<212>  PRT
<213>  Artificial Sequence

<220>
<223>  epitope

<400>  182

Gly Lys Asp Leu Leu
1               5


<210>  183
<211>  15
<212>  PRT
<213>  Artificial Sequence

<220>
<223>  epitope


<220>
<221>  misc_feature
<222>  (1)..(2)
<223>  Xaa can be any naturally occurring amino acid

<220>
<221>  misc_feature
<222>  (4)..(4)
<223>  Xaa can be any naturally occurring amino acid

<220>
<221>  misc_feature
<222>  (6)..(7)
<223>  Xaa can be any naturally occurring amino acid

<220>
<221>  misc_feature
<222>  (11)..(15)
<223>  Xaa can be any naturally occurring amino acid

<400>  183

Xaa Xaa His Xaa Lys Xaa Xaa Leu Phe Lys Xaa Xaa Xaa Xaa Xaa
1               5                   10                  15


<210>  184
<211>  6
<212>  PRT
<213>  Artificial Sequence

<220>
<223>  epitope

<400>  184

Lys Asp Leu Leu Phe Lys
1               5


<210>  185
<211>  5
<212>  PRT
<213>  Artificial Sequence

<220>
<223>  epitope

<400>  185

Asp Leu Leu Phe Lys
1               5


<210>  186
<211>  6
<212>  PRT
<213>  Artificial Sequence

<220>
<223>  epitope

<400>  186

Lys Asp Ser Ala Asp Gly
1               5


<210>  187
<211>  15
<212>  PRT
<213>  Artificial Sequence

<220>
<223>  epitope


<220>
<221>  misc_feature
<222>  (1)..(2)
<223>  Xaa can be any naturally occurring amino acid

<220>
<221>  misc_feature
<222>  (4)..(4)
<223>  Xaa can be any naturally occurring amino acid

<220>
<221>  misc_feature
<222>  (7)..(7)
<223>  Xaa can be any naturally occurring amino acid

<220>
<221>  misc_feature
<222>  (13)..(15)
<223>  Xaa can be any naturally occurring amino acid

<400>  187

Xaa Xaa His Xaa Lys Asp Xaa Leu Phe Lys Asp Ser Xaa Xaa Xaa
1               5                   10                  15


<210>  188
<211>  15
<212>  PRT
<213>  Artificial Sequence

<220>
<223>  epitope


<220>
<221>  misc_feature
<222>  (1)..(2)
<223>  Xaa can be any naturally occurring amino acid

<220>
<221>  misc_feature
<222>  (4)..(4)
<223>  Xaa can be any naturally occurring amino acid

<220>
<221>  misc_feature
<222>  (6)..(9)
<223>  Xaa can be any naturally occurring amino acid

<220>
<221>  misc_feature
<222>  (12)..(15)
<223>  Xaa can be any naturally occurring amino acid

<400>  188

Xaa Xaa His Xaa Lys Xaa Xaa Xaa Xaa Lys Asp Xaa Xaa Xaa Xaa
1               5                   10                  15


<210>  189
<211>  6
<212>  PRT
<213>  Artificial Sequence

<220>
<223>  epitope

<400>  189

Lys Asp Leu Leu Phe Lys
1               5


<210>  190
<211>  6
<212>  PRT
<213>  Artificial Sequence

<220>
<223>  epitope


<220>
<221>  misc_feature
<222>  (3)..(3)
<223>  Xaa can be any naturally occurring amino acid

<400>  190

Lys Asp Xaa Leu Phe Lys
1               5


<210>  191
<211>  15
<212>  PRT
<213>  Artificial Sequence

<220>
<223>  epitope


<220>
<221>  misc_feature
<222>  (1)..(4)
<223>  Xaa can be any naturally occurring amino acid

<220>
<221>  misc_feature
<222>  (6)..(6)
<223>  Xaa can be any naturally occurring amino acid

<220>
<221>  misc_feature
<222>  (11)..(15)
<223>  Xaa can be any naturally occurring amino acid

<400>  191

Xaa Xaa Xaa Xaa Lys Xaa Leu Leu Phe Lys Xaa Xaa Xaa Xaa Xaa
1               5                   10                  15


<210>  192
<211>  4
<212>  PRT
<213>  Artificial Sequence

<220>

<223>  epitope

<400>  192

Leu Leu Phe Lys
1



<210>  193
<211>  6
<212>  PRT
<213>  Artificial Sequence

<220>
<223>  epitope

<400>  193

Ser Pro His Gly Lys Asp
1               5



<210>  194
<211>  7
<212>  PRT
<213>  Artificial Sequence

<220>
<223>  epitope

<400>  194

Phe Lys Asp Ser Ala Asp Gly
1               5



<210>  195
<211>  4
<212>  PRT
<213>  Artificial Sequence

<220>
<223>  epitope

<400>  195

Lys Asp Ser Ala
1



<210>  196
<211>  15
<212>  PRT
<213>  Artificial Sequence

<220>
<223>  epitope

<400>  196

His Ser Thr Val Phe Asp Asn Leu Pro Asn Pro Glu Asp Arg Lys
1               5                   10                  15

<210>  197
<211>  15
<212>  PRT
<213>  Artificial Sequence

<220>
<223>  epitope

<400>  197

His Ser Thr Val Phe Asp Asn Leu Pro Asn Pro Glu Asp Arg Lys
1               5                   10                  15


<210>  198
<211>  15
<212>  PRT
<213>  Artificial Sequence

<220>
<223>  epitope


<220>
<221>  misc_feature
<222>  (1)..(9)
<223>  Xaa can be any naturally occurring amino acid

<400>  198

Xaa Xaa Xaa Xaa Xaa Xaa Xaa Xaa Xaa Asn Pro Glu Asp Arg Lys
1               5                   10                  15


<210>  199
<211>  10
<212>  PRT
<213>  Artificial Sequence

<220>
<223>  epitope

<400>  199

Phe Val Lys His Ser Thr Val Phe Asp Asn
1               5                   10


<210>  200
<211>  8
<212>  PRT
<213>  Artificial Sequence

<220>
<223>  epitope

<400>  200

Val Lys His Ser Thr Val Phe Asp
1               5

<210>  201
<211>  7
<212>  PRT
<213>  Artificial Sequence

<220>
<223>  epitope

<400>  201

Lys His Ser Thr Val Phe Asp
1               5


<210>  202
<211>  6
<212>  PRT
<213>  Artificial Sequence

<220>
<223>  epitope

<400>  202

Asn Pro Glu Asp Arg Lys
1               5


<210>  203
<211>  5
<212>  PRT
<213>  Artificial Sequence

<220>
<223>  epitope

<400>  203

Asn Pro Glu Asp Arg
1               5


<210>  204
<211>  15
<212>  PRT
<213>  Artificial Sequence

<220>
<223>  epitope

<400>  204

His Ser Thr Val Phe Asp Asn Leu Pro Asn Pro Glu Asp Arg Lys
1               5                   10                  15


<210>  205
<211>  7
<212>  PRT
<213>  Artificial Sequence

<220>
<223>  epitope

<400>  205

Lys His Ser Thr Val Phe Asp
1               5



<210>  206
<211>  6
<212>  PRT
<213>  Artificial Sequence

<220>
<223>  epitope

<400>  206

His Ser Thr Val Phe Asp
1               5



<210>  207
<211>  6
<212>  PRT
<213>  Artificial Sequence

<220>
<223>  epitope

<400>  207

Asn Pro Glu Asp Arg Lys
1               5



<210>  208
<211>  5
<212>  PRT
<213>  Artificial Sequence

<220>
<223>  epitope

<400>  208

Asn Pro Glu Asp Arg
1               5



<210>  209
<211>  15
<212>  PRT
<213>  Artificial Sequence

<220>
<223>  epitope

<400>  209

His Ser Thr Val Phe Asp Asn Leu Pro Asn Pro Glu Asp Arg Lys
1               5                   10                  15

<210>  210
<211>  9
<212>  PRT
<213>  Artificial Sequence

<220>
<223>  epitope

<400>  210

Phe Val Lys His Ser Thr Val Phe Asp
1               5


<210>  211
<211>  7
<212>  PRT
<213>  Artificial Sequence

<220>
<223>  epitope

<400>  211

Lys His Ser Thr Val Phe Asp
1               5


<210>  212
<211>  6
<212>  PRT
<213>  Artificial Sequence

<220>
<223>  epitope

<400>  212

His Ser Thr Val Phe Asp
1               5


<210>  213
<211>  5
<212>  PRT
<213>  Artificial Sequence

<220>
<223>  epitope

<400>  213

Asn Pro Glu Asp Arg
1               5


<210>  214
<211>  15
<212>  PRT
<213>  Artificial Sequence

<220>
<223>  epitope

<400>  214

His Ser Thr Val Phe Asp Asn Leu Pro Asn Pro Glu Asp Arg Lys
1               5                   10                  15


<210>  215
<211>  7
<212>  PRT
<213>  Artificial Sequence

<220>
<223>  epitope

<400>  215

Lys His Ser Thr Val Phe Asp
1               5


<210>  216
<211>  6
<212>  PRT
<213>  Artificial Sequence

<220>
<223>  epitope

<400>  216

His Ser Thr Val Phe Asp
1               5


<210>  217
<211>  5
<212>  PRT
<213>  Artificial Sequence

<220>
<223>  epitope

<400>  217

Asn Pro Glu Asp Arg
1               5


<210>  218
<211>  15
<212>  PRT
<213>  Artificial Sequence

<220>
<223>  epitope

<400>  218

His Ser Thr Val Phe Asp Asn Leu Pro Asn Pro Glu Asp Arg Lys
1               5                   10                  15

<210>  219
<211>  6
<212>  PRT
<213>  Artificial Sequence

<220>
<223>  epitope

<400>  219

His Ser Thr Val Phe Asp
1               5


<210>  220
<211>  5
<212>  PRT
<213>  Artificial Sequence

<220>
<223>  epitope

<400>  220

Asn Pro Glu Asp Arg
1               5


<210>  221
<211>  15
<212>  PRT
<213>  Artificial Sequence

<220>
<223>  epitope

<400>  221

His Ser Thr Val Phe Asp Asn Leu Pro Asn Pro Glu Asp Arg Lys
1               5                   10                  15


<210>  222
<211>  15
<212>  PRT
<213>  Artificial Sequence

<220>
<223>  epitope

<400>  222

His Ser Thr Val Phe Asp Asn Leu Pro Asn Pro Glu Asp Arg Lys
1               5                   10                  15


<210>  223
<211>  15
<212>  PRT
<213>  Artificial Sequence

<220>
<223>  epitope

<220>
<221>  misc_feature
<222>  (1)..(10)
<223>  Xaa can be any naturally occurring amino acid

<400>  223

Xaa Xaa Xaa Xaa Xaa Xaa Xaa Xaa Xaa Xaa Pro Glu Asp Arg Lys
1               5                   10                  15


<210>  224
<211>  7
<212>  PRT
<213>  Artificial Sequence

<220>
<223>  epitope

<400>  224

Lys His Ser Thr Val Phe Asp
1               5


<210>  225
<211>  6
<212>  PRT
<213>  Artificial Sequence

<220>
<223>  epitope

<400>  225

Asn Pro Glu Asp Arg Lys
1               5


<210>  226
<211>  6
<212>  PRT
<213>  Artificial Sequence

<220>
<223>  epitope


<220>
<221>  misc_feature
<222>  (1)..(1)
<223>  Xaa can be any naturally occurring amino acid

<400>  226

Xaa Pro Glu Asp Arg Lys
1               5


<210>  227
<211>  15

<212>  PRT
<213>  Artificial Sequence

<220>
<223>  epitope

<400>  227

His Ser Thr Val Phe Asp Asn Leu Pro Asn Pro Glu Asp Arg Lys
1               5                   10                  15


<210>  228
<211>  15
<212>  PRT
<213>  Artificial Sequence

<220>
<223>  epitope

<400>  228

His Ser Thr Val Phe Asp Asn Leu Pro Asn Pro Glu Asp Arg Lys
1               5                   10                  15


<210>  229
<211>  4
<212>  PRT
<213>  Artificial Sequence

<220>
<223>  epitope

<400>  229

Thr Val Phe Asp
1


<210>  230
<211>  5
<212>  PRT
<213>  Artificial Sequence

<220>
<223>  epitope

<400>  230

Asn Pro Glu Asp Arg
1               5


<210>  231
<211>  5
<212>  PRT
<213>  Artificial Sequence

<220>
<223>  epitope

<400>  231

Ser Thr Val Phe Asp
1               5

<210>  232
<211>  6
<212>  PRT
<213>  Artificial Sequence

<220>
<223>  epitope

<400>  232

Asn Pro Glu Asp Arg Lys
1               5

<210>  233
<211>  5
<212>  PRT
<213>  Artificial Sequence

<220>
<223>  epitope

<400>  233

Asn Pro Glu Asp Arg
1               5

<210>  234
<211>  5
<212>  PRT
<213>  Artificial Sequence

<220>
<223>  epitope

<400>  234

Ser Thr Val Phe Asp
1               5

<210>  235
<211>  5
<212>  PRT
<213>  Artificial Sequence

<220>
<223>  epitope

<400>  235

Asn Pro Glu Asp Arg
1               5

<210>  236
<211>  6

<212>  PRT
<213>  Artificial Sequence

<220>
<223>  epitope

<400>  236

His Ser Thr Val Phe Asp
1               5


<210>  237
<211>  4
<212>  PRT
<213>  Artificial Sequence

<220>
<223>  epitope

<400>  237

Thr Val Phe Asp
1


<210>  238
<211>  5
<212>  PRT
<213>  Artificial Sequence

<220>
<223>  epitope

<400>  238

Asn Pro Glu Asp Arg
1               5


<210>  239
<211>  15
<212>  PRT
<213>  Artificial Sequence

<220>
<223>  epitope


<220>
<221>  misc_feature
<222>  (1)..(1)
<223>  Xaa can be any naturally occurring amino acid

<220>
<221>  misc_feature
<222>  (3)..(10)
<223>  Xaa can be any naturally occurring amino acid

<220>
<221>  misc_feature
<222>  (15)..(15)
<223>  Xaa can be any naturally occurring amino acid

<400>  239

Xaa Ser Xaa Xaa Xaa Xaa Xaa Xaa Xaa Xaa Pro Glu Asp Arg Xaa
1               5                   10                  15


<210>  240
<211>  10
<212>  PRT
<213>  Artificial Sequence

<220>
<223>  epitope

<400>  240

Asp Asn Leu Pro Asn Pro Glu Asp Arg Lys
1               5                   10


<210>  241
<211>  5
<212>  PRT
<213>  Artificial Sequence

<220>
<223>  epitope

<400>  241

Asn Pro Glu Asp Arg
1               5


<210>  242
<211>  5
<212>  PRT
<213>  Artificial Sequence

<220>
<223>  epitope


<220>
<221>  misc_feature
<222>  (1)..(1)
<223>  Xaa can be any naturally occurring amino acid

<400>  242

Xaa Pro Glu Asp Arg
1               5


<210>  243
<211>  6
<212>  PRT
<213>  Artificial Sequence

<220>
<223>  epitope

<400>  243

Asn Pro Glu Asp Arg Lys
1               5



<210>  244
<211>  6
<212>  PRT
<213>  Artificial Sequence

<220>
<223>  epitope

<400>  244

His Ser Thr Val Phe Asp
1               5



<210>  245
<211>  6
<212>  PRT
<213>  Artificial Sequence

<220>
<223>  epitope

<400>  245

Asn Pro Glu Asp Arg Lys
1               5



<210>  246
<211>  5
<212>  PRT
<213>  Artificial Sequence

<220>
<223>  epitope

<400>  246

Ser Thr Val Phe Asp
1               5



<210>  247
<211>  4
<212>  PRT
<213>  Artificial Sequence

<220>
<223>  epitope

<400>  247

Thr Val Phe Asp
1



<210>  248

<211>  6
<212>  PRT
<213>  Artificial Sequence

<220>
<223>  epitope

<400>  248

Asn Pro Glu Asp Arg Lys
1               5


<210>  249
<211>  15
<212>  PRT
<213>  Artificial Sequence

<220>
<223>  epitope


<220>
<221>  misc_feature
<222>  (1)..(9)
<223>  Xaa can be any naturally occurring amino acid

<400>  249

Xaa Xaa Xaa Xaa Xaa Xaa Xaa Xaa Xaa Asn Pro Glu Asp Arg Lys
1               5                   10                  15


<210>  250
<211>  15
<212>  PRT
<213>  Artificial Sequence

<220>
<223>  epitope


<220>
<221>  misc_feature
<222>  (1)..(10)
<223>  Xaa can be any naturally occurring amino acid

<400>  250

Xaa Xaa Xaa Xaa Xaa Xaa Xaa Xaa Xaa Xaa Pro Glu Asp Arg Lys
1               5                   10                  15


<210>  251
<211>  15
<212>  PRT
<213>  Artificial Sequence

<220>
<223>  epitope


<220>

<221>  misc_feature
<222>  (1)..(10)
<223>  Xaa can be any naturally occurring amino acid

<220>
<221>  misc_feature
<222>  (15)..(15)
<223>  Xaa can be any naturally occurring amino acid

<400>  251

Xaa Xaa Xaa Xaa Xaa Xaa Xaa Xaa Xaa Xaa Pro Glu Asp Arg Xaa
1               5                   10                  15


<210>  252
<211>  6
<212>  PRT
<213>  Artificial Sequence

<220>
<223>  epitope

<400>  252

Asn Pro Glu Asp Arg Lys
1               5


<210>  253
<211>  5
<212>  PRT
<213>  Artificial Sequence

<220>
<223>  epitope

<400>  253

Asn Pro Glu Asp Arg
1               5


<210>  254
<211>  5
<212>  PRT
<213>  Artificial Sequence

<220>
<223>  epitope


<220>
<221>  misc_feature
<222>  (1)..(1)
<223>  Xaa can be any naturally occurring amino acid

<400>  254

Xaa Pro Glu Asp Arg
1               5

<210>  255
<211>  15
<212>  PRT
<213>  Artificial Sequence

<220>
<223>  epitope

<400>  255

Asp Cys Thr Ser Asn Phe Cys Leu Phe Gln Ser Asn Ser Lys Asp
1               5                   10                  15


<210>  256
<211>  15
<212>  PRT
<213>  Artificial Sequence

<220>
<223>  epitope

<400>  256

Asp Cys Thr Ser Asn Phe Cys Leu Phe Gln Ser Asn Ser Lys Asp
1               5                   10                  15


<210>  257
<211>  5
<212>  PRT
<213>  Artificial Sequence

<220>
<223>  epitope

<400>  257

Gln Ser Asn Ser Lys
1               5


<210>  258
<211>  15
<212>  PRT
<213>  Artificial Sequence

<220>
<223>  epitope

<400>  258

Asp Cys Thr Ser Asn Phe Cys Leu Phe Gln Ser Asn Ser Lys Asp
1               5                   10                  15


<210>  259
<211>  15
<212>  PRT
<213>  Artificial Sequence

<220>
<223>  epitope

<220>
<221>  misc_feature
<222>  (1)..(8)
<223>  Xaa can be any naturally occurring amino acid

<400>  259

Xaa Xaa Xaa Xaa Xaa Xaa Xaa Xaa Phe Gln Ser Asn Ser Lys Asp
1               5                   10                  15


<210>  260
<211>  8
<212>  PRT
<213>  Artificial Sequence

<220>
<223>  epitope

<400>  260

Cys Leu Phe Gln Ser Asn Ser Lys
1               5


<210>  261
<211>  5
<212>  PRT
<213>  Artificial Sequence

<220>
<223>  epitope

<400>  261

Gln Ser Asn Ser Lys
1               5


<210>  262
<211>  15
<212>  PRT
<213>  Artificial Sequence

<220>
<223>  epitope

<400>  262

Asp Cys Thr Ser Asn Phe Cys Leu Phe Gln Ser Asn Ser Lys Asp
1               5                   10                  15


<210>  263
<211>  15
<212>  PRT
<213>  Artificial Sequence

<220>
<223>  epitope

<220>
<221>  misc_feature
<222>  (1)..(8)
<223>  Xaa can be any naturally occurring amino acid

<400>  263

Xaa Xaa Xaa Xaa Xaa Xaa Xaa Xaa Phe Gln Ser Asn Ser Lys Asp
1               5                   10                  15


<210>  264
<211>  5
<212>  PRT
<213>  Artificial Sequence

<220>
<223>  epitope

<400>  264

Gln Ser Asn Ser Lys
1               5


<210>  265
<211>  15
<212>  PRT
<213>  Artificial Sequence

<220>
<223>  epitope

<400>  265

Asp Cys Thr Ser Asn Phe Cys Leu Phe Gln Ser Asn Ser Lys Asp
1               5                   10                  15


<210>  266
<211>  15
<212>  PRT
<213>  Artificial Sequence

<220>
<223>  epitope


<220>
<221>  misc_feature
<222>  (1)..(5)
<223>  Xaa can be any naturally occurring amino acid

<220>
<221>  misc_feature
<222>  (12)..(12)
<223>  Xaa can be any naturally occurring amino acid

<400>  266

Xaa Xaa Xaa Xaa Xaa Phe Cys Leu Phe Gln Ser Xaa Ser Lys Asp
1               5                   10                  15

<210>  267
<211>  8
<212>  PRT
<213>  Artificial Sequence

<220>
<223>  epitope

<400>  267

Leu Phe Gln Ser Asn Ser Lys Asp
1               5


<210>  268
<211>  6
<212>  PRT
<213>  Artificial Sequence

<220>
<223>  epitope

<400>  268

Phe Gln Ser Asn Ser Lys
1               5


<210>  269
<211>  6
<212>  PRT
<213>  Artificial Sequence

<220>
<223>  epitope


<220>
<221>  misc_feature
<222>  (4)..(4)
<223>  Xaa can be any naturally occurring amino acid

<400>  269

Phe Gln Ser Xaa Ser Lys
1               5


<210>  270
<211>  4
<212>  PRT
<213>  Artificial Sequence

<220>
<223>  epitope

<400>  270

Gln Ser Asn Ser
1

<210>  271
<211>  15
<212>  PRT
<213>  Artificial Sequence

<220>
<223>  epitope

<400>  271

Asp Cys Thr Ser Asn Phe Cys Leu Phe Gln Ser Asn Ser Lys Asp
1               5                   10                  15


<210>  272
<211>  15
<212>  PRT
<213>  Artificial Sequence

<220>
<223>  epitope


<220>
<221>  misc_feature
<222>  (1)..(6)
<223>  Xaa can be any naturally occurring amino acid

<220>
<221>  misc_feature
<222>  (10)..(13)
<223>  Xaa can be any naturally occurring amino acid

<220>
<221>  misc_feature
<222>  (15)..(15)
<223>  Xaa can be any naturally occurring amino acid

<400>  272

Xaa Xaa Xaa Xaa Xaa Xaa Cys Leu Phe Xaa Xaa Xaa Xaa Lys Xaa
1               5                   10                  15


<210>  273
<211>  6
<212>  PRT
<213>  Artificial Sequence

<220>
<223>  epitope

<400>  273

Phe Gln Ser Asn Ser Lys
1               5


<210>  274
<211>  5
<212>  PRT
<213>  Artificial Sequence

<220>
<223>  epitope

<400>  274

Gln Ser Asn Ser Lys
1               5


<210>  275
<211>  4
<212>  PRT
<213>  Artificial Sequence

<220>
<223>  epitope

<400>  275

Gln Ser Asn Ser
1


<210>  276
<211>  5
<212>  PRT
<213>  Artificial Sequence

<220>
<223>  epitope

<400>  276

Gln Ser Asn Ser Lys
1               5


<210>  277
<211>  15
<212>  PRT
<213>  Artificial Sequence

<220>
<223>  epitope


<220>
<221>  misc_feature
<222>  (1)..(8)
<223>  Xaa can be any naturally occurring amino acid

<220>
<221>  misc_feature
<222>  (10)..(11)
<223>  Xaa can be any naturally occurring amino acid

<400>  277

Xaa Xaa Xaa Xaa Xaa Xaa Xaa Xaa Phe Xaa Xaa Asn Ser Lys Asp
1               5                   10                  15

<210> 278
<211> 5
<212> PRT
<213> Artificial Sequence

<220>
<223> epitope

<400> 278

Gln Ser Asn Ser Lys
1               5

<210> 279
<211> 8
<212> PRT
<213> Artificial Sequence

<220>
<223> epitope

<400> 279

Cys Leu Phe Gln Ser Asn Ser Lys
1               5

<210> 280
<211> 5
<212> PRT
<213> Artificial Sequence

<220>
<223> epitope

<400> 280

Gln Ser Asn Ser Lys
1               5

<210> 281
<211> 5
<212> PRT
<213> Artificial Sequence

<220>
<223> epitope

<400> 281

Gln Ser Asn Ser Lys
1               5

<210> 282
<211> 15
<212> PRT
<213> Artificial Sequence

<220>
<223> epitope

<220>
<221>  misc_feature
<222>  (1)..(6)
<223>  Xaa can be any naturally occurring amino acid

<220>
<221>  misc_feature
<222>  (10)..(12)
<223>  Xaa can be any naturally occurring amino acid

<400>  282

Xaa Xaa Xaa Xaa Xaa Xaa Cys Leu Phe Xaa Xaa Xaa Ser Lys Asp
1               5                   10                  15


<210>  283
<211>  8
<212>  PRT
<213>  Artificial Sequence

<220>
<223>  epitope

<400>  283

Cys Leu Phe Gln Ser Asn Ser Lys
1               5


<210>  284
<211>  5
<212>  PRT
<213>  Artificial Sequence

<220>
<223>  epitope

<400>  284

Gln Ser Asn Ser Lys
1               5


<210>  285
<211>  5
<212>  PRT
<213>  Artificial Sequence

<220>
<223>  epitope

<400>  285

Gln Ser Asn Ser Lys
1               5


<210>  286
<211>  4
<212>  PRT

<213>  Artificial Sequence

<220>
<223>  epitope

<400>  286

Gln Ser Asn Ser
1



<210>  287
<211>  15
<212>  PRT
<213>  Artificial Sequence

<220>
<223>  epitope



<220>
<221>  misc_feature
<222>  (1)..(8)
<223>  Xaa can be any naturally occurring amino acid

<220>
<221>  misc_feature
<222>  (10)..(11)
<223>  Xaa can be any naturally occurring amino acid

<400>  287

Xaa Xaa Xaa Xaa Xaa Xaa Xaa Xaa Phe Xaa Xaa Asn Ser Lys Asp
1               5                   10                  15



<210>  288
<211>  5
<212>  PRT
<213>  Artificial Sequence

<220>
<223>  epitope

<400>  288

Gln Ser Asn Ser Lys
1               5



<210>  289
<211>  15
<212>  PRT
<213>  Artificial Sequence

<220>
<223>  epitope

<400>  289

Leu Ala Glu Asn Tyr Lys Thr Glu Gly Glu Ser Cys Lys Asn Thr
1               5                   10                  15

<210>  290
<211>  15
<212>  PRT
<213>  Artificial Sequence

<220>
<223>  epitope

<400>  290

Leu Ala Glu Asn Tyr Lys Thr Glu Gly Glu Ser Cys Lys Asn Thr
1               5                   10                  15


<210>  291
<211>  15
<212>  PRT
<213>  Artificial Sequence

<220>
<223>  epitope


<220>
<221>  misc_feature
<222>  (1)..(5)
<223>  Xaa can be any naturally occurring amino acid

<220>
<221>  misc_feature
<222>  (7)..(9)
<223>  Xaa can be any naturally occurring amino acid

<220>
<221>  misc_feature
<222>  (12)..(12)
<223>  Xaa can be any naturally occurring amino acid

<220>
<221>  misc_feature
<222>  (14)..(14)
<223>  Xaa can be any naturally occurring amino acid

<400>  291

Xaa Xaa Xaa Xaa Xaa Lys Xaa Xaa Xaa Glu Ser Xaa Lys Xaa Thr
1               5                   10                  15


<210>  292
<211>  8
<212>  PRT
<213>  Artificial Sequence

<220>
<223>  epitope

<400>  292

Ala Glu Asn Tyr Lys Thr Glu Gly
1               5

<210>  293
<211>  9
<212>  PRT
<213>  Artificial Sequence

<220>
<223>  epitope

<400>  293

Tyr Lys Thr Glu Gly Glu Ser Cys Lys
1               5


<210>  294
<211>  9
<212>  PRT
<213>  Artificial Sequence

<220>
<223>  epitope


<220>
<221>  misc_feature
<222>  (1)..(1)
<223>  Xaa can be any naturally occurring amino acid

<220>
<221>  misc_feature
<222>  (3)..(5)
<223>  Xaa can be any naturally occurring amino acid

<220>
<221>  misc_feature
<222>  (8)..(8)
<223>  Xaa can be any naturally occurring amino acid

<400>  294

Xaa Lys Xaa Xaa Xaa Glu Ser Xaa Lys
1               5


<210>  295
<211>  9
<212>  PRT
<213>  Artificial Sequence

<220>
<223>  epitope

<400>  295

Glu Ser Cys Lys Asn Thr Pro Glu Lys
1               5


<210>  296
<211>  8
<212>  PRT
<213>  Artificial Sequence

<220>
<223>  epitope

<400>  296

Glu Ser Cys Lys Asn Thr Pro Glu
1               5


<210>  297
<211>  8
<212>  PRT
<213>  Artificial Sequence

<220>
<223>  epitope


<220>
<221>  misc_feature
<222>  (3)..(3)
<223>  Xaa can be any naturally occurring amino acid

<220>
<221>  misc_feature
<222>  (5)..(5)
<223>  Xaa can be any naturally occurring amino acid

<400>  297

Glu Ser Xaa Lys Xaa Thr Pro Glu
1               5


<210>  298
<211>  15
<212>  PRT
<213>  Artificial Sequence

<220>
<223>  epitope

<400>  298

Leu Ala Glu Asn Tyr Lys Thr Glu Gly Glu Ser Cys Lys Asn Thr
1               5                   10                  15


<210>  299
<211>  15
<212>  PRT
<213>  Artificial Sequence

<220>
<223>  epitope


<220>
<221>  misc_feature
<222>  (1)..(4)
<223>  Xaa can be any naturally occurring amino acid

<220>
<221>  misc_feature
<222>  (7)..(8)
<223>  Xaa can be any naturally occurring amino acid

<400>  299

Xaa Xaa Xaa Xaa Tyr Lys Xaa Xaa Gly Glu Ser Cys Lys Asn Thr
1               5                   10                  15


<210>  300
<211>  9
<212>  PRT
<213>  Artificial Sequence

<220>
<223>  epitope

<400>  300

Tyr Lys Thr Glu Gly Glu Ser Cys Lys
1               5


<210>  301
<211>  5
<212>  PRT
<213>  Artificial Sequence

<220>
<223>  epitope

<400>  301

Lys Thr Glu Gly Glu
1               5


<210>  302
<211>  9
<212>  PRT
<213>  Artificial Sequence

<220>
<223>  epitope


<220>
<221>  misc_feature
<222>  (3)..(4)
<223>  Xaa can be any naturally occurring amino acid

<400>  302

Tyr Lys Xaa Xaa Gly Glu Ser Cys Lys
1               5


<210>  303
<211>  8
<212>  PRT
<213>  Artificial Sequence

<220>
<223>  epitope

<400>  303

Glu Ser Cys Lys Asn Thr Pro Glu
1               5


<210>  304
<211>  15
<212>  PRT
<213>  Artificial Sequence

<220>
<223>  epitope

<400>  304

Leu Ala Glu Asn Tyr Lys Thr Glu Gly Glu Ser Cys Lys Asn Thr
1               5                   10                  15


<210>  305
<211>  15
<212>  PRT
<213>  Artificial Sequence

<220>
<223>  epitope


<220>
<221>  misc_feature
<222>  (1)..(2)
<223>  Xaa can be any naturally occurring amino acid

<220>
<221>  misc_feature
<222>  (4)..(5)
<223>  Xaa can be any naturally occurring amino acid

<220>
<221>  misc_feature
<222>  (7)..(7)
<223>  Xaa can be any naturally occurring amino acid

<220>
<221>  misc_feature
<222>  (11)..(11)
<223>  Xaa can be any naturally occurring amino acid

<220>
<221>  misc_feature
<222>  (14)..(15)
<223>  Xaa can be any naturally occurring amino acid

<400>  305

Xaa Xaa Glu Xaa Xaa Lys Xaa Glu Gly Glu Xaa Cys Lys Xaa Xaa
1               5                   10                  15

<210>  306
<211>  9
<212>  PRT
<213>  Artificial Sequence

<220>
<223>  epitope

<400>  306

Gly Glu Ser Cys Lys Asn Thr Pro Glu
1               5


<210>  307
<211>  9
<212>  PRT
<213>  Artificial Sequence

<220>
<223>  epitope


<220>
<221>  misc_feature
<222>  (3)..(3)
<223>  Xaa can be any naturally occurring amino acid

<220>
<221>  misc_feature
<222>  (6)..(7)
<223>  Xaa can be any naturally occurring amino acid

<400>  307

Gly Glu Xaa Cys Lys Xaa Xaa Pro Glu
1               5


<210>  308
<211>  15
<212>  PRT
<213>  Artificial Sequence

<220>
<223>  epitope

<400>  308

Leu Ala Glu Asn Tyr Lys Thr Glu Gly Glu Ser Cys Lys Asn Thr
1               5                   10                  15


<210>  309
<211>  15
<212>  PRT
<213>  Artificial Sequence

<220>
<223>  epitope

<220>
<221>  misc_feature
<222>  (1)..(9)
<223>  Xaa can be any naturally occurring amino acid

<220>
<221>  misc_feature
<222>  (12)..(12)
<223>  Xaa can be any naturally occurring amino acid

<220>
<221>  misc_feature
<222>  (15)..(15)
<223>  Xaa can be any naturally occurring amino acid

<400>  309

Xaa Xaa Xaa Xaa Xaa Xaa Xaa Xaa Xaa Glu Ser Xaa Lys Asn Xaa
1               5                   10                  15


<210>  310
<211>  8
<212>  PRT
<213>  Artificial Sequence

<220>
<223>  epitope

<400>  310

Lys Thr Glu Gly Glu Ser Cys Lys
1               5


<210>  311
<211>  9
<212>  PRT
<213>  Artificial Sequence

<220>
<223>  epitope

<400>  311

Glu Ser Cys Lys Asn Thr Pro Glu Lys
1               5


<210>  312
<211>  9
<212>  PRT
<213>  Artificial Sequence

<220>
<223>  epitope


<220>
<221>  misc_feature
<222>  (3)..(3)
<223>  Xaa can be any naturally occurring amino acid

<220>
<221>  misc_feature
<222>  (6)..(6)
<223>  Xaa can be any naturally occurring amino acid

<400>  312

Glu Ser Xaa Lys Asn Xaa Pro Glu Lys
1               5


<210>  313
<211>  15
<212>  PRT
<213>  Artificial Sequence

<220>
<223>  epitope

<400>  313

Leu Ala Glu Asn Tyr Lys Thr Glu Gly Glu Ser Cys Lys Asn Thr
1               5                   10                  15


<210>  314
<211>  15
<212>  PRT
<213>  Artificial Sequence

<220>
<223>  epitope


<220>
<221>  misc_feature
<222>  (1)..(7)
<223>  Xaa can be any naturally occurring amino acid

<220>
<221>  misc_feature
<222>  (12)..(12)
<223>  Xaa can be any naturally occurring amino acid

<220>
<221>  misc_feature
<222>  (14)..(15)
<223>  Xaa can be any naturally occurring amino acid

<400>  314

Xaa Xaa Xaa Xaa Xaa Xaa Xaa Glu Gly Glu Ser Xaa Lys Xaa Xaa
1               5                   10                  15


<210>  315
<211>  10
<212>  PRT
<213>  Artificial Sequence

<220>
<223>  epitope

<400>  315

Asn Tyr Lys Thr Glu Gly Glu Ser Cys Lys
1               5                   10


<210>  316
<211>  10
<212>  PRT
<213>  Artificial Sequence

<220>
<223>  epitope


<220>
<221>  misc_feature
<222>  (1)..(4)
<223>  Xaa can be any naturally occurring amino acid

<220>
<221>  misc_feature
<222>  (9)..(9)
<223>  Xaa can be any naturally occurring amino acid

<400>  316

Xaa Xaa Xaa Xaa Glu Gly Glu Ser Xaa Lys
1               5                   10


<210>  317
<211>  9
<212>  PRT
<213>  Artificial Sequence

<220>
<223>  epitope

<400>  317

Glu Ser Cys Lys Asn Thr Pro Glu Lys
1               5


<210>  318
<211>  9
<212>  PRT
<213>  Artificial Sequence

<220>
<223>  epitope


<220>
<221>  misc_feature
<222>  (3)..(3)
<223>  Xaa can be any naturally occurring amino acid

<220>
<221>  misc_feature
<222>  (5)..(6)
<223>  Xaa can be any naturally occurring amino acid

<400>  318

Glu Ser Xaa Lys Xaa Xaa Pro Glu Lys
1               5

<210>  319
<211>  15
<212>  PRT
<213>  Artificial Sequence

<220>
<223>  epitope

<220>
<221>  misc_feature
<222>  (1)..(4)
<223>  Xaa can be any naturally occurring amino acid

<220>
<221>  misc_feature
<222>  (7)..(7)
<223>  Xaa can be any naturally occurring amino acid

<220>
<221>  misc_feature
<222>  (11)..(11)
<223>  Xaa can be any naturally occurring amino acid

<400>  319

Xaa Xaa Xaa Xaa Tyr Lys Xaa Glu Gly Glu Xaa Cys Lys Asn Thr
1               5                   10                  15

<210>  320
<211>  15
<212>  PRT
<213>  Artificial Sequence

<220>
<223>  epitope

<220>
<221>  misc_feature
<222>  (1)..(8)
<223>  Xaa can be any naturally occurring amino acid

<220>
<221>  misc_feature
<222>  (10)..(12)
<223>  Xaa can be any naturally occurring amino acid

<400>  320

Xaa Xaa Xaa Xaa Xaa Xaa Xaa Xaa Gly Xaa Xaa Xaa Lys Asn Thr
1               5                   10                  15

<210>  321

<211>  9
<212>  PRT
<213>  Artificial Sequence

<220>
<223>  epitope

<400>  321

Tyr Lys Thr Glu Gly Glu Ser Cys Lys
1               5


<210>  322
<211>  9
<212>  PRT
<213>  Artificial Sequence

<220>
<223>  epitope


<220>
<221>  misc_feature
<222>  (3)..(3)
<223>  Xaa can be any naturally occurring amino acid

<220>
<221>  misc_feature
<222>  (7)..(7)
<223>  Xaa can be any naturally occurring amino acid

<400>  322

Tyr Lys Xaa Glu Gly Glu Xaa Cys Lys
1               5


<210>  323
<211>  15
<212>  PRT
<213>  Artificial Sequence

<220>
<223>  epitope


<220>
<221>  misc_feature
<222>  (1)..(2)
<223>  Xaa can be any naturally occurring amino acid

<220>
<221>  misc_feature
<222>  (4)..(4)
<223>  Xaa can be any naturally occurring amino acid

<220>
<221>  misc_feature
<222>  (7)..(8)
<223>  Xaa can be any naturally occurring amino acid

<220>

<221>  misc_feature
<222>  (11)..(11)
<223>  Xaa can be any naturally occurring amino acid

<400>  323

Xaa Xaa Glu Xaa Tyr Lys Xaa Xaa Gly Glu Xaa Cys Lys Asn Thr
1               5                   10                  15


<210>  324
<211>  9
<212>  PRT
<213>  Artificial Sequence

<220>
<223>  epitope

<400>  324

Tyr Lys Thr Glu Gly Glu Ser Cys Lys
1               5


<210>  325
<211>  7
<212>  PRT
<213>  Artificial Sequence

<220>
<223>  epitope

<400>  325

Lys Thr Glu Gly Glu Ser Cys
1               5


<210>  326
<211>  7
<212>  PRT
<213>  Artificial Sequence

<220>
<223>  epitope


<220>
<221>  misc_feature
<222>  (2)..(3)
<223>  Xaa can be any naturally occurring amino acid

<220>
<221>  misc_feature
<222>  (6)..(6)
<223>  Xaa can be any naturally occurring amino acid

<400>  326

Lys Xaa Xaa Gly Glu Xaa Cys
1               5

<210>  327
<211>  15
<212>  PRT
<213>  Artificial Sequence

<220>
<223>  epitope


<220>
<221>  misc_feature
<222>  (1)..(2)
<223>  Xaa can be any naturally occurring amino acid

<220>
<221>  misc_feature
<222>  (7)..(11)
<223>  Xaa can be any naturally occurring amino acid

<400>  327

Xaa Xaa Glu Asn Tyr Lys Xaa Xaa Xaa Xaa Xaa Cys Lys Asn Thr
1               5                   10                  15


<210>  328
<211>  9
<212>  PRT
<213>  Artificial Sequence

<220>
<223>  epitope

<400>  328

Tyr Lys Thr Glu Gly Glu Ser Cys Lys
1               5


<210>  329
<211>  6
<212>  PRT
<213>  Artificial Sequence

<220>
<223>  epitope

<400>  329

Lys Thr Glu Gly Glu Ser
1               5


<210>  330
<211>  15
<212>  PRT
<213>  Artificial Sequence

<220>
<223>  epitope


<220>

<221>  misc_feature
<222>  (1)..(4)
<223>  Xaa can be any naturally occurring amino acid

<220>
<221>  misc_feature
<222>  (6)..(9)
<223>  Xaa can be any naturally occurring amino acid

<400>  330

Xaa Xaa Xaa Xaa Tyr Xaa Xaa Xaa Xaa Glu Ser Cys Lys Asn Thr
1               5                   10                  15


<210>  331
<211>  15
<212>  PRT
<213>  Artificial Sequence

<220>
<223>  epitope


<220>
<221>  misc_feature
<222>  (1)..(10)
<223>  Xaa can be any naturally occurring amino acid

<400>  331

Xaa Xaa Xaa Xaa Xaa Xaa Xaa Xaa Xaa Xaa Ser Cys Lys Asn Thr
1               5                   10                  15


<210>  332
<211>  6
<212>  PRT
<213>  Artificial Sequence

<220>
<223>  epitope

<400>  332

Lys Thr Glu Gly Glu Ser
1               5


<210>  333
<211>  15
<212>  PRT
<213>  Artificial Sequence

<220>
<223>  epitope


<220>
<221>  misc_feature
<222>  (1)..(5)
<223>  Xaa can be any naturally occurring amino acid

<220>
<221>  misc_feature
<222>  (7)..(8)
<223>  Xaa can be any naturally occurring amino acid

<220>
<221>  misc_feature
<222>  (12)..(12)
<223>  Xaa can be any naturally occurring amino acid

<220>
<221>  misc_feature
<222>  (14)..(15)
<223>  Xaa can be any naturally occurring amino acid

<400>  333

Xaa Xaa Xaa Xaa Xaa Lys Xaa Xaa Gly Glu Ser Xaa Lys Xaa Xaa
1               5                   10                  15


<210>  334
<211>  15
<212>  PRT
<213>  Artificial Sequence

<220>
<223>  epitope

<400>  334

Asn His Ala Val Val Ser Arg Lys Asp Lys Ala Thr Cys Val Glu
1               5                   10                  15


<210>  335
<211>  15
<212>  PRT
<213>  Artificial Sequence

<220>
<223>  epitope

<400>  335

Asn His Ala Val Val Ser Arg Lys Asp Lys Ala Thr Cys Val Glu
1               5                   10                  15


<210>  336
<211>  15
<212>  PRT
<213>  Artificial Sequence

<220>
<223>  epitope

<400>  336

Asn His Ala Val Val Ser Arg Lys Asp Lys Ala Thr Cys Val Glu
1               5                   10                  15

<210>  337
<211>  15
<212>  PRT
<213>  Artificial Sequence

<220>
<223>  epitope

<400>  337

Asn His Ala Val Val Ser Arg Lys Asp Lys Ala Thr Cys Val Glu
1               5                   10                  15


<210>  338
<211>  9
<212>  PRT
<213>  Artificial Sequence

<220>
<223>  epitope

<400>  338

Arg Gly Pro Asn His Ala Val Val Ser
1               5


<210>  339
<211>  15
<212>  PRT
<213>  Artificial Sequence

<220>
<223>  epitope

<400>  339

Asn His Ala Val Val Ser Arg Lys Asp Lys Ala Thr Cys Val Glu
1               5                   10                  15


<210>  340
<211>  15
<212>  PRT
<213>  Artificial Sequence

<220>
<223>  epitope


<220>
<221>  misc_feature
<222>  (1)..(4)
<223>  Xaa can be any naturally occurring amino acid

<220>
<221>  misc_feature
<222>  (6)..(7)
<223>  Xaa can be any naturally occurring amino acid

<220>
<221>  misc_feature

<222>  (10)..(10)
<223>  Xaa can be any naturally occurring amino acid

<220>
<221>  misc_feature
<222>  (12)..(15)
<223>  Xaa can be any naturally occurring amino acid

<400>  340

Xaa Xaa Xaa Xaa Val Xaa Xaa Lys Asp Xaa Ala Xaa Xaa Xaa Xaa
1               5                   10                  15


<210>  341
<211>  6
<212>  PRT
<213>  Artificial Sequence

<220>
<223>  epitope

<400>  341

Arg Lys Asp Lys Ala Thr
1               5


<210>  342
<211>  15
<212>  PRT
<213>  Artificial Sequence

<220>
<223>  epitope

<400>  342

Asn His Ala Val Val Ser Arg Lys Asp Lys Ala Thr Cys Val Glu
1               5                   10                  15


<210>  343
<211>  15
<212>  PRT
<213>  Artificial Sequence

<220>
<223>  epitope

<400>  343

Asn His Ala Val Val Ser Arg Lys Asp Lys Ala Thr Cys Val Glu
1               5                   10                  15


<210>  344
<211>  6
<212>  PRT
<213>  Artificial Sequence

<220>
<223>  epitope

<400>  344

Arg Lys Asp Lys Ala Thr
1               5


<210>  345
<211>  15
<212>  PRT
<213>  Artificial Sequence

<220>
<223>  epitope


<220>
<221>  misc_feature
<222>  (1)..(3)
<223>  Xaa can be any naturally occurring amino acid

<220>
<221>  misc_feature
<222>  (12)..(15)
<223>  Xaa can be any naturally occurring amino acid

<400>  345

Xaa Xaa Xaa Val Val Ser Arg Lys Asp Lys Ala Xaa Xaa Xaa Xaa
1               5                   10                  15


<210>  346
<211>  4
<212>  PRT
<213>  Artificial Sequence

<220>
<223>  epitope

<400>  346

Arg Lys Asp Lys
1


<210>  347
<211>  15
<212>  PRT
<213>  Artificial Sequence

<220>
<223>  epitope


<220>
<221>  misc_feature
<222>  (1)..(4)
<223>  Xaa can be any naturally occurring amino acid

<220>
<221>  misc_feature
<222>  (6)..(6)

<223>  Xaa can be any naturally occurring amino acid

<220>
<221>  misc_feature
<222>  (9)..(9)
<223>  Xaa can be any naturally occurring amino acid

<220>
<221>  misc_feature
<222>  (13)..(15)
<223>  Xaa can be any naturally occurring amino acid

<400>  347

Xaa Xaa Xaa Xaa Val Xaa Arg Lys Xaa Lys Ala Thr Xaa Xaa Xaa
1               5                   10                  15


<210>  348
<211>  6
<212>  PRT
<213>  Artificial Sequence

<220>
<223>  epitope

<400>  348

Arg Lys Asp Lys Ala Thr
1               5


<210>  349
<211>  6
<212>  PRT
<213>  Artificial Sequence

<220>
<223>  epitope


<220>
<221>  misc_feature
<222>  (3)..(3)
<223>  Xaa can be any naturally occurring amino acid

<400>  349

Arg Lys Xaa Lys Ala Thr
1               5


<210>  350
<211>  15
<212>  PRT
<213>  Artificial Sequence

<220>
<223>  epitope


<220>
<221>  misc_feature

<222>  (1)..(4)
<223>  Xaa can be any naturally occurring amino acid

<220>
<221>  misc_feature
<222>  (7)..(7)
<223>  Xaa can be any naturally occurring amino acid

<220>
<221>  misc_feature
<222>  (9)..(9)
<223>  Xaa can be any naturally occurring amino acid

<220>
<221>  misc_feature
<222>  (12)..(15)
<223>  Xaa can be any naturally occurring amino acid

<400>  350

Xaa Xaa Xaa Xaa Val Ser Xaa Lys Xaa Lys Ala Xaa Xaa Xaa Xaa
1               5                   10                  15


<210>  351
<211>  15
<212>  PRT
<213>  Artificial Sequence

<220>
<223>  epitope


<220>
<221>  misc_feature
<222>  (1)..(3)
<223>  Xaa can be any naturally occurring amino acid

<220>
<221>  misc_feature
<222>  (13)..(15)
<223>  Xaa can be any naturally occurring amino acid

<400>  351

Xaa Xaa Xaa Val Val Ser Arg Lys Asp Lys Ala Thr Xaa Xaa Xaa
1               5                   10                  15


<210>  352
<211>  15
<212>  PRT
<213>  Artificial Sequence

<220>
<223>  epitope


<220>
<221>  misc_feature
<222>  (1)..(4)
<223>  Xaa can be any naturally occurring amino acid

<220>
<221>  misc_feature
<222>  (11)..(15)
<223>  Xaa can be any naturally occurring amino acid

<400>  352

Xaa Xaa Xaa Xaa Val Ser Arg Lys Asp Lys Xaa Xaa Xaa Xaa Xaa
1               5                   10                  15


<210>  353
<211>  15
<212>  PRT
<213>  Artificial Sequence

<220>
<223>  epitope

<400>  353

Glu Thr Glu Leu Gly Ser Asn Glu His Ser Lys Thr Gln Cys Glu
1               5                   10                  15


<210>  354
<211>  15
<212>  PRT
<213>  Artificial Sequence

<220>
<223>  epitope

<400>  354

Glu Thr Glu Leu Gly Ser Asn Glu His Ser Lys Thr Gln Cys Glu
1               5                   10                  15


<210>  355
<211>  5
<212>  PRT
<213>  Artificial Sequence

<220>
<223>  epitope

<400>  355

Ser Lys Thr Gln Cys
1               5


<210>  356
<211>  4
<212>  PRT
<213>  Artificial Sequence

<220>
<223>  epitope

<400>  356

Ser Lys Thr Gln
1

<210> 357
<211> 15
<212> PRT
<213> Artificial Sequence

<220>
<223> epitope

<400> 357

Glu Thr Glu Leu Gly Ser Asn Glu His Ser Lys Thr Gln Cys Glu
1               5                   10                  15

<210> 358
<211> 5
<212> PRT
<213> Artificial Sequence

<220>
<223> epitope

<400> 358

Ser Lys Thr Gln Cys
1               5

<210> 359
<211> 15
<212> PRT
<213> Artificial Sequence

<220>
<223> epitope

<400> 359

Glu Thr Glu Leu Gly Ser Asn Glu His Ser Lys Thr Gln Cys Glu
1               5                   10                  15

<210> 360
<211> 6
<212> PRT
<213> Artificial Sequence

<220>
<223> epitope

<400> 360

His Ser Lys Thr Gln Cys
1               5

<210> 361
<211> 5
<212> PRT

<213>  Artificial Sequence

<220>
<223>  epitope

<400>  361

Ser Lys Thr Gln Cys
1               5


<210>  362
<211>  15
<212>  PRT
<213>  Artificial Sequence

<220>
<223>  epitope

<400>  362

Glu Thr Glu Leu Gly Ser Asn Glu His Ser Lys Thr Gln Cys Glu
1               5                   10                  15


<210>  363
<211>  9
<212>  PRT
<213>  Artificial Sequence

<220>
<223>  epitope

<400>  363

Phe Ala Pro Glu Thr Glu Leu Gly Ser
1               5


<210>  364
<211>  5
<212>  PRT
<213>  Artificial Sequence

<220>
<223>  epitope

<400>  364

Ser Lys Thr Gln Cys
1               5


<210>  365
<211>  4
<212>  PRT
<213>  Artificial Sequence

<220>
<223>  epitope

<400>  365

Ser Lys Thr Gln
1

<210> 366
<211> 15
<212> PRT
<213> Artificial Sequence

<220>
<223> epitope

<400> 366

Glu Thr Glu Leu Gly Ser Asn Glu His Ser Lys Thr Gln Cys Glu
1               5                   10                  15

<210> 367
<211> 15
<212> PRT
<213> Artificial Sequence

<220>
<223> epitope

<400> 367

Glu Thr Glu Leu Gly Ser Asn Glu His Ser Lys Thr Gln Cys Glu
1               5                   10                  15

<210> 368
<211> 15
<212> PRT
<213> Artificial Sequence

<220>
<223> epitope

<400> 368

Glu Thr Glu Leu Gly Ser Asn Glu His Ser Lys Thr Gln Cys Glu
1               5                   10                  15

<210> 369
<211> 7
<212> PRT
<213> Artificial Sequence

<220>
<223> epitope

<400> 369

Glu His Ser Lys Thr Gln Cys
1               5

<210> 370
<211> 4
<212> PRT

<213>  Artificial Sequence

<220>
<223>  epitope

<400>  370

Ser Lys Thr Gln
1


<210>  371
<211>  4
<212>  PRT
<213>  Artificial Sequence

<220>
<223>  epitope

<400>  371

Ser Lys Thr Gln
1


<210>  372
<211>  15
<212>  PRT
<213>  Artificial Sequence

<220>
<223>  epitope


<220>
<221>  misc_feature
<222>  (1)..(7)
<223>  Xaa can be any naturally occurring amino acid

<220>
<221>  misc_feature
<222>  (9)..(10)
<223>  Xaa can be any naturally occurring amino acid

<220>
<221>  misc_feature
<222>  (13)..(14)
<223>  Xaa can be any naturally occurring amino acid

<400>  372

Xaa Xaa Xaa Xaa Xaa Xaa Xaa Glu Xaa Xaa Lys Thr Xaa Xaa Glu
1               5                   10                  15


<210>  373
<211>  4
<212>  PRT
<213>  Artificial Sequence

<220>
<223>  epitope

<400>  373

Ser Lys Thr Gln
1



<210>  374
<211>  5
<212>  PRT
<213>  Artificial Sequence

<220>
<223>  epitope

<400>  374

Ser Lys Thr Gln Cys
1               5



<210>  375
<211>  4
<212>  PRT
<213>  Artificial Sequence

<220>
<223>  epitope

<400>  375

Ser Lys Thr Gln
1



<210>  376
<211>  4
<212>  PRT
<213>  Artificial Sequence

<220>
<223>  epitope

<400>  376

Ser Lys Thr Gln
1



<210>  377
<211>  15
<212>  PRT
<213>  Artificial Sequence

<220>
<223>  epitope

<400>  377

Leu His Thr Leu Phe Phe Asn Thr Trp Arg Ile Ile Lys Asp Gly
1               5                   10                  15



<210>  378

<211>  15
<212>  PRT
<213>  Artificial Sequence

<220>
<223>  epitope

<400>  378

Leu His Thr Leu Phe Phe Asn Thr Trp Arg Ile Ile Lys Asp Gly
1               5                   10                  15


<210>  379
<211>  15
<212>  PRT
<213>  Artificial Sequence

<220>
<223>  epitope


<220>
<221>  misc_feature
<222>  (1)..(11)
<223>  Xaa can be any naturally occurring amino acid

<400>  379

Xaa Xaa Xaa Xaa Xaa Xaa Xaa Xaa Xaa Xaa Xaa Ile Lys Asp Gly
1               5                   10                  15


<210>  380
<211>  10
<212>  PRT
<213>  Artificial Sequence

<220>
<223>  epitope

<400>  380

Leu Pro Leu His Thr Leu Phe Phe Asn Thr
1               5                   10


<210>  381
<211>  15
<212>  PRT
<213>  Artificial Sequence

<220>
<223>  epitope

<400>  381

Leu His Thr Leu Phe Phe Asn Thr Trp Arg Ile Ile Lys Asp Gly
1               5                   10                  15


<210>  382
<211>  15

<212>  PRT
<213>  Artificial Sequence

<220>
<223>  epitope

<400>  382

Leu His Thr Leu Phe Phe Asn Thr Trp Arg Ile Ile Lys Asp Gly
1               5                   10                  15


<210>  383
<211>  15
<212>  PRT
<213>  Artificial Sequence

<220>
<223>  epitope

<400>  383

Leu His Thr Leu Phe Phe Asn Thr Trp Arg Ile Ile Lys Asp Gly
1               5                   10                  15


<210>  384
<211>  4
<212>  PRT
<213>  Artificial Sequence

<220>
<223>  epitope

<400>  384

Arg Ile Ile Lys
1


<210>  385
<211>  15
<212>  PRT
<213>  Artificial Sequence

<220>
<223>  epitope

<400>  385

Ser Phe Glu Lys Tyr Trp Cys Lys Trp Ser Asn Arg Gly Cys Ser
1               5                   10                  15


<210>  386
<211>  15
<212>  PRT
<213>  Artificial Sequence

<220>
<223>  epitope

<400>  386

Ser Phe Glu Lys Tyr Trp Cys Lys Trp Ser Asn Arg Gly Cys Ser
1               5                   10                  15


<210>  387
<211>  15
<212>  PRT
<213>  Artificial Sequence

<220>
<223>  epitope


<220>
<221>  misc_feature
<222>  (1)..(1)
<223>  Xaa can be any naturally occurring amino acid

<400>  387

Xaa Phe Glu Lys Tyr Trp Cys Lys Trp Ser Asn Arg Gly Cys Ser
1               5                   10                  15


<210>  388
<211>  15
<212>  PRT
<213>  Artificial Sequence

<220>
<223>  epitope


<220>
<221>  misc_feature
<222>  (1)..(2)
<223>  Xaa can be any naturally occurring amino acid

<220>
<221>  misc_feature
<222>  (12)..(15)
<223>  Xaa can be any naturally occurring amino acid

<400>  388

Xaa Xaa Glu Lys Tyr Trp Cys Lys Trp Ser Asn Xaa Xaa Xaa Xaa
1               5                   10                  15


<210>  389
<211>  5
<212>  PRT
<213>  Artificial Sequence

<220>
<223>  epitope

<400>  389

Phe Glu Lys Tyr Trp
1               5

<210>  390
<211>  5
<212>  PRT
<213>  Artificial Sequence

<220>
<223>  epitope


<220>
<221>  misc_feature
<222>  (1)..(1)
<223>  Xaa can be any naturally occurring amino acid

<400>  390

Xaa Glu Lys Tyr Trp
1               5


<210>  391
<211>  10
<212>  PRT
<213>  Artificial Sequence

<220>
<223>  epitope

<400>  391

Tyr Trp Cys Lys Trp Ser Asn Arg Gly Cys
1               5                   10


<210>  392
<211>  10
<212>  PRT
<213>  Artificial Sequence

<220>
<223>  epitope


<220>
<221>  misc_feature
<222>  (8)..(10)
<223>  Xaa can be any naturally occurring amino acid

<400>  392

Tyr Trp Cys Lys Trp Ser Asn Xaa Xaa Xaa
1               5                   10


<210>  393
<211>  15
<212>  PRT
<213>  Artificial Sequence

<220>
<223>  epitope

<400>  393

Ser Phe Glu Lys Tyr Trp Cys Lys Trp Ser Asn Arg Gly Cys Ser
1               5                   10                  15


<210>  394
<211>  15
<212>  PRT
<213>  Artificial Sequence

<220>
<223>  epitope


<220>
<221>  misc_feature
<222>  (1)..(1)
<223>  Xaa can be any naturally occurring amino acid

<400>  394

Xaa Phe Glu Lys Tyr Trp Cys Lys Trp Ser Asn Arg Gly Cys Ser
1               5                   10                  15


<210>  395
<211>  15
<212>  PRT
<213>  Artificial Sequence

<220>
<223>  epitope


<220>
<221>  misc_feature
<222>  (1)..(2)
<223>  Xaa can be any naturally occurring amino acid

<220>
<221>  misc_feature
<222>  (15)..(15)
<223>  Xaa can be any naturally occurring amino acid

<400>  395

Xaa Xaa Glu Lys Tyr Trp Cys Lys Trp Ser Asn Arg Gly Cys Xaa
1               5                   10                  15


<210>  396
<211>  15
<212>  PRT
<213>  Artificial Sequence

<220>
<223>  epitope

<400>  396

Ser Phe Glu Lys Tyr Trp Cys Lys Trp Ser Asn Arg Gly Cys Ser
1               5                   10                  15

<210>  397
<211>  15
<212>  PRT
<213>  Artificial Sequence

<220>
<223>  epitope



<220>
<221>  misc_feature
<222>  (1)..(1)
<223>  Xaa can be any naturally occurring amino acid

<220>
<221>  misc_feature
<222>  (6)..(6)
<223>  Xaa can be any naturally occurring amino acid

<220>
<221>  misc_feature
<222>  (12)..(15)
<223>  Xaa can be any naturally occurring amino acid

<400>  397

Xaa Phe Glu Lys Tyr Xaa Cys Lys Trp Ser Asn Xaa Xaa Xaa Xaa
1               5                   10                  15


<210>  398
<211>  15
<212>  PRT
<213>  Artificial Sequence

<220>
<223>  epitope



<220>
<221>  misc_feature
<222>  (1)..(2)
<223>  Xaa can be any naturally occurring amino acid

<220>
<221>  misc_feature
<222>  (6)..(6)
<223>  Xaa can be any naturally occurring amino acid

<220>
<221>  misc_feature
<222>  (12)..(15)
<223>  Xaa can be any naturally occurring amino acid

<400>  398

Xaa Xaa Glu Lys Tyr Xaa Cys Lys Trp Ser Asn Xaa Xaa Xaa Xaa
1               5                   10                  15


<210>  399

<211>  15
<212>  PRT
<213>  Artificial Sequence

<220>
<223>  epitope


<220>
<221>  misc_feature
<222>  (1)..(2)
<223>  Xaa can be any naturally occurring amino acid

<220>
<221>  misc_feature
<222>  (4)..(8)
<223>  Xaa can be any naturally occurring amino acid

<220>
<221>  misc_feature
<222>  (12)..(15)
<223>  Xaa can be any naturally occurring amino acid

<400>  399

Xaa Xaa Glu Xaa Xaa Xaa Xaa Xaa Trp Ser Asn Xaa Xaa Xaa Xaa
1               5                   10                  15


<210>  400
<211>  15
<212>  PRT
<213>  Artificial Sequence

<220>
<223>  epitope

<400>  400

Ser Phe Glu Lys Tyr Trp Cys Lys Trp Ser Asn Arg Gly Cys Ser
1               5                   10                  15


<210>  401
<211>  15
<212>  PRT
<213>  Artificial Sequence

<220>
<223>  epitope

<400>  401

Ser Phe Glu Lys Tyr Trp Cys Lys Trp Ser Asn Arg Gly Cys Ser
1               5                   10                  15


<210>  402
<211>  15
<212>  PRT
<213>  Artificial Sequence

<220>

<223>  epitope

<220>
<221>  misc_feature
<222>  (1)..(1)
<223>  Xaa can be any naturally occurring amino acid

<400>  402

Xaa Phe Glu Lys Tyr Trp Cys Lys Trp Ser Asn Arg Gly Cys Ser
1               5                   10                  15

<210>  403
<211>  15
<212>  PRT
<213>  Artificial Sequence

<220>
<223>  epitope

<220>
<221>  misc_feature
<222>  (1)..(2)
<223>  Xaa can be any naturally occurring amino acid

<220>
<221>  misc_feature
<222>  (6)..(6)
<223>  Xaa can be any naturally occurring amino acid

<220>
<221>  misc_feature
<222>  (13)..(15)
<223>  Xaa can be any naturally occurring amino acid

<400>  403

Xaa Xaa Glu Lys Tyr Xaa Cys Lys Trp Ser Asn Arg Xaa Xaa Xaa
1               5                   10                  15

<210>  404
<211>  15
<212>  PRT
<213>  Artificial Sequence

<220>
<223>  epitope

<400>  404

Ser Phe Glu Lys Tyr Trp Cys Lys Trp Ser Asn Arg Gly Cys Ser
1               5                   10                  15

<210>  405
<211>  15
<212>  PRT
<213>  Artificial Sequence

<220>
<223>  epitope


<220>
<221>  misc_feature
<222>  (1)..(3)
<223>  Xaa can be any naturally occurring amino acid

<220>
<221>  misc_feature
<222>  (8)..(9)
<223>  Xaa can be any naturally occurring amino acid

<220>
<221>  misc_feature
<222>  (11)..(11)
<223>  Xaa can be any naturally occurring amino acid

<220>
<221>  misc_feature
<222>  (14)..(15)
<223>  Xaa can be any naturally occurring amino acid

<400>  405

Xaa Xaa Xaa Lys Tyr Trp Cys Xaa Xaa Ser Xaa Arg Gly Xaa Xaa
1               5                   10                  15


<210>  406
<211>  15
<212>  PRT
<213>  Artificial Sequence

<220>
<223>  epitope

<400>  406

Ser Phe Glu Lys Tyr Trp Cys Lys Trp Ser Asn Arg Gly Cys Ser
1               5                   10                  15


<210>  407
<211>  15
<212>  PRT
<213>  Artificial Sequence

<220>
<223>  epitope


<220>
<221>  misc_feature
<222>  (1)..(3)
<223>  Xaa can be any naturally occurring amino acid

<220>
<221>  misc_feature
<222>  (13)..(15)
<223>  Xaa can be any naturally occurring amino acid

<400>  407

Xaa Xaa Xaa Lys Tyr Trp Cys Lys Trp Ser Asn Arg Xaa Xaa Xaa
1               5                   10                  15


<210>  408
<211>  15
<212>  PRT
<213>  Artificial Sequence

<220>
<223>  epitope


<220>
<221>  misc_feature
<222>  (1)..(1)
<223>  Xaa can be any naturally occurring amino acid

<220>
<221>  misc_feature
<222>  (3)..(3)
<223>  Xaa can be any naturally occurring amino acid

<220>
<221>  misc_feature
<222>  (13)..(15)
<223>  Xaa can be any naturally occurring amino acid

<400>  408

Xaa Phe Xaa Lys Tyr Trp Cys Lys Trp Ser Asn Arg Xaa Xaa Xaa
1               5                   10                  15


<210>  409
<211>  15
<212>  PRT
<213>  Artificial Sequence

<220>
<223>  epitope


<220>
<221>  misc_feature
<222>  (1)..(3)
<223>  Xaa can be any naturally occurring amino acid

<220>
<221>  misc_feature
<222>  (6)..(7)
<223>  Xaa can be any naturally occurring amino acid

<220>
<221>  misc_feature
<222>  (12)..(15)
<223>  Xaa can be any naturally occurring amino acid

<400>  409

Xaa Xaa Xaa Lys Tyr Xaa Xaa Lys Trp Ser Asn Xaa Xaa Xaa Xaa

1               5                   10                  15


<210>  410
<211>  15
<212>  PRT
<213>  Artificial Sequence

<220>
<223>  epitope


<220>
<221>  misc_feature
<222>  (1)..(1)
<223>  Xaa can be any naturally occurring amino acid

<220>
<221>  misc_feature
<222>  (3)..(3)
<223>  Xaa can be any naturally occurring amino acid

<220>
<221>  misc_feature
<222>  (14)..(15)
<223>  Xaa can be any naturally occurring amino acid

<400>  410

Xaa Phe Xaa Lys Tyr Trp Cys Lys Trp Ser Asn Arg Gly Xaa Xaa
1               5                   10                  15


<210>  411
<211>  15
<212>  PRT
<213>  Artificial Sequence

<220>
<223>  epitope


<220>
<221>  misc_feature
<222>  (1)..(10)
<223>  Xaa can be any naturally occurring amino acid

<400>  411

Xaa Xaa Xaa Xaa Xaa Xaa Xaa Xaa Xaa Xaa Asn Arg Gly Cys Ser
1               5                   10                  15


<210>  412
<211>  15
<212>  PRT
<213>  Artificial Sequence

<220>
<223>  epitope

<400>  412

Asn Leu Asp Thr Val Thr Lys Glu Asp Glu Gly Trp Tyr Trp Cys
1               5                   10                  15


<210>  413
<211>  15
<212>  PRT
<213>  Artificial Sequence

<220>
<223>  epitope

<400>  413

Asn Leu Asp Thr Val Thr Lys Glu Asp Glu Gly Trp Tyr Trp Cys
1               5                   10                  15


<210>  414
<211>  8
<212>  PRT
<213>  Artificial Sequence

<220>
<223>  epitope

<400>  414

Ser Leu Asn Leu Asp Thr Val Thr
1               5


<210>  415
<211>  7
<212>  PRT
<213>  Artificial Sequence

<220>
<223>  epitope

<400>  415

Leu Asn Leu Asp Thr Val Thr
1               5


<210>  416
<211>  5
<212>  PRT
<213>  Artificial Sequence

<220>
<223>  epitope

<400>  416

Leu Asp Thr Val Thr
1               5


<210>  417
<211>  10
<212>  PRT

<213>  Artificial Sequence

<220>
<223>  epitope

<400>  417

Thr Lys Glu Asp Glu Gly Trp Tyr Trp Cys
1               5                   10


<210>  418
<211>  4
<212>  PRT
<213>  Artificial Sequence

<220>
<223>  epitope

<400>  418

Glu Gly Trp Tyr
1


<210>  419
<211>  15
<212>  PRT
<213>  Artificial Sequence

<220>
<223>  epitope

<400>  419

Asn Leu Asp Thr Val Thr Lys Glu Asp Glu Gly Trp Tyr Trp Cys
1               5                   10                  15


<210>  420
<211>  10
<212>  PRT
<213>  Artificial Sequence

<220>
<223>  epitope

<400>  420

Val Val Ser Leu Asn Leu Asp Thr Val Thr
1               5                   10


<210>  421
<211>  8
<212>  PRT
<213>  Artificial Sequence

<220>
<223>  epitope

<400>  421

Ser Leu Asn Leu Asp Thr Val Thr
1               5

<210>  422
<211>  10
<212>  PRT
<213>  Artificial Sequence

<220>
<223>  epitope

<400>  422

Val Thr Lys Glu Asp Glu Gly Trp Tyr Trp
1               5                   10

<210>  423
<211>  7
<212>  PRT
<213>  Artificial Sequence

<220>
<223>  epitope

<400>  423

Lys Glu Asp Glu Gly Trp Tyr
1               5

<210>  424
<211>  10
<212>  PRT
<213>  Artificial Sequence

<220>
<223>  epitope

<400>  424

Glu Gly Trp Tyr Trp Cys Gly Val Lys Glu
1               5                   10

<210>  425
<211>  15
<212>  PRT
<213>  Artificial Sequence

<220>
<223>  epitope

<400>  425

Asn Leu Asp Thr Val Thr Lys Glu Asp Glu Gly Trp Tyr Trp Cys
1               5                   10                  15

<210>  426
<211>  10
<212>  PRT

<213>  Artificial Sequence

<220>
<223>  epitope

<400>  426

Val Val Ser Leu Asn Leu Asp Thr Val Thr
1               5                   10


<210>  427
<211>  9
<212>  PRT
<213>  Artificial Sequence

<220>
<223>  epitope

<400>  427

Val Ser Leu Asn Leu Asp Thr Val Thr
1               5


<210>  428
<211>  10
<212>  PRT
<213>  Artificial Sequence

<220>
<223>  epitope

<400>  428

Thr Lys Glu Asp Glu Gly Trp Tyr Trp Cys
1               5                   10


<210>  429
<211>  9
<212>  PRT
<213>  Artificial Sequence

<220>
<223>  epitope

<400>  429

Lys Glu Asp Glu Gly Trp Tyr Trp Cys
1               5


<210>  430
<211>  15
<212>  PRT
<213>  Artificial Sequence

<220>
<223>  epitope

<400>  430

Asn Leu Asp Thr Val Thr Lys Glu Asp Glu Gly Trp Tyr Trp Cys
1               5                   10                  15


<210>  431
<211>  15
<212>  PRT
<213>  Artificial Sequence

<220>
<223>  epitope


<220>
<221>  misc_feature
<222>  (1)..(3)
<223>  Xaa can be any naturally occurring amino acid

<220>
<221>  misc_feature
<222>  (6)..(6)
<223>  Xaa can be any naturally occurring amino acid

<220>
<221>  misc_feature
<222>  (9)..(9)
<223>  Xaa can be any naturally occurring amino acid

<220>
<221>  misc_feature
<222>  (11)..(15)
<223>  Xaa can be any naturally occurring amino acid

<400>  431

Xaa Xaa Xaa Thr Val Xaa Lys Glu Xaa Glu Xaa Xaa Xaa Xaa Xaa
1               5                   10                  15


<210>  432
<211>  9
<212>  PRT
<213>  Artificial Sequence

<220>
<223>  epitope

<400>  432

Val Ser Leu Asn Leu Asp Thr Val Thr
1               5


<210>  433
<211>  9
<212>  PRT
<213>  Artificial Sequence

<220>
<223>  epitope


<220>

<221>  misc_feature
<222>  (4)..(6)
<223>  Xaa can be any naturally occurring amino acid

<220>
<221>  misc_feature
<222>  (9)..(9)
<223>  Xaa can be any naturally occurring amino acid

<400>  433

Val Ser Leu Xaa Xaa Xaa Thr Val Xaa
1               5


<210>  434
<211>  10
<212>  PRT
<213>  Artificial Sequence

<220>
<223>  epitope

<400>  434

Thr Lys Glu Asp Glu Gly Trp Tyr Trp Cys
1               5                   10


<210>  435
<211>  9
<212>  PRT
<213>  Artificial Sequence

<220>
<223>  epitope

<400>  435

Thr Lys Glu Asp Glu Gly Trp Tyr Trp
1               5


<210>  436
<211>  8
<212>  PRT
<213>  Artificial Sequence

<220>
<223>  epitope

<400>  436

Lys Glu Asp Glu Gly Trp Tyr Trp
1               5


<210>  437
<211>  10
<212>  PRT
<213>  Artificial Sequence

<220>

<223>  epitope

<400>  437

Leu Asp Thr Val Thr Lys Glu Asp Glu Gly
1               5                   10


<210>  438
<211>  7
<212>  PRT
<213>  Artificial Sequence

<220>
<223>  epitope

<400>  438

Lys Glu Asp Glu Gly Trp Tyr
1               5


<210>  439
<211>  15
<212>  PRT
<213>  Artificial Sequence

<220>
<223>  epitope


<220>
<221>  misc_feature
<222>  (1)..(4)
<223>  Xaa can be any naturally occurring amino acid

<220>
<221>  misc_feature
<222>  (6)..(6)
<223>  Xaa can be any naturally occurring amino acid

<220>
<221>  misc_feature
<222>  (11)..(15)
<223>  Xaa can be any naturally occurring amino acid

<400>  439

Xaa Xaa Xaa Xaa Val Xaa Lys Glu Asp Glu Xaa Xaa Xaa Xaa Xaa
1               5                   10                  15


<210>  440
<211>  15
<212>  PRT
<213>  Artificial Sequence

<220>
<223>  epitope


<220>
<221>  misc_feature

<222>  (1)..(6)
<223>  Xaa can be any naturally occurring amino acid

<220>
<221>  misc_feature
<222>  (11)..(15)
<223>  Xaa can be any naturally occurring amino acid

<400>  440

Xaa Xaa Xaa Xaa Xaa Xaa Lys Glu Asp Glu Xaa Xaa Xaa Xaa Xaa
1               5                   10                  15


<210>  441
<211>  10
<212>  PRT
<213>  Artificial Sequence

<220>
<223>  epitope

<400>  441

Val Thr Lys Glu Asp Glu Gly Trp Tyr Trp
1               5                   10


<210>  442
<211>  9
<212>  PRT
<213>  Artificial Sequence

<220>
<223>  epitope

<400>  442

Thr Lys Glu Asp Glu Gly Trp Tyr Trp
1               5


<210>  443
<211>  9
<212>  PRT
<213>  Artificial Sequence

<220>
<223>  epitope


<220>
<221>  misc_feature
<222>  (1)..(1)
<223>  Xaa can be any naturally occurring amino acid

<220>
<221>  misc_feature
<222>  (6)..(9)
<223>  Xaa can be any naturally occurring amino acid

<400>  443

Xaa Lys Glu Asp Glu Xaa Xaa Xaa Xaa
1               5

<210>  444
<211>  6
<212>  PRT
<213>  Artificial Sequence

<220>
<223>  epitope

<400>  444

Asn Leu Asp Thr Val Thr
1               5

<210>  445
<211>  9
<212>  PRT
<213>  Artificial Sequence

<220>
<223>  epitope

<400>  445

Thr Lys Glu Asp Glu Gly Trp Tyr Trp
1               5

<210>  446
<211>  7
<212>  PRT
<213>  Artificial Sequence

<220>
<223>  epitope

<400>  446

Lys Glu Asp Glu Gly Trp Tyr
1               5

<210>  447
<211>  15
<212>  PRT
<213>  Artificial Sequence

<220>
<223>  epitope

<220>
<221>  misc_feature
<222>  (1)..(3)
<223>  Xaa can be any naturally occurring amino acid

<220>
<221>  misc_feature
<222>  (11)..(15)

<223>  Xaa can be any naturally occurring amino acid

<400>  447

Xaa Xaa Xaa Thr Val Thr Lys Glu Asp Glu Xaa Xaa Xaa Xaa Xaa
1               5                   10                  15


<210>  448
<211>  9
<212>  PRT
<213>  Artificial Sequence

<220>
<223>  epitope

<400>  448

Lys Glu Asp Glu Gly Trp Tyr Trp Cys
1               5


<210>  449
<211>  5
<212>  PRT
<213>  Artificial Sequence

<220>
<223>  epitope

<400>  449

Lys Glu Asp Glu Gly
1               5


<210>  450
<211>  5
<212>  PRT
<213>  Artificial Sequence

<220>
<223>  epitope


<220>
<221>  misc_feature
<222>  (5)..(5)
<223>  Xaa can be any naturally occurring amino acid

<400>  450

Lys Glu Asp Glu Xaa
1               5


<210>  451
<211>  15
<212>  PRT
<213>  Artificial Sequence

<220>
<223>  epitope

<400>  451

Val Lys Asn Gly Gly Lys Glu Glu Lys His His Arg Pro Gly Gln
1               5                   10                  15


<210>  452
<211>  15
<212>  PRT
<213>  Artificial Sequence

<220>
<223>  epitope

<400>  452

Val Lys Asn Gly Gly Lys Glu Glu Lys His His Arg Pro Gly Gln
1               5                   10                  15


<210>  453
<211>  7
<212>  PRT
<213>  Artificial Sequence

<220>
<223>  epitope

<400>  453

Lys Asn Gly Gly Lys Glu Glu
1               5


<210>  454
<211>  4
<212>  PRT
<213>  Artificial Sequence

<220>
<223>  epitope

<400>  454

Gly Gly Lys Glu
1


<210>  455
<211>  15
<212>  PRT
<213>  Artificial Sequence

<220>
<223>  epitope

<400>  455

Val Lys Asn Gly Gly Lys Glu Glu Lys His His Arg Pro Gly Gln
1               5                   10                  15

<210>  456
<211>  15
<212>  PRT
<213>  Artificial Sequence

<220>
<223>  epitope


<220>
<221>  misc_feature
<222>  (1)..(2)
<223>  Xaa can be any naturally occurring amino acid

<220>
<221>  misc_feature
<222>  (4)..(6)
<223>  Xaa can be any naturally occurring amino acid

<220>
<221>  misc_feature
<222>  (9)..(12)
<223>  Xaa can be any naturally occurring amino acid

<400>  456

Xaa Xaa Asn Xaa Xaa Xaa Glu Glu Xaa Xaa Xaa Xaa Pro Gly Gln
1               5                   10                  15


<210>  457
<211>  8
<212>  PRT
<213>  Artificial Sequence

<220>
<223>  epitope

<400>  457

Lys Asn Gly Gly Lys Glu Glu Lys
1               5


<210>  458
<211>  6
<212>  PRT
<213>  Artificial Sequence

<220>
<223>  epitope

<400>  458

Lys Asn Gly Gly Lys Glu
1               5


<210>  459
<211>  10
<212>  PRT
<213>  Artificial Sequence

<220>
<223>  epitope

<400>  459

Glu Lys His His Arg Pro Gly Gln Gln Ile
1               5                   10


<210>  460
<211>  10
<212>  PRT
<213>  Artificial Sequence

<220>
<223>  epitope


<220>
<221>  misc_feature
<222>  (2)..(5)
<223>  Xaa can be any naturally occurring amino acid

<400>  460

Glu Xaa Xaa Xaa Xaa Pro Gly Gln Gln Ile
1               5                   10


<210>  461
<211>  15
<212>  PRT
<213>  Artificial Sequence

<220>
<223>  epitope

<400>  461

Val Lys Asn Gly Gly Lys Glu Glu Lys His His Arg Pro Gly Gln
1               5                   10                  15


<210>  462
<211>  10
<212>  PRT
<213>  Artificial Sequence

<220>
<223>  epitope

<400>  462

Thr Leu Val Val Lys Asn Gly Gly Lys Glu
1               5                   10


<210>  463
<211>  7
<212>  PRT
<213>  Artificial Sequence

<220>

<223>  epitope

<400>  463

Asn Gly Gly Lys Glu Glu Lys
1               5


<210>  464
<211>  6
<212>  PRT
<213>  Artificial Sequence

<220>
<223>  epitope

<400>  464

Gly Gly Lys Glu Glu Lys
1               5


<210>  465
<211>  10
<212>  PRT
<213>  Artificial Sequence

<220>
<223>  epitope

<400>  465

Glu Lys His His Arg Pro Gly Gln Gln Ile
1               5                   10


<210>  466
<211>  15
<212>  PRT
<213>  Artificial Sequence

<220>
<223>  epitope

<400>  466

Val Lys Asn Gly Gly Lys Glu Glu Lys His His Arg Pro Gly Gln
1               5                   10                  15


<210>  467
<211>  15
<212>  PRT
<213>  Artificial Sequence

<220>
<223>  epitope


<220>
<221>  misc_feature
<222>  (1)..(3)
<223>  Xaa can be any naturally occurring amino acid

<220>
<221>  misc_feature
<222>  (5)..(5)
<223>  Xaa can be any naturally occurring amino acid

<220>
<221>  misc_feature
<222>  (9)..(13)
<223>  Xaa can be any naturally occurring amino acid

<400>  467

Xaa Xaa Xaa Gly Xaa Lys Glu Glu Xaa Xaa Xaa Xaa Xaa Gly Gln
1               5                   10                  15


<210>  468
<211>  10
<212>  PRT
<213>  Artificial Sequence

<220>
<223>  epitope

<400>  468

Lys Asn Gly Gly Lys Glu Glu Lys His His
1               5                   10


<210>  469
<211>  9
<212>  PRT
<213>  Artificial Sequence

<220>
<223>  epitope

<400>  469

Lys Asn Gly Gly Lys Glu Glu Lys His
1               5


<210>  470
<211>  9
<212>  PRT
<213>  Artificial Sequence

<220>
<223>  epitope

<400>  470

Lys Asn Gly Gly Lys Glu Glu Lys His
1               5


<210>  471
<211>  7
<212>  PRT
<213>  Artificial Sequence

<220>
<223>  epitope

<400>  471

Asn Gly Gly Lys Glu Glu Lys
1               5


<210>  472
<211>  7
<212>  PRT
<213>  Artificial Sequence

<220>
<223>  epitope


<220>
<221>  misc_feature
<222>  (1)..(1)
<223>  Xaa can be any naturally occurring amino acid

<220>
<221>  misc_feature
<222>  (3)..(3)
<223>  Xaa can be any naturally occurring amino acid

<220>
<221>  misc_feature
<222>  (7)..(7)
<223>  Xaa can be any naturally occurring amino acid

<400>  472

Xaa Gly Xaa Lys Glu Glu Xaa
1               5


<210>  473
<211>  10
<212>  PRT
<213>  Artificial Sequence

<220>
<223>  epitope

<400>  473

Lys Glu Glu Lys His His Arg Pro Gly Gln
1               5                   10


<210>  474
<211>  10
<212>  PRT
<213>  Artificial Sequence

<220>
<223>  epitope

<220>
<221>  misc_feature
<222>  (4)..(8)
<223>  Xaa can be any naturally occurring amino acid

<400>  474

Lys Glu Glu Xaa Xaa Xaa Xaa Xaa Gly Gln
1               5                   10


<210>  475
<211>  10
<212>  PRT
<213>  Artificial Sequence

<220>
<223>  epitope

<400>  475

Glu Lys His His Arg Pro Gly Gln Gln Ile
1               5                   10


<210>  476
<211>  10
<212>  PRT
<213>  Artificial Sequence

<220>
<223>  epitope


<220>
<221>  misc_feature
<222>  (2)..(6)
<223>  Xaa can be any naturally occurring amino acid

<400>  476

Glu Xaa Xaa Xaa Xaa Xaa Gly Gln Gln Ile
1               5                   10


<210>  477
<211>  15
<212>  PRT
<213>  Artificial Sequence

<220>
<223>  epitope

<400>  477

Val Lys Asn Gly Gly Lys Glu Glu Lys His His Arg Pro Gly Gln
1               5                   10                  15


<210>  478
<211>  10
<212>  PRT
<213>  Artificial Sequence

<220>
<223>  epitope

<400>  478

Val Lys Asn Gly Gly Lys Glu Glu Lys His
1               5                   10


<210>  479
<211>  8
<212>  PRT
<213>  Artificial Sequence

<220>
<223>  epitope

<400>  479

Lys Asn Gly Gly Lys Glu Glu Lys
1               5


<210>  480
<211>  8
<212>  PRT
<213>  Artificial Sequence

<220>
<223>  epitope

<400>  480

Lys Glu Glu Lys His His Arg Pro
1               5


<210>  481
<211>  10
<212>  PRT
<213>  Artificial Sequence

<220>
<223>  epitope

<400>  481

Glu Lys His His Arg Pro Gly Gln Gln Ile
1               5                   10


<210>  482
<211>  15
<212>  PRT
<213>  Artificial Sequence

<220>
<223>  epitope

<400>  482

Val Lys Asn Gly Gly Lys Glu Glu Lys His His Arg Pro Gly Gln

1               5                   10                  15


<210>  483
<211>  9
<212>  PRT
<213>  Artificial Sequence

<220>
<223>  epitope

<400>  483

Thr Leu Val Val Lys Asn Gly Gly Lys
1               5


<210>  484
<211>  4
<212>  PRT
<213>  Artificial Sequence

<220>
<223>  epitope

<400>  484

Lys Glu Glu Lys
1


<210>  485
<211>  9
<212>  PRT
<213>  Artificial Sequence

<220>
<223>  epitope

<400>  485

Lys His His Arg Pro Gly Gln Gln Ile
1               5


<210>  486
<211>  15
<212>  PRT
<213>  Artificial Sequence

<220>
<223>  epitope

<400>  486

Val Lys Asn Gly Gly Lys Glu Glu Lys His His Arg Pro Gly Gln
1               5                   10                  15


<210>  487
<211>  10
<212>  PRT
<213>  Artificial Sequence

<220>
<223>  epitope

<400>  487

Thr Leu Val Val Lys Asn Gly Gly Lys Glu
1               5                   10


<210>  488
<211>  9
<212>  PRT
<213>  Artificial Sequence

<220>
<223>  epitope

<400>  488

Thr Leu Val Val Lys Asn Gly Gly Lys
1               5


<210>  489
<211>  15
<212>  PRT
<213>  Artificial Sequence

<220>
<223>  epitope


<220>
<221>  misc_feature
<222>  (1)..(6)
<223>  Xaa can be any naturally occurring amino acid

<220>
<221>  misc_feature
<222>  (9)..(9)
<223>  Xaa can be any naturally occurring amino acid

<220>
<221>  misc_feature
<222>  (12)..(12)
<223>  Xaa can be any naturally occurring amino acid

<400>  489

Xaa Xaa Xaa Xaa Xaa Xaa Glu Glu Xaa His His Xaa Pro Gly Gln
1               5                   10                  15


<210>  490
<211>  8
<212>  PRT
<213>  Artificial Sequence

<220>
<223>  epitope

<400>  490

Lys Asn Gly Gly Lys Glu Glu Lys
1               5

<210>  491
<211>  9
<212>  PRT
<213>  Artificial Sequence

<220>
<223>  epitope

<400>  491

Gly Lys Glu Glu Lys His His Arg Pro
1               5

<210>  492
<211>  4
<212>  PRT
<213>  Artificial Sequence

<220>
<223>  epitope

<400>  492

Lys Glu Glu Lys
1

<210>  493
<211>  8
<212>  PRT
<213>  Artificial Sequence

<220>
<223>  epitope

<400>  493

Lys Asn Gly Gly Lys Glu Glu Lys
1               5

<210>  494
<211>  6
<212>  PRT
<213>  Artificial Sequence

<220>
<223>  epitope

<400>  494

Gly Gly Lys Glu Glu Lys
1               5

<210>  495
<211>  5

<212>  PRT
<213>  Artificial Sequence

<220>
<223>  epitope

<400>  495

Gly Lys Glu Glu Lys
1               5


<210>  496
<211>  8
<212>  PRT
<213>  Artificial Sequence

<220>
<223>  epitope

<400>  496

Lys Asn Gly Gly Lys Glu Glu Lys
1               5


<210>  497
<211>  10
<212>  PRT
<213>  Artificial Sequence

<220>
<223>  epitope

<400>  497

Gly Gly Lys Glu Glu Lys His His Arg Pro
1               5                   10


<210>  498
<211>  4
<212>  PRT
<213>  Artificial Sequence

<220>
<223>  epitope

<400>  498

Lys Glu Glu Lys
1


<210>  499
<211>  6
<212>  PRT
<213>  Artificial Sequence

<220>
<223>  epitope

<400>  499

Gly Gly Lys Glu Glu Lys
1               5

<210>  500
<211>  15
<212>  PRT
<213>  Artificial Sequence

<220>
<223>  epitope

<220>
<221>  misc_feature
<222>  (1)..(3)
<223>  Xaa can be any naturally occurring amino acid

<220>
<221>  misc_feature
<222>  (5)..(6)
<223>  Xaa can be any naturally occurring amino acid

<220>
<221>  misc_feature
<222>  (10)..(14)
<223>  Xaa can be any naturally occurring amino acid

<400>  500

Xaa Xaa Xaa Gly Xaa Xaa Glu Glu Lys Xaa Xaa Xaa Xaa Xaa Gln
1               5                   10                  15

<210>  501
<211>  15
<212>  PRT
<213>  Artificial Sequence

<220>
<223>  epitope

<400>  501

Glu Ser Glu Asp Lys Tyr Gly Thr Thr Pro Pro Gln Leu Asp Ala
1               5                   10                  15

<210>  502
<211>  15
<212>  PRT
<213>  Artificial Sequence

<220>
<223>  epitope

<400>  502

Glu Ser Glu Asp Lys Tyr Gly Thr Thr Pro Pro Gln Leu Asp Ala
1               5                   10                  15

<210>  503
<211>  15
<212>  PRT
<213>  Artificial Sequence

<220>
<223>  epitope


<220>
<221>  misc_feature
<222>  (1)..(2)
<223>  Xaa can be any naturally occurring amino acid

<220>
<221>  misc_feature
<222>  (7)..(9)
<223>  Xaa can be any naturally occurring amino acid

<220>
<221>  misc_feature
<222>  (11)..(15)
<223>  Xaa can be any naturally occurring amino acid

<400>  503

Xaa Xaa Glu Asp Lys Tyr Xaa Xaa Xaa Pro Xaa Xaa Xaa Xaa Xaa
1               5                   10                  15


<210>  504
<211>  15
<212>  PRT
<213>  Artificial Sequence

<220>
<223>  epitope


<220>
<221>  misc_feature
<222>  (1)..(2)
<223>  Xaa can be any naturally occurring amino acid

<220>
<221>  misc_feature
<222>  (7)..(15)
<223>  Xaa can be any naturally occurring amino acid

<400>  504

Xaa Xaa Glu Asp Lys Tyr Xaa Xaa Xaa Xaa Xaa Xaa Xaa Xaa Xaa
1               5                   10                  15


<210>  505
<211>  7
<212>  PRT
<213>  Artificial Sequence

<220>
<223>  epitope

<400>  505

Asp Lys Tyr Gly Thr Thr Pro
1               5


<210>  506
<211>  7
<212>  PRT
<213>  Artificial Sequence

<220>
<223>  epitope


<220>
<221>  misc_feature
<222>  (4)..(6)
<223>  Xaa can be any naturally occurring amino acid

<400>  506

Asp Lys Tyr Xaa Xaa Xaa Pro
1               5


<210>  507
<211>  10
<212>  PRT
<213>  Artificial Sequence

<220>
<223>  epitope

<400>  507

Tyr Gly Thr Thr Pro Pro Gln Leu Asp Ala
1               5                   10


<210>  508
<211>  15
<212>  PRT
<213>  Artificial Sequence

<220>
<223>  epitope

<400>  508

Glu Ser Glu Asp Lys Tyr Gly Thr Thr Pro Pro Gln Leu Asp Ala
1               5                   10                  15


<210>  509
<211>  15
<212>  PRT
<213>  Artificial Sequence

<220>
<223>  epitope

<220>
<221>  misc_feature
<222>  (1)..(1)
<223>  Xaa can be any naturally occurring amino acid

<220>
<221>  misc_feature
<222>  (3)..(3)
<223>  Xaa can be any naturally occurring amino acid

<220>
<221>  misc_feature
<222>  (7)..(15)
<223>  Xaa can be any naturally occurring amino acid

<400>  509

Xaa Ser Xaa Asp Lys Tyr Xaa Xaa Xaa Xaa Xaa Xaa Xaa Xaa Xaa
1               5                   10                  15


<210>  510
<211>  7
<212>  PRT
<213>  Artificial Sequence

<220>
<223>  epitope

<400>  510

Glu Asp Lys Tyr Gly Thr Thr
1               5


<210>  511
<211>  6
<212>  PRT
<213>  Artificial Sequence

<220>
<223>  epitope

<400>  511

Glu Asp Lys Tyr Gly Thr
1               5


<210>  512
<211>  10
<212>  PRT
<213>  Artificial Sequence

<220>
<223>  epitope

<400>  512

Tyr Gly Thr Thr Pro Pro Gln Leu Asp Ala
1               5                   10

<210>  513
<211>  9
<212>  PRT
<213>  Artificial Sequence

<220>
<223>  epitope

<400>  513

Tyr Gly Thr Thr Pro Pro Gln Leu Asp
1               5


<210>  514
<211>  15
<212>  PRT
<213>  Artificial Sequence

<220>
<223>  epitope

<400>  514

Glu Ser Glu Asp Lys Tyr Gly Thr Thr Pro Pro Gln Leu Asp Ala
1               5                   10                  15


<210>  515
<211>  15
<212>  PRT
<213>  Artificial Sequence

<220>
<223>  epitope


<220>
<221>  misc_feature
<222>  (1)..(1)
<223>  Xaa can be any naturally occurring amino acid

<220>
<221>  misc_feature
<222>  (7)..(15)
<223>  Xaa can be any naturally occurring amino acid

<400>  515

Xaa Ser Glu Asp Lys Tyr Xaa Xaa Xaa Xaa Xaa Xaa Xaa Xaa Xaa
1               5                   10                  15


<210>  516
<211>  4
<212>  PRT
<213>  Artificial Sequence

<220>
<223>  epitope

<400>  516

Glu Asp Lys Tyr
1

<210> 517
<211> 10
<212> PRT
<213> Artificial Sequence

<220>
<223> epitope

<400> 517

Tyr Gly Thr Thr Pro Pro Gln Leu Asp Ala
1               5                   10

<210> 518
<211> 15
<212> PRT
<213> Artificial Sequence

<220>
<223> epitope

<400> 518

Glu Ser Glu Asp Lys Tyr Gly Thr Thr Pro Pro Gln Leu Asp Ala
1               5                   10                  15

<210> 519
<211> 15
<212> PRT
<213> Artificial Sequence

<220>
<223> epitope

<220>
<221> misc_feature
<222> (1)..(1)
<223> Xaa can be any naturally occurring amino acid

<220>
<221> misc_feature
<222> (8)..(8)
<223> Xaa can be any naturally occurring amino acid

<220>
<221> misc_feature
<222> (14)..(15)
<223> Xaa can be any naturally occurring amino acid

<400> 519

Xaa Ser Glu Asp Lys Tyr Gly Xaa Thr Pro Pro Gln Leu Xaa Xaa
1               5                   10                  15

<210> 520

<211>  15
<212>  PRT
<213>  Artificial Sequence

<220>
<223>  epitope



<220>
<221>  misc_feature
<222>  (1)..(2)
<223>  Xaa can be any naturally occurring amino acid

<220>
<221>  misc_feature
<222>  (8)..(10)
<223>  Xaa can be any naturally occurring amino acid

<220>
<221>  misc_feature
<222>  (13)..(15)
<223>  Xaa can be any naturally occurring amino acid

<400>  520

Xaa Xaa Glu Asp Lys Tyr Gly Xaa Xaa Xaa Pro Gln Xaa Xaa Xaa
1               5                   10                  15


<210>  521
<211>  15
<212>  PRT
<213>  Artificial Sequence

<220>
<223>  epitope



<220>
<221>  misc_feature
<222>  (1)..(3)
<223>  Xaa can be any naturally occurring amino acid

<220>
<221>  misc_feature
<222>  (8)..(10)
<223>  Xaa can be any naturally occurring amino acid

<220>
<221>  misc_feature
<222>  (13)..(15)
<223>  Xaa can be any naturally occurring amino acid

<400>  521

Xaa Xaa Xaa Asp Lys Tyr Gly Xaa Xaa Xaa Pro Gln Xaa Xaa Xaa
1               5                   10                  15


<210>  522
<211>  5
<212>  PRT
<213>  Artificial Sequence

<220>
<223>  epitope

<400>  522

Asp Lys Tyr Gly Thr
1               5


<210>  523
<211>  5
<212>  PRT
<213>  Artificial Sequence

<220>
<223>  epitope


<220>
<221>  misc_feature
<222>  (5)..(5)
<223>  Xaa can be any naturally occurring amino acid

<400>  523

Asp Lys Tyr Gly Xaa
1               5


<210>  524
<211>  10
<212>  PRT
<213>  Artificial Sequence

<220>
<223>  epitope

<400>  524

Tyr Gly Thr Thr Pro Pro Gln Leu Asp Ala
1               5                   10


<210>  525
<211>  9
<212>  PRT
<213>  Artificial Sequence

<220>
<223>  epitope

<400>  525

Gly Thr Thr Pro Pro Gln Leu Asp Ala
1               5


<210>  526
<211>  9
<212>  PRT
<213>  Artificial Sequence

<220>
<223>  epitope

<220>
<221>  misc_feature
<222>  (2)..(2)
<223>  Xaa can be any naturally occurring amino acid

<220>
<221>  misc_feature
<222>  (8)..(9)
<223>  Xaa can be any naturally occurring amino acid

<400>  526

Gly Xaa Thr Pro Pro Gln Leu Xaa Xaa
1               5

<210>  527
<211>  15
<212>  PRT
<213>  Artificial Sequence

<220>
<223>  epitope

<400>  527

Glu Ser Glu Asp Lys Tyr Gly Thr Thr Pro Pro Gln Leu Asp Ala
1               5                   10                  15

<210>  528
<211>  15
<212>  PRT
<213>  Artificial Sequence

<220>
<223>  epitope

<220>
<221>  misc_feature
<222>  (1)..(1)
<223>  Xaa can be any naturally occurring amino acid

<220>
<221>  misc_feature
<222>  (3)..(3)
<223>  Xaa can be any naturally occurring amino acid

<220>
<221>  misc_feature
<222>  (7)..(8)
<223>  Xaa can be any naturally occurring amino acid

<220>
<221>  misc_feature
<222>  (10)..(10)
<223>  Xaa can be any naturally occurring amino acid

<220>
<221>  misc_feature
<222>  (12)..(15)
<223>  Xaa can be any naturally occurring amino acid

<400>  528

Xaa Ser Xaa Asp Lys Tyr Xaa Xaa Thr Xaa Pro Xaa Xaa Xaa Xaa
1               5                   10                  15


<210>  529
<211>  10
<212>  PRT
<213>  Artificial Sequence

<220>
<223>  epitope

<400>  529

Ser Glu Asp Lys Tyr Gly Thr Thr Pro Pro
1               5                   10


<210>  530
<211>  7
<212>  PRT
<213>  Artificial Sequence

<220>
<223>  epitope

<400>  530

Glu Asp Lys Tyr Gly Thr Thr
1               5


<210>  531
<211>  7
<212>  PRT
<213>  Artificial Sequence

<220>
<223>  epitope


<220>
<221>  misc_feature
<222>  (1)..(1)
<223>  Xaa can be any naturally occurring amino acid

<220>
<221>  misc_feature
<222>  (5)..(6)
<223>  Xaa can be any naturally occurring amino acid

<400>  531

Xaa Asp Lys Tyr Xaa Xaa Thr
1               5

<210>  532
<211>  10
<212>  PRT
<213>  Artificial Sequence

<220>
<223>  epitope

<400>  532

Tyr Gly Thr Thr Pro Pro Gln Leu Asp Ala
1               5                   10


<210>  533
<211>  9
<212>  PRT
<213>  Artificial Sequence

<220>
<223>  epitope

<400>  533

Tyr Gly Thr Thr Pro Pro Gln Leu Asp
1               5


<210>  534
<211>  15
<212>  PRT
<213>  Artificial Sequence

<220>
<223>  epitope

<400>  534

Glu Ser Glu Asp Lys Tyr Gly Thr Thr Pro Pro Gln Leu Asp Ala
1               5                   10                  15


<210>  535
<211>  15
<212>  PRT
<213>  Artificial Sequence

<220>
<223>  epitope


<220>
<221>  misc_feature
<222>  (1)..(1)
<223>  Xaa can be any naturally occurring amino acid

<220>
<221>  misc_feature
<222>  (8)..(8)
<223>  Xaa can be any naturally occurring amino acid

<220>

<221>  misc_feature
<222>  (10)..(15)
<223>  Xaa can be any naturally occurring amino acid

<400>  535

Xaa Ser Glu Asp Lys Tyr Gly Xaa Thr Xaa Xaa Xaa Xaa Xaa Xaa
1               5                   10                  15


<210>  536
<211>  9
<212>  PRT
<213>  Artificial Sequence

<220>
<223>  epitope

<400>  536

Ser Glu Asp Lys Tyr Gly Thr Thr Pro
1               5


<210>  537
<211>  8
<212>  PRT
<213>  Artificial Sequence

<220>
<223>  epitope

<400>  537

Glu Asp Lys Tyr Gly Thr Thr Pro
1               5


<210>  538
<211>  7
<212>  PRT
<213>  Artificial Sequence

<220>
<223>  epitope

<400>  538

Glu Asp Lys Tyr Gly Thr Thr
1               5


<210>  539
<211>  7
<212>  PRT
<213>  Artificial Sequence

<220>
<223>  epitope


<220>
<221>  misc_feature

<222>  (6)..(6)
<223>  Xaa can be any naturally occurring amino acid

<400>  539

Glu Asp Lys Tyr Gly Xaa Thr
1               5


<210>  540
<211>  15
<212>  PRT
<213>  Artificial Sequence

<220>
<223>  epitope

<400>  540

Glu Ser Glu Asp Lys Tyr Gly Thr Thr Pro Pro Gln Leu Asp Ala
1               5                   10                  15


<210>  541
<211>  6
<212>  PRT
<213>  Artificial Sequence

<220>
<223>  epitope

<400>  541

Glu Asp Lys Tyr Gly Thr
1               5


<210>  542
<211>  4
<212>  PRT
<213>  Artificial Sequence

<220>
<223>  epitope

<400>  542

Glu Asp Lys Tyr
1


<210>  543
<211>  10
<212>  PRT
<213>  Artificial Sequence

<220>
<223>  epitope

<400>  543

Tyr Gly Thr Thr Pro Pro Gln Leu Asp Ala
1               5                   10

<210>  544
<211>  15
<212>  PRT
<213>  Artificial Sequence

<220>
<223>  epitope


<220>
<221>  misc_feature
<222>  (1)..(1)
<223>  Xaa can be any naturally occurring amino acid

<220>
<221>  misc_feature
<222>  (7)..(8)
<223>  Xaa can be any naturally occurring amino acid

<220>
<221>  misc_feature
<222>  (10)..(10)
<223>  Xaa can be any naturally occurring amino acid

<220>
<221>  misc_feature
<222>  (12)..(15)
<223>  Xaa can be any naturally occurring amino acid

<400>  544

Xaa Ser Glu Asp Lys Tyr Xaa Xaa Thr Xaa Pro Xaa Xaa Xaa Xaa
1               5                   10                  15


<210>  545
<211>  15
<212>  PRT
<213>  Artificial Sequence

<220>
<223>  epitope


<220>
<221>  misc_feature
<222>  (1)..(1)
<223>  Xaa can be any naturally occurring amino acid

<220>
<221>  misc_feature
<222>  (3)..(3)
<223>  Xaa can be any naturally occurring amino acid

<220>
<221>  misc_feature
<222>  (7)..(8)
<223>  Xaa can be any naturally occurring amino acid

<220>
<221>  misc_feature

<222>  (10)..(10)
<223>  Xaa can be any naturally occurring amino acid

<220>
<221>  misc_feature
<222>  (12)..(15)
<223>  Xaa can be any naturally occurring amino acid

<400>  545

Xaa Ser Xaa Asp Lys Tyr Xaa Xaa Thr Xaa Pro Xaa Xaa Xaa Xaa
1               5                   10                  15


<210>  546
<211>  15
<212>  PRT
<213>  Artificial Sequence

<220>
<223>  epitope


<220>
<221>  misc_feature
<222>  (1)..(1)
<223>  Xaa can be any naturally occurring amino acid

<220>
<221>  misc_feature
<222>  (3)..(3)
<223>  Xaa can be any naturally occurring amino acid

<220>
<221>  misc_feature
<222>  (7)..(15)
<223>  Xaa can be any naturally occurring amino acid

<400>  546

Xaa Ser Xaa Asp Lys Tyr Xaa Xaa Xaa Xaa Xaa Xaa Xaa Xaa Xaa
1               5                   10                  15


<210>  547
<211>  5
<212>  PRT
<213>  Artificial Sequence

<220>
<223>  epitope

<400>  547

Glu Asp Lys Tyr Gly
1               5


<210>  548
<211>  4
<212>  PRT
<213>  Artificial Sequence

<220>
<223>  epitope

<400>  548

Glu Asp Lys Tyr
1


<210>  549
<211>  10
<212>  PRT
<213>  Artificial Sequence

<220>
<223>  epitope

<400>  549

Tyr Gly Thr Thr Pro Pro Gln Leu Asp Ala
1               5                   10


<210>  550
<211>  9
<212>  PRT
<213>  Artificial Sequence

<220>
<223>  epitope

<400>  550

Tyr Gly Thr Thr Pro Pro Gln Leu Asp
1               5


<210>  551
<211>  15
<212>  PRT
<213>  Artificial Sequence

<220>
<223>  epitope


<220>
<221>  misc_feature
<222>  (1)..(3)
<223>  Xaa can be any naturally occurring amino acid

<220>
<221>  misc_feature
<222>  (7)..(8)
<223>  Xaa can be any naturally occurring amino acid

<220>
<221>  misc_feature
<222>  (12)..(12)
<223>  Xaa can be any naturally occurring amino acid

<400>  551

Xaa Xaa Xaa Asp Lys Tyr Xaa Xaa Thr Pro Pro Xaa Leu Asp Ala
1               5                   10                  15


<210>  552
<211>  15
<212>  PRT
<213>  Artificial Sequence

<220>
<223>  epitope


<220>
<221>  misc_feature
<222>  (1)..(3)
<223>  Xaa can be any naturally occurring amino acid

<220>
<221>  misc_feature
<222>  (7)..(8)
<223>  Xaa can be any naturally occurring amino acid

<220>
<221>  misc_feature
<222>  (10)..(15)
<223>  Xaa can be any naturally occurring amino acid

<400>  552

Xaa Xaa Xaa Asp Lys Tyr Xaa Xaa Thr Xaa Xaa Xaa Xaa Xaa Xaa
1               5                   10                  15


<210>  553
<211>  10
<212>  PRT
<213>  Artificial Sequence

<220>
<223>  epitope

<400>  553

Ser Glu Asp Lys Tyr Gly Thr Thr Pro Pro
1               5                   10


<210>  554
<211>  8
<212>  PRT
<213>  Artificial Sequence

<220>
<223>  epitope

<400>  554

Glu Asp Lys Tyr Gly Thr Thr Pro
1               5


<210>  555

<211>  8
<212>  PRT
<213>  Artificial Sequence

<220>
<223>  epitope


<220>
<221>  misc_feature
<222>  (1)..(1)
<223>  Xaa can be any naturally occurring amino acid

<220>
<221>  misc_feature
<222>  (5)..(6)
<223>  Xaa can be any naturally occurring amino acid

<400>  555

Xaa Asp Lys Tyr Xaa Xaa Thr Pro
1               5


<210>  556
<211>  8
<212>  PRT
<213>  Artificial Sequence

<220>
<223>  epitope


<220>
<221>  misc_feature
<222>  (1)..(1)
<223>  Xaa can be any naturally occurring amino acid

<220>
<221>  misc_feature
<222>  (5)..(6)
<223>  Xaa can be any naturally occurring amino acid

<220>
<221>  misc_feature
<222>  (8)..(8)
<223>  Xaa can be any naturally occurring amino acid

<400>  556

Xaa Asp Lys Tyr Xaa Xaa Thr Xaa
1               5


<210>  557
<211>  10
<212>  PRT
<213>  Artificial Sequence

<220>
<223>  epitope

<400>  557

Tyr Gly Thr Thr Pro Pro Gln Leu Asp Ala
1               5                   10


<210>  558
<211>  8
<212>  PRT
<213>  Artificial Sequence

<220>
<223>  epitope

<400>  558

Gly Thr Thr Pro Pro Gln Leu Asp
1               5


<210>  559
<211>  8
<212>  PRT
<213>  Artificial Sequence

<220>
<223>  epitope


<220>
<221>  misc_feature
<222>  (1)..(2)
<223>  Xaa can be any naturally occurring amino acid

<220>
<221>  misc_feature
<222>  (6)..(6)
<223>  Xaa can be any naturally occurring amino acid

<400>  559

Xaa Xaa Thr Pro Pro Xaa Leu Asp
1               5


<210>  560
<211>  15
<212>  PRT
<213>  Artificial Sequence

<220>
<223>  epitope


<220>
<221>  misc_feature
<222>  (1)..(1)
<223>  Xaa can be any naturally occurring amino acid

<220>
<221>  misc_feature
<222>  (3)..(3)
<223>  Xaa can be any naturally occurring amino acid

<220>
<221>  misc_feature
<222>  (7)..(8)
<223>  Xaa can be any naturally occurring amino acid

<220>
<221>  misc_feature
<222>  (13)..(15)
<223>  Xaa can be any naturally occurring amino acid

<400>  560

Xaa Ser Xaa Asp Lys Tyr Xaa Xaa Thr Pro Pro Gln Xaa Xaa Xaa
1               5                   10                  15


<210>  561
<211>  15
<212>  PRT
<213>  Artificial Sequence

<220>
<223>  epitope


<220>
<221>  misc_feature
<222>  (1)..(3)
<223>  Xaa can be any naturally occurring amino acid

<220>
<221>  misc_feature
<222>  (7)..(11)
<223>  Xaa can be any naturally occurring amino acid

<220>
<221>  misc_feature
<222>  (13)..(15)
<223>  Xaa can be any naturally occurring amino acid

<400>  561

Xaa Xaa Xaa Asp Lys Tyr Xaa Xaa Xaa Xaa Xaa Gln Xaa Xaa Xaa
1               5                   10                  15


<210>  562
<211>  9
<212>  PRT
<213>  Artificial Sequence

<220>
<223>  epitope

<400>  562

Ser Glu Asp Lys Tyr Gly Thr Thr Pro
1               5


<210>  563
<211>  4
<212>  PRT

<213>  Artificial Sequence

<220>
<223>  epitope

<400>  563

Glu Asp Lys Tyr
1


<210>  564
<211>  10
<212>  PRT
<213>  Artificial Sequence

<220>
<223>  epitope

<400>  564

Tyr Gly Thr Thr Pro Pro Gln Leu Asp Ala
1               5                   10


<210>  565
<211>  7
<212>  PRT
<213>  Artificial Sequence

<220>
<223>  epitope

<400>  565

Thr Thr Pro Pro Gln Leu Asp
1               5


<210>  566
<211>  7
<212>  PRT
<213>  Artificial Sequence

<220>
<223>  epitope


<220>
<221>  misc_feature
<222>  (1)..(1)
<223>  Xaa can be any naturally occurring amino acid

<220>
<221>  misc_feature
<222>  (6)..(7)
<223>  Xaa can be any naturally occurring amino acid

<400>  566

Xaa Thr Pro Pro Gln Xaa Xaa
1               5

<210>  567
<211>  15
<212>  PRT
<213>  Artificial Sequence

<220>
<223>  epitope



<220>
<221>  misc_feature
<222>  (1)..(1)
<223>  Xaa can be any naturally occurring amino acid

<220>
<221>  misc_feature
<222>  (3)..(3)
<223>  Xaa can be any naturally occurring amino acid

<220>
<221>  misc_feature
<222>  (7)..(8)
<223>  Xaa can be any naturally occurring amino acid

<220>
<221>  misc_feature
<222>  (13)..(15)
<223>  Xaa can be any naturally occurring amino acid

<400>  567

Xaa Ser Xaa Asp Lys Tyr Xaa Xaa Thr Pro Pro Gln Xaa Xaa Xaa
1               5                   10                  15


<210>  568
<211>  15
<212>  PRT
<213>  Artificial Sequence

<220>
<223>  epitope



<220>
<221>  misc_feature
<222>  (1)..(3)
<223>  Xaa can be any naturally occurring amino acid

<220>
<221>  misc_feature
<222>  (7)..(10)
<223>  Xaa can be any naturally occurring amino acid

<220>
<221>  misc_feature
<222>  (12)..(15)
<223>  Xaa can be any naturally occurring amino acid

<400>  568

Xaa Xaa Xaa Asp Lys Tyr Xaa Xaa Xaa Xaa Pro Xaa Xaa Xaa Xaa

1                 5                   10                  15


<210>  569
<211>  8
<212>  PRT
<213>  Artificial Sequence

<220>
<223>  epitope

<400>  569

Glu Asp Lys Tyr Gly Thr Thr Pro
1               5


<210>  570
<211>  10
<212>  PRT
<213>  Artificial Sequence

<220>
<223>  epitope

<400>  570

Tyr Gly Thr Thr Pro Pro Gln Leu Asp Ala
1               5                   10


<210>  571
<211>  5
<212>  PRT
<213>  Artificial Sequence

<220>
<223>  epitope

<400>  571

Tyr Gly Thr Thr Pro
1               5


<210>  572
<211>  15
<212>  PRT
<213>  Artificial Sequence

<220>
<223>  epitope


<220>
<221>  misc_feature
<222>  (1)..(2)
<223>  Xaa can be any naturally occurring amino acid

<220>
<221>  misc_feature
<222>  (7)..(8)
<223>  Xaa can be any naturally occurring amino acid

<220>
<221>  misc_feature
<222>  (13)..(13)
<223>  Xaa can be any naturally occurring amino acid

<400>  572

Xaa Xaa Glu Asp Lys Tyr Xaa Xaa Thr Pro Pro Gln Xaa Asp Ala
1               5                   10                  15


<210>  573
<211>  15
<212>  PRT
<213>  Artificial Sequence

<220>
<223>  epitope


<220>
<221>  misc_feature
<222>  (1)..(2)
<223>  Xaa can be any naturally occurring amino acid

<220>
<221>  misc_feature
<222>  (7)..(14)
<223>  Xaa can be any naturally occurring amino acid

<400>  573

Xaa Xaa Glu Asp Lys Tyr Xaa Xaa Xaa Xaa Xaa Xaa Xaa Xaa Ala
1               5                   10                  15


<210>  574
<211>  9
<212>  PRT
<213>  Artificial Sequence

<220>
<223>  epitope

<400>  574

Glu Asp Lys Tyr Gly Thr Thr Pro Pro
1               5


<210>  575
<211>  9
<212>  PRT
<213>  Artificial Sequence

<220>
<223>  epitope


<220>
<221>  misc_feature
<222>  (5)..(6)

<223>  Xaa can be any naturally occurring amino acid

<400>  575

Glu Asp Lys Tyr Xaa Xaa Thr Pro Pro
1               5


<210>  576
<211>  9
<212>  PRT
<213>  Artificial Sequence

<220>
<223>  epitope


<220>
<221>  misc_feature
<222>  (5)..(9)
<223>  Xaa can be any naturally occurring amino acid

<400>  576

Glu Asp Lys Tyr Xaa Xaa Xaa Xaa Xaa
1               5


<210>  577
<211>  10
<212>  PRT
<213>  Artificial Sequence

<220>
<223>  epitope

<400>  577

Tyr Gly Thr Thr Pro Pro Gln Leu Asp Ala
1               5                   10


<210>  578
<211>  9
<212>  PRT
<213>  Artificial Sequence

<220>
<223>  epitope

<400>  578

Tyr Gly Thr Thr Pro Pro Gln Leu Asp
1               5


<210>  579
<211>  9
<212>  PRT
<213>  Artificial Sequence

<220>
<223>  epitope

<220>
<221>  misc_feature
<222>  (2)..(3)
<223>  Xaa can be any naturally occurring amino acid

<220>
<221>  misc_feature
<222>  (8)..(8)
<223>  Xaa can be any naturally occurring amino acid

<400>  579

Tyr Xaa Xaa Thr Pro Pro Gln Xaa Asp
1               5


<210>  580
<211>  15
<212>  PRT
<213>  Artificial Sequence

<220>
<223>  epitope


<220>
<221>  misc_feature
<222>  (1)..(1)
<223>  Xaa can be any naturally occurring amino acid

<220>
<221>  misc_feature
<222>  (3)..(3)
<223>  Xaa can be any naturally occurring amino acid

<220>
<221>  misc_feature
<222>  (7)..(8)
<223>  Xaa can be any naturally occurring amino acid

<400>  580

Xaa Ser Xaa Asp Lys Tyr Xaa Xaa Thr Pro Pro Gln Leu Asp Ala
1               5                   10                  15


<210>  581
<211>  15
<212>  PRT
<213>  Artificial Sequence

<220>
<223>  epitope


<220>
<221>  misc_feature
<222>  (1)..(3)
<223>  Xaa can be any naturally occurring amino acid

<220>

<221>  misc_feature
<222>  (7)..(8)
<223>  Xaa can be any naturally occurring amino acid

<220>
<221>  misc_feature
<222>  (10)..(10)
<223>  Xaa can be any naturally occurring amino acid

<220>
<221>  misc_feature
<222>  (14)..(14)
<223>  Xaa can be any naturally occurring amino acid

<400>  581

Xaa Xaa Xaa Asp Lys Tyr Xaa Xaa Thr Xaa Pro Gln Leu Xaa Ala
1               5                   10                  15


<210>  582
<211>  15
<212>  PRT
<213>  Artificial Sequence

<220>
<223>  epitope


<220>
<221>  misc_feature
<222>  (1)..(3)
<223>  Xaa can be any naturally occurring amino acid

<220>
<221>  misc_feature
<222>  (7)..(8)
<223>  Xaa can be any naturally occurring amino acid

<220>
<221>  misc_feature
<222>  (10)..(10)
<223>  Xaa can be any naturally occurring amino acid

<220>
<221>  misc_feature
<222>  (13)..(15)
<223>  Xaa can be any naturally occurring amino acid

<400>  582

Xaa Xaa Xaa Asp Lys Tyr Xaa Xaa Thr Xaa Pro Gln Xaa Xaa Xaa
1               5                   10                  15


<210>  583
<211>  9
<212>  PRT
<213>  Artificial Sequence

<220>
<223>  epitope

<400>  583

Ser Glu Asp Lys Tyr Gly Thr Thr Pro
1               5


<210>  584
<211>  5
<212>  PRT
<213>  Artificial Sequence

<220>
<223>  epitope

<400>  584

Ser Glu Asp Lys Tyr
1               5


<210>  585
<211>  5
<212>  PRT
<213>  Artificial Sequence

<220>
<223>  epitope


<220>
<221>  misc_feature
<222>  (2)..(2)
<223>  Xaa can be any naturally occurring amino acid

<400>  585

Ser Xaa Asp Lys Tyr
1               5


<210>  586
<211>  9
<212>  PRT
<213>  Artificial Sequence

<220>
<223>  epitope

<400>  586

Tyr Gly Thr Thr Pro Pro Gln Leu Asp
1               5


<210>  587
<211>  8
<212>  PRT
<213>  Artificial Sequence

<220>
<223>  epitope

<400>  587

Tyr Gly Thr Thr Pro Pro Gln Leu
1               5

<210>  588
<211>  8
<212>  PRT
<213>  Artificial Sequence

<220>
<223>  epitope

<220>
<221>  misc_feature
<222>  (2)..(3)
<223>  Xaa can be any naturally occurring amino acid

<400>  588

Tyr Xaa Xaa Thr Pro Pro Gln Leu
1               5

<210>  589
<211>  8
<212>  PRT
<213>  Artificial Sequence

<220>
<223>  epitope

<220>
<221>  misc_feature
<222>  (2)..(3)
<223>  Xaa can be any naturally occurring amino acid

<220>
<221>  misc_feature
<222>  (5)..(5)
<223>  Xaa can be any naturally occurring amino acid

<400>  589

Tyr Xaa Xaa Thr Xaa Pro Gln Leu
1               5

<210>  590
<211>  8
<212>  PRT
<213>  Artificial Sequence

<220>
<223>  epitope

<220>
<221>  misc_feature
<222>  (2)..(3)
<223>  Xaa can be any naturally occurring amino acid

<220>
<221>  misc_feature
<222>  (5)..(5)
<223>  Xaa can be any naturally occurring amino acid

<220>
<221>  misc_feature
<222>  (8)..(8)
<223>  Xaa can be any naturally occurring amino acid

<400>  590

Tyr Xaa Xaa Thr Xaa Pro Gln Xaa
1               5


<210>  591
<211>  15
<212>  PRT
<213>  Artificial Sequence

<220>
<223>  epitope


<220>
<221>  misc_feature
<222>  (1)..(1)
<223>  Xaa can be any naturally occurring amino acid

<220>
<221>  misc_feature
<222>  (3)..(4)
<223>  Xaa can be any naturally occurring amino acid

<220>
<221>  misc_feature
<222>  (7)..(7)
<223>  Xaa can be any naturally occurring amino acid

<220>
<221>  misc_feature
<222>  (10)..(10)
<223>  Xaa can be any naturally occurring amino acid

<220>
<221>  misc_feature
<222>  (15)..(15)
<223>  Xaa can be any naturally occurring amino acid

<400>  591

Xaa Ser Xaa Xaa Lys Tyr Xaa Thr Thr Xaa Pro Gln Leu Asp Xaa
1               5                   10                  15


<210>  592
<211>  15
<212>  PRT
<213>  Artificial Sequence

<220>

<223>  epitope

<220>
<221>  misc_feature
<222>  (1)..(4)
<223>  Xaa can be any naturally occurring amino acid

<220>
<221>  misc_feature
<222>  (7)..(7)
<223>  Xaa can be any naturally occurring amino acid

<220>
<221>  misc_feature
<222>  (10)..(10)
<223>  Xaa can be any naturally occurring amino acid

<220>
<221>  misc_feature
<222>  (14)..(15)
<223>  Xaa can be any naturally occurring amino acid

<400>  592

Xaa Xaa Xaa Xaa Lys Tyr Xaa Thr Thr Xaa Pro Gln Leu Xaa Xaa
1               5                   10                  15

<210>  593
<211>  15
<212>  PRT
<213>  Artificial Sequence

<220>
<223>  epitope

<220>
<221>  misc_feature
<222>  (1)..(4)
<223>  Xaa can be any naturally occurring amino acid

<220>
<221>  misc_feature
<222>  (7)..(8)
<223>  Xaa can be any naturally occurring amino acid

<220>
<221>  misc_feature
<222>  (10)..(10)
<223>  Xaa can be any naturally occurring amino acid

<220>
<221>  misc_feature
<222>  (13)..(15)
<223>  Xaa can be any naturally occurring amino acid

<400>  593

Xaa Xaa Xaa Xaa Lys Tyr Xaa Xaa Thr Xaa Pro Gln Xaa Xaa Xaa
1               5                   10                  15

<210>  594
<211>  9
<212>  PRT
<213>  Artificial Sequence

<220>
<223>  epitope

<400>  594

Tyr Gly Thr Thr Pro Pro Gln Leu Asp
1               5


<210>  595
<211>  8
<212>  PRT
<213>  Artificial Sequence

<220>
<223>  epitope

<400>  595

Tyr Gly Thr Thr Pro Pro Gln Leu
1               5


<210>  596
<211>  8
<212>  PRT
<213>  Artificial Sequence

<220>
<223>  epitope


<220>
<221>  misc_feature
<222>  (2)..(2)
<223>  Xaa can be any naturally occurring amino acid

<220>
<221>  misc_feature
<222>  (5)..(5)
<223>  Xaa can be any naturally occurring amino acid

<400>  596

Tyr Xaa Thr Thr Xaa Pro Gln Leu
1               5


<210>  597
<211>  8
<212>  PRT
<213>  Artificial Sequence

<220>
<223>  epitope

<220>
<221>  misc_feature
<222>  (2)..(3)
<223>  Xaa can be any naturally occurring amino acid

<220>
<221>  misc_feature
<222>  (5)..(5)
<223>  Xaa can be any naturally occurring amino acid

<220>
<221>  misc_feature
<222>  (8)..(8)
<223>  Xaa can be any naturally occurring amino acid

<400>  597

Tyr Xaa Xaa Thr Xaa Pro Gln Xaa
1               5


<210>  598
<211>  15
<212>  PRT
<213>  Artificial Sequence

<220>
<223>  epitope


<220>
<221>  misc_feature
<222>  (1)..(2)
<223>  Xaa can be any naturally occurring amino acid

<220>
<221>  misc_feature
<222>  (7)..(10)
<223>  Xaa can be any naturally occurring amino acid

<220>
<221>  misc_feature
<222>  (12)..(15)
<223>  Xaa can be any naturally occurring amino acid

<400>  598

Xaa Xaa Glu Asp Lys Tyr Xaa Xaa Xaa Xaa Pro Xaa Xaa Xaa Xaa
1               5                   10                  15


<210>  599
<211>  15
<212>  PRT
<213>  Artificial Sequence

<220>
<223>  epitope


<220>
<221>  misc_feature
<222>  (1)..(2)

<223>  Xaa can be any naturally occurring amino acid

<220>
<221>  misc_feature
<222>  (7)..(15)
<223>  Xaa can be any naturally occurring amino acid

<400>  599

Xaa Xaa Glu Asp Lys Tyr Xaa Xaa Xaa Xaa Xaa Xaa Xaa Xaa Xaa
1               5                   10                  15


<210>  600
<211>  8
<212>  PRT
<213>  Artificial Sequence

<220>
<223>  epitope

<400>  600

Glu Asp Lys Tyr Gly Thr Thr Pro
1               5


<210>  601
<211>  7
<212>  PRT
<213>  Artificial Sequence

<220>
<223>  epitope

<400>  601

Glu Asp Lys Tyr Gly Thr Thr
1               5


<210>  602
<211>  7
<212>  PRT
<213>  Artificial Sequence

<220>
<223>  epitope


<220>
<221>  misc_feature
<222>  (5)..(7)
<223>  Xaa can be any naturally occurring amino acid

<400>  602

Glu Asp Lys Tyr Xaa Xaa Xaa
1               5


<210>  603
<211>  10

<212>  PRT
<213>  Artificial Sequence

<220>
<223>  epitope

<400>  603

Tyr Gly Thr Thr Pro Pro Gln Leu Asp Ala
1               5                   10


<210>  604
<211>  8
<212>  PRT
<213>  Artificial Sequence

<220>
<223>  epitope

<400>  604

Gly Thr Thr Pro Pro Gln Leu Asp
1               5


<210>  605
<211>  15
<212>  PRT
<213>  Artificial Sequence

<220>
<223>  epitope

<400>  605

Thr Lys Glu Asn Ser Lys Ser Gln Val Ser Leu Ser Val Ser Ser
1               5                   10                  15


<210>  606
<211>  15
<212>  PRT
<213>  Artificial Sequence

<220>
<223>  epitope

<400>  606

Thr Lys Glu Asn Ser Lys Ser Gln Val Ser Leu Ser Val Ser Ser
1               5                   10                  15


<210>  607
<211>  15
<212>  PRT
<213>  Artificial Sequence

<220>
<223>  epitope

<220>
<221>  misc_feature
<222>  (1)..(1)
<223>  Xaa can be any naturally occurring amino acid

<220>
<221>  misc_feature
<222>  (3)..(3)
<223>  Xaa can be any naturally occurring amino acid

<220>
<221>  misc_feature
<222>  (15)..(15)
<223>  Xaa can be any naturally occurring amino acid

<400>  607

Xaa Lys Xaa Asn Ser Lys Ser Gln Val Ser Leu Ser Val Ser Xaa
1               5                   10                  15


<210>  608
<211>  15
<212>  PRT
<213>  Artificial Sequence

<220>
<223>  epitope


<220>
<221>  misc_feature
<222>  (1)..(1)
<223>  Xaa can be any naturally occurring amino acid

<220>
<221>  misc_feature
<222>  (3)..(4)
<223>  Xaa can be any naturally occurring amino acid

<220>
<221>  misc_feature
<222>  (9)..(9)
<223>  Xaa can be any naturally occurring amino acid

<220>
<221>  misc_feature
<222>  (13)..(13)
<223>  Xaa can be any naturally occurring amino acid

<220>
<221>  misc_feature
<222>  (15)..(15)
<223>  Xaa can be any naturally occurring amino acid

<400>  608

Xaa Lys Xaa Xaa Ser Lys Ser Gln Xaa Ser Leu Ser Xaa Ser Xaa
1               5                   10                  15


<210>  609
<211>  15

<212>  PRT
<213>  Artificial Sequence

<220>
<223>  epitope



<220>
<221>  misc_feature
<222>  (1)..(1)
<223>  Xaa can be any naturally occurring amino acid

<220>
<221>  misc_feature
<222>  (3)..(4)
<223>  Xaa can be any naturally occurring amino acid

<220>
<221>  misc_feature
<222>  (9)..(9)
<223>  Xaa can be any naturally occurring amino acid

<220>
<221>  misc_feature
<222>  (11)..(11)
<223>  Xaa can be any naturally occurring amino acid

<220>
<221>  misc_feature
<222>  (13)..(15)
<223>  Xaa can be any naturally occurring amino acid

<400>  609

Xaa Lys Xaa Xaa Ser Lys Ser Gln Xaa Ser Xaa Ser Xaa Xaa Xaa
1               5                   10                  15


<210>  610
<211>  5
<212>  PRT
<213>  Artificial Sequence

<220>
<223>  epitope

<400>  610

Lys Glu Asn Ser Lys
1               5


<210>  611
<211>  5
<212>  PRT
<213>  Artificial Sequence

<220>
<223>  epitope


<220>
<221>  misc_feature

<222>  (2)..(2)
<223>  Xaa can be any naturally occurring amino acid

<400>  611

Lys Xaa Asn Ser Lys
1               5


<210>  612
<211>  4
<212>  PRT
<213>  Artificial Sequence

<220>
<223>  epitope

<400>  612

Glu Asn Ser Lys
1


<210>  613
<211>  15
<212>  PRT
<213>  Artificial Sequence

<220>
<223>  epitope

<400>  613

Thr Lys Glu Asn Ser Lys Ser Gln Val Ser Leu Ser Val Ser Ser
1               5                   10                  15


<210>  614
<211>  15
<212>  PRT
<213>  Artificial Sequence

<220>
<223>  epitope

<400>  614

Thr Lys Glu Asn Ser Lys Ser Gln Val Ser Leu Ser Val Ser Ser
1               5                   10                  15


<210>  615
<211>  10
<212>  PRT
<213>  Artificial Sequence

<220>
<223>  epitope

<400>  615

Val Ser Leu Ser Val Ser Ser Val Thr Pro
1               5                   10

<210>  616
<211>  15
<212>  PRT
<213>  Artificial Sequence

<220>
<223>  epitope

<400>  616

Thr Lys Glu Asn Ser Lys Ser Gln Val Ser Leu Ser Val Ser Ser
1               5                   10                  15


<210>  617
<211>  15
<212>  PRT
<213>  Artificial Sequence

<220>
<223>  epitope


<220>
<221>  misc_feature
<222>  (1)..(2)
<223>  Xaa can be any naturally occurring amino acid

<220>
<221>  misc_feature
<222>  (9)..(15)
<223>  Xaa can be any naturally occurring amino acid

<400>  617

Xaa Xaa Glu Asn Ser Lys Ser Gln Xaa Xaa Xaa Xaa Xaa Xaa Xaa
1               5                   10                  15


<210>  618
<211>  15
<212>  PRT
<213>  Artificial Sequence

<220>
<223>  epitope


<220>
<221>  misc_feature
<222>  (1)..(3)
<223>  Xaa can be any naturally occurring amino acid

<220>
<221>  misc_feature
<222>  (8)..(15)
<223>  Xaa can be any naturally occurring amino acid

<400>  618

Xaa Xaa Xaa Asn Ser Lys Ser Xaa Xaa Xaa Xaa Xaa Xaa Xaa Xaa

1               5                   10                  15


<210>  619
<211>  15
<212>  PRT
<213>  Artificial Sequence

<220>
<223>  epitope

<400>  619

Thr Lys Glu Asn Ser Lys Ser Gln Val Ser Leu Ser Val Ser Ser
1               5                   10                  15


<210>  620
<211>  15
<212>  PRT
<213>  Artificial Sequence

<220>
<223>  epitope


<220>
<221>  misc_feature
<222>  (1)..(1)
<223>  Xaa can be any naturally occurring amino acid

<220>
<221>  misc_feature
<222>  (9)..(15)
<223>  Xaa can be any naturally occurring amino acid

<400>  620

Xaa Lys Glu Asn Ser Lys Ser Gln Xaa Xaa Xaa Xaa Xaa Xaa Xaa
1               5                   10                  15


<210>  621
<211>  15
<212>  PRT
<213>  Artificial Sequence

<220>
<223>  epitope

<400>  621

Thr Lys Glu Asn Ser Lys Ser Gln Val Ser Leu Ser Val Ser Ser
1               5                   10                  15


<210>  622
<211>  9
<212>  PRT
<213>  Artificial Sequence

<220>
<223>  epitope

<400>  622

Ser Leu Ser Val Ser Ser Val Thr Pro
1               5


<210>  623
<211>  15
<212>  PRT
<213>  Artificial Sequence

<220>
<223>  epitope

<400>  623

Thr Lys Glu Asn Ser Lys Ser Gln Val Ser Leu Ser Val Ser Ser
1               5                   10                  15


<210>  624
<211>  15
<212>  PRT
<213>  Artificial Sequence

<220>
<223>  epitope

<400>  624

Thr Lys Glu Asn Ser Lys Ser Gln Val Ser Leu Ser Val Ser Ser
1               5                   10                  15


<210>  625
<211>  15
<212>  PRT
<213>  Artificial Sequence

<220>
<223>  epitope


<220>
<221>  misc_feature
<222>  (1)..(1)
<223>  Xaa can be any naturally occurring amino acid

<220>
<221>  misc_feature
<222>  (3)..(4)
<223>  Xaa can be any naturally occurring amino acid

<220>
<221>  misc_feature
<222>  (7)..(13)
<223>  Xaa can be any naturally occurring amino acid

<400>  625

Xaa Lys Xaa Xaa Ser Lys Xaa Xaa Xaa Xaa Xaa Xaa Xaa Ser Ser
1               5                   10                  15

<210>  626
<211>  4
<212>  PRT
<213>  Artificial Sequence

<220>
<223>  epitope

<400>  626

Asn Ser Lys Ser
1


<210>  627
<211>  15
<212>  PRT
<213>  Artificial Sequence

<220>
<223>  epitope


<220>
<221>  misc_feature
<222>  (1)..(2)
<223>  Xaa can be any naturally occurring amino acid

<220>
<221>  misc_feature
<222>  (7)..(15)
<223>  Xaa can be any naturally occurring amino acid

<400>  627

Xaa Xaa Glu Asn Ser Lys Xaa Xaa Xaa Xaa Xaa Xaa Xaa Xaa Xaa
1               5                   10                  15


<210>  628
<211>  15
<212>  PRT
<213>  Artificial Sequence

<220>
<223>  epitope


<220>
<221>  misc_feature
<222>  (1)..(3)
<223>  Xaa can be any naturally occurring amino acid

<220>
<221>  misc_feature
<222>  (8)..(15)
<223>  Xaa can be any naturally occurring amino acid

<400>  628

Xaa Xaa Xaa Asn Ser Lys Ser Xaa Xaa Xaa Xaa Xaa Xaa Xaa Xaa

1                 5                   10                  15

<210>  629
<211>  15
<212>  PRT
<213>  Artificial Sequence

<220>
<223>  epitope


<220>
<221>  misc_feature
<222>  (1)..(2)
<223>  Xaa can be any naturally occurring amino acid

<220>
<221>  misc_feature
<222>  (4)..(4)
<223>  Xaa can be any naturally occurring amino acid

<220>
<221>  misc_feature
<222>  (7)..(7)
<223>  Xaa can be any naturally occurring amino acid

<220>
<221>  misc_feature
<222>  (9)..(10)
<223>  Xaa can be any naturally occurring amino acid

<400>  629

Xaa Xaa Glu Xaa Ser Lys Xaa Gln Xaa Xaa Leu Ser Val Ser Ser
1               5                   10                  15


<210>  630
<211>  15
<212>  PRT
<213>  Artificial Sequence

<220>
<223>  epitope


<220>
<221>  misc_feature
<222>  (1)..(2)
<223>  Xaa can be any naturally occurring amino acid

<220>
<221>  misc_feature
<222>  (4)..(4)
<223>  Xaa can be any naturally occurring amino acid

<220>
<221>  misc_feature
<222>  (7)..(10)
<223>  Xaa can be any naturally occurring amino acid

<220>

<221>  misc_feature
<222>  (12)..(12)
<223>  Xaa can be any naturally occurring amino acid

<400>  630

Xaa Xaa Glu Xaa Ser Lys Xaa Xaa Xaa Xaa Leu Xaa Val Ser Ser
1               5                   10                  15


<210>  631
<211>  15
<212>  PRT
<213>  Artificial Sequence

<220>
<223>  epitope


<220>
<221>  misc_feature
<222>  (2)..(2)
<223>  Xaa can be any naturally occurring amino acid

<220>
<221>  misc_feature
<222>  (7)..(15)
<223>  Xaa can be any naturally occurring amino acid

<400>  631

Thr Xaa Glu Asn Ser Lys Xaa Xaa Xaa Xaa Xaa Xaa Xaa Xaa Xaa
1               5                   10                  15


<210>  632
<211>  4
<212>  PRT
<213>  Artificial Sequence

<220>
<223>  epitope

<400>  632

Glu Asn Ser Lys
1


<210>  633
<211>  15
<212>  PRT
<213>  Artificial Sequence

<220>
<223>  epitope

<400>  633

Thr Thr Val Thr Gly Ala Lys Asp Ser Val Ala Ser Thr Ile Thr
1               5                   10                  15

<210>  634
<211>  15
<212>  PRT
<213>  Artificial Sequence

<220>
<223>  epitope

<400>  634

Thr Thr Val Thr Gly Ala Lys Asp Ser Val Ala Ser Thr Ile Thr
1               5                   10                  15


<210>  635
<211>  15
<212>  PRT
<213>  Artificial Sequence

<220>
<223>  epitope


<220>
<221>  misc_feature
<222>  (1)..(6)
<223>  Xaa can be any naturally occurring amino acid

<220>
<221>  misc_feature
<222>  (10)..(11)
<223>  Xaa can be any naturally occurring amino acid

<220>
<221>  misc_feature
<222>  (13)..(15)
<223>  Xaa can be any naturally occurring amino acid

<400>  635

Xaa Xaa Xaa Xaa Xaa Xaa Lys Asp Ser Xaa Xaa Ser Xaa Xaa Xaa
1               5                   10                  15


<210>  636
<211>  10
<212>  PRT
<213>  Artificial Sequence

<220>
<223>  epitope

<400>  636

Ala Val Thr Thr Thr Val Thr Gly Ala Lys
1               5                   10


<210>  637
<211>  9
<212>  PRT
<213>  Artificial Sequence

<220>
<223>  epitope

<400>  637

Ala Val Thr Thr Thr Val Thr Gly Ala
1               5


<210>  638
<211>  4
<212>  PRT
<213>  Artificial Sequence

<220>
<223>  epitope

<400>  638

Lys Asp Ser Val
1


<210>  639
<211>  15
<212>  PRT
<213>  Artificial Sequence

<220>
<223>  epitope

<400>  639

Thr Thr Val Thr Gly Ala Lys Asp Ser Val Ala Ser Thr Ile Thr
1               5                   10                  15


<210>  640
<211>  15
<212>  PRT
<213>  Artificial Sequence

<220>
<223>  epitope


<220>
<221>  misc_feature
<222>  (6)..(6)
<223>  Xaa can be any naturally occurring amino acid

<220>
<221>  misc_feature
<222>  (9)..(9)
<223>  Xaa can be any naturally occurring amino acid

<220>
<221>  misc_feature
<222>  (12)..(15)
<223>  Xaa can be any naturally occurring amino acid

<400>  640

Thr Thr Val Thr Gly Xaa Lys Asp Xaa Val Ala Xaa Xaa Xaa Xaa
1               5                   10                  15

<210>  641
<211>  15
<212>  PRT
<213>  Artificial Sequence

<220>
<223>  epitope

<220>
<221>  misc_feature
<222>  (2)..(2)
<223>  Xaa can be any naturally occurring amino acid

<220>
<221>  misc_feature
<222>  (4)..(4)
<223>  Xaa can be any naturally occurring amino acid

<220>
<221>  misc_feature
<222>  (6)..(6)
<223>  Xaa can be any naturally occurring amino acid

<220>
<221>  misc_feature
<222>  (8)..(9)
<223>  Xaa can be any naturally occurring amino acid

<220>
<221>  misc_feature
<222>  (11)..(15)
<223>  Xaa can be any naturally occurring amino acid

<400>  641

Thr Xaa Val Xaa Gly Xaa Lys Xaa Xaa Val Xaa Xaa Xaa Xaa Xaa
1               5                   10                  15

<210>  642
<211>  10
<212>  PRT
<213>  Artificial Sequence

<220>
<223>  epitope

<400>  642

Asp Ala Val Thr Thr Thr Val Thr Gly Ala
1               5                   10

<210>  643
<211>  9
<212>  PRT
<213>  Artificial Sequence

<220>
<223>  epitope

<400>  643

Ala Val Thr Thr Thr Val Thr Gly Ala
1               5


<210>  644
<211>  7
<212>  PRT
<213>  Artificial Sequence

<220>
<223>  epitope

<400>  644

Thr Thr Thr Val Thr Gly Ala
1               5


<210>  645
<211>  7
<212>  PRT
<213>  Artificial Sequence

<220>
<223>  epitope


<220>
<221>  misc_feature
<222>  (7)..(7)
<223>  Xaa can be any naturally occurring amino acid

<400>  645

Thr Thr Thr Val Thr Gly Xaa
1               5


<210>  646
<211>  7
<212>  PRT
<213>  Artificial Sequence

<220>
<223>  epitope


<220>
<221>  misc_feature
<222>  (3)..(3)
<223>  Xaa can be any naturally occurring amino acid

<220>
<221>  misc_feature
<222>  (5)..(5)
<223>  Xaa can be any naturally occurring amino acid

<220>

<221>  misc_feature
<222>  (7)..(7)
<223>  Xaa can be any naturally occurring amino acid

<400>  646

Thr Thr Xaa Val Xaa Gly Xaa
1               5


<210>  647
<211>  9
<212>  PRT
<213>  Artificial Sequence

<220>
<223>  epitope

<400>  647

Thr Gly Ala Lys Asp Ser Val Ala Ser
1               5


<210>  648
<211>  5
<212>  PRT
<213>  Artificial Sequence

<220>
<223>  epitope

<400>  648

Asp Ser Val Ala Ser
1               5


<210>  649
<211>  4
<212>  PRT
<213>  Artificial Sequence

<220>
<223>  epitope

<400>  649

Asp Ser Val Ala
1


<210>  650
<211>  15
<212>  PRT
<213>  Artificial Sequence

<220>
<223>  epitope

<400>  650

Thr Thr Val Thr Gly Ala Lys Asp Ser Val Ala Ser Thr Ile Thr

1               5                   10                  15


<210>  651
<211>  9
<212>  PRT
<213>  Artificial Sequence

<220>
<223>  epitope

<400>  651

Ala Val Thr Thr Thr Val Thr Gly Ala
1               5


<210>  652
<211>  6
<212>  PRT
<213>  Artificial Sequence

<220>
<223>  epitope

<400>  652

Asp Ser Val Ala Ser Thr
1               5


<210>  653
<211>  15
<212>  PRT
<213>  Artificial Sequence

<220>
<223>  epitope

<400>  653

Thr Thr Val Thr Gly Ala Lys Asp Ser Val Ala Ser Thr Ile Thr
1               5                   10                  15


<210>  654
<211>  15
<212>  PRT
<213>  Artificial Sequence

<220>
<223>  epitope

<400>  654

Thr Thr Val Thr Gly Ala Lys Asp Ser Val Ala Ser Thr Ile Thr
1               5                   10                  15


<210>  655
<211>  10
<212>  PRT
<213>  Artificial Sequence

<220>
<223>  epitope

<400>  655

Ala Val Thr Thr Thr Val Thr Gly Ala Lys
1               5                   10


<210>  656
<211>  7
<212>  PRT
<213>  Artificial Sequence

<220>
<223>  epitope

<400>  656

Thr Thr Thr Val Thr Gly Ala
1               5


<210>  657
<211>  10
<212>  PRT
<213>  Artificial Sequence

<220>
<223>  epitope

<400>  657

Thr Val Thr Gly Ala Lys Asp Ser Val Ala
1               5                   10


<210>  658
<211>  8
<212>  PRT
<213>  Artificial Sequence

<220>
<223>  epitope

<400>  658

Lys Asp Ser Val Ala Ser Thr Ile
1               5


<210>  659
<211>  6
<212>  PRT
<213>  Artificial Sequence

<220>
<223>  epitope

<400>  659

Asp Ser Val Ala Ser Thr

1               5


<210>  660
<211>  15
<212>  PRT
<213>  Artificial Sequence

<220>
<223>  epitope

<400>  660

Thr Thr Val Thr Gly Ala Lys Asp Ser Val Ala Ser Thr Ile Thr
1               5                   10                  15


<210>  661
<211>  15
<212>  PRT
<213>  Artificial Sequence

<220>
<223>  epitope


<220>
<221>  misc_feature
<222>  (1)..(6)
<223>  Xaa can be any naturally occurring amino acid

<220>
<221>  misc_feature
<222>  (8)..(9)
<223>  Xaa can be any naturally occurring amino acid

<220>
<221>  misc_feature
<222>  (13)..(13)
<223>  Xaa can be any naturally occurring amino acid

<220>
<221>  misc_feature
<222>  (15)..(15)
<223>  Xaa can be any naturally occurring amino acid

<400>  661

Xaa Xaa Xaa Xaa Xaa Xaa Lys Xaa Xaa Val Ala Ser Xaa Ile Xaa
1               5                   10                  15


<210>  662
<211>  9
<212>  PRT
<213>  Artificial Sequence

<220>
<223>  epitope

<400>  662

Ala Val Thr Thr Thr Val Thr Gly Ala

1               5


<210>  663
<211>  8
<212>  PRT
<213>  Artificial Sequence

<220>
<223>  epitope

<400>  663

Lys Asp Ser Val Ala Ser Thr Ile
1               5


<210>  664
<211>  8
<212>  PRT
<213>  Artificial Sequence

<220>
<223>  epitope


<220>
<221>  misc_feature
<222>  (2)..(3)
<223>  Xaa can be any naturally occurring amino acid

<220>
<221>  misc_feature
<222>  (7)..(7)
<223>  Xaa can be any naturally occurring amino acid

<400>  664

Lys Xaa Xaa Val Ala Ser Xaa Ile
1               5


<210>  665
<211>  15
<212>  PRT
<213>  Artificial Sequence

<220>
<223>  epitope


<220>
<221>  misc_feature
<222>  (1)..(4)
<223>  Xaa can be any naturally occurring amino acid

<220>
<221>  misc_feature
<222>  (6)..(6)
<223>  Xaa can be any naturally occurring amino acid

<220>
<221>  misc_feature

<222>  (12)..(15)
<223>  Xaa can be any naturally occurring amino acid

<400>  665

Xaa Xaa Xaa Xaa Gly Xaa Lys Asp Ser Val Ala Xaa Xaa Xaa Xaa
1               5                   10                  15


<210>  666
<211>  15
<212>  PRT
<213>  Artificial Sequence

<220>
<223>  epitope


<220>
<221>  misc_feature
<222>  (1)..(6)
<223>  Xaa can be any naturally occurring amino acid

<220>
<221>  misc_feature
<222>  (12)..(15)
<223>  Xaa can be any naturally occurring amino acid

<400>  666

Xaa Xaa Xaa Xaa Xaa Xaa Lys Asp Ser Val Ala Xaa Xaa Xaa Xaa
1               5                   10                  15


<210>  667
<211>  15
<212>  PRT
<213>  Artificial Sequence

<220>
<223>  epitope


<220>
<221>  misc_feature
<222>  (1)..(6)
<223>  Xaa can be any naturally occurring amino acid

<220>
<221>  misc_feature
<222>  (10)..(10)
<223>  Xaa can be any naturally occurring amino acid

<220>
<221>  misc_feature
<222>  (12)..(15)
<223>  Xaa can be any naturally occurring amino acid

<400>  667

Xaa Xaa Xaa Xaa Xaa Xaa Lys Asp Ser Xaa Ala Xaa Xaa Xaa Xaa
1               5                   10                  15

<210>  668
<211>  5
<212>  PRT
<213>  Artificial Sequence

<220>
<223>  epitope

<400>  668

Lys Asp Ser Val Ala
1               5


<210>  669
<211>  4
<212>  PRT
<213>  Artificial Sequence

<220>
<223>  epitope

<400>  669

Lys Asp Ser Val
1


<210>  670
<211>  15
<212>  PRT
<213>  Artificial Sequence

<220>
<223>  epitope


<220>
<221>  misc_feature
<222>  (2)..(3)
<223>  Xaa can be any naturally occurring amino acid

<220>
<221>  misc_feature
<222>  (8)..(8)
<223>  Xaa can be any naturally occurring amino acid

<220>
<221>  misc_feature
<222>  (10)..(15)
<223>  Xaa can be any naturally occurring amino acid

<400>  670

Thr Xaa Xaa Thr Gly Ala Lys Xaa Ser Xaa Xaa Xaa Xaa Xaa Xaa
1               5                   10                  15


<210>  671
<211>  15
<212>  PRT
<213>  Artificial Sequence

<220>
<223>  epitope

<220>
<221>  misc_feature
<222>  (2)..(3)
<223>  Xaa can be any naturally occurring amino acid

<220>
<221>  misc_feature
<222>  (6)..(6)
<223>  Xaa can be any naturally occurring amino acid

<220>
<221>  misc_feature
<222>  (8)..(8)
<223>  Xaa can be any naturally occurring amino acid

<220>
<221>  misc_feature
<222>  (10)..(15)
<223>  Xaa can be any naturally occurring amino acid

<400>  671

Thr Xaa Xaa Thr Gly Xaa Lys Xaa Ser Xaa Xaa Xaa Xaa Xaa Xaa
1               5                   10                  15

<210>  672
<211>  7
<212>  PRT
<213>  Artificial Sequence

<220>
<223>  epitope

<400>  672

Gly Ala Lys Asp Ser Val Ala
1               5

<210>  673
<211>  7
<212>  PRT
<213>  Artificial Sequence

<220>
<223>  epitope

<220>
<221>  misc_feature
<222>  (4)..(4)
<223>  Xaa can be any naturally occurring amino acid

<220>
<221>  misc_feature
<222>  (6)..(7)
<223>  Xaa can be any naturally occurring amino acid

<400>  673

Gly Ala Lys Xaa Ser Xaa Xaa
1               5


<210>  674
<211>  15
<212>  PRT
<213>  Artificial Sequence

<220>
<223>  epitope

<400>  674

Asn Gln Lys Ile Gln Asp Ala Gln Asp Lys Leu Tyr Leu Ser Trp
1               5                   10                  15


<210>  675
<211>  15
<212>  PRT
<213>  Artificial Sequence

<220>
<223>  epitope

<400>  675

Asn Gln Lys Ile Gln Asp Ala Gln Asp Lys Leu Tyr Leu Ser Trp
1               5                   10                  15


<210>  676
<211>  10
<212>  PRT
<213>  Artificial Sequence

<220>
<223>  epitope

<400>  676

Ala Gln Asp Lys Leu Tyr Leu Ser Trp Leu
1               5                   10


<210>  677
<211>  15
<212>  PRT
<213>  Artificial Sequence

<220>
<223>  epitope

<400>  677

Asn Gln Lys Ile Gln Asp Ala Gln Asp Lys Leu Tyr Leu Ser Trp
1               5                   10                  15

<210>  678
<211>  15
<212>  PRT
<213>  Artificial Sequence

<220>
<223>  epitope

<400>  678

Asn Gln Lys Ile Gln Asp Ala Gln Asp Lys Leu Tyr Leu Ser Trp
1               5                   10                  15


<210>  679
<211>  15
<212>  PRT
<213>  Artificial Sequence

<220>
<223>  epitope


<220>
<221>  misc_feature
<222>  (1)..(1)
<223>  Xaa can be any naturally occurring amino acid

<220>
<221>  misc_feature
<222>  (4)..(9)
<223>  Xaa can be any naturally occurring amino acid

<400>  679

Xaa Gln Lys Xaa Xaa Xaa Xaa Xaa Xaa Lys Leu Tyr Leu Ser Trp
1               5                   10                  15


<210>  680
<211>  15
<212>  PRT
<213>  Artificial Sequence

<220>
<223>  epitope


<220>
<221>  misc_feature
<222>  (1)..(1)
<223>  Xaa can be any naturally occurring amino acid

<220>
<221>  misc_feature
<222>  (4)..(10)
<223>  Xaa can be any naturally occurring amino acid

<400>  680

Xaa Gln Lys Xaa Xaa Xaa Xaa Xaa Xaa Xaa Leu Tyr Leu Ser Trp
1               5                   10                  15

<210>  681
<211>  10
<212>  PRT
<213>  Artificial Sequence

<220>
<223>  epitope

<400>  681

Ala Gln Asp Lys Leu Tyr Leu Ser Trp Leu
1               5                   10


<210>  682
<211>  10
<212>  PRT
<213>  Artificial Sequence

<220>
<223>  epitope


<220>
<221>  misc_feature
<222>  (1)..(3)
<223>  Xaa can be any naturally occurring amino acid

<400>  682

Xaa Xaa Xaa Lys Leu Tyr Leu Ser Trp Leu
1               5                   10


<210>  683
<211>  10
<212>  PRT
<213>  Artificial Sequence

<220>
<223>  epitope


<220>
<221>  misc_feature
<222>  (1)..(4)
<223>  Xaa can be any naturally occurring amino acid

<400>  683

Xaa Xaa Xaa Xaa Leu Tyr Leu Ser Trp Leu
1               5                   10


<210>  684
<211>  15
<212>  PRT
<213>  Artificial Sequence

<220>
<223>  epitope

<400>  684

Asn Gln Lys Ile Gln Asp Ala Gln Asp Lys Leu Tyr Leu Ser Trp
1               5                   10                  15


<210>  685
<211>  15
<212>  PRT
<213>  Artificial Sequence

<220>
<223>  epitope

<400>  685

Asn Gln Lys Ile Gln Asp Ala Gln Asp Lys Leu Tyr Leu Ser Trp
1               5                   10                  15


<210>  686
<211>  10
<212>  PRT
<213>  Artificial Sequence

<220>
<223>  epitope

<400>  686

Asp Lys Leu Tyr Leu Ser Trp Leu Glu Trp
1               5                   10


<210>  687
<211>  15
<212>  PRT
<213>  Artificial Sequence

<220>
<223>  epitope


<220>
<221>  misc_feature
<222>  (6)..(15)
<223>  Xaa can be any naturally occurring amino acid

<400>  687

Asn Gln Lys Ile Gln Xaa Xaa Xaa Xaa Xaa Xaa Xaa Xaa Xaa Xaa
1               5                   10                  15


<210>  688
<211>  15
<212>  PRT
<213>  Artificial Sequence

<220>
<223>  epitope

<220>
<221>  misc_feature
<222>  (6)..(7)
<223>  Xaa can be any naturally occurring amino acid

<220>
<221>  misc_feature
<222>  (9)..(9)
<223>  Xaa can be any naturally occurring amino acid

<220>
<221>  misc_feature
<222>  (15)..(15)
<223>  Xaa can be any naturally occurring amino acid

<400>  688

Asn Gln Lys Ile Gln Xaa Xaa Gln Xaa Lys Leu Tyr Leu Ser Xaa
1               5                   10                  15


<210>  689
<211>  15
<212>  PRT
<213>  Artificial Sequence

<220>
<223>  epitope


<220>
<221>  misc_feature
<222>  (1)..(1)
<223>  Xaa can be any naturally occurring amino acid

<220>
<221>  misc_feature
<222>  (6)..(9)
<223>  Xaa can be any naturally occurring amino acid

<220>
<221>  misc_feature
<222>  (15)..(15)
<223>  Xaa can be any naturally occurring amino acid

<400>  689

Xaa Gln Lys Ile Gln Xaa Xaa Xaa Xaa Lys Leu Tyr Leu Ser Xaa
1               5                   10                  15


<210>  690
<211>  15
<212>  PRT
<213>  Artificial Sequence

<220>
<223>  epitope


<220>
<221>  misc_feature
<222>  (1)..(1)

<223>  Xaa can be any naturally occurring amino acid

<220>
<221>  misc_feature
<222>  (6)..(11)
<223>  Xaa can be any naturally occurring amino acid

<220>
<221>  misc_feature
<222>  (14)..(15)
<223>  Xaa can be any naturally occurring amino acid

<400>  690

Xaa Gln Lys Ile Gln Xaa Xaa Xaa Xaa Xaa Xaa Tyr Leu Xaa Xaa
1               5                   10                  15


<210>  691
<211>  8
<212>  PRT
<213>  Artificial Sequence

<220>
<223>  epitope

<400>  691

Gln Asp Lys Leu Tyr Leu Ser Trp
1               5


<210>  692
<211>  8
<212>  PRT
<213>  Artificial Sequence

<220>
<223>  epitope



<220>
<221>  misc_feature
<222>  (2)..(2)
<223>  Xaa can be any naturally occurring amino acid

<220>
<221>  misc_feature
<222>  (8)..(8)
<223>  Xaa can be any naturally occurring amino acid

<400>  692

Gln Xaa Lys Leu Tyr Leu Ser Xaa
1               5


<210>  693
<211>  8
<212>  PRT
<213>  Artificial Sequence

<220>

<223>  epitope

<220>
<221>  misc_feature
<222>  (1)..(2)
<223>  Xaa can be any naturally occurring amino acid

<220>
<221>  misc_feature
<222>  (8)..(8)
<223>  Xaa can be any naturally occurring amino acid

<400>  693

Xaa Xaa Lys Leu Tyr Leu Ser Xaa
1               5

<210>  694
<211>  15
<212>  PRT
<213>  Artificial Sequence

<220>
<223>  epitope

<400>  694

Glu Asp Trp Glu Lys Glu Ser Ala Leu Gln Arg Ala Arg Glu Asp
1               5                   10                  15

<210>  695
<211>  15
<212>  PRT
<213>  Artificial Sequence

<220>
<223>  epitope

<400>  695

Glu Asp Trp Glu Lys Glu Ser Ala Leu Gln Arg Ala Arg Glu Asp
1               5                   10                  15

<210>  696
<211>  15
<212>  PRT
<213>  Artificial Sequence

<220>
<223>  epitope

<220>
<221>  misc_feature
<222>  (1)..(2)
<223>  Xaa can be any naturally occurring amino acid

<220>
<221>  misc_feature

<222>  (7)..(8)
<223>  Xaa can be any naturally occurring amino acid

<220>
<221>  misc_feature
<222>  (11)..(13)
<223>  Xaa can be any naturally occurring amino acid

<400>  696

Xaa Xaa Trp Glu Lys Glu Xaa Xaa Leu Gln Xaa Xaa Xaa Glu Asp
1               5                   10                  15


<210>  697
<211>  15
<212>  PRT
<213>  Artificial Sequence

<220>
<223>  epitope



<220>
<221>  misc_feature
<222>  (1)..(2)
<223>  Xaa can be any naturally occurring amino acid

<220>
<221>  misc_feature
<222>  (7)..(15)
<223>  Xaa can be any naturally occurring amino acid

<400>  697

Xaa Xaa Trp Glu Lys Glu Xaa Xaa Xaa Xaa Xaa Xaa Xaa Xaa Xaa
1               5                   10                  15


<210>  698
<211>  7
<212>  PRT
<213>  Artificial Sequence

<220>
<223>  epitope

<400>  698

Trp Glu Lys Glu Ser Ala Leu
1               5


<210>  699
<211>  7
<212>  PRT
<213>  Artificial Sequence

<220>
<223>  epitope


<220>

<221>  misc_feature
<222>  (5)..(6)
<223>  Xaa can be any naturally occurring amino acid

<400>  699

Trp Glu Lys Glu Xaa Xaa Leu
1               5


<210>  700
<211>  7
<212>  PRT
<213>  Artificial Sequence

<220>
<223>  epitope


<220>
<221>  misc_feature
<222>  (5)..(7)
<223>  Xaa can be any naturally occurring amino acid

<400>  700

Trp Glu Lys Glu Xaa Xaa Xaa
1               5


<210>  701
<211>  6
<212>  PRT
<213>  Artificial Sequence

<220>
<223>  epitope

<400>  701

Glu Lys Glu Ser Ala Leu
1               5


<210>  702
<211>  6
<212>  PRT
<213>  Artificial Sequence

<220>
<223>  epitope


<220>
<221>  misc_feature
<222>  (4)..(5)
<223>  Xaa can be any naturally occurring amino acid

<400>  702

Glu Lys Glu Xaa Xaa Leu
1               5

<210>  703
<211>  15
<212>  PRT
<213>  Artificial Sequence

<220>
<223>  epitope

<400>  703

Glu Asp Trp Glu Lys Glu Ser Ala Leu Gln Arg Ala Arg Glu Asp
1               5                   10                  15


<210>  704
<211>  15
<212>  PRT
<213>  Artificial Sequence

<220>
<223>  epitope


<220>
<221>  misc_feature
<222>  (1)..(2)
<223>  Xaa can be any naturally occurring amino acid

<220>
<221>  misc_feature
<222>  (7)..(15)
<223>  Xaa can be any naturally occurring amino acid

<400>  704

Xaa Xaa Trp Glu Lys Glu Xaa Xaa Xaa Xaa Xaa Xaa Xaa Xaa Xaa
1               5                   10                  15


<210>  705
<211>  10
<212>  PRT
<213>  Artificial Sequence

<220>
<223>  epitope

<400>  705

Met Glu Asp Trp Glu Lys Glu Ser Ala Leu
1               5                   10


<210>  706
<211>  10
<212>  PRT
<213>  Artificial Sequence

<220>
<223>  epitope

<400>  706

Asp Trp Glu Lys Glu Ser Ala Leu Gln Arg
1               5                   10


<210>  707
<211>  8
<212>  PRT
<213>  Artificial Sequence

<220>
<223>  epitope

<400>  707

Asp Trp Glu Lys Glu Ser Ala Leu
1               5


<210>  708
<211>  7
<212>  PRT
<213>  Artificial Sequence

<220>
<223>  epitope

<400>  708

Asp Trp Glu Lys Glu Ser Ala
1               5


<210>  709
<211>  7
<212>  PRT
<213>  Artificial Sequence

<220>
<223>  epitope


<220>
<221>  misc_feature
<222>  (1)..(1)
<223>  Xaa can be any naturally occurring amino acid

<220>
<221>  misc_feature
<222>  (6)..(7)
<223>  Xaa can be any naturally occurring amino acid

<400>  709

Xaa Trp Glu Lys Glu Xaa Xaa
1               5


<210>  710
<211>  15
<212>  PRT
<213>  Artificial Sequence

<220>
<223>  epitope

<400>  710

Glu Asp Trp Glu Lys Glu Ser Ala Leu Gln Arg Ala Arg Glu Asp
1               5                   10                  15


<210>  711
<211>  4
<212>  PRT
<213>  Artificial Sequence

<220>
<223>  epitope

<400>  711

Trp Glu Lys Glu
1


<210>  712
<211>  15
<212>  PRT
<213>  Artificial Sequence

<220>
<223>  epitope

<400>  712

Glu Asp Trp Glu Lys Glu Ser Ala Leu Gln Arg Ala Arg Glu Asp
1               5                   10                  15


<210>  713
<211>  8
<212>  PRT
<213>  Artificial Sequence

<220>
<223>  epitope

<400>  713

Asp Trp Glu Lys Glu Ser Ala Leu
1               5


<210>  714
<211>  8
<212>  PRT
<213>  Artificial Sequence

<220>
<223>  epitope

<400>  714

Asp Trp Glu Lys Glu Ser Ala Leu
1               5

<210>  715
<211>  5
<212>  PRT
<213>  Artificial Sequence

<220>
<223>  epitope

<400>  715

Trp Glu Lys Glu Ser
1               5


<210>  716
<211>  4
<212>  PRT
<213>  Artificial Sequence

<220>
<223>  epitope

<400>  716

Trp Glu Lys Glu
1


<210>  717
<211>  15
<212>  PRT
<213>  Artificial Sequence

<220>
<223>  epitope

<400>  717

Glu Asp Trp Glu Lys Glu Ser Ala Leu Gln Arg Ala Arg Glu Asp
1               5                   10                  15


<210>  718
<211>  15
<212>  PRT
<213>  Artificial Sequence

<220>
<223>  epitope


<220>
<221>  misc_feature
<222>  (1)..(4)
<223>  Xaa can be any naturally occurring amino acid

<220>
<221>  misc_feature
<222>  (7)..(7)
<223>  Xaa can be any naturally occurring amino acid

<220>
<221>  misc_feature
<222>  (10)..(10)
<223>  Xaa can be any naturally occurring amino acid

<220>
<221>  misc_feature
<222>  (12)..(12)
<223>  Xaa can be any naturally occurring amino acid

<220>
<221>  misc_feature
<222>  (14)..(14)
<223>  Xaa can be any naturally occurring amino acid

<400>  718

Xaa Xaa Xaa Xaa Lys Glu Xaa Ala Leu Xaa Arg Xaa Arg Xaa Asp
1               5                   10                  15


<210>  719
<211>  7
<212>  PRT
<213>  Artificial Sequence

<220>
<223>  epitope

<400>  719

Trp Glu Lys Glu Ser Ala Leu
1               5


<210>  720
<211>  5
<212>  PRT
<213>  Artificial Sequence

<220>
<223>  epitope

<400>  720

Trp Glu Lys Glu Ser
1               5


<210>  721
<211>  10
<212>  PRT
<213>  Artificial Sequence

<220>
<223>  epitope

<400>  721

Lys Glu Ser Ala Leu Gln Arg Ala Arg Glu
1               5                   10

<210>  722
<211>  10
<212>  PRT
<213>  Artificial Sequence

<220>
<223>  epitope


<220>
<221>  misc_feature
<222>  (3)..(3)
<223>  Xaa can be any naturally occurring amino acid

<220>
<221>  misc_feature
<222>  (6)..(6)
<223>  Xaa can be any naturally occurring amino acid

<220>
<221>  misc_feature
<222>  (8)..(8)
<223>  Xaa can be any naturally occurring amino acid

<220>
<221>  misc_feature
<222>  (10)..(10)
<223>  Xaa can be any naturally occurring amino acid

<400>  722

Lys Glu Xaa Ala Leu Xaa Arg Xaa Arg Xaa
1               5                   10


<210>  723
<211>  15
<212>  PRT
<213>  Artificial Sequence

<220>
<223>  epitope

<400>  723

Glu Asp Trp Glu Lys Glu Ser Ala Leu Gln Arg Ala Arg Glu Asp
1               5                   10                  15


<210>  724
<211>  6
<212>  PRT
<213>  Artificial Sequence

<220>
<223>  epitope

<400>  724

Trp Glu Lys Glu Ser Ala
1               5

<210>  725
<211>  4
<212>  PRT
<213>  Artificial Sequence

<220>
<223>  epitope

<400>  725

Trp Glu Lys Glu
1


<210>  726
<211>  10
<212>  PRT
<213>  Artificial Sequence

<220>
<223>  epitope

<400>  726

Lys Glu Ser Ala Leu Gln Arg Ala Arg Glu
1               5                   10


<210>  727
<211>  15
<212>  PRT
<213>  Artificial Sequence

<220>
<223>  epitope

<400>  727

Glu Asp Trp Glu Lys Glu Ser Ala Leu Gln Arg Ala Arg Glu Asp
1               5                   10                  15


<210>  728
<211>  7
<212>  PRT
<213>  Artificial Sequence

<220>
<223>  epitope

<400>  728

Met Glu Asp Trp Glu Lys Glu
1               5


<210>  729
<211>  7
<212>  PRT
<213>  Artificial Sequence

<220>
<223>  epitope

<400>  729

Lys Glu Ser Ala Leu Gln Arg
1               5

<210>  730
<211>  10
<212>  PRT
<213>  Artificial Sequence

<220>
<223>  epitope

<400>  730

Leu Gln Arg Ala Arg Glu Asp Ile Phe Met
1               5                   10

<210>  731
<211>  15
<212>  PRT
<213>  Artificial Sequence

<220>
<223>  epitope

<400>  731

Glu Asp Trp Glu Lys Glu Ser Ala Leu Gln Arg Ala Arg Glu Asp
1               5                   10                  15

<210>  732
<211>  4
<212>  PRT
<213>  Artificial Sequence

<220>
<223>  epitope

<400>  732

Trp Glu Lys Glu
1

<210>  733
<211>  15
<212>  PRT
<213>  Artificial Sequence

<220>
<223>  epitope

<220>
<221>  misc_feature
<222>  (1)..(4)
<223>  Xaa can be any naturally occurring amino acid

<220>
<221>  misc_feature
<222>  (7)..(7)
<223>  Xaa can be any naturally occurring amino acid

<220>
<221>  misc_feature
<222>  (9)..(10)
<223>  Xaa can be any naturally occurring amino acid

<220>
<221>  misc_feature
<222>  (12)..(12)
<223>  Xaa can be any naturally occurring amino acid

<220>
<221>  misc_feature
<222>  (14)..(14)
<223>  Xaa can be any naturally occurring amino acid

<400>  733

Xaa Xaa Xaa Xaa Lys Glu Xaa Ala Xaa Xaa Arg Xaa Arg Xaa Asp
1               5                   10                  15


<210>  734
<211>  10
<212>  PRT
<213>  Artificial Sequence

<220>
<223>  epitope

<400>  734

Asp Trp Glu Lys Glu Ser Ala Leu Gln Arg
1               5                   10


<210>  735
<211>  9
<212>  PRT
<213>  Artificial Sequence

<220>
<223>  epitope

<400>  735

Trp Glu Lys Glu Ser Ala Leu Gln Arg
1               5


<210>  736
<211>  7
<212>  PRT
<213>  Artificial Sequence

<220>
<223>  epitope

<400>  736

Trp Glu Lys Glu Ser Ala Leu
1               5

<210>  737
<211>  10
<212>  PRT
<213>  Artificial Sequence

<220>
<223>  epitope

<400>  737

Lys Glu Ser Ala Leu Gln Arg Ala Arg Glu
1               5                   10

<210>  738
<211>  10
<212>  PRT
<213>  Artificial Sequence

<220>
<223>  epitope

<220>
<221>  misc_feature
<222>  (3)..(3)
<223>  Xaa can be any naturally occurring amino acid

<220>
<221>  misc_feature
<222>  (5)..(6)
<223>  Xaa can be any naturally occurring amino acid

<220>
<221>  misc_feature
<222>  (8)..(8)
<223>  Xaa can be any naturally occurring amino acid

<220>
<221>  misc_feature
<222>  (10)..(10)
<223>  Xaa can be any naturally occurring amino acid

<400>  738

Lys Glu Xaa Ala Xaa Xaa Arg Xaa Arg Xaa
1               5                   10

<210>  739
<211>  7
<212>  PRT
<213>  Artificial Sequence

<220>
<223>  epitope

<400>  739

Trp Glu Lys Glu Ser Ala Leu
1               5

<210>  740
<211>  6
<212>  PRT
<213>  Artificial Sequence

<220>
<223>  epitope

<400>  740

Glu Lys Glu Ser Ala Leu
1               5

<210>  741
<211>  15
<212>  PRT
<213>  Artificial Sequence

<220>
<223>  epitope

<220>
<221>  misc_feature
<222>  (1)..(2)
<223>  Xaa can be any naturally occurring amino acid

<220>
<221>  misc_feature
<222>  (7)..(12)
<223>  Xaa can be any naturally occurring amino acid

<400>  741

Xaa Xaa Trp Glu Lys Glu Xaa Xaa Xaa Xaa Xaa Xaa Arg Glu Asp
1               5                   10                  15

<210>  742
<211>  15
<212>  PRT
<213>  Artificial Sequence

<220>
<223>  epitope

<220>
<221>  misc_feature
<222>  (1)..(4)
<223>  Xaa can be any naturally occurring amino acid

<220>
<221>  misc_feature
<222>  (6)..(12)
<223>  Xaa can be any naturally occurring amino acid

<400>  742

Xaa Xaa Xaa Xaa Lys Xaa Xaa Xaa Xaa Xaa Xaa Xaa Arg Glu Asp
1               5                   10                  15


<210>  743
<211>  9
<212>  PRT
<213>  Artificial Sequence

<220>
<223>  epitope

<400>  743

Trp Glu Lys Glu Ser Ala Leu Gln Arg
1               5


<210>  744
<211>  5
<212>  PRT
<213>  Artificial Sequence

<220>
<223>  epitope

<400>  744

Lys Glu Ser Ala Leu
1               5


<210>  745
<211>  15
<212>  PRT
<213>  Artificial Sequence

<220>
<223>  epitope


<220>
<221>  misc_feature
<222>  (1)..(2)
<223>  Xaa can be any naturally occurring amino acid

<220>
<221>  misc_feature
<222>  (7)..(12)
<223>  Xaa can be any naturally occurring amino acid

<220>
<221>  misc_feature
<222>  (14)..(15)
<223>  Xaa can be any naturally occurring amino acid

<400>  745

Xaa Xaa Trp Glu Lys Glu Xaa Xaa Xaa Xaa Xaa Xaa Arg Xaa Xaa
1               5                   10                  15

<210>  746
<211>  10
<212>  PRT
<213>  Artificial Sequence

<220>
<223>  epitope

<400>  746

Asp Trp Glu Lys Glu Ser Ala Leu Gln Arg
1               5                   10


<210>  747
<211>  10
<212>  PRT
<213>  Artificial Sequence

<220>
<223>  epitope


<220>
<221>  misc_feature
<222>  (1)..(1)
<223>  Xaa can be any naturally occurring amino acid

<220>
<221>  misc_feature
<222>  (6)..(10)
<223>  Xaa can be any naturally occurring amino acid

<400>  747

Xaa Trp Glu Lys Glu Xaa Xaa Xaa Xaa Xaa
1               5                   10


<210>  748
<211>  10
<212>  PRT
<213>  Artificial Sequence

<220>
<223>  epitope

<400>  748

Asp Trp Glu Lys Glu Ser Ala Leu Gln Arg
1               5                   10


<210>  749
<211>  8
<212>  PRT
<213>  Artificial Sequence

<220>
<223>  epitope

<400>  749

Glu Ser Ala Leu Gln Arg Ala Arg
1               5


<210>  750
<211>  5
<212>  PRT
<213>  Artificial Sequence

<220>
<223>  epitope

<400>  750

Trp Glu Lys Glu Ser
1               5


<210>  751
<211>  4
<212>  PRT
<213>  Artificial Sequence

<220>
<223>  epitope

<400>  751

Trp Glu Lys Glu
1


<210>  752
<211>  15
<212>  PRT
<213>  Artificial Sequence

<220>
<223>  epitope


<220>
<221>  misc_feature
<222>  (1)..(2)
<223>  Xaa can be any naturally occurring amino acid

<220>
<221>  misc_feature
<222>  (7)..(7)
<223>  Xaa can be any naturally occurring amino acid

<220>
<221>  misc_feature
<222>  (9)..(12)
<223>  Xaa can be any naturally occurring amino acid

<220>
<221>  misc_feature
<222>  (14)..(15)
<223>  Xaa can be any naturally occurring amino acid

<400>  752

Xaa Xaa Trp Glu Lys Glu Xaa Ala Xaa Xaa Xaa Xaa Arg Xaa Xaa
1               5                   10                  15


<210>  753
<211>  15
<212>  PRT
<213>  Artificial Sequence

<220>
<223>  epitope



<220>
<221>  misc_feature
<222>  (1)..(2)
<223>  Xaa can be any naturally occurring amino acid

<220>
<221>  misc_feature
<222>  (7)..(15)
<223>  Xaa can be any naturally occurring amino acid

<400>  753

Xaa Xaa Trp Glu Lys Glu Xaa Xaa Xaa Xaa Xaa Xaa Xaa Xaa Xaa
1               5                   10                  15


<210>  754
<211>  9
<212>  PRT
<213>  Artificial Sequence

<220>
<223>  epitope

<400>  754

Trp Glu Lys Glu Ser Ala Leu Gln Arg
1               5


<210>  755
<211>  7
<212>  PRT
<213>  Artificial Sequence

<220>
<223>  epitope

<400>  755

Trp Glu Lys Glu Ser Ala Leu
1               5


<210>  756
<211>  7
<212>  PRT
<213>  Artificial Sequence

<220>
<223>  epitope


<220>
<221>  misc_feature
<222>  (5)..(5)
<223>  Xaa can be any naturally occurring amino acid

<220>
<221>  misc_feature
<222>  (7)..(7)
<223>  Xaa can be any naturally occurring amino acid

<400>  756

Trp Glu Lys Glu Xaa Ala Xaa
1               5


<210>  757
<211>  7
<212>  PRT
<213>  Artificial Sequence

<220>
<223>  epitope


<220>
<221>  misc_feature
<222>  (5)..(7)
<223>  Xaa can be any naturally occurring amino acid

<400>  757

Trp Glu Lys Glu Xaa Xaa Xaa
1               5


<210>  758
<211>  15
<212>  PRT
<213>  Artificial Sequence

<220>
<223>  epitope

<400>  758

Leu Ser Asp Ile Met Asp Tyr Tyr Lys Asp Arg Glu Gly Ser His
1               5                   10                  15


<210>  759
<211>  15
<212>  PRT
<213>  Artificial Sequence

<220>
<223>  epitope

<400>  759

Leu Ser Asp Ile Met Asp Tyr Tyr Lys Asp Arg Glu Gly Ser His
1               5                   10                  15


<210>  760
<211>  6
<212>  PRT
<213>  Artificial Sequence

<220>
<223>  epitope

<400>  760

Lys Asp Arg Glu Gly Ser
1               5


<210>  761
<211>  15
<212>  PRT
<213>  Artificial Sequence

<220>
<223>  epitope

<400>  761

Leu Ser Asp Ile Met Asp Tyr Tyr Lys Asp Arg Glu Gly Ser His
1               5                   10                  15


<210>  762
<211>  15
<212>  PRT
<213>  Artificial Sequence

<220>
<223>  epitope


<220>
<221>  misc_feature
<222>  (1)..(8)
<223>  Xaa can be any naturally occurring amino acid

<220>
<221>  misc_feature
<222>  (10)..(10)
<223>  Xaa can be any naturally occurring amino acid

<400>  762

Xaa Xaa Xaa Xaa Xaa Xaa Xaa Xaa Lys Xaa Arg Glu Gly Ser His
1               5                   10                  15


<210>  763
<211>  15
<212>  PRT
<213>  Artificial Sequence

<220>
<223>  epitope


<220>
<221>  misc_feature
<222>  (1)..(8)
<223>  Xaa can be any naturally occurring amino acid

<220>
<221>  misc_feature
<222>  (10)..(11)
<223>  Xaa can be any naturally occurring amino acid

<400>  763

Xaa Xaa Xaa Xaa Xaa Xaa Xaa Xaa Lys Xaa Xaa Glu Gly Ser His
1               5                   10                  15


<210>  764
<211>  6
<212>  PRT
<213>  Artificial Sequence

<220>
<223>  epitope

<400>  764

Lys Asp Arg Glu Gly Ser
1               5


<210>  765
<211>  5
<212>  PRT
<213>  Artificial Sequence

<220>
<223>  epitope

<400>  765

Lys Asp Arg Glu Gly
1               5


<210>  766
<211>  5
<212>  PRT
<213>  Artificial Sequence

<220>
<223>  epitope


<220>
<221>  misc_feature
<222>  (2)..(2)
<223>  Xaa can be any naturally occurring amino acid

<400>  766

Lys Xaa Arg Glu Gly
1               5


<210>  767
<211>  4
<212>  PRT
<213>  Artificial Sequence

<220>
<223>  epitope

<400>  767

Asp Arg Glu Gly
1


<210>  768
<211>  15
<212>  PRT
<213>  Artificial Sequence

<220>
<223>  epitope

<400>  768

Leu Ser Asp Ile Met Asp Tyr Tyr Lys Asp Arg Glu Gly Ser His
1               5                   10                  15


<210>  769
<211>  15
<212>  PRT
<213>  Artificial Sequence

<220>
<223>  epitope


<220>
<221>  misc_feature
<222>  (1)..(8)
<223>  Xaa can be any naturally occurring amino acid

<220>
<221>  misc_feature
<222>  (10)..(10)
<223>  Xaa can be any naturally occurring amino acid

<220>
<221>  misc_feature
<222>  (14)..(15)
<223>  Xaa can be any naturally occurring amino acid

<400>  769

Xaa Xaa Xaa Xaa Xaa Xaa Xaa Xaa Lys Xaa Arg Glu Gly Xaa Xaa
1               5                   10                  15

<210>  770
<211>  5
<212>  PRT
<213>  Artificial Sequence

<220>
<223>  epitope

<400>  770

Lys Asp Arg Glu Gly
1               5


<210>  771
<211>  5
<212>  PRT
<213>  Artificial Sequence

<220>
<223>  epitope


<220>
<221>  misc_feature
<222>  (2)..(2)
<223>  Xaa can be any naturally occurring amino acid

<400>  771

Lys Xaa Arg Glu Gly
1               5


<210>  772
<211>  4
<212>  PRT
<213>  Artificial Sequence

<220>
<223>  epitope

<400>  772

Asp Arg Glu Gly
1


<210>  773
<211>  15
<212>  PRT
<213>  Artificial Sequence

<220>
<223>  epitope

<400>  773

Leu Ser Asp Ile Met Asp Tyr Tyr Lys Asp Arg Glu Gly Ser His
1               5                   10                  15


<210>  774

<211>  6
<212>  PRT
<213>  Artificial Sequence

<220>
<223>  epitope

<400>  774

Lys Asp Arg Glu Gly Ser
1               5


<210>  775
<211>  15
<212>  PRT
<213>  Artificial Sequence

<220>
<223>  epitope

<400>  775

Leu Ser Asp Ile Met Asp Tyr Tyr Lys Asp Arg Glu Gly Ser His
1               5                   10                  15


<210>  776
<211>  4
<212>  PRT
<213>  Artificial Sequence

<220>
<223>  epitope

<400>  776

Asp Arg Glu Gly
1


<210>  777
<211>  6
<212>  PRT
<213>  Artificial Sequence

<220>
<223>  epitope

<400>  777

Lys Asp Arg Glu Gly Ser
1               5


<210>  778
<211>  5
<212>  PRT
<213>  Artificial Sequence

<220>
<223>  epitope

<400>  778

Lys Asp Arg Glu Gly
1               5



<210>  779
<211>  4
<212>  PRT
<213>  Artificial Sequence

<220>
<223>  epitope

<400>  779

Asp Arg Glu Gly
1



<210>  780
<211>  6
<212>  PRT
<213>  Artificial Sequence

<220>
<223>  epitope

<400>  780

Lys Asp Arg Glu Gly Ser
1               5



<210>  781
<211>  4
<212>  PRT
<213>  Artificial Sequence

<220>
<223>  epitope

<400>  781

Lys Asp Arg Glu
1



<210>  782
<211>  5
<212>  PRT
<213>  Artificial Sequence

<220>
<223>  epitope

<400>  782

Lys Asp Arg Glu Gly
1               5



<210>  783

<211>  15
<212>  PRT
<213>  Artificial Sequence

<220>
<223>  epitope


<220>
<221>  misc_feature
<222>  (1)..(6)
<223>  Xaa can be any naturally occurring amino acid

<220>
<221>  misc_feature
<222>  (11)..(15)
<223>  Xaa can be any naturally occurring amino acid

<400>  783

Xaa Xaa Xaa Xaa Xaa Xaa Tyr Tyr Lys Asp Xaa Xaa Xaa Xaa Xaa
1               5                   10                  15


<210>  784
<211>  5
<212>  PRT
<213>  Artificial Sequence

<220>
<223>  epitope

<400>  784

Lys Asp Arg Glu Gly
1               5


<210>  785
<211>  15
<212>  PRT
<213>  Artificial Sequence

<220>
<223>  epitope


<220>
<221>  misc_feature
<222>  (1)..(8)
<223>  Xaa can be any naturally occurring amino acid

<400>  785

Xaa Xaa Xaa Xaa Xaa Xaa Xaa Xaa Lys Asp Arg Glu Gly Ser His
1               5                   10                  15


<210>  786
<211>  15
<212>  PRT
<213>  Artificial Sequence

<220>
<223>  epitope

<220>
<221>  misc_feature
<222>  (1)..(8)
<223>  Xaa can be any naturally occurring amino acid

<220>
<221>  misc_feature
<222>  (10)..(11)
<223>  Xaa can be any naturally occurring amino acid

<400>  786

Xaa Xaa Xaa Xaa Xaa Xaa Xaa Xaa Lys Xaa Xaa Glu Gly Ser His
1               5                   10                  15

<210>  787
<211>  6
<212>  PRT
<213>  Artificial Sequence

<220>
<223>  epitope

<400>  787

Lys Asp Arg Glu Gly Ser
1               5

<210>  788
<211>  5
<212>  PRT
<213>  Artificial Sequence

<220>
<223>  epitope

<400>  788

Lys Asp Arg Glu Gly
1               5

<210>  789
<211>  4
<212>  PRT
<213>  Artificial Sequence

<220>
<223>  epitope

<400>  789

Asp Arg Glu Gly
1

<210>  790

<211>  15
<212>  PRT
<213>  Artificial Sequence

<220>
<223>  epitope


<220>
<221>  misc_feature
<222>  (1)..(8)
<223>  Xaa can be any naturally occurring amino acid

<220>
<221>  misc_feature
<222>  (11)..(11)
<223>  Xaa can be any naturally occurring amino acid

<220>
<221>  misc_feature
<222>  (14)..(15)
<223>  Xaa can be any naturally occurring amino acid

<400>  790

Xaa Xaa Xaa Xaa Xaa Xaa Xaa Xaa Lys Asp Xaa Glu Gly Xaa Xaa
1               5                   10                  15


<210>  791
<211>  4
<212>  PRT
<213>  Artificial Sequence

<220>
<223>  epitope

<400>  791

Asp Arg Glu Gly
1


<210>  792
<211>  15
<212>  PRT
<213>  Artificial Sequence

<220>
<223>  epitope

<400>  792

Asp Pro Ala Thr Ala Glu Asp Lys Cys Asp Arg Thr Cys Arg Pro
1               5                   10                  15


<210>  793
<211>  15
<212>  PRT
<213>  Artificial Sequence

<220>

<223>  epitope

<400>  793

Asp Pro Ala Thr Ala Glu Asp Lys Cys Asp Arg Thr Cys Arg Pro
1               5                   10                  15


<210>  794
<211>  15
<212>  PRT
<213>  Artificial Sequence

<220>
<223>  epitope


<220>
<221>  misc_feature
<222>  (1)..(2)
<223>  Xaa can be any naturally occurring amino acid

<400>  794

Xaa Xaa Ala Thr Ala Glu Asp Lys Cys Asp Arg Thr Cys Arg Pro
1               5                   10                  15


<210>  795
<211>  10
<212>  PRT
<213>  Artificial Sequence

<220>
<223>  epitope

<400>  795

Ala Thr Ala Glu Asp Lys Cys Asp Arg Thr
1               5                   10


<210>  796
<211>  10
<212>  PRT
<213>  Artificial Sequence

<220>
<223>  epitope

<400>  796

Ala Glu Asp Lys Cys Asp Arg Thr Cys Arg
1               5                   10


<210>  797
<211>  15
<212>  PRT
<213>  Artificial Sequence

<220>
<223>  epitope

<400>  797

Asp Pro Ala Thr Ala Glu Asp Lys Cys Asp Arg Thr Cys Arg Pro
1               5                   10                  15


<210>  798
<211>  15
<212>  PRT
<213>  Artificial Sequence

<220>
<223>  epitope


<220>
<221>  misc_feature
<222>  (13)..(13)
<223>  Xaa can be any naturally occurring amino acid

<400>  798

Asp Pro Ala Thr Ala Glu Asp Lys Cys Asp Arg Thr Xaa Arg Pro
1               5                   10                  15


<210>  799
<211>  15
<212>  PRT
<213>  Artificial Sequence

<220>
<223>  epitope


<220>
<221>  misc_feature
<222>  (1)..(2)
<223>  Xaa can be any naturally occurring amino acid

<220>
<221>  misc_feature
<222>  (12)..(14)
<223>  Xaa can be any naturally occurring amino acid

<400>  799

Xaa Xaa Ala Thr Ala Glu Asp Lys Cys Asp Arg Xaa Xaa Xaa Pro
1               5                   10                  15


<210>  800
<211>  15
<212>  PRT
<213>  Artificial Sequence

<220>
<223>  epitope


<220>
<221>  misc_feature

<222>  (1)..(2)
<223>  Xaa can be any naturally occurring amino acid

<220>
<221>  misc_feature
<222>  (12)..(15)
<223>  Xaa can be any naturally occurring amino acid

<400>  800

Xaa Xaa Ala Thr Ala Glu Asp Lys Cys Asp Arg Xaa Xaa Xaa Xaa
1               5                   10                  15


<210>  801
<211>  4
<212>  PRT
<213>  Artificial Sequence

<220>
<223>  epitope

<400>  801

Glu Asp Lys Cys
1


<210>  802
<211>  15
<212>  PRT
<213>  Artificial Sequence

<220>
<223>  epitope

<400>  802

Asp Pro Ala Thr Ala Glu Asp Lys Cys Asp Arg Thr Cys Arg Pro
1               5                   10                  15


<210>  803
<211>  15
<212>  PRT
<213>  Artificial Sequence

<220>
<223>  epitope


<220>
<221>  misc_feature
<222>  (1)..(2)
<223>  Xaa can be any naturally occurring amino acid

<220>
<221>  misc_feature
<222>  (5)..(5)
<223>  Xaa can be any naturally occurring amino acid

<220>
<221>  misc_feature

<222>  (13)..(15)
<223>  Xaa can be any naturally occurring amino acid

<400>  803

Xaa Xaa Ala Thr Xaa Glu Asp Lys Cys Asp Arg Thr Xaa Xaa Xaa
1               5                   10                  15


<210>  804
<211>  15
<212>  PRT
<213>  Artificial Sequence

<220>
<223>  epitope


<220>
<221>  misc_feature
<222>  (1)..(6)
<223>  Xaa can be any naturally occurring amino acid

<220>
<221>  misc_feature
<222>  (9)..(9)
<223>  Xaa can be any naturally occurring amino acid

<220>
<221>  misc_feature
<222>  (12)..(15)
<223>  Xaa can be any naturally occurring amino acid

<400>  804

Xaa Xaa Xaa Xaa Xaa Xaa Asp Lys Xaa Asp Arg Xaa Xaa Xaa Xaa
1               5                   10                  15


<210>  805
<211>  15
<212>  PRT
<213>  Artificial Sequence

<220>
<223>  epitope

<400>  805

Asp Pro Ala Thr Ala Glu Asp Lys Cys Asp Arg Thr Cys Arg Pro
1               5                   10                  15


<210>  806
<211>  15
<212>  PRT
<213>  Artificial Sequence

<220>
<223>  epitope


<220>

<221>  misc_feature
<222>  (5)..(6)
<223>  Xaa can be any naturally occurring amino acid

<400>  806

Asp Pro Ala Thr Xaa Xaa Asp Lys Cys Asp Arg Thr Cys Arg Pro
1               5                   10                  15


<210>  807
<211>  15
<212>  PRT
<213>  Artificial Sequence

<220>
<223>  epitope


<220>
<221>  misc_feature
<222>  (1)..(1)
<223>  Xaa can be any naturally occurring amino acid

<220>
<221>  misc_feature
<222>  (5)..(6)
<223>  Xaa can be any naturally occurring amino acid

<400>  807

Xaa Pro Ala Thr Xaa Xaa Asp Lys Cys Asp Arg Thr Cys Arg Pro
1               5                   10                  15


<210>  808
<211>  8
<212>  PRT
<213>  Artificial Sequence

<220>
<223>  epitope

<400>  808

Ala Glu Asp Lys Cys Asp Arg Thr
1               5


<210>  809
<211>  8
<212>  PRT
<213>  Artificial Sequence

<220>
<223>  epitope

<400>  809

Ala Glu Asp Lys Cys Asp Arg Thr
1               5

<210>  810
<211>  6
<212>  PRT
<213>  Artificial Sequence

<220>
<223>  epitope

<400>  810

Glu Asp Lys Cys Asp Arg
1               5


<210>  811
<211>  15
<212>  PRT
<213>  Artificial Sequence

<220>
<223>  epitope

<400>  811

Asp Pro Ala Thr Ala Glu Asp Lys Cys Asp Arg Thr Cys Arg Pro
1               5                   10                  15


<210>  812
<211>  15
<212>  PRT
<213>  Artificial Sequence

<220>
<223>  epitope


<220>
<221>  misc_feature
<222>  (13)..(13)
<223>  Xaa can be any naturally occurring amino acid

<400>  812

Asp Pro Ala Thr Ala Glu Asp Lys Cys Asp Arg Thr Xaa Arg Pro
1               5                   10                  15


<210>  813
<211>  15
<212>  PRT
<213>  Artificial Sequence

<220>
<223>  epitope


<220>
<221>  misc_feature
<222>  (1)..(1)
<223>  Xaa can be any naturally occurring amino acid

<220>

<221>  misc_feature
<222>  (13)..(15)
<223>  Xaa can be any naturally occurring amino acid

<400>  813

Xaa Pro Ala Thr Ala Glu Asp Lys Cys Asp Arg Thr Xaa Xaa Xaa
1               5                   10                  15


<210>  814
<211>  15
<212>  PRT
<213>  Artificial Sequence

<220>
<223>  epitope


<220>
<221>  misc_feature
<222>  (1)..(1)
<223>  Xaa can be any naturally occurring amino acid

<220>
<221>  misc_feature
<222>  (5)..(6)
<223>  Xaa can be any naturally occurring amino acid

<220>
<221>  misc_feature
<222>  (12)..(15)
<223>  Xaa can be any naturally occurring amino acid

<400>  814

Xaa Pro Ala Thr Xaa Xaa Asp Lys Cys Asp Arg Xaa Xaa Xaa Xaa
1               5                   10                  15


<210>  815
<211>  10
<212>  PRT
<213>  Artificial Sequence

<220>
<223>  epitope

<400>  815

Arg Tyr Cys Thr Asp Pro Ala Thr Ala Glu
1               5                   10


<210>  816
<211>  10
<212>  PRT
<213>  Artificial Sequence

<220>
<223>  epitope

<220>
<221>  misc_feature
<222>  (5)..(5)
<223>  Xaa can be any naturally occurring amino acid

<400>  816

Arg Tyr Cys Thr Xaa Pro Ala Thr Ala Glu
1               5                   10


<210>  817
<211>  10
<212>  PRT
<213>  Artificial Sequence

<220>
<223>  epitope


<220>
<221>  misc_feature
<222>  (5)..(5)
<223>  Xaa can be any naturally occurring amino acid

<220>
<221>  misc_feature
<222>  (9)..(10)
<223>  Xaa can be any naturally occurring amino acid

<400>  817

Arg Tyr Cys Thr Xaa Pro Ala Thr Xaa Xaa
1               5                   10


<210>  818
<211>  10
<212>  PRT
<213>  Artificial Sequence

<220>
<223>  epitope

<400>  818

Ala Glu Asp Lys Cys Asp Arg Thr Cys Arg
1               5                   10


<210>  819
<211>  10
<212>  PRT
<213>  Artificial Sequence

<220>
<223>  epitope


<220>
<221>  misc_feature
<222>  (1)..(2)
<223>  Xaa can be any naturally occurring amino acid

<220>
<221>  misc_feature
<222>  (8)..(10)
<223>  Xaa can be any naturally occurring amino acid

<400>  819

Xaa Xaa Asp Lys Cys Asp Arg Xaa Xaa Xaa
1               5                   10


<210>  820
<211>  15
<212>  PRT
<213>  Artificial Sequence

<220>
<223>  epitope

<400>  820

Asp Pro Ala Thr Ala Glu Asp Lys Cys Asp Arg Thr Cys Arg Pro
1               5                   10                  15


<210>  821
<211>  15
<212>  PRT
<213>  Artificial Sequence

<220>
<223>  epitope


<220>
<221>  misc_feature
<222>  (1)..(3)
<223>  Xaa can be any naturally occurring amino acid

<220>
<221>  misc_feature
<222>  (5)..(5)
<223>  Xaa can be any naturally occurring amino acid

<220>
<221>  misc_feature
<222>  (9)..(9)
<223>  Xaa can be any naturally occurring amino acid

<220>
<221>  misc_feature
<222>  (13)..(15)
<223>  Xaa can be any naturally occurring amino acid

<400>  821

Xaa Xaa Xaa Thr Xaa Glu Asp Lys Xaa Asp Arg Thr Xaa Xaa Xaa
1               5                   10                  15


<210>  822
<211>  15

<212>  PRT
<213>  Artificial Sequence

<220>
<223>  epitope


<220>
<221>  misc_feature
<222>  (1)..(6)
<223>  Xaa can be any naturally occurring amino acid

<220>
<221>  misc_feature
<222>  (9)..(9)
<223>  Xaa can be any naturally occurring amino acid

<220>
<221>  misc_feature
<222>  (12)..(15)
<223>  Xaa can be any naturally occurring amino acid

<400>  822

Xaa Xaa Xaa Xaa Xaa Xaa Asp Lys Xaa Asp Arg Xaa Xaa Xaa Xaa
1               5                   10                  15


<210>  823
<211>  5
<212>  PRT
<213>  Artificial Sequence

<220>
<223>  epitope

<400>  823

Glu Asp Lys Cys Asp
1               5


<210>  824
<211>  15
<212>  PRT
<213>  Artificial Sequence

<220>
<223>  epitope

<400>  824

Asp Pro Ala Thr Ala Glu Asp Lys Cys Asp Arg Thr Cys Arg Pro
1               5                   10                  15


<210>  825
<211>  9
<212>  PRT
<213>  Artificial Sequence

<220>
<223>  epitope

<400>  825

Tyr Cys Thr Asp Pro Ala Thr Ala Glu
1               5


<210>  826
<211>  10
<212>  PRT
<213>  Artificial Sequence

<220>
<223>  epitope

<400>  826

Thr Asp Pro Ala Thr Ala Glu Asp Lys Cys
1               5                   10


<210>  827
<211>  9
<212>  PRT
<213>  Artificial Sequence

<220>
<223>  epitope

<400>  827

Ala Thr Ala Glu Asp Lys Cys Asp Arg
1               5


<210>  828
<211>  15
<212>  PRT
<213>  Artificial Sequence

<220>
<223>  epitope

<400>  828

Asp Pro Ala Thr Ala Glu Asp Lys Cys Asp Arg Thr Cys Arg Pro
1               5                   10                  15


<210>  829
<211>  15
<212>  PRT
<213>  Artificial Sequence

<220>
<223>  epitope


<220>
<221>  misc_feature
<222>  (1)..(6)
<223>  Xaa can be any naturally occurring amino acid

<220>
<221>  misc_feature
<222>  (9)..(9)
<223>  Xaa can be any naturally occurring amino acid

<220>
<221>  misc_feature
<222>  (11)..(11)
<223>  Xaa can be any naturally occurring amino acid

<220>
<221>  misc_feature
<222>  (14)..(15)
<223>  Xaa can be any naturally occurring amino acid

<400>  829

Xaa Xaa Xaa Xaa Xaa Xaa Asp Lys Xaa Asp Xaa Thr Cys Xaa Xaa
1               5                   10                  15


<210>  830
<211>  5
<212>  PRT
<213>  Artificial Sequence

<220>
<223>  epitope

<400>  830

Glu Asp Lys Cys Asp
1               5


<210>  831
<211>  15
<212>  PRT
<213>  Artificial Sequence

<220>
<223>  epitope


<220>
<221>  misc_feature
<222>  (1)..(3)
<223>  Xaa can be any naturally occurring amino acid

<220>
<221>  misc_feature
<222>  (13)..(15)
<223>  Xaa can be any naturally occurring amino acid

<400>  831

Xaa Xaa Xaa Thr Ala Glu Asp Lys Cys Asp Arg Thr Xaa Xaa Xaa
1               5                   10                  15


<210>  832
<211>  15
<212>  PRT

<213>  Artificial Sequence

<220>
<223>  epitope



<220>
<221>  misc_feature
<222>  (1)..(6)
<223>  Xaa can be any naturally occurring amino acid

<220>
<221>  misc_feature
<222>  (12)..(15)
<223>  Xaa can be any naturally occurring amino acid

<400>  832

Xaa Xaa Xaa Xaa Xaa Xaa Asp Lys Cys Asp Arg Xaa Xaa Xaa Xaa
1               5                   10                  15


<210>  833
<211>  5
<212>  PRT
<213>  Artificial Sequence

<220>
<223>  epitope

<400>  833

Ala Glu Asp Lys Cys
1               5


<210>  834
<211>  15
<212>  PRT
<213>  Artificial Sequence

<220>
<223>  epitope



<220>
<221>  misc_feature
<222>  (2)..(3)
<223>  Xaa can be any naturally occurring amino acid

<220>
<221>  misc_feature
<222>  (6)..(6)
<223>  Xaa can be any naturally occurring amino acid

<220>
<221>  misc_feature
<222>  (12)..(15)
<223>  Xaa can be any naturally occurring amino acid

<400>  834

Asp Xaa Xaa Thr Ala Xaa Asp Lys Cys Asp Arg Xaa Xaa Xaa Xaa

1               5                   10                  15


<210>  835
<211>  15
<212>  PRT
<213>  Artificial Sequence

<220>
<223>  epitope



<220>
<221>  misc_feature
<222>  (1)..(3)
<223>  Xaa can be any naturally occurring amino acid

<220>
<221>  misc_feature
<222>  (5)..(6)
<223>  Xaa can be any naturally occurring amino acid

<220>
<221>  misc_feature
<222>  (9)..(10)
<223>  Xaa can be any naturally occurring amino acid

<220>
<221>  misc_feature
<222>  (12)..(15)
<223>  Xaa can be any naturally occurring amino acid

<400>  835

Xaa Xaa Xaa Thr Xaa Xaa Asp Lys Xaa Xaa Arg Xaa Xaa Xaa Xaa
1               5                   10                  15


<210>  836
<211>  10
<212>  PRT
<213>  Artificial Sequence

<220>
<223>  epitope

<400>  836

Asp Pro Ala Thr Ala Glu Asp Lys Cys Asp
1               5                   10


<210>  837
<211>  10
<212>  PRT
<213>  Artificial Sequence

<220>
<223>  epitope



<220>
<221>  misc_feature

<222>  (2)..(3)
<223>  Xaa can be any naturally occurring amino acid

<220>
<221>  misc_feature
<222>  (6)..(6)
<223>  Xaa can be any naturally occurring amino acid

<400>  837

Asp Xaa Xaa Thr Ala Xaa Asp Lys Cys Asp
1               5                   10


<210>  838
<211>  9
<212>  PRT
<213>  Artificial Sequence

<220>
<223>  epitope

<400>  838

Ala Glu Asp Lys Cys Asp Arg Thr Cys
1               5


<210>  839
<211>  8
<212>  PRT
<213>  Artificial Sequence

<220>
<223>  epitope

<400>  839

Ala Glu Asp Lys Cys Asp Arg Thr
1               5


<210>  840
<211>  8
<212>  PRT
<213>  Artificial Sequence

<220>
<223>  epitope


<220>
<221>  misc_feature
<222>  (2)..(2)
<223>  Xaa can be any naturally occurring amino acid

<220>
<221>  misc_feature
<222>  (8)..(8)
<223>  Xaa can be any naturally occurring amino acid

<400>  840

Ala Xaa Asp Lys Cys Asp Arg Xaa
1               5

<210>  841
<211>  15
<212>  PRT
<213>  Artificial Sequence

<220>
<223>  epitope

<220>
<221>  misc_feature
<222>  (1)..(6)
<223>  Xaa can be any naturally occurring amino acid

<220>
<221>  misc_feature
<222>  (13)..(13)
<223>  Xaa can be any naturally occurring amino acid

<400>  841

Xaa Xaa Xaa Xaa Xaa Xaa Asp Lys Cys Asp Arg Thr Xaa Arg Pro
1               5                   10                  15

<210>  842
<211>  15
<212>  PRT
<213>  Artificial Sequence

<220>
<223>  epitope

<220>
<221>  misc_feature
<222>  (1)..(6)
<223>  Xaa can be any naturally occurring amino acid

<220>
<221>  misc_feature
<222>  (13)..(15)
<223>  Xaa can be any naturally occurring amino acid

<400>  842

Xaa Xaa Xaa Xaa Xaa Xaa Asp Lys Cys Asp Arg Thr Xaa Xaa Xaa
1               5                   10                  15

<210>  843
<211>  9
<212>  PRT
<213>  Artificial Sequence

<220>
<223>  epitope

<400>  843

Ala Thr Ala Glu Asp Lys Cys Asp Arg
1               5


<210>  844
<211>  9
<212>  PRT
<213>  Artificial Sequence

<220>
<223>  epitope

<400>  844

Glu Asp Lys Cys Asp Arg Thr Cys Arg
1               5


<210>  845
<211>  6
<212>  PRT
<213>  Artificial Sequence

<220>
<223>  epitope

<400>  845

Glu Asp Lys Cys Asp Arg
1               5


<210>  846
<211>  6
<212>  PRT
<213>  Artificial Sequence

<220>
<223>  epitope


<220>
<221>  misc_feature
<222>  (1)..(1)
<223>  Xaa can be any naturally occurring amino acid

<400>  846

Xaa Asp Lys Cys Asp Arg
1               5


<210>  847
<211>  15
<212>  PRT
<213>  Artificial Sequence

<220>
<223>  epitope


<220>

<221>  misc_feature
<222>  (1)..(1)
<223>  Xaa can be any naturally occurring amino acid

<220>
<221>  misc_feature
<222>  (9)..(15)
<223>  Xaa can be any naturally occurring amino acid

<400>  847

Xaa Pro Ala Thr Ala Glu Asp Lys Xaa Xaa Xaa Xaa Xaa Xaa Xaa
1               5                   10                  15


<210>  848
<211>  15
<212>  PRT
<213>  Artificial Sequence

<220>
<223>  epitope


<220>
<221>  misc_feature
<222>  (1)..(1)
<223>  Xaa can be any naturally occurring amino acid

<220>
<221>  misc_feature
<222>  (4)..(6)
<223>  Xaa can be any naturally occurring amino acid

<220>
<221>  misc_feature
<222>  (9)..(15)
<223>  Xaa can be any naturally occurring amino acid

<400>  848

Xaa Pro Ala Xaa Xaa Xaa Asp Lys Xaa Xaa Xaa Xaa Xaa Xaa Xaa
1               5                   10                  15


<210>  849
<211>  15
<212>  PRT
<213>  Artificial Sequence

<220>
<223>  epitope


<220>
<221>  misc_feature
<222>  (1)..(3)
<223>  Xaa can be any naturally occurring amino acid

<220>
<221>  misc_feature
<222>  (6)..(8)
<223>  Xaa can be any naturally occurring amino acid

<220>
<221>  misc_feature
<222>  (12)..(15)
<223>  Xaa can be any naturally occurring amino acid

<400>  849

Xaa Xaa Xaa Thr Ala Xaa Xaa Xaa Cys Asp Arg Xaa Xaa Xaa Xaa
1               5                   10                  15


<210>  850
<211>  15
<212>  PRT
<213>  Artificial Sequence

<220>
<223>  epitope


<220>
<221>  misc_feature
<222>  (1)..(3)
<223>  Xaa can be any naturally occurring amino acid

<220>
<221>  misc_feature
<222>  (6)..(9)
<223>  Xaa can be any naturally occurring amino acid

<220>
<221>  misc_feature
<222>  (12)..(15)
<223>  Xaa can be any naturally occurring amino acid

<400>  850

Xaa Xaa Xaa Thr Ala Xaa Xaa Xaa Xaa Asp Arg Xaa Xaa Xaa Xaa
1               5                   10                  15


<210>  851
<211>  9
<212>  PRT
<213>  Artificial Sequence

<220>
<223>  epitope

<400>  851

Ala Glu Asp Lys Cys Asp Arg Thr Cys
1               5


<210>  852
<211>  6
<212>  PRT
<213>  Artificial Sequence

<220>
<223>  epitope

<400>  852

Ala Glu Asp Lys Cys Asp
1               5


<210>  853
<211>  15
<212>  PRT
<213>  Artificial Sequence

<220>
<223>  epitope


<220>
<221>  misc_feature
<222>  (1)..(4)
<223>  Xaa can be any naturally occurring amino acid

<220>
<221>  misc_feature
<222>  (6)..(6)
<223>  Xaa can be any naturally occurring amino acid

<220>
<221>  misc_feature
<222>  (12)..(15)
<223>  Xaa can be any naturally occurring amino acid

<400>  853

Xaa Xaa Xaa Xaa Ala Xaa Asp Lys Cys Asp Arg Xaa Xaa Xaa Xaa
1               5                   10                  15


<210>  854
<211>  15
<212>  PRT
<213>  Artificial Sequence

<220>
<223>  epitope


<220>
<221>  misc_feature
<222>  (1)..(6)
<223>  Xaa can be any naturally occurring amino acid

<220>
<221>  misc_feature
<222>  (12)..(15)
<223>  Xaa can be any naturally occurring amino acid

<400>  854

Xaa Xaa Xaa Xaa Xaa Xaa Asp Lys Cys Asp Arg Xaa Xaa Xaa Xaa
1               5                   10                  15


<210>  855

<211>  15
<212>  PRT
<213>  Artificial Sequence

<220>
<223>  epitope


<220>
<221>  misc_feature
<222>  (1)..(6)
<223>  Xaa can be any naturally occurring amino acid

<220>
<221>  misc_feature
<222>  (9)..(9)
<223>  Xaa can be any naturally occurring amino acid

<220>
<221>  misc_feature
<222>  (12)..(15)
<223>  Xaa can be any naturally occurring amino acid

<400>  855

Xaa Xaa Xaa Xaa Xaa Xaa Asp Lys Xaa Asp Arg Xaa Xaa Xaa Xaa
1               5                   10                  15


<210>  856
<211>  7
<212>  PRT
<213>  Artificial Sequence

<220>
<223>  epitope

<400>  856

Ala Glu Asp Lys Cys Asp Arg
1               5


<210>  857
<211>  6
<212>  PRT
<213>  Artificial Sequence

<220>
<223>  epitope

<400>  857

Ala Glu Asp Lys Cys Asp
1               5


<210>  858
<211>  6
<212>  PRT
<213>  Artificial Sequence

<220>

<223>  epitope

<220>
<221>  misc_feature
<222>  (2)..(2)
<223>  Xaa can be any naturally occurring amino acid

<400>  858

Ala Xaa Asp Lys Cys Asp
1               5

<210>  859
<211>  6
<212>  PRT
<213>  Artificial Sequence

<220>
<223>  epitope

<220>
<221>  misc_feature
<222>  (1)..(2)
<223>  Xaa can be any naturally occurring amino acid

<400>  859

Xaa Xaa Asp Lys Cys Asp
1               5

<210>  860
<211>  15
<212>  PRT
<213>  Artificial Sequence

<220>
<223>  epitope

<220>
<221>  misc_feature
<222>  (2)..(3)
<223>  Xaa can be any naturally occurring amino acid

<400>  860

Asp Xaa Xaa Thr Ala Glu Asp Lys Cys Asp Arg Thr Cys Arg Pro
1               5                   10                  15

<210>  861
<211>  15
<212>  PRT
<213>  Artificial Sequence

<220>
<223>  epitope

<220>
<221>  misc_feature
<222>  (1)..(3)
<223>  Xaa can be any naturally occurring amino acid

<220>
<221>  misc_feature
<222>  (5)..(5)
<223>  Xaa can be any naturally occurring amino acid

<220>
<221>  misc_feature
<222>  (13)..(13)
<223>  Xaa can be any naturally occurring amino acid

<400>  861

Xaa Xaa Xaa Thr Xaa Glu Asp Lys Cys Asp Arg Thr Xaa Arg Pro
1               5                   10                  15


<210>  862
<211>  15
<212>  PRT
<213>  Artificial Sequence

<220>
<223>  epitope


<220>
<221>  misc_feature
<222>  (1)..(3)
<223>  Xaa can be any naturally occurring amino acid

<220>
<221>  misc_feature
<222>  (5)..(6)
<223>  Xaa can be any naturally occurring amino acid

<220>
<221>  misc_feature
<222>  (13)..(13)
<223>  Xaa can be any naturally occurring amino acid

<400>  862

Xaa Xaa Xaa Thr Xaa Xaa Asp Lys Cys Asp Arg Thr Xaa Arg Pro
1               5                   10                  15


<210>  863
<211>  10
<212>  PRT
<213>  Artificial Sequence

<220>
<223>  epitope

<400>  863

Pro Ala Thr Ala Glu Asp Lys Cys Asp Arg
1               5                   10

<210>  864
<211>  9
<212>  PRT
<213>  Artificial Sequence

<220>
<223>  epitope

<400>  864

Ala Glu Asp Lys Cys Asp Arg Thr Cys
1               5


<210>  865
<211>  7
<212>  PRT
<213>  Artificial Sequence

<220>
<223>  epitope

<400>  865

Ala Glu Asp Lys Cys Asp Arg
1               5


<210>  866
<211>  6
<212>  PRT
<213>  Artificial Sequence

<220>
<223>  epitope

<400>  866

Ala Glu Asp Lys Cys Asp
1               5


<210>  867
<211>  6
<212>  PRT
<213>  Artificial Sequence

<220>
<223>  epitope


<220>
<221>  misc_feature
<222>  (1)..(1)
<223>  Xaa can be any naturally occurring amino acid

<400>  867

Xaa Glu Asp Lys Cys Asp
1               5

<210>  868
<211>  6
<212>  PRT
<213>  Artificial Sequence

<220>
<223>  epitope


<220>
<221>  misc_feature
<222>  (1)..(2)
<223>  Xaa can be any naturally occurring amino acid

<400>  868

Xaa Xaa Asp Lys Cys Asp
1               5


<210>  869
<211>  15
<212>  PRT
<213>  Artificial Sequence

<220>
<223>  epitope

<400>  869

Thr Glu Asp Ile Gln Gly Cys Asp Ser Asp Lys His Gly Trp Tyr
1               5                   10                  15


<210>  870
<211>  15
<212>  PRT
<213>  Artificial Sequence

<220>
<223>  epitope

<400>  870

Thr Glu Asp Ile Gln Gly Cys Asp Ser Asp Lys His Gly Trp Tyr
1               5                   10                  15


<210>  871
<211>  15
<212>  PRT
<213>  Artificial Sequence

<220>
<223>  epitope


<220>
<221>  misc_feature
<222>  (2)..(2)
<223>  Xaa can be any naturally occurring amino acid

<220>
<221>  misc_feature
<222>  (6)..(7)
<223>  Xaa can be any naturally occurring amino acid

<220>
<221>  misc_feature
<222>  (9)..(9)
<223>  Xaa can be any naturally occurring amino acid

<220>
<221>  misc_feature
<222>  (13)..(14)
<223>  Xaa can be any naturally occurring amino acid

<400>  871

Thr Xaa Asp Ile Gln Xaa Xaa Asp Xaa Asp Lys His Xaa Xaa Tyr
1               5                   10                  15


<210>  872
<211>  15
<212>  PRT
<213>  Artificial Sequence

<220>
<223>  epitope


<220>
<221>  misc_feature
<222>  (2)..(2)
<223>  Xaa can be any naturally occurring amino acid

<220>
<221>  misc_feature
<222>  (6)..(7)
<223>  Xaa can be any naturally occurring amino acid

<220>
<221>  misc_feature
<222>  (9)..(9)
<223>  Xaa can be any naturally occurring amino acid

<220>
<221>  misc_feature
<222>  (12)..(14)
<223>  Xaa can be any naturally occurring amino acid

<400>  872

Thr Xaa Asp Ile Gln Xaa Xaa Asp Xaa Asp Lys Xaa Xaa Xaa Tyr
1               5                   10                  15


<210>  873
<211>  10
<212>  PRT
<213>  Artificial Sequence

<220>
<223>  epitope

<400>  873

Ile Gln Gly Cys Asp Ser Asp Lys His Gly
1               5                   10


<210>  874
<211>  10
<212>  PRT
<213>  Artificial Sequence

<220>
<223>  epitope


<220>
<221>  misc_feature
<222>  (3)..(4)
<223>  Xaa can be any naturally occurring amino acid

<220>
<221>  misc_feature
<222>  (6)..(6)
<223>  Xaa can be any naturally occurring amino acid

<220>
<221>  misc_feature
<222>  (10)..(10)
<223>  Xaa can be any naturally occurring amino acid

<400>  874

Ile Gln Xaa Xaa Asp Xaa Asp Lys His Xaa
1               5                   10


<210>  875
<211>  10
<212>  PRT
<213>  Artificial Sequence

<220>
<223>  epitope


<220>
<221>  misc_feature
<222>  (3)..(4)
<223>  Xaa can be any naturally occurring amino acid

<220>
<221>  misc_feature
<222>  (6)..(6)
<223>  Xaa can be any naturally occurring amino acid

<220>
<221>  misc_feature
<222>  (9)..(10)
<223>  Xaa can be any naturally occurring amino acid

<400>  875

Ile Gln Xaa Xaa Asp Xaa Asp Lys Xaa Xaa
1               5                   10


<210>  876
<211>  9
<212>  PRT
<213>  Artificial Sequence

<220>
<223>  epitope

<400>  876

Asp Ser Asp Lys His Gly Trp Tyr Arg
1               5


<210>  877
<211>  9
<212>  PRT
<213>  Artificial Sequence

<220>
<223>  epitope


<220>
<221>  misc_feature
<222>  (2)..(2)
<223>  Xaa can be any naturally occurring amino acid

<220>
<221>  misc_feature
<222>  (6)..(7)
<223>  Xaa can be any naturally occurring amino acid

<400>  877

Asp Xaa Asp Lys His Xaa Xaa Tyr Arg
1               5


<210>  878
<211>  9
<212>  PRT
<213>  Artificial Sequence

<220>
<223>  epitope


<220>
<221>  misc_feature
<222>  (2)..(2)
<223>  Xaa can be any naturally occurring amino acid

<220>
<221>  misc_feature
<222>  (5)..(7)
<223>  Xaa can be any naturally occurring amino acid

<400>  878

Asp Xaa Asp Lys Xaa Xaa Xaa Tyr Arg
1               5

<210>  879
<211>  10
<212>  PRT
<213>  Artificial Sequence

<220>
<223>  epitope

<400>  879

Asp Lys His Gly Trp Tyr Arg Phe Val Gly
1               5                   10

<210>  880
<211>  10
<212>  PRT
<213>  Artificial Sequence

<220>
<223>  epitope

<220>
<221>  misc_feature
<222>  (4)..(5)
<223>  Xaa can be any naturally occurring amino acid

<400>  880

Asp Lys His Xaa Xaa Tyr Arg Phe Val Gly
1               5                   10

<210>  881
<211>  10
<212>  PRT
<213>  Artificial Sequence

<220>
<223>  epitope

<220>
<221>  misc_feature
<222>  (3)..(5)
<223>  Xaa can be any naturally occurring amino acid

<400>  881

Asp Lys Xaa Xaa Xaa Tyr Arg Phe Val Gly
1               5                   10

<210>  882
<211>  15
<212>  PRT
<213>  Artificial Sequence

<220>
<223>  epitope

<400>  882

Thr Glu Asp Ile Gln Gly Cys Asp Ser Asp Lys His Gly Trp Tyr
1               5                   10                  15


<210>  883
<211>  15
<212>  PRT
<213>  Artificial Sequence

<220>
<223>  epitope


<220>
<221>  misc_feature
<222>  (2)..(2)
<223>  Xaa can be any naturally occurring amino acid

<220>
<221>  misc_feature
<222>  (6)..(15)
<223>  Xaa can be any naturally occurring amino acid

<400>  883

Thr Xaa Asp Ile Gln Xaa Xaa Xaa Xaa Xaa Xaa Xaa Xaa Xaa Xaa
1               5                   10                  15


<210>  884
<211>  8
<212>  PRT
<213>  Artificial Sequence

<220>
<223>  epitope

<400>  884

Glu Asn Thr Glu Asp Ile Gln Gly
1               5


<210>  885
<211>  8
<212>  PRT
<213>  Artificial Sequence

<220>
<223>  epitope


<220>
<221>  misc_feature
<222>  (4)..(4)
<223>  Xaa can be any naturally occurring amino acid

<220>
<221>  misc_feature
<222>  (8)..(8)
<223>  Xaa can be any naturally occurring amino acid

<400>  885

Glu Asn Thr Xaa Asp Ile Gln Xaa
1               5


<210>  886
<211>  10
<212>  PRT
<213>  Artificial Sequence

<220>
<223>  epitope

<400>  886

Ile Gln Gly Cys Asp Ser Asp Lys His Gly
1               5                   10


<210>  887
<211>  6
<212>  PRT
<213>  Artificial Sequence

<220>
<223>  epitope

<400>  887

Asp Lys His Gly Trp Tyr
1               5


<210>  888
<211>  15
<212>  PRT
<213>  Artificial Sequence

<220>
<223>  epitope

<400>  888

Thr Glu Asp Ile Gln Gly Cys Asp Ser Asp Lys His Gly Trp Tyr
1               5                   10                  15


<210>  889
<211>  10
<212>  PRT
<213>  Artificial Sequence

<220>
<223>  epitope

<400>  889

Glu Asn Thr Glu Asp Ile Gln Gly Cys Asp
1               5                   10

<210>  890
<211>  15
<212>  PRT
<213>  Artificial Sequence

<220>
<223>  epitope

<400>  890

Thr Glu Asp Ile Gln Gly Cys Asp Ser Asp Lys His Gly Trp Tyr
1               5                   10                  15

<210>  891
<211>  4
<212>  PRT
<213>  Artificial Sequence

<220>
<223>  epitope

<400>  891

Asp Ser Asp Lys
1

<210>  892
<211>  10
<212>  PRT
<213>  Artificial Sequence

<220>
<223>  epitope

<400>  892

Asp Lys His Gly Trp Tyr Arg Phe Val Gly
1               5                   10

<210>  893
<211>  15
<212>  PRT
<213>  Artificial Sequence

<220>
<223>  epitope

<400>  893

Thr Glu Asp Ile Gln Gly Cys Asp Ser Asp Lys His Gly Trp Tyr
1               5                   10                  15

<210>  894
<211>  7
<212>  PRT

<213>  Artificial Sequence

<220>
<223>  epitope

<400>  894

Asn Thr Glu Asp Ile Gln Gly
1               5


<210>  895
<211>  15
<212>  PRT
<213>  Artificial Sequence

<220>
<223>  epitope

<400>  895

Thr Glu Asp Ile Gln Gly Cys Asp Ser Asp Lys His Gly Trp Tyr
1               5                   10                  15


<210>  896
<211>  10
<212>  PRT
<213>  Artificial Sequence

<220>
<223>  epitope

<400>  896

Ser Thr Glu Asn Thr Glu Asp Ile Gln Gly
1               5                   10


<210>  897
<211>  10
<212>  PRT
<213>  Artificial Sequence

<220>
<223>  epitope

<400>  897

Glu Asp Ile Gln Gly Cys Asp Ser Asp Lys
1               5                   10


<210>  898
<211>  10
<212>  PRT
<213>  Artificial Sequence

<220>
<223>  epitope

<400>  898

Ile Gln Gly Cys Asp Ser Asp Lys His Gly
1               5                   10

<210>  899
<211>  8
<212>  PRT
<213>  Artificial Sequence

<220>
<223>  epitope

<400>  899

Ile Gln Gly Cys Asp Ser Asp Lys
1               5

<210>  900
<211>  7
<212>  PRT
<213>  Artificial Sequence

<220>
<223>  epitope

<400>  900

Asp Lys His Gly Trp Tyr Arg
1               5

<210>  901
<211>  6
<212>  PRT
<213>  Artificial Sequence

<220>
<223>  epitope

<400>  901

Asp Lys His Gly Trp Tyr
1               5

<210>  902
<211>  15
<212>  PRT
<213>  Artificial Sequence

<220>
<223>  epitope

<400>  902

Thr Glu Asp Ile Gln Gly Cys Asp Ser Asp Lys His Gly Trp Tyr
1               5                   10                  15

<210>  903
<211>  10
<212>  PRT

<213>  Artificial Sequence

<220>
<223>  epitope

<400>  903

Ile Gln Gly Cys Asp Ser Asp Lys His Gly
1               5                   10


<210>  904
<211>  6
<212>  PRT
<213>  Artificial Sequence

<220>
<223>  epitope

<400>  904

Asp Lys His Gly Trp Tyr
1               5


<210>  905
<211>  15
<212>  PRT
<213>  Artificial Sequence

<220>
<223>  epitope

<400>  905

Thr Glu Asp Ile Gln Gly Cys Asp Ser Asp Lys His Gly Trp Tyr
1               5                   10                  15


<210>  906
<211>  15
<212>  PRT
<213>  Artificial Sequence

<220>
<223>  epitope

<400>  906

Thr Glu Asp Ile Gln Gly Cys Asp Ser Asp Lys His Gly Trp Tyr
1               5                   10                  15


<210>  907
<211>  15
<212>  PRT
<213>  Artificial Sequence

<220>
<223>  epitope


<220>

<221>  misc_feature
<222>  (1)..(2)
<223>  Xaa can be any naturally occurring amino acid

<220>
<221>  misc_feature
<222>  (4)..(4)
<223>  Xaa can be any naturally occurring amino acid

<220>
<221>  misc_feature
<222>  (6)..(10)
<223>  Xaa can be any naturally occurring amino acid

<220>
<221>  misc_feature
<222>  (12)..(12)
<223>  Xaa can be any naturally occurring amino acid

<400>  907

Xaa Xaa Asp Xaa Gln Xaa Xaa Xaa Xaa Xaa Lys Xaa Gly Trp Tyr
1               5                   10                  15


<210>  908
<211>  15
<212>  PRT
<213>  Artificial Sequence

<220>
<223>  epitope


<220>
<221>  misc_feature
<222>  (1)..(4)
<223>  Xaa can be any naturally occurring amino acid

<220>
<221>  misc_feature
<222>  (6)..(12)
<223>  Xaa can be any naturally occurring amino acid

<400>  908

Xaa Xaa Xaa Xaa Gln Xaa Xaa Xaa Xaa Xaa Xaa Xaa Gly Trp Tyr
1               5                   10                  15


<210>  909
<211>  6
<212>  PRT
<213>  Artificial Sequence

<220>
<223>  epitope

<400>  909

Asp Lys His Gly Trp Tyr
1               5

<210>  910
<211>  6
<212>  PRT
<213>  Artificial Sequence

<220>
<223>  epitope


<220>
<221>  misc_feature
<222>  (1)..(1)
<223>  Xaa can be any naturally occurring amino acid

<220>
<221>  misc_feature
<222>  (3)..(3)
<223>  Xaa can be any naturally occurring amino acid

<400>  910

Xaa Lys Xaa Gly Trp Tyr
1               5


<210>  911
<211>  15
<212>  PRT
<213>  Artificial Sequence

<220>
<223>  epitope


<220>
<221>  misc_feature
<222>  (1)..(2)
<223>  Xaa can be any naturally occurring amino acid

<220>
<221>  misc_feature
<222>  (6)..(10)
<223>  Xaa can be any naturally occurring amino acid

<220>
<221>  misc_feature
<222>  (12)..(15)
<223>  Xaa can be any naturally occurring amino acid

<400>  911

Xaa Xaa Asp Ile Gln Xaa Xaa Xaa Xaa Xaa Lys Xaa Xaa Xaa Xaa
1               5                   10                  15


<210>  912
<211>  10
<212>  PRT
<213>  Artificial Sequence

<220>
<223>  epitope

<400>  912

Ile Gln Gly Cys Asp Ser Asp Lys His Gly
1               5                   10



<210>  913
<211>  9
<212>  PRT
<213>  Artificial Sequence

<220>
<223>  epitope

<400>  913

Ile Gln Gly Cys Asp Ser Asp Lys His
1               5



<210>  914
<211>  6
<212>  PRT
<213>  Artificial Sequence

<220>
<223>  epitope

<400>  914

Asp Lys His Gly Trp Tyr
1               5



<210>  915
<211>  6
<212>  PRT
<213>  Artificial Sequence

<220>
<223>  epitope

<400>  915

Asp Lys His Gly Trp Tyr
1               5



<210>  916
<211>  4
<212>  PRT
<213>  Artificial Sequence

<220>
<223>  epitope

<400>  916

Asp Lys His Gly
1

<210>  917
<211>  10
<212>  PRT
<213>  Artificial Sequence

<220>
<223>  epitope

<400>  917

Glu Asp Ile Gln Gly Cys Asp Ser Asp Lys
1               5                   10


<210>  918
<211>  10
<212>  PRT
<213>  Artificial Sequence

<220>
<223>  epitope

<400>  918

Ile Gln Gly Cys Asp Ser Asp Lys His Gly
1               5                   10


<210>  919
<211>  8
<212>  PRT
<213>  Artificial Sequence

<220>
<223>  epitope

<400>  919

Ile Gln Gly Cys Asp Ser Asp Lys
1               5


<210>  920
<211>  6
<212>  PRT
<213>  Artificial Sequence

<220>
<223>  epitope

<400>  920

Asp Lys His Gly Trp Tyr
1               5


<210>  921
<211>  10
<212>  PRT
<213>  Artificial Sequence

<220>
<223>  epitope

<400>  921

Ile Gln Gly Cys Asp Ser Asp Lys His Gly
1               5                   10



<210>  922
<211>  7
<212>  PRT
<213>  Artificial Sequence

<220>
<223>  epitope

<400>  922

Ser Asp Lys His Gly Trp Tyr
1               5



<210>  923
<211>  4
<212>  PRT
<213>  Artificial Sequence

<220>
<223>  epitope

<400>  923

Ser Asp Lys His
1



<210>  924
<211>  15
<212>  PRT
<213>  Artificial Sequence

<220>
<223>  epitope



<220>
<221>  misc_feature
<222>  (1)..(2)
<223>  Xaa can be any naturally occurring amino acid

<220>
<221>  misc_feature
<222>  (6)..(6)
<223>  Xaa can be any naturally occurring amino acid

<220>
<221>  misc_feature
<222>  (9)..(9)
<223>  Xaa can be any naturally occurring amino acid

<400>  924

Xaa Xaa Asp Ile Gln Xaa Cys Asp Xaa Asp Lys His Gly Trp Tyr
1               5                   10                  15

<210>  925
<211>  15
<212>  PRT
<213>  Artificial Sequence

<220>
<223>  epitope


<220>
<221>  misc_feature
<222>  (1)..(2)
<223>  Xaa can be any naturally occurring amino acid

<220>
<221>  misc_feature
<222>  (4)..(4)
<223>  Xaa can be any naturally occurring amino acid

<220>
<221>  misc_feature
<222>  (6)..(9)
<223>  Xaa can be any naturally occurring amino acid

<220>
<221>  misc_feature
<222>  (12)..(15)
<223>  Xaa can be any naturally occurring amino acid

<400>  925

Xaa Xaa Asp Xaa Gln Xaa Xaa Xaa Xaa Asp Lys Xaa Xaa Xaa Xaa
1               5                   10                  15


<210>  926
<211>  10
<212>  PRT
<213>  Artificial Sequence

<220>
<223>  epitope

<400>  926

Ile Gln Gly Cys Asp Ser Asp Lys His Gly
1               5                   10


<210>  927
<211>  6
<212>  PRT
<213>  Artificial Sequence

<220>
<223>  epitope

<400>  927

Asp Lys His Gly Trp Tyr
1               5

<210>  928
<211>  15
<212>  PRT
<213>  Artificial Sequence

<220>
<223>  epitope

<400>  928

Ser Glu Ser Lys Ile Phe Pro Gly Asn Met Asp Asn Asn Ser His
1               5                   10                  15


<210>  929
<211>  15
<212>  PRT
<213>  Artificial Sequence

<220>
<223>  epitope

<400>  929

Ser Glu Ser Lys Ile Phe Pro Gly Asn Met Asp Asn Asn Ser His
1               5                   10                  15


<210>  930
<211>  5
<212>  PRT
<213>  Artificial Sequence

<220>
<223>  epitope

<400>  930

Glu Ser Lys Ile Phe
1               5


<210>  931
<211>  10
<212>  PRT
<213>  Artificial Sequence

<220>
<223>  epitope

<400>  931

Asn Met Asp Asn Asn Ser His Lys Lys Asn
1               5                   10


<210>  932
<211>  8
<212>  PRT
<213>  Artificial Sequence

<220>
<223>  epitope

<400>  932

Met Asp Asn Asn Ser His Lys Lys
1               5


<210>  933
<211>  15
<212>  PRT
<213>  Artificial Sequence

<220>
<223>  epitope

<400>  933

Ser Glu Ser Lys Ile Phe Pro Gly Asn Met Asp Asn Asn Ser His
1               5                   10                  15


<210>  934
<211>  9
<212>  PRT
<213>  Artificial Sequence

<220>
<223>  epitope

<400>  934

Glu Ser Lys Ile Phe Pro Gly Asn Met
1               5


<210>  935
<211>  8
<212>  PRT
<213>  Artificial Sequence

<220>
<223>  epitope

<400>  935

Glu Ser Lys Ile Phe Pro Gly Asn
1               5


<210>  936
<211>  9
<212>  PRT
<213>  Artificial Sequence

<220>
<223>  epitope

<400>  936

Asn Met Asp Asn Asn Ser His Lys Lys
1               5

<210>  937
<211>  8
<212>  PRT
<213>  Artificial Sequence

<220>
<223>  epitope

<400>  937

Met Asp Asn Asn Ser His Lys Lys
1               5


<210>  938
<211>  5
<212>  PRT
<213>  Artificial Sequence

<220>
<223>  epitope

<400>  938

Met Asp Asn Asn Ser
1               5


<210>  939
<211>  15
<212>  PRT
<213>  Artificial Sequence

<220>
<223>  epitope

<400>  939

Ser Glu Ser Lys Ile Phe Pro Gly Asn Met Asp Asn Asn Ser His
1               5                   10                  15


<210>  940
<211>  4
<212>  PRT
<213>  Artificial Sequence

<220>
<223>  epitope

<400>  940

Ser Lys Ile Phe
1


<210>  941
<211>  10
<212>  PRT
<213>  Artificial Sequence

<220>
<223>  epitope

<400>  941

Asn Met Asp Asn Asn Ser His Lys Lys Asn
1               5                   10


<210>  942
<211>  15
<212>  PRT
<213>  Artificial Sequence

<220>
<223>  epitope

<400>  942

Ser Glu Ser Lys Ile Phe Pro Gly Asn Met Asp Asn Asn Ser His
1               5                   10                  15


<210>  943
<211>  15
<212>  PRT
<213>  Artificial Sequence

<220>
<223>  epitope


<220>
<221>  misc_feature
<222>  (5)..(12)
<223>  Xaa can be any naturally occurring amino acid

<220>
<221>  misc_feature
<222>  (14)..(15)
<223>  Xaa can be any naturally occurring amino acid

<400>  943

Ser Glu Ser Lys Xaa Xaa Xaa Xaa Xaa Xaa Xaa Xaa Asn Xaa Xaa
1               5                   10                  15


<210>  944
<211>  6
<212>  PRT
<213>  Artificial Sequence

<220>
<223>  epitope

<400>  944

Ser Glu Ser Lys Ile Phe
1               5


<210>  945

<211>  10
<212>  PRT
<213>  Artificial Sequence

<220>
<223>  epitope

<400>  945

Ser Lys Ile Phe Pro Gly Asn Met Asp Asn
1               5                   10


<210>  946
<211>  7
<212>  PRT
<213>  Artificial Sequence

<220>
<223>  epitope

<400>  946

Met Asp Asn Asn Ser His Lys
1               5


<210>  947
<211>  6
<212>  PRT
<213>  Artificial Sequence

<220>
<223>  epitope

<400>  947

Met Asp Asn Asn Ser His
1               5


<210>  948
<211>  5
<212>  PRT
<213>  Artificial Sequence

<220>
<223>  epitope

<400>  948

Met Asp Asn Asn Ser
1               5


<210>  949
<211>  15
<212>  PRT
<213>  Artificial Sequence

<220>
<223>  epitope

<400>  949

Ser Glu Ser Lys Ile Phe Pro Gly Asn Met Asp Asn Asn Ser His
1               5                   10                  15


<210>  950
<211>  15
<212>  PRT
<213>  Artificial Sequence

<220>
<223>  epitope


<220>
<221>  misc_feature
<222>  (1)..(5)
<223>  Xaa can be any naturally occurring amino acid

<400>  950

Xaa Xaa Xaa Xaa Xaa Phe Pro Gly Asn Met Asp Asn Asn Ser His
1               5                   10                  15


<210>  951
<211>  9
<212>  PRT
<213>  Artificial Sequence

<220>
<223>  epitope

<400>  951

Glu Ser Lys Ile Phe Pro Gly Asn Met
1               5


<210>  952
<211>  9
<212>  PRT
<213>  Artificial Sequence

<220>
<223>  epitope


<220>
<221>  misc_feature
<222>  (1)..(4)
<223>  Xaa can be any naturally occurring amino acid

<400>  952

Xaa Xaa Xaa Xaa Phe Pro Gly Asn Met
1               5


<210>  953
<211>  10
<212>  PRT

<213>  Artificial Sequence

<220>
<223>  epitope

<400>  953

Phe Pro Gly Asn Met Asp Asn Asn Ser His
1               5                   10


<210>  954
<211>  9
<212>  PRT
<213>  Artificial Sequence

<220>
<223>  epitope

<400>  954

Asn Met Asp Asn Asn Ser His Lys Lys
1               5


<210>  955
<211>  6
<212>  PRT
<213>  Artificial Sequence

<220>
<223>  epitope

<400>  955

Met Asp Asn Asn Ser His
1               5


<210>  956
<211>  15
<212>  PRT
<213>  Artificial Sequence

<220>
<223>  epitope

<400>  956

Ser Glu Ser Lys Ile Phe Pro Gly Asn Met Asp Asn Asn Ser His
1               5                   10                  15


<210>  957
<211>  5
<212>  PRT
<213>  Artificial Sequence

<220>
<223>  epitope

<400>  957

Glu Ser Lys Ile Phe
1               5


<210>  958
<211>  4
<212>  PRT
<213>  Artificial Sequence

<220>
<223>  epitope

<400>  958

Ser Lys Ile Phe
1


<210>  959
<211>  15
<212>  PRT
<213>  Artificial Sequence

<220>
<223>  epitope

<400>  959

Ser Glu Ser Lys Ile Phe Pro Gly Asn Met Asp Asn Asn Ser His
1               5                   10                  15


<210>  960
<211>  15
<212>  PRT
<213>  Artificial Sequence

<220>
<223>  epitope

<400>  960

Ser Glu Ser Lys Ile Phe Pro Gly Asn Met Asp Asn Asn Ser His
1               5                   10                  15


<210>  961
<211>  7
<212>  PRT
<213>  Artificial Sequence

<220>
<223>  epitope

<400>  961

Ala Ser Glu Ser Lys Ile Phe
1               5


<210>  962
<211>  5
<212>  PRT

<213>  Artificial Sequence

<220>
<223>  epitope

<400>  962

Met Asp Asn Asn Ser
1               5


<210>  963
<211>  15
<212>  PRT
<213>  Artificial Sequence

<220>
<223>  epitope

<400>  963

Ser Glu Ser Lys Ile Phe Pro Gly Asn Met Asp Asn Asn Ser His
1               5                   10                  15


<210>  964
<211>  5
<212>  PRT
<213>  Artificial Sequence

<220>
<223>  epitope

<400>  964

Glu Ser Lys Ile Phe
1               5


<210>  965
<211>  15
<212>  PRT
<213>  Artificial Sequence

<220>
<223>  epitope


<220>
<221>  misc_feature
<222>  (8)..(15)
<223>  Xaa can be any naturally occurring amino acid

<400>  965

Ser Glu Ser Lys Ile Phe Pro Xaa Xaa Xaa Xaa Xaa Xaa Xaa Xaa
1               5                   10                  15


<210>  966
<211>  5
<212>  PRT
<213>  Artificial Sequence

<220>
<223>  epitope

<400>  966

Glu Ser Lys Ile Phe
1               5


<210>  967
<211>  8
<212>  PRT
<213>  Artificial Sequence

<220>
<223>  epitope

<400>  967

Glu Ser Lys Ile Phe Pro Gly Asn
1               5


<210>  968
<211>  5
<212>  PRT
<213>  Artificial Sequence

<220>
<223>  epitope

<400>  968

Glu Ser Lys Ile Phe
1               5


<210>  969
<211>  15
<212>  PRT
<213>  Artificial Sequence

<220>
<223>  epitope


<220>
<221>  misc_feature
<222>  (1)..(1)
<223>  Xaa can be any naturally occurring amino acid

<220>
<221>  misc_feature
<222>  (3)..(3)
<223>  Xaa can be any naturally occurring amino acid

<220>
<221>  misc_feature
<222>  (5)..(13)
<223>  Xaa can be any naturally occurring amino acid

<400>  969

Xaa Glu Xaa Lys Xaa Xaa Xaa Xaa Xaa Xaa Xaa Xaa Xaa Ser His
1               5                   10                  15

<210>  970
<211>  7
<212>  PRT
<213>  Artificial Sequence

<220>
<223>  epitope

<400>  970

Glu Ser Lys Ile Phe Pro Gly
1               5

<210>  971
<211>  5
<212>  PRT
<213>  Artificial Sequence

<220>
<223>  epitope

<400>  971

Glu Ser Lys Ile Phe
1               5

<210>  972
<211>  6
<212>  PRT
<213>  Artificial Sequence

<220>
<223>  epitope

<400>  972

Met Asp Asn Asn Ser His
1               5

<210>  973
<211>  5
<212>  PRT
<213>  Artificial Sequence

<220>
<223>  epitope

<400>  973

Met Asp Asn Asn Ser
1               5

<210>  974
<211>  15

<212>  PRT
<213>  Artificial Sequence

<220>
<223>  epitope


<220>
<221>  misc_feature
<222>  (1)..(5)
<223>  Xaa can be any naturally occurring amino acid

<220>
<221>  misc_feature
<222>  (7)..(7)
<223>  Xaa can be any naturally occurring amino acid

<400>  974

Xaa Xaa Xaa Xaa Xaa Phe Xaa Gly Asn Met Asp Asn Asn Ser His
1               5                   10                  15


<210>  975
<211>  5
<212>  PRT
<213>  Artificial Sequence

<220>
<223>  epitope

<400>  975

Glu Ser Lys Ile Phe
1               5


<210>  976
<211>  6
<212>  PRT
<213>  Artificial Sequence

<220>
<223>  epitope

<400>  976

Met Asp Asn Asn Ser His
1               5


<210>  977
<211>  15
<212>  PRT
<213>  Artificial Sequence

<220>
<223>  epitope


<220>
<221>  misc_feature
<222>  (1)..(8)

<223>  Xaa can be any naturally occurring amino acid

<400>  977

Xaa Xaa Xaa Xaa Xaa Xaa Xaa Xaa Asn Met Asp Asn Asn Ser His
1               5                   10                  15


<210>  978
<211>  15
<212>  PRT
<213>  Artificial Sequence

<220>
<223>  epitope


<220>
<221>  misc_feature
<222>  (1)..(8)
<223>  Xaa can be any naturally occurring amino acid

<220>
<221>  misc_feature
<222>  (12)..(12)
<223>  Xaa can be any naturally occurring amino acid

<400>  978

Xaa Xaa Xaa Xaa Xaa Xaa Xaa Xaa Asn Met Asp Xaa Asn Ser His
1               5                   10                  15


<210>  979
<211>  10
<212>  PRT
<213>  Artificial Sequence

<220>
<223>  epitope

<400>  979

Ser Glu Ser Lys Ile Phe Pro Gly Asn Met
1               5                   10


<210>  980
<211>  10
<212>  PRT
<213>  Artificial Sequence

<220>
<223>  epitope

<400>  980

Ser Lys Ile Phe Pro Gly Asn Met Asp Asn
1               5                   10


<210>  981
<211>  10

<212>  PRT
<213>  Artificial Sequence

<220>
<223>  epitope


<220>
<221>  misc_feature
<222>  (1)..(6)
<223>  Xaa can be any naturally occurring amino acid

<400>  981

Xaa Xaa Xaa Xaa Xaa Xaa Asn Met Asp Asn
1               5                   10


<210>  982
<211>  5
<212>  PRT
<213>  Artificial Sequence

<220>
<223>  epitope

<400>  982

Met Asp Asn Asn Ser
1               5


<210>  983
<211>  5
<212>  PRT
<213>  Artificial Sequence

<220>
<223>  epitope


<220>
<221>  misc_feature
<222>  (3)..(3)
<223>  Xaa can be any naturally occurring amino acid

<400>  983

Met Asp Xaa Asn Ser
1               5


<210>  984
<211>  9
<212>  PRT
<213>  Artificial Sequence

<220>
<223>  epitope

<400>  984

Glu Ser Lys Ile Phe Pro Gly Asn Met

1               5


<210>  985
<211>  8
<212>  PRT
<213>  Artificial Sequence

<220>
<223>  epitope

<400>  985

Ser Lys Ile Phe Pro Gly Asn Met
1               5


<210>  986
<211>  4
<212>  PRT
<213>  Artificial Sequence

<220>
<223>  epitope

<400>  986

Ser Lys Ile Phe
1


<210>  987
<211>  5
<212>  PRT
<213>  Artificial Sequence

<220>
<223>  epitope

<400>  987

Met Asp Asn Asn Ser
1               5


<210>  988
<211>  7
<212>  PRT
<213>  Artificial Sequence

<220>
<223>  epitope

<400>  988

Glu Ser Lys Ile Phe Pro Gly
1               5


<210>  989
<211>  5
<212>  PRT
<213>  Artificial Sequence

<220>
<223>  epitope

<400>  989

Glu Ser Lys Ile Phe
1               5


<210>  990
<211>  15
<212>  PRT
<213>  Artificial Sequence

<220>
<223>  epitope


<220>
<221>  misc_feature
<222>  (8)..(13)
<223>  Xaa can be any naturally occurring amino acid

<220>
<221>  misc_feature
<222>  (15)..(15)
<223>  Xaa can be any naturally occurring amino acid

<400>  990

Ser Glu Ser Lys Ile Phe Pro Xaa Xaa Xaa Xaa Xaa Xaa Ser Xaa
1               5                   10                  15


<210>  991
<211>  15
<212>  PRT
<213>  Artificial Sequence

<220>
<223>  epitope


<220>
<221>  misc_feature
<222>  (2)..(3)
<223>  Xaa can be any naturally occurring amino acid

<220>
<221>  misc_feature
<222>  (7)..(15)
<223>  Xaa can be any naturally occurring amino acid

<400>  991

Ser Xaa Xaa Lys Ile Phe Xaa Xaa Xaa Xaa Xaa Xaa Xaa Xaa Xaa
1               5                   10                  15


<210>  992
<211>  10
<212>  PRT

<213>  Artificial Sequence

<220>
<223>  epitope

<400>  992

Ser Glu Ser Lys Ile Phe Pro Gly Asn Met
1               5                   10


<210>  993
<211>  10
<212>  PRT
<213>  Artificial Sequence

<220>
<223>  epitope


<220>
<221>  misc_feature
<222>  (8)..(10)
<223>  Xaa can be any naturally occurring amino acid

<400>  993

Ser Glu Ser Lys Ile Phe Pro Xaa Xaa Xaa
1               5                   10


<210>  994
<211>  10
<212>  PRT
<213>  Artificial Sequence

<220>
<223>  epitope


<220>
<221>  misc_feature
<222>  (2)..(3)
<223>  Xaa can be any naturally occurring amino acid

<220>
<221>  misc_feature
<222>  (7)..(10)
<223>  Xaa can be any naturally occurring amino acid

<400>  994

Ser Xaa Xaa Lys Ile Phe Xaa Xaa Xaa Xaa
1               5                   10


<210>  995
<211>  10
<212>  PRT
<213>  Artificial Sequence

<220>
<223>  epitope

<400>  995

Ser Lys Ile Phe Pro Gly Asn Met Asp Asn
1               5                   10


<210>  996
<211>  10
<212>  PRT
<213>  Artificial Sequence

<220>
<223>  epitope


<220>
<221>  misc_feature
<222>  (6)..(10)
<223>  Xaa can be any naturally occurring amino acid

<400>  996

Ser Lys Ile Phe Pro Xaa Xaa Xaa Xaa Xaa
1               5                   10


<210>  997
<211>  15
<212>  PRT
<213>  Artificial Sequence

<220>
<223>  epitope

<400>  997

Gln Pro Lys Ser Pro Pro Ser Val Thr Leu Phe Pro Pro Ser Thr
1               5                   10                  15


<210>  998
<211>  15
<212>  PRT
<213>  Artificial Sequence

<220>
<223>  epitope

<400>  998

Gln Pro Lys Ser Pro Pro Ser Val Thr Leu Phe Pro Pro Ser Thr
1               5                   10                  15


<210>  999
<211>  15
<212>  PRT
<213>  Artificial Sequence

<220>
<223>  epitope

<220>
<221>  misc_feature
<222>  (1)..(2)
<223>  Xaa can be any naturally occurring amino acid

<220>
<221>  misc_feature
<222>  (5)..(6)
<223>  Xaa can be any naturally occurring amino acid

<220>
<221>  misc_feature
<222>  (8)..(8)
<223>  Xaa can be any naturally occurring amino acid

<220>
<221>  misc_feature
<222>  (12)..(12)
<223>  Xaa can be any naturally occurring amino acid

<400>  999

Xaa Xaa Lys Ser Xaa Xaa Ser Xaa Thr Leu Phe Xaa Pro Ser Thr
1               5                   10                  15


<210>  1000
<211>  15
<212>  PRT
<213>  Artificial Sequence

<220>
<223>  epitope


<220>
<221>  misc_feature
<222>  (1)..(2)
<223>  Xaa can be any naturally occurring amino acid

<220>
<221>  misc_feature
<222>  (4)..(6)
<223>  Xaa can be any naturally occurring amino acid

<220>
<221>  misc_feature
<222>  (8)..(8)
<223>  Xaa can be any naturally occurring amino acid

<220>
<221>  misc_feature
<222>  (12)..(12)
<223>  Xaa can be any naturally occurring amino acid

<220>
<221>  misc_feature
<222>  (15)..(15)
<223>  Xaa can be any naturally occurring amino acid

<400>  1000

Xaa Xaa Lys Xaa Xaa Xaa Ser Xaa Thr Leu Phe Xaa Pro Ser Xaa
1               5                   10                  15

<210>  1001
<211>  15
<212>  PRT
<213>  Artificial Sequence

<220>
<223>  epitope

<220>
<221>  misc_feature
<222>  (1)..(2)
<223>  Xaa can be any naturally occurring amino acid

<220>
<221>  misc_feature
<222>  (4)..(9)
<223>  Xaa can be any naturally occurring amino acid

<220>
<221>  misc_feature
<222>  (12)..(12)
<223>  Xaa can be any naturally occurring amino acid

<220>
<221>  misc_feature
<222>  (15)..(15)
<223>  Xaa can be any naturally occurring amino acid

<400>  1001

Xaa Xaa Lys Xaa Xaa Xaa Xaa Xaa Xaa Leu Phe Xaa Pro Ser Xaa
1               5                   10                  15

<210>  1002
<211>  6
<212>  PRT
<213>  Artificial Sequence

<220>
<223>  epitope

<400>  1002

Lys Ser Pro Pro Ser Val
1               5

<210>  1003
<211>  5
<212>  PRT
<213>  Artificial Sequence

<220>
<223>  epitope

<400>  1003

Ser Pro Pro Ser Val
1               5

<210>  1004
<211>  10
<212>  PRT
<213>  Artificial Sequence

<220>
<223>  epitope

<400>  1004

Ser Val Thr Leu Phe Pro Pro Ser Thr Glu
1               5                   10

<210>  1005
<211>  10
<212>  PRT
<213>  Artificial Sequence

<220>
<223>  epitope

<220>
<221>  misc_feature
<222>  (2)..(2)
<223>  Xaa can be any naturally occurring amino acid

<220>
<221>  misc_feature
<222>  (6)..(6)
<223>  Xaa can be any naturally occurring amino acid

<400>  1005

Ser Xaa Thr Leu Phe Xaa Pro Ser Thr Glu
1               5                   10

<210>  1006
<211>  10
<212>  PRT
<213>  Artificial Sequence

<220>
<223>  epitope

<220>
<221>  misc_feature
<222>  (2)..(2)
<223>  Xaa can be any naturally occurring amino acid

<220>
<221>  misc_feature
<222>  (6)..(6)
<223>  Xaa can be any naturally occurring amino acid

<220>

<221>  misc_feature
<222>  (9)..(9)
<223>  Xaa can be any naturally occurring amino acid

<400>  1006

Ser Xaa Thr Leu Phe Xaa Pro Ser Xaa Glu
1               5                   10


<210>  1007
<211>  10
<212>  PRT
<213>  Artificial Sequence

<220>
<223>  epitope


<220>
<221>  misc_feature
<222>  (1)..(3)
<223>  Xaa can be any naturally occurring amino acid

<220>
<221>  misc_feature
<222>  (6)..(6)
<223>  Xaa can be any naturally occurring amino acid

<220>
<221>  misc_feature
<222>  (9)..(9)
<223>  Xaa can be any naturally occurring amino acid

<400>  1007

Xaa Xaa Xaa Leu Phe Xaa Pro Ser Xaa Glu
1               5                   10


<210>  1008
<211>  15
<212>  PRT
<213>  Artificial Sequence

<220>
<223>  epitope

<400>  1008

Gln Pro Lys Ser Pro Pro Ser Val Thr Leu Phe Pro Pro Ser Thr
1               5                   10                  15


<210>  1009
<211>  10
<212>  PRT
<213>  Artificial Sequence

<220>
<223>  epitope

<400>  1009

Val Leu Gly Gln Pro Lys Ser Pro Pro Ser
1               5                   10

<210>  1010
<211>  10
<212>  PRT
<213>  Artificial Sequence

<220>
<223>  epitope

<400>  1010

Gly Gln Pro Lys Ser Pro Pro Ser Val Thr
1               5                   10

<210>  1011
<211>  9
<212>  PRT
<213>  Artificial Sequence

<220>
<223>  epitope

<400>  1011

Lys Ser Pro Pro Ser Val Thr Leu Phe
1               5

<210>  1012
<211>  4
<212>  PRT
<213>  Artificial Sequence

<220>
<223>  epitope

<400>  1012

Ser Pro Pro Ser
1

<210>  1013
<211>  7
<212>  PRT
<213>  Artificial Sequence

<220>
<223>  epitope

<400>  1013

Leu Phe Pro Pro Ser Thr Glu
1               5

<210>  1014
<211>  15

<212>  PRT
<213>  Artificial Sequence

<220>
<223>  epitope

<400>  1014

Gln Pro Lys Ser Pro Pro Ser Val Thr Leu Phe Pro Pro Ser Thr
1               5                   10                  15


<210>  1015
<211>  15
<212>  PRT
<213>  Artificial Sequence

<220>
<223>  epitope


<220>
<221>  misc_feature
<222>  (2)..(2)
<223>  Xaa can be any naturally occurring amino acid

<220>
<221>  misc_feature
<222>  (5)..(5)
<223>  Xaa can be any naturally occurring amino acid

<220>
<221>  misc_feature
<222>  (8)..(15)
<223>  Xaa can be any naturally occurring amino acid

<400>  1015

Gln Xaa Lys Ser Xaa Pro Ser Xaa Xaa Xaa Xaa Xaa Xaa Xaa Xaa
1               5                   10                  15


<210>  1016
<211>  5
<212>  PRT
<213>  Artificial Sequence

<220>
<223>  epitope

<400>  1016

Lys Ser Pro Pro Ser
1               5


<210>  1017
<211>  5
<212>  PRT
<213>  Artificial Sequence

<220>
<223>  epitope

<220>
<221>  misc_feature
<222>  (3)..(3)
<223>  Xaa can be any naturally occurring amino acid

<400>  1017

Lys Ser Xaa Pro Ser
1               5


<210>  1018
<211>  15
<212>  PRT
<213>  Artificial Sequence

<220>
<223>  epitope

<400>  1018

Gln Pro Lys Ser Pro Pro Ser Val Thr Leu Phe Pro Pro Ser Thr
1               5                   10                  15


<210>  1019
<211>  15
<212>  PRT
<213>  Artificial Sequence

<220>
<223>  epitope


<220>
<221>  misc_feature
<222>  (1)..(2)
<223>  Xaa can be any naturally occurring amino acid

<220>
<221>  misc_feature
<222>  (5)..(6)
<223>  Xaa can be any naturally occurring amino acid

<220>
<221>  misc_feature
<222>  (9)..(9)
<223>  Xaa can be any naturally occurring amino acid

<220>
<221>  misc_feature
<222>  (12)..(15)
<223>  Xaa can be any naturally occurring amino acid

<400>  1019

Xaa Xaa Lys Ser Xaa Xaa Ser Val Xaa Leu Phe Xaa Xaa Xaa Xaa
1               5                   10                  15


<210>  1020

<211>  15
<212>  PRT
<213>  Artificial Sequence

<220>
<223>  epitope



<220>
<221>  misc_feature
<222>  (1)..(2)
<223>  Xaa can be any naturally occurring amino acid

<220>
<221>  misc_feature
<222>  (5)..(7)
<223>  Xaa can be any naturally occurring amino acid

<220>
<221>  misc_feature
<222>  (9)..(9)
<223>  Xaa can be any naturally occurring amino acid

<220>
<221>  misc_feature
<222>  (12)..(15)
<223>  Xaa can be any naturally occurring amino acid

<400>  1020

Xaa Xaa Lys Ser Xaa Xaa Xaa Val Xaa Leu Phe Xaa Xaa Xaa Xaa
1               5                   10                  15


<210>  1021
<211>  10
<212>  PRT
<213>  Artificial Sequence

<220>
<223>  epitope

<400>  1021

Thr Val Leu Gly Gln Pro Lys Ser Pro Pro
1               5                   10


<210>  1022
<211>  5
<212>  PRT
<213>  Artificial Sequence

<220>
<223>  epitope

<400>  1022

Ser Pro Pro Ser Val
1               5


<210>  1023

<211>  10
<212>  PRT
<213>  Artificial Sequence

<220>
<223>  epitope

<400>  1023

Ser Val Thr Leu Phe Pro Pro Ser Thr Glu
1               5                   10


<210>  1024
<211>  10
<212>  PRT
<213>  Artificial Sequence

<220>
<223>  epitope

<400>  1024

Leu Phe Pro Pro Ser Thr Glu Glu Leu Asn
1               5                   10


<210>  1025
<211>  15
<212>  PRT
<213>  Artificial Sequence

<220>
<223>  epitope

<400>  1025

Gln Pro Lys Ser Pro Pro Ser Val Thr Leu Phe Pro Pro Ser Thr
1               5                   10                  15


<210>  1026
<211>  9
<212>  PRT
<213>  Artificial Sequence

<220>
<223>  epitope

<400>  1026

Val Leu Gly Gln Pro Lys Ser Pro Pro
1               5


<210>  1027
<211>  8
<212>  PRT
<213>  Artificial Sequence

<220>
<223>  epitope

<400>  1027

Lys Ser Pro Pro Ser Val Thr Leu
1               5


<210>  1028
<211>  7
<212>  PRT
<213>  Artificial Sequence

<220>
<223>  epitope

<400>  1028

Lys Ser Pro Pro Ser Val Thr
1               5


<210>  1029
<211>  4
<212>  PRT
<213>  Artificial Sequence

<220>
<223>  epitope

<400>  1029

Lys Ser Pro Pro
1


<210>  1030
<211>  10
<212>  PRT
<213>  Artificial Sequence

<220>
<223>  epitope

<400>  1030

Leu Phe Pro Pro Ser Thr Glu Glu Leu Asn
1               5                   10


<210>  1031
<211>  8
<212>  PRT
<213>  Artificial Sequence

<220>
<223>  epitope

<400>  1031

Leu Phe Pro Pro Ser Thr Glu Glu
1               5


<210>  1032

<211>  15
<212>  PRT
<213>  Artificial Sequence

<220>
<223>  epitope


<220>
<221>  misc_feature
<222>  (5)..(10)
<223>  Xaa can be any naturally occurring amino acid

<400>  1032

Gln Pro Lys Ser Xaa Xaa Xaa Xaa Xaa Xaa Phe Pro Pro Ser Thr
1               5                   10                  15


<210>  1033
<211>  15
<212>  PRT
<213>  Artificial Sequence

<220>
<223>  epitope


<220>
<221>  misc_feature
<222>  (1)..(2)
<223>  Xaa can be any naturally occurring amino acid

<220>
<221>  misc_feature
<222>  (5)..(12)
<223>  Xaa can be any naturally occurring amino acid

<400>  1033

Xaa Xaa Lys Ser Xaa Xaa Xaa Xaa Xaa Xaa Xaa Xaa Pro Ser Thr
1               5                   10                  15


<210>  1034
<211>  4
<212>  PRT
<213>  Artificial Sequence

<220>
<223>  epitope

<400>  1034

Ser Pro Pro Ser
1


<210>  1035
<211>  15
<212>  PRT
<213>  Artificial Sequence

<220>
<223>  epitope

<220>
<221>  misc_feature
<222>  (1)..(2)
<223>  Xaa can be any naturally occurring amino acid

<220>
<221>  misc_feature
<222>  (5)..(6)
<223>  Xaa can be any naturally occurring amino acid

<220>
<221>  misc_feature
<222>  (8)..(10)
<223>  Xaa can be any naturally occurring amino acid

<220>
<221>  misc_feature
<222>  (12)..(15)
<223>  Xaa can be any naturally occurring amino acid

<400>  1035

Xaa Xaa Lys Ser Xaa Xaa Ser Xaa Xaa Xaa Phe Xaa Xaa Xaa Xaa
1               5                   10                  15

<210>  1036
<211>  4
<212>  PRT
<213>  Artificial Sequence

<220>
<223>  epitope

<400>  1036

Ser Pro Pro Ser
1

<210>  1037
<211>  9
<212>  PRT
<213>  Artificial Sequence

<220>
<223>  epitope

<400>  1037

Ser Val Thr Leu Phe Pro Pro Ser Thr
1               5

<210>  1038
<211>  15
<212>  PRT
<213>  Artificial Sequence

<220>
<223>  epitope

<400>  1038

Gly Ile Pro Val Pro Phe Pro Ile Pro Glu Ser Asp Gly Cys Lys
1               5                   10                  15


<210>  1039
<211>  15
<212>  PRT
<213>  Artificial Sequence

<220>
<223>  epitope

<400>  1039

Gly Ile Pro Val Pro Phe Pro Ile Pro Glu Ser Asp Gly Cys Lys
1               5                   10                  15


<210>  1040
<211>  15
<212>  PRT
<213>  Artificial Sequence

<220>
<223>  epitope


<220>
<221>  misc_feature
<222>  (1)..(2)
<223>  Xaa can be any naturally occurring amino acid

<220>
<221>  misc_feature
<222>  (9)..(9)
<223>  Xaa can be any naturally occurring amino acid

<220>
<221>  misc_feature
<222>  (14)..(14)
<223>  Xaa can be any naturally occurring amino acid

<400>  1040

Xaa Xaa Pro Val Pro Phe Pro Ile Xaa Glu Ser Asp Gly Xaa Lys
1               5                   10                  15


<210>  1041
<211>  15
<212>  PRT
<213>  Artificial Sequence

<220>
<223>  epitope


<220>

<221>  misc_feature
<222>  (1)..(3)
<223>  Xaa can be any naturally occurring amino acid

<220>
<221>  misc_feature
<222>  (8)..(9)
<223>  Xaa can be any naturally occurring amino acid

<220>
<221>  misc_feature
<222>  (13)..(15)
<223>  Xaa can be any naturally occurring amino acid

<400>  1041

Xaa Xaa Xaa Val Pro Phe Pro Xaa Xaa Glu Ser Asp Xaa Xaa Xaa
1               5                   10                  15


<210>  1042
<211>  6
<212>  PRT
<213>  Artificial Sequence

<220>
<223>  epitope

<400>  1042

Glu Ser Asp Gly Cys Lys
1               5


<210>  1043
<211>  6
<212>  PRT
<213>  Artificial Sequence

<220>
<223>  epitope


<220>
<221>  misc_feature
<222>  (5)..(5)
<223>  Xaa can be any naturally occurring amino acid

<400>  1043

Glu Ser Asp Gly Xaa Lys
1               5


<210>  1044
<211>  15
<212>  PRT
<213>  Artificial Sequence

<220>
<223>  epitope

<400>  1044

Gly Ile Pro Val Pro Phe Pro Ile Pro Glu Ser Asp Gly Cys Lys
1               5                   10                  15


<210>  1045
<211>  6
<212>  PRT
<213>  Artificial Sequence

<220>
<223>  epitope

<400>  1045

Glu Ser Asp Gly Cys Lys
1               5


<210>  1046
<211>  15
<212>  PRT
<213>  Artificial Sequence

<220>
<223>  epitope

<400>  1046

Gly Ile Pro Val Pro Phe Pro Ile Pro Glu Ser Asp Gly Cys Lys
1               5                   10                  15


<210>  1047
<211>  9
<212>  PRT
<213>  Artificial Sequence

<220>
<223>  epitope

<400>  1047

Ile Val Met Gly Ile Pro Val Pro Phe
1               5


<210>  1048
<211>  5
<212>  PRT
<213>  Artificial Sequence

<220>
<223>  epitope

<400>  1048

Ile Pro Val Pro Phe
1               5


<210>  1049
<211>  6

<212>  PRT
<213>  Artificial Sequence

<220>
<223>  epitope

<400>  1049

Glu Ser Asp Gly Cys Lys
1               5


<210>  1050
<211>  4
<212>  PRT
<213>  Artificial Sequence

<220>
<223>  epitope

<400>  1050

Glu Ser Asp Gly
1


<210>  1051
<211>  15
<212>  PRT
<213>  Artificial Sequence

<220>
<223>  epitope

<400>  1051

Gly Ile Pro Val Pro Phe Pro Ile Pro Glu Ser Asp Gly Cys Lys
1               5                   10                  15


<210>  1052
<211>  6
<212>  PRT
<213>  Artificial Sequence

<220>
<223>  epitope

<400>  1052

Glu Ser Asp Gly Cys Lys
1               5


<210>  1053
<211>  15
<212>  PRT
<213>  Artificial Sequence

<220>
<223>  epitope

<400>  1053

Gly Ile Pro Val Pro Phe Pro Ile Pro Glu Ser Asp Gly Cys Lys
1               5                   10                  15


<210>  1054
<211>  15
<212>  PRT
<213>  Artificial Sequence

<220>
<223>  epitope


<220>
<221>  misc_feature
<222>  (1)..(9)
<223>  Xaa can be any naturally occurring amino acid

<220>
<221>  misc_feature
<222>  (14)..(14)
<223>  Xaa can be any naturally occurring amino acid

<400>  1054

Xaa Xaa Xaa Xaa Xaa Xaa Xaa Xaa Xaa Glu Ser Asp Gly Xaa Lys
1               5                   10                  15


<210>  1055
<211>  6
<212>  PRT
<213>  Artificial Sequence

<220>
<223>  epitope

<400>  1055

Glu Ser Asp Gly Cys Lys
1               5


<210>  1056
<211>  6
<212>  PRT
<213>  Artificial Sequence

<220>
<223>  epitope


<220>
<221>  misc_feature
<222>  (5)..(5)
<223>  Xaa can be any naturally occurring amino acid

<400>  1056

Glu Ser Asp Gly Xaa Lys
1               5

<210>  1057
<211>  15
<212>  PRT
<213>  Artificial Sequence

<220>
<223>  epitope


<220>
<221>  misc_feature
<222>  (2)..(5)
<223>  Xaa can be any naturally occurring amino acid

<220>
<221>  misc_feature
<222>  (12)..(14)
<223>  Xaa can be any naturally occurring amino acid

<400>  1057

Gly Xaa Xaa Xaa Xaa Phe Pro Ile Pro Glu Ser Xaa Xaa Xaa Lys
1               5                   10                  15


<210>  1058
<211>  15
<212>  PRT
<213>  Artificial Sequence

<220>
<223>  epitope


<220>
<221>  misc_feature
<222>  (1)..(5)
<223>  Xaa can be any naturally occurring amino acid

<220>
<221>  misc_feature
<222>  (7)..(8)
<223>  Xaa can be any naturally occurring amino acid

<220>
<221>  misc_feature
<222>  (12)..(14)
<223>  Xaa can be any naturally occurring amino acid

<400>  1058

Xaa Xaa Xaa Xaa Xaa Phe Xaa Xaa Pro Glu Ser Xaa Xaa Xaa Lys
1               5                   10                  15


<210>  1059
<211>  10
<212>  PRT
<213>  Artificial Sequence

<220>
<223>  epitope

<400>  1059

Ile Pro Val Pro Phe Pro Ile Pro Glu Ser
1               5                   10


<210>  1060
<211>  10
<212>  PRT
<213>  Artificial Sequence

<220>
<223>  epitope


<220>
<221>  misc_feature
<222>  (1)..(4)
<223>  Xaa can be any naturally occurring amino acid

<400>  1060

Xaa Xaa Xaa Xaa Phe Pro Ile Pro Glu Ser
1               5                   10


<210>  1061
<211>  10
<212>  PRT
<213>  Artificial Sequence

<220>
<223>  epitope


<220>
<221>  misc_feature
<222>  (1)..(4)
<223>  Xaa can be any naturally occurring amino acid

<220>
<221>  misc_feature
<222>  (6)..(7)
<223>  Xaa can be any naturally occurring amino acid

<400>  1061

Xaa Xaa Xaa Xaa Phe Xaa Xaa Pro Glu Ser
1               5                   10


<210>  1062
<211>  6
<212>  PRT
<213>  Artificial Sequence

<220>
<223>  epitope

<400>  1062

Glu Ser Asp Gly Cys Lys

1               5


<210>  1063
<211>  4
<212>  PRT
<213>  Artificial Sequence

<220>
<223>  epitope

<400>  1063

Glu Ser Asp Gly
1


<210>  1064
<211>  9
<212>  PRT
<213>  Artificial Sequence

<220>
<223>  epitope

<400>  1064

Gly Ile Pro Val Pro Phe Pro Ile Pro
1               5


<210>  1065
<211>  8
<212>  PRT
<213>  Artificial Sequence

<220>
<223>  epitope

<400>  1065

Ile Pro Val Pro Phe Pro Ile Pro
1               5


<210>  1066
<211>  7
<212>  PRT
<213>  Artificial Sequence

<220>
<223>  epitope

<400>  1066

Ile Pro Val Pro Phe Pro Ile
1               5


<210>  1067
<211>  10
<212>  PRT
<213>  Artificial Sequence

<220>
<223>  epitope

<400>  1067

Phe Pro Ile Pro Glu Ser Asp Gly Cys Lys
1               5                   10


<210>  1068
<211>  15
<212>  PRT
<213>  Artificial Sequence

<220>
<223>  epitope


<220>
<221>  misc_feature
<222>  (1)..(10)
<223>  Xaa can be any naturally occurring amino acid

<400>  1068

Xaa Xaa Xaa Xaa Xaa Xaa Xaa Xaa Xaa Xaa Ser Asp Gly Cys Lys
1               5                   10                  15


<210>  1069
<211>  15
<212>  PRT
<213>  Artificial Sequence

<220>
<223>  epitope


<220>
<221>  misc_feature
<222>  (1)..(10)
<223>  Xaa can be any naturally occurring amino acid

<220>
<221>  misc_feature
<222>  (14)..(14)
<223>  Xaa can be any naturally occurring amino acid

<400>  1069

Xaa Xaa Xaa Xaa Xaa Xaa Xaa Xaa Xaa Xaa Ser Asp Gly Xaa Lys
1               5                   10                  15


<210>  1070
<211>  6
<212>  PRT
<213>  Artificial Sequence

<220>
<223>  epitope

<400>  1070

Glu Ser Asp Gly Cys Lys
1               5


<210>  1071
<211>  6
<212>  PRT
<213>  Artificial Sequence

<220>
<223>  epitope


<220>
<221>  misc_feature
<222>  (1)..(1)
<223>  Xaa can be any naturally occurring amino acid

<400>  1071

Xaa Ser Asp Gly Cys Lys
1               5


<210>  1072
<211>  6
<212>  PRT
<213>  Artificial Sequence

<220>
<223>  epitope


<220>
<221>  misc_feature
<222>  (1)..(1)
<223>  Xaa can be any naturally occurring amino acid

<220>
<221>  misc_feature
<222>  (5)..(5)
<223>  Xaa can be any naturally occurring amino acid

<400>  1072

Xaa Ser Asp Gly Xaa Lys
1               5


<210>  1073
<211>  15
<212>  PRT
<213>  Artificial Sequence

<220>
<223>  epitope


<220>
<221>  misc_feature
<222>  (1)..(9)

<223>  Xaa can be any naturally occurring amino acid

<220>
<221>  misc_feature
<222>  (13)..(13)
<223>  Xaa can be any naturally occurring amino acid

<400>  1073

Xaa Xaa Xaa Xaa Xaa Xaa Xaa Xaa Xaa Glu Ser Asp Xaa Cys Lys
1               5                   10                  15


<210>  1074
<211>  6
<212>  PRT
<213>  Artificial Sequence

<220>
<223>  epitope

<400>  1074

Glu Ser Asp Gly Cys Lys
1               5


<210>  1075
<211>  6
<212>  PRT
<213>  Artificial Sequence

<220>
<223>  epitope


<220>
<221>  misc_feature
<222>  (4)..(4)
<223>  Xaa can be any naturally occurring amino acid

<400>  1075

Glu Ser Asp Xaa Cys Lys
1               5


<210>  1076
<211>  15
<212>  PRT
<213>  Artificial Sequence

<220>
<223>  epitope

<400>  1076

Leu Thr Lys Asp Ser Glu Lys Leu Lys Glu Glu Ile Arg Lys Glu
1               5                   10                  15


<210>  1077
<211>  15

<212>  PRT
<213>  Artificial Sequence

<220>
<223>  epitope

<400>  1077

Leu Thr Lys Asp Ser Glu Lys Leu Lys Glu Glu Ile Arg Lys Glu
1               5                   10                  15


<210>  1078
<211>  15
<212>  PRT
<213>  Artificial Sequence

<220>
<223>  epitope


<220>
<221>  misc_feature
<222>  (1)..(6)
<223>  Xaa can be any naturally occurring amino acid

<220>
<221>  misc_feature
<222>  (12)..(12)
<223>  Xaa can be any naturally occurring amino acid

<220>
<221>  misc_feature
<222>  (14)..(14)
<223>  Xaa can be any naturally occurring amino acid

<400>  1078

Xaa Xaa Xaa Xaa Xaa Xaa Lys Leu Lys Glu Glu Xaa Arg Xaa Glu
1               5                   10                  15


<210>  1079
<211>  4
<212>  PRT
<213>  Artificial Sequence

<220>
<223>  epitope

<400>  1079

Lys Glu Glu Ile
1


<210>  1080
<211>  15
<212>  PRT
<213>  Artificial Sequence

<220>
<223>  epitope

<400>  1080

Leu Thr Lys Asp Ser Glu Lys Leu Lys Glu Glu Ile Arg Lys Glu
1               5                   10                  15


<210>  1081
<211>  4
<212>  PRT
<213>  Artificial Sequence

<220>
<223>  epitope

<400>  1081

Lys Asp Ser Glu
1


<210>  1082
<211>  9
<212>  PRT
<213>  Artificial Sequence

<220>
<223>  epitope

<400>  1082

Lys Leu Lys Glu Glu Ile Arg Lys Glu
1               5


<210>  1083
<211>  10
<212>  PRT
<213>  Artificial Sequence

<220>
<223>  epitope

<400>  1083

Lys Glu Glu Ile Arg Lys Glu Leu Glu Asp
1               5                   10


<210>  1084
<211>  15
<212>  PRT
<213>  Artificial Sequence

<220>
<223>  epitope

<400>  1084

Leu Thr Lys Asp Ser Glu Lys Leu Lys Glu Glu Ile Arg Lys Glu
1               5                   10                  15

<210>  1085
<211>  15
<212>  PRT
<213>  Artificial Sequence

<220>
<223>  epitope


<220>
<221>  misc_feature
<222>  (1)..(3)
<223>  Xaa can be any naturally occurring amino acid

<220>
<221>  misc_feature
<222>  (5)..(5)
<223>  Xaa can be any naturally occurring amino acid

<220>
<221>  misc_feature
<222>  (7)..(8)
<223>  Xaa can be any naturally occurring amino acid

<220>
<221>  misc_feature
<222>  (10)..(12)
<223>  Xaa can be any naturally occurring amino acid

<220>
<221>  misc_feature
<222>  (14)..(14)
<223>  Xaa can be any naturally occurring amino acid

<400>  1085

Xaa Xaa Xaa Asp Xaa Glu Xaa Xaa Lys Xaa Xaa Xaa Arg Xaa Glu
1               5                   10                  15


<210>  1086
<211>  15
<212>  PRT
<213>  Artificial Sequence

<220>
<223>  epitope

<400>  1086

Leu Thr Lys Asp Ser Glu Lys Leu Lys Glu Glu Ile Arg Lys Glu
1               5                   10                  15


<210>  1087
<211>  15
<212>  PRT
<213>  Artificial Sequence

<220>
<223>  epitope

<220>
<221>  misc_feature
<222>  (1)..(5)
<223>  Xaa can be any naturally occurring amino acid

<220>
<221>  misc_feature
<222>  (10)..(12)
<223>  Xaa can be any naturally occurring amino acid

<400>  1087

Xaa Xaa Xaa Xaa Xaa Glu Lys Leu Lys Xaa Xaa Xaa Arg Lys Glu
1               5                   10                  15


<210>  1088
<211>  8
<212>  PRT
<213>  Artificial Sequence

<220>
<223>  epitope

<400>  1088

Glu Arg Leu Thr Lys Asp Ser Glu
1               5


<210>  1089
<211>  10
<212>  PRT
<213>  Artificial Sequence

<220>
<223>  epitope

<400>  1089

Leu Thr Lys Asp Ser Glu Lys Leu Lys Glu
1               5                   10


<210>  1090
<211>  10
<212>  PRT
<213>  Artificial Sequence

<220>
<223>  epitope


<220>
<221>  misc_feature
<222>  (1)..(5)
<223>  Xaa can be any naturally occurring amino acid

<220>
<221>  misc_feature
<222>  (10)..(10)
<223>  Xaa can be any naturally occurring amino acid

<400>  1090

Xaa Xaa Xaa Xaa Xaa Glu Lys Leu Lys Xaa
1               5                   10


<210>  1091
<211>  7
<212>  PRT
<213>  Artificial Sequence

<220>
<223>  epitope

<400>  1091

Leu Lys Glu Glu Ile Arg Lys
1               5


<210>  1092
<211>  7
<212>  PRT
<213>  Artificial Sequence

<220>
<223>  epitope


<220>
<221>  misc_feature
<222>  (3)..(5)
<223>  Xaa can be any naturally occurring amino acid

<400>  1092

Leu Lys Xaa Xaa Xaa Arg Lys
1               5


<210>  1093
<211>  15
<212>  PRT
<213>  Artificial Sequence

<220>
<223>  epitope

<400>  1093

Leu Thr Lys Asp Ser Glu Lys Leu Lys Glu Glu Ile Arg Lys Glu
1               5                   10                  15


<210>  1094
<211>  10
<212>  PRT
<213>  Artificial Sequence

<220>
<223>  epitope

<400>  1094

Arg Leu Thr Lys Asp Ser Glu Lys Leu Lys
1               5                   10


<210>  1095
<211>  6
<212>  PRT
<213>  Artificial Sequence

<220>
<223>  epitope

<400>  1095

Lys Leu Lys Glu Glu Ile
1               5


<210>  1096
<211>  15
<212>  PRT
<213>  Artificial Sequence

<220>
<223>  epitope


<220>
<221>  misc_feature
<222>  (1)..(2)
<223>  Xaa can be any naturally occurring amino acid

<220>
<221>  misc_feature
<222>  (12)..(15)
<223>  Xaa can be any naturally occurring amino acid

<400>  1096

Xaa Xaa Lys Asp Ser Glu Lys Leu Lys Glu Glu Xaa Xaa Xaa Xaa
1               5                   10                  15


<210>  1097
<211>  15
<212>  PRT
<213>  Artificial Sequence

<220>
<223>  epitope


<220>
<221>  misc_feature
<222>  (1)..(2)
<223>  Xaa can be any naturally occurring amino acid

<220>
<221>  misc_feature
<222>  (5)..(7)
<223>  Xaa can be any naturally occurring amino acid

<220>
<221>  misc_feature
<222>  (12)..(15)
<223>  Xaa can be any naturally occurring amino acid

<400>  1097

Xaa Xaa Lys Asp Xaa Xaa Xaa Leu Lys Glu Glu Xaa Xaa Xaa Xaa
1               5                   10                  15


<210>  1098
<211>  4
<212>  PRT
<213>  Artificial Sequence

<220>
<223>  epitope

<400>  1098

Lys Asp Ser Glu
1


<210>  1099
<211>  9
<212>  PRT
<213>  Artificial Sequence

<220>
<223>  epitope

<400>  1099

Lys Leu Lys Glu Glu Ile Arg Lys Glu
1               5


<210>  1100
<211>  9
<212>  PRT
<213>  Artificial Sequence

<220>
<223>  epitope


<220>
<221>  misc_feature
<222>  (6)..(9)
<223>  Xaa can be any naturally occurring amino acid

<400>  1100

Lys Leu Lys Glu Glu Xaa Xaa Xaa Xaa
1               5


<210>  1101
<211>  9
<212>  PRT
<213>  Artificial Sequence

<220>
<223>  epitope


<220>
<221>  misc_feature
<222>  (1)..(1)
<223>  Xaa can be any naturally occurring amino acid

<220>
<221>  misc_feature
<222>  (6)..(9)
<223>  Xaa can be any naturally occurring amino acid

<400>  1101

Xaa Leu Lys Glu Glu Xaa Xaa Xaa Xaa
1               5


<210>  1102
<211>  6
<212>  PRT
<213>  Artificial Sequence

<220>
<223>  epitope

<400>  1102

Leu Thr Lys Asp Ser Glu
1               5


<210>  1103
<211>  10
<212>  PRT
<213>  Artificial Sequence

<220>
<223>  epitope

<400>  1103

Lys Asp Ser Glu Lys Leu Lys Glu Glu Ile
1               5                   10


<210>  1104
<211>  9
<212>  PRT
<213>  Artificial Sequence

<220>
<223>  epitope

<400>  1104

Lys Leu Lys Glu Glu Ile Arg Lys Glu
1               5

<210>  1105
<211>  6
<212>  PRT
<213>  Artificial Sequence

<220>
<223>  epitope

<400>  1105

Lys Leu Lys Glu Glu Ile
1               5


<210>  1106
<211>  15
<212>  PRT
<213>  Artificial Sequence

<220>
<223>  epitope


<220>
<221>  misc_feature
<222>  (1)..(1)
<223>  Xaa can be any naturally occurring amino acid

<220>
<221>  misc_feature
<222>  (3)..(3)
<223>  Xaa can be any naturally occurring amino acid

<220>
<221>  misc_feature
<222>  (5)..(5)
<223>  Xaa can be any naturally occurring amino acid

<220>
<221>  misc_feature
<222>  (12)..(12)
<223>  Xaa can be any naturally occurring amino acid

<400>  1106

Xaa Thr Xaa Asp Xaa Glu Lys Leu Lys Glu Glu Xaa Arg Lys Glu
1               5                   10                  15


<210>  1107
<211>  15
<212>  PRT
<213>  Artificial Sequence

<220>
<223>  epitope


<220>
<221>  misc_feature
<222>  (1)..(3)
<223>  Xaa can be any naturally occurring amino acid

<220>
<221>  misc_feature
<222>  (5)..(6)
<223>  Xaa can be any naturally occurring amino acid

<220>
<221>  misc_feature
<222>  (11)..(12)
<223>  Xaa can be any naturally occurring amino acid

<220>
<221>  misc_feature
<222>  (15)..(15)
<223>  Xaa can be any naturally occurring amino acid

<400>  1107

Xaa Xaa Xaa Asp Xaa Xaa Lys Leu Lys Glu Xaa Xaa Arg Lys Xaa
1               5                   10                  15


<210>  1108
<211>  8
<212>  PRT
<213>  Artificial Sequence

<220>
<223>  epitope

<400>  1108

Glu Lys Leu Lys Glu Glu Ile Arg
1               5


<210>  1109
<211>  6
<212>  PRT
<213>  Artificial Sequence

<220>
<223>  epitope

<400>  1109

Glu Lys Leu Lys Glu Glu
1               5


<210>  1110
<211>  5
<212>  PRT
<213>  Artificial Sequence

<220>
<223>  epitope

<400>  1110

Lys Leu Lys Glu Glu
1               5

<210>  1111
<211>  5
<212>  PRT
<213>  Artificial Sequence

<220>
<223>  epitope



<220>
<221>  misc_feature
<222>  (5)..(5)
<223>  Xaa can be any naturally occurring amino acid

<400>  1111

Lys Leu Lys Glu Xaa
1               5


<210>  1112
<211>  15
<212>  PRT
<213>  Artificial Sequence

<220>
<223>  epitope



<220>
<221>  misc_feature
<222>  (3)..(4)
<223>  Xaa can be any naturally occurring amino acid

<220>
<221>  misc_feature
<222>  (6)..(8)
<223>  Xaa can be any naturally occurring amino acid

<220>
<221>  misc_feature
<222>  (13)..(14)
<223>  Xaa can be any naturally occurring amino acid

<400>  1112

Leu Thr Xaa Xaa Ser Xaa Xaa Xaa Lys Glu Glu Ile Xaa Xaa Glu
1               5                   10                  15


<210>  1113
<211>  5
<212>  PRT
<213>  Artificial Sequence

<220>
<223>  epitope

<400>  1113

Lys Glu Glu Ile Arg
1               5

<210>  1114
<211>  4
<212>  PRT
<213>  Artificial Sequence

<220>
<223>  epitope

<400>  1114

Lys Glu Glu Ile
1


<210>  1115
<211>  10
<212>  PRT
<213>  Artificial Sequence

<220>
<223>  epitope

<400>  1115

Tyr Leu Ser Gln Thr Glu Glu Asn Lys Glu
1               5                   10


<210>  1116
<211>  6
<212>  PRT
<213>  Artificial Sequence

<220>
<223>  epitope

<400>  1116

His Pro Glu Asp Arg Lys
1               5


<210>  1117
<211>  5
<212>  PRT
<213>  Artificial Sequence

<220>
<223>  epitope

<400>  1117

Lys Pro Glu Asp Arg
1               5


<210>  1118
<211>  5
<212>  PRT
<213>  Artificial Sequence

<220>

<223>  epitope

<400>  1118

Val Pro Glu Asp Arg
1               5


<210>  1119
<211>  7
<212>  PRT
<213>  Artificial Sequence

<220>
<223>  epitope

<400>  1119

Lys Arg Arg Gly Glu Ser Cys
1               5


<210>  1120
<211>  7
<212>  PRT
<213>  Artificial Sequence

<220>
<223>  epitope

<400>  1120

Lys Ala Asp Gly Glu Ser Cys
1               5


<210>  1121
<211>  15
<212>  PRT
<213>  Artificial Sequence

<220>
<223>  epitope

<400>  1121

Glu Trp Gln Asn Arg Leu Glu Glu Ile Lys Lys Thr Met Phe Glu
1               5                   10                  15


<210>  1122
<211>  15
<212>  PRT
<213>  Artificial Sequence

<220>
<223>  epitope

<400>  1122

Glu Lys Thr Phe Glu Cys Gly Glu Cys Gly Lys Thr Phe Trp Glu
1               5                   10                  15

<210>  1123
<211>  7
<212>  PRT
<213>  Artificial Sequence

<220>
<223>  epitope

<400>  1123

Thr Gly Gly Lys Glu Glu Lys
1               5


<210>  1124
<211>  10
<212>  PRT
<213>  Artificial Sequence

<220>
<223>  epitope

<400>  1124

Lys Glu Glu Ser Val Pro Leu Val Gly Gln
1               5                   10


<210>  1125
<211>  7
<212>  PRT
<213>  Artificial Sequence

<220>
<223>  epitope

<400>  1125

Asp Lys Tyr Glu Leu Pro Pro
1               5


<210>  1126
<211>  9
<212>  PRT
<213>  Artificial Sequence

<220>
<223>  epitope

<400>  1126

Glu Asp Lys Tyr Arg Lys Ile Asn Glu
1               5


<210>  1127
<211>  9
<212>  PRT
<213>  Artificial Sequence

<220>

<223>  epitope

<400>  1127

Glu Asp Lys Tyr Gly Thr Thr Thr Ser
1               5

<210>  1128
<211>  5
<212>  PRT
<213>  Artificial Sequence

<220>
<223>  epitope

<400>  1128

Lys Ile Asn Ser Lys
1               5

<210>  1129
<211>  7
<212>  PRT
<213>  Artificial Sequence

<220>
<223>  epitope

<400>  1129

Trp Glu Lys Glu Met Lys Leu
1               5

<210>  1130
<211>  10
<212>  PRT
<213>  Artificial Sequence

<220>
<223>  epitope

<400>  1130

Leu Asn Asp Lys Cys Asp Arg Leu Arg Lys
1               5                   10

<210>  1131
<211>  5
<212>  PRT
<213>  Artificial Sequence

<220>
<223>  epitope

<400>  1131

Lys Leu Lys Glu Arg
1               5